(19) **Europäisches Patentamt / European Patent Office / Office européen des brevets**

(11) **EP 4 095 258 B1**

(12) **EUROPEAN PATENT SPECIFICATION**

(45) Date of publication and mention of the grant of the patent:
**29.04.2026 Bulletin 2026/18**

(21) Application number: **22172589.8**

(22) Date of filing: **06.07.2018**

(51) International Patent Classification (IPC):
**C12Q 1/6806** (2018.01)     **C12Q 1/6883** (2018.01)
**C12Q 1/6886** (2018.01)

(52) Cooperative Patent Classification (CPC):
(C-Sets available)
**C12Q 1/6883; C12Q 1/6806; C12Q 1/6886**   (Cont.)

(54) **TARGET-ENRICHED MULTIPLEXED PARALLEL ANALYSIS FOR ASSESMENT OF TUMOR BIOMARKERS**

ZIELANGEREICHERTE MULTIPLEXIERTE PARALLELANALYSE ZUR BEURTEILUNG VON TUMORBIOMARKERN

ANALYSE PARALLÈLE MULTIPLEXÉE ENRICHIE EN CIBLE POUR L'ÉVALUATION DE BIOMARQUEURS TUMORAUX

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.07.2017 US 201762529779 P**

(43) Date of publication of application:
**30.11.2022 Bulletin 2022/48**

(62) Document number(s) of the earlier application(s) in accordance with Art. 76 EPC:
**18745511.8 / 3 649 260**

(73) Proprietor: **MEDICOVER PUBLIC CO LTD**
**Nicosia 2409 (CY)**

(72) Inventors:
- **KOUMBARIS, George**
  **2565 Lithrodontas (CY)**
- **ACHILLEOS, Acilleas**
  **4045 Limassol (CY)**
- **IOANNIDES, Marios**
  **2416 Nicosia (CY)**
- **KYPRI, Elena**
  **2007 Nicosia (CY)**
- **MINA, Petros**
  **2015 Nicosia (CY)**
- **ELIADES, Alexia**
  **2102 Nicosia (CY)**
- **LOIZIDES, Charalambos**
  **2013 Nicosia (CY)**
- **PATSALIS, Philippos**
  **2402 Nicosia (CY)**

(74) Representative: **CH Kilger Anwaltspartnerschaft mbB**
**Fasanenstraße 29**
**10719 Berlin (DE)**

(56) References cited:
EP-A1- 2 902 500     WO-A1-2012/092426
WO-A1-2014/130890     WO-A1-2016/189388
WO-A2-2016/024134     US-A1- 2008 194 414
US-A1- 2011 039 304     US-A1- 2015 203 907
US-A1- 2016 068 889     US-A1- 2017 051 355

- ERIC J DUNCAVAGE ET AL: "Targeted next generation sequencing of clinically significant gene mutations and translocations in leukemia", MODERN PATHOLOGY, vol. 25, no. 6, 16 March 2012 (2012-03-16), GB, pages 795 - 804, XP055235397, ISSN: 0893-3952, DOI: 10.1038/modpathol.2012.29
- XI LIN ET AL: "Applications of targeted gene capture and next-generation sequencing technologies in studies of human deafness and other genetic disabilities", HEARING RESEARCH, ELSEVIER SCIENCE PUBLISHERS, AMSTERDAM, NL, vol. 288, no. 1, 6 January 2012 (2012-01-06), pages 67 - 76, XP028519246, ISSN: 0378-5955, [retrieved on 20120114], DOI: 10.1016/J.HEARES.2012.01.004

Remarks:
The complete document including Reference Table(s) and the Sequence Listing(s) can be downloaded from the EPO website

(52) Cooperative Patent Classification (CPC): (Cont.)

C-Sets
**C12Q 1/6806, C12Q 2525/185, C12Q 2525/204, C12Q 2535/122, C12Q 2537/159**

## Description

### Field of the Invention

The invention is in the field of biology, medicine and chemistry, more in particular in the field of molecular biology and more in particular in the field of molecular diagnostics.

### Background of the Invention

The identification of tumor biomarkers has been an important advance in the detection, diagnosis and treatment of a wide variety of cancers. Various methods of detecting tumor biomarkers are known in the art; however, additional methods are still needed, in particular methods that allow for detection of tumor biomarkers non-invasively, such as in a plasma sample (liquid biopsy). The identification of hereditary (germline) mutations in patients with cancer or high risk individuals suspected of cancer-predisposing syndrome is a useful clinical tool that enables early medical intervention, prophylactic surgery and close monitoring. These germline mutations can be identified in an individual's healthy tissue (such as buccal swab or lymphocytes).

Next generation sequencing (NGS) technologies have been implemented in the development of non-invasive prenatal testing (NIPT). In 2008, two independent groups demonstrated that NIPT of trisomy 21 could be achieved using next generation massively parallel shotgun sequencing (MPSS) (Chiu, R. W. et al.(2008) Proc. Natl. Acad. Sci. USA 105:20458-20463; Fan, H.C. et al.(2008) Proc. Natl. Acad. Sci. USA 105:16266-162710). Large-scale clinical studies using NGS for NIPT have been described (Palomaki, G.E. et al. (2011) Genet. Med. 13:913-920; Ehrich, M. et al. (2011) Am. J. Obstet. Gynecol. 204:205e1-11; Chen, E.Z. et al. (2011) PLoS One 6:e21791; Sehnert, A.J. et al. (2011) Clin. Chem. 57:1042-1049; Palomaki, G.E. et al. (2012); Genet. Med. 14:296-305; Bianchi, D.W. et al. (2012) Obstet. Gynecol. 119:890-901; Zimmerman, B. et al. (2012) Prenat. Diag. 32:1233-1241; Nicolaides, K.H. et al. (2013) Prenat. Diagn. 33:575-579; Sparks, A.B. et al. (2012) Prenat. Diagn. 32:3-9).

Initial NIPT approaches used massively parallel shotgun sequencing (MPSS) NGS methodologies (see e.g., US Patent No. 7,888,017; US Patent No. 8,008,018; US Patent No. 8,195,415; US Patent No. 8,296,076; US Patent No. 8,682,594; US Patent Publication 20110201507; US Patent Publication 20120270739). Thus, these approaches are whole genome-based. More recently, targeted-based NGS approaches for NIPT, in which only specific sequences of interest are sequenced, have been developed. For example, a targeted NIPT approach using TArget Capture Sequences (TACS) for identifying fetal chromosomal abnormalities using a maternal blood sample has been described (PCT Publication WO 2016/189388; US Patent Publication 2016/0340733; Koumbaris, G. et al. (2016) Clinical chemistry, 62(6), pp.848-855.). Such targeted approaches require significantly less sequencing than the MPSS approaches, since sequencing is only performed on specific loci on the target sequence of interest rather than across the WO2014/130890 discloses a method of assessing cancer by determining a profile of genetic abnormalities for said set of genes based on targeted sequencing.

Additional methodologies for NGS-based approaches are still needed, in particular approaches that can target specific sequences of interest, such as for example tumor biomarkers, thereby greatly reducing the amount of sequencing needed as compared to whole genome-based approaches, as well as increasing the read-depth of regions of interest, thus enabling detection of low signal to noise ratio regions. In particular, additional methodologies are still needed that allow for genetic aberrations present in diminutive amounts in a sample to be reliably detected, such as for example in the early detection of cancer.

### Summary of the Invention

This invention provides improved methods for enriching targeted genomic regions of interest to be analyzed by multiplexed parallel sequencing, wherein the enriched sequences are tumor biomarker sequences and the DNA sample used in the method is from a subject having or suspected of having a tumor. Accordingly, the methods allow for detection of tumor biomarkers in a variety of biological samples, including liquid samples, such as plasma samples (liquid biopsy), thereby providing non-invasive means for tumor detection and monitoring. The methods of the invention utilize a pool of TArget Capture Sequences (TACS) designed such that the sequences within the pool have features that optimize the efficiency, specificity and accuracy of genetic assessment of tumor biomarkers. The methods of the invention can be used, for example, in cancer diagnosis, cancer screening, cancer treatment regimen selection and/or cancer therapy monitoring.

Accordingly, in one aspect, the invention pertains to
method of diagnosing cancer in a subject suspected of having cancer based on the detection of at least one tumor biomarker sequence in a DNA sample from said subject, wherein the DNA sample is a blood sample or urine sample, and comprises cell free tumor DNA (cftDNA), wherein the method comprises: (a) preparing a sequencing library from the DNA sample; (b) hybridizing the sequencing library to a pool of double-stranded TArget Capture Sequences (TACS) that bind to one or more tumor biomarker sequences of interest, wherein: (i) the pool of TACS comprises a plurality of TACS families directed to different tumor biomarker sequences of interest, wherein each TACS family comprises a plurality of member sequences, wherein each member sequence binds to the same tumor biomarker sequence of interest but has different start and/or stop positions with respect to a reference coordinate system for the tumor biomarker sequence of interest, wherein the start and/or stop positions are staggered by 5-10 base pairs; (ii) each member sequence within the pool of TACS is between 150-260 base pairs in length, each member sequence having a 5' end and a 3' end; (iii) each member sequence binds to the tumor biomarker sequence of interest at least 50 base pairs away, on both the 5' end and the 3' end, from regions harboring Copy Number Variations (CNVs), Segmental duplications or repetitive DNA elements; and (iv) the GC content of the pool of TACS is between 19% and 80%, as determined by calculating the GC content of each member within the pool of TACS; (c) isolating members of the sequencing library that bind to the pool of TACS to obtain an enriched library; (d) amplifying and sequencing the enriched library; and (e) analyzing the enriched library sequences for the presence or absence of the at least one tumor biomarker sequence, thereby diagnosing the patient.

embodiment, the pool of TACS comprises a plurality of TACS families, wherein each member of a TACS family binds to the same tumor biomarker sequence of interest but with different start and/or stop positions on the sequence with respect to a reference coordinate system (i.e., binding of TACS family members to the target sequence is staggered) to thereby enrich for target sequences of interest, followed by massive parallel sequencing and statistical analysis of the enriched population. The use of families of TACS with the TACS pool that bind to each target sequence of interest, as compared to use of a single TACS within the TACS pool that binds to each target sequence of interest, significantly increases enrichment for the target sequences of interest, as evidenced by a greater than 50% average increase in read-depth for the family of TACS versus a single TACS. Herein, the mutations detected or biomarkers detected may be due to somatic mutation or may be hereditary, i.e already present in the germ line.

Accordingly, in one embodiment, the pool of TACS comprises a plurality of TACS families directed to different tumor biomarker sequences of interest, wherein each TACS family comprises a plurality of member sequences, wherein each member sequence binds to the same tumor biomarker sequence of interest but has different start and/or stop positions with respect to a reference coordinate system for the genomic sequence of interest.

In certain embodiments, each TACS family comprises at least 3 member sequences or at least 5 member sequences. Alternative numbers of member sequences in each TACS family are described herein. In one embodiment, the pool of TACS comprises at least 50 different TACS families. Alternative numbers of different TACS families within the pool of TACS are described herein. In certain embodiments, the start and/or stop positions for the member sequences within a TACS family, with respect to a reference coordinate system for the genomic sequence of interest, are staggered by at least 3 base pairs or at least 5 base pairs or by at least 10 base pairs. Alternative lengths (sizes) for the number of base pairs within the stagger are described herein.

In one embodiment, each member sequence within the pool of TACS is at least 160 base pairs in length. In certain embodiments, the GC content of the pool of TACS is between 19% and 80% or is between 19% and 46%. Alternative % ranges for the GC content of the pool of TACS are described herein.

In one embodiment, the pool of TACS is fixed to a solid support. For example, in one embodiment, the TACS are biotinylated and are bound to streptavidin-coated magnetic beads.

In one embodiment, amplification of the enriched library is performed in the presence of blocking sequences that inhibit amplification of wild-type sequences.

In one embodiment, members of the sequencing library that bind to the pool of TACS are partially complementary to the TACS.

In one embodiment, the statistical analysis comprises a segmentation algorithm, for example, likelihood-based segmentation, segmentation using small overlapping windows, segmentation using parallel pairwise testing, and combinations thereof. In one embodiment, the statistical analysis comprises a score-based classification system. In one embodiment, sequencing of the enriched library provides a read-depth for the genomic sequences of interest and read-depths for reference loci and the statistical analysis comprises applying an algorithm that tests sequentially the read-depth of the loci of from the genomic sequences of interest against the read-depth of the reference loci, the algorithm comprising steps for: (a) removal of inadequately sequenced loci; (b) GC-content bias alleviation; and (c) genetic status determination. In one embodiment, GC-content bias is alleviated by grouping together loci of matching GC content. In one embodiment, sequencing of the enriched library provides the number and size of sequenced fragments for TACS-specific coordinates and the statistical analysis comprises applying an algorithm that tests sequentially the fragment-size proportion for the genomic sequence of interest against the fragment-size proportion of the reference loci, the algorithm comprising steps for: (a) removal of fragment-size outliers; (b) fragment-size proportion calculation; and (c) genetic status determination.

In one embodiment, the DNA sample comprises cell free tumor DNA (cftDNA). In various embodiments, the DNA sample is selected from a group comprising of a plasma sample, a urine sample, a sputum sample, a cerebrospinal fluid sample, an ascites sample and a pleural effusion sample from subject having or suspected of having a tumor. In one embodiment, the DNA sample is from a tissue sample from a subject having or suspected of having a tumor.

In one embodiment, the pool of TACS binds to a plurality of tumor biomarker sequences of interest selected from a group comprising ABL, AKT, AKT1, ALK, APC, AR, ARAF, ATM, BAP1, BARD1, BCL, BMPR1A, BRAF, BRCA, BRCA1, BRCA2, BRIP1, CDH1, CDKN, CHEK2, CTNNB1, DDB2, DDR2, DICER1, EGFR, EPCAM, ErbB, ErcC, ESR1, FANCA, FANCB, FANCC, FANCD2, FANCE, FANCF, FANCG, FANCI, FANCL, FANCM, FBXW7, FGFR, FLT, FLT3, FOXA1, FOXL2, GATA3, GNA11, GNAQ, GNAS, GREM1, HOX, HOXB13, HRAS, IDH1, JAK, JAK2, KEAP1, KIT, KRAS, MAP2Ks, MAP3Ks, MET, MLH1, MPL, MRE11A, MSH2, MSH6, MTOR, MUTYH, NBN, NPM1, NRAS, NTRK1, PALB2, PDGFRs, PI3KCs, PMS2, POLD1, POLE, POLH, PTEN, RAD50, RAD51C, RAD51D, RAF1, RB1, RET, RUNX1, SLX4, SMAD, SMAD4, SMARCA4, SPOP, STAT, STK11, TP53, VHL, XPA and XPC, and combinations thereof.

In another embodiment, the pool of TACS binds to a plurality of tumor biomarker sequences of interest selected from a group comprising , AKT1, ALK, APC, AR, ARAF, ATM, BAP1, BARD1, BMPR1A, BRAF, BRCA1, BRCA2, BRIP1, CDH1, CDK4, CDKN2A (p14ARF), CDKN2A (p16INK4a), CHEK2, CTNNB1, DDB2, DDR2, DICER1, EGFR, EPCAM, ERBB2, ERBB3, ERBB4, ERCC1, ERCC2, ERCC3, ERCC4, ERCC5, ESR1, FANCA, FANCB, FANCC, FANCD2, FANCE, FANCF, FANCG, FANCI, FANCL, FANCM, FBXW7, FGFR1, FGFR2, FLT3, FOXA1, FOXL2, GATA3, GNA11, GNAQ, GNAS, GREM1, HOXB13, IDH1, IDH2, JAK2, KEAP1, KIT, KRAS, MAP2K1, MAP3K1, MEN1, MET, MLH1, MPL, MRE11A, MSH2, MSH6, MTOR, MUTYH, MYC, MYCN, NBN, NPM1, NRAS, NTRK1, PALB2, PDGFRA, PIK3CA, PIK3CB, PMS2, POLD1, POLE, POLH, PTEN, RAD50, RAD51C, RAD51D, RAF1, RB1, RET, ROS1, RUNX1, SDHA, SDHAF2, SDHB, SDHC, SDHD, SLX4, SMAD4, SMARCA4, SPOP, STAT, STK11, TMPRSS2, TP53, VHL, XPA, XPC and combinations thereof.

In one embodiment, the pool of TACS binds to a plurality of tumor biomarker sequences of interest selected from a group comprising EGFR_6240, KRAS_521, EGFR_6225, NRAS_578, NRAS_580, PIK3CA_763, EGFR_13553, EGFR_18430, BRAF_476, KIT_1314, NRAS_584, EGFR_12378, and combinations thereof.

In another embodiment, the pool of TACS binds to a plurality of tumor biomarker sequences of interest selected from a group comprising COSM6240 (EGFR_6240), COSM521 (KRAS_521), COSM6225 (EGFR_6225), COSM578 (NRAS_578), COSM580 (NRAS_580), COSM763 (PIK3CA_763), COSM13553 (EGFR_13553), COSM18430 (EGFR_18430), COSM476 (BRAF_476), COSM1314 (KIT_1314), COSM584 (NRAS_584), COSM12378 (EGFR_12378), and combinations thereof, wherein the identifiers refer to the COSMIC database ID number of the biomarker.

In one embodiment, the method further comprises making a diagnosis of the subject based on detection of at least one tumor biomarker sequence. In another embodiment, the method further comprises selecting a therapeutic regimen for the subject based on detection of at least one tumor biomarker sequence. In yet another embodiment, the method further comprises monitoring treatment efficacy of a therapeutic regimen in the subject based on detection of at least one tumor biomarker sequence.

In another aspect, kits for performing the methods of the invention are also encompassed.

**Brief Description of the Figures**

The patent or application file contains at least one drawing executed in color. Copies of this patent or patent application publication with color drawing(s) will be provided by the Office upon request and payment of the necessary fee.

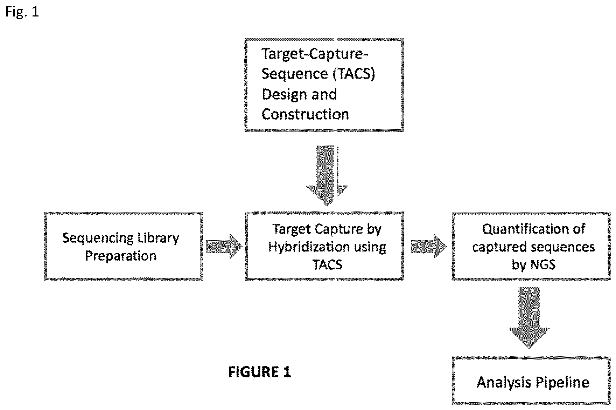

Figure 1 is a schematic diagram of multiplexed parallel analysis of targeted genomic regions for non-invasive prenatal testing using TArget Capture Sequences (TACS).

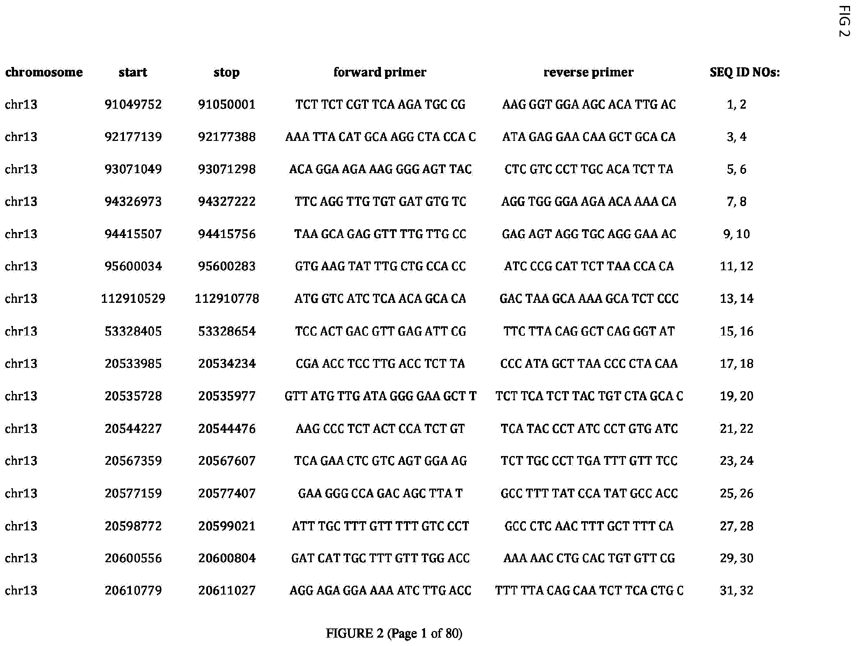

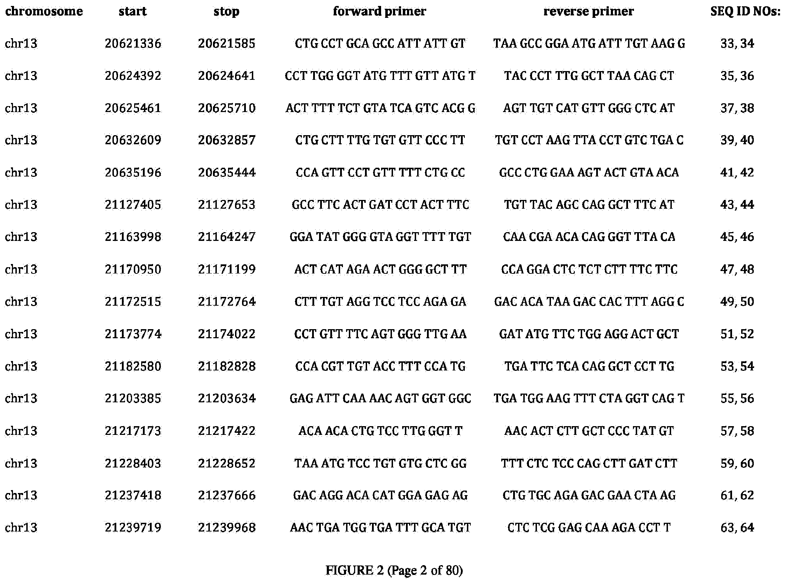

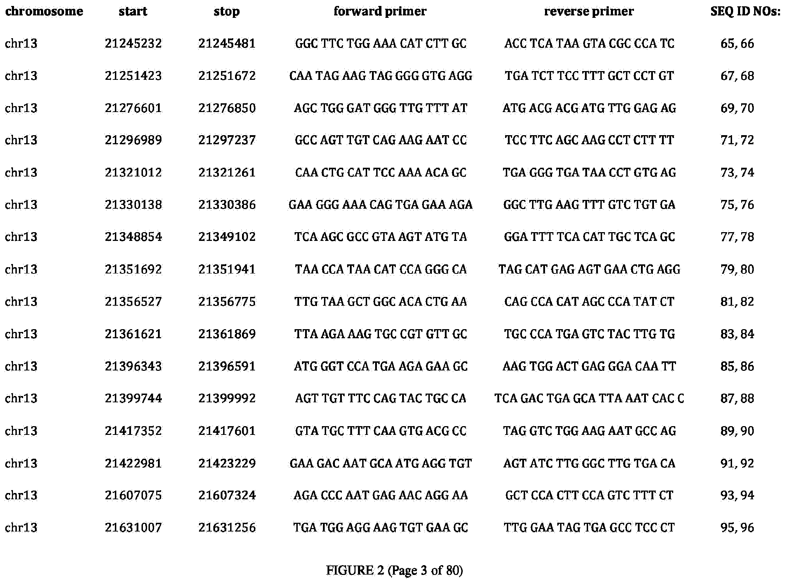

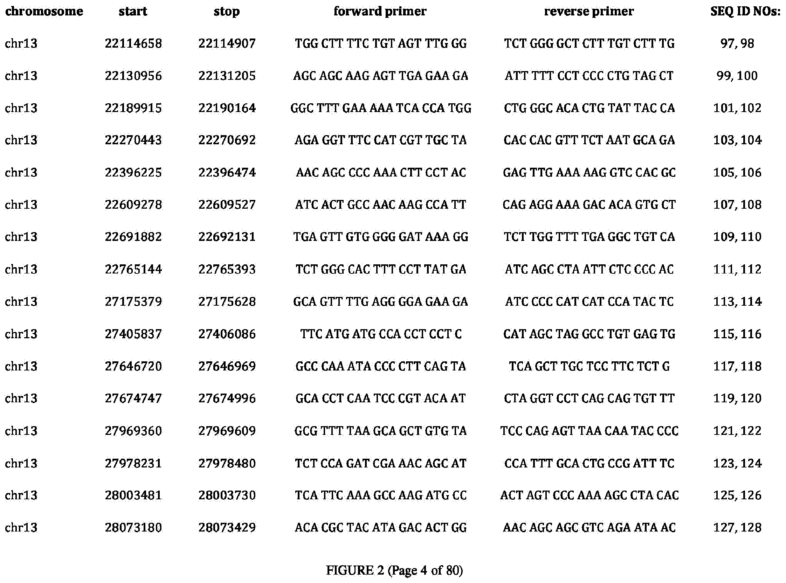

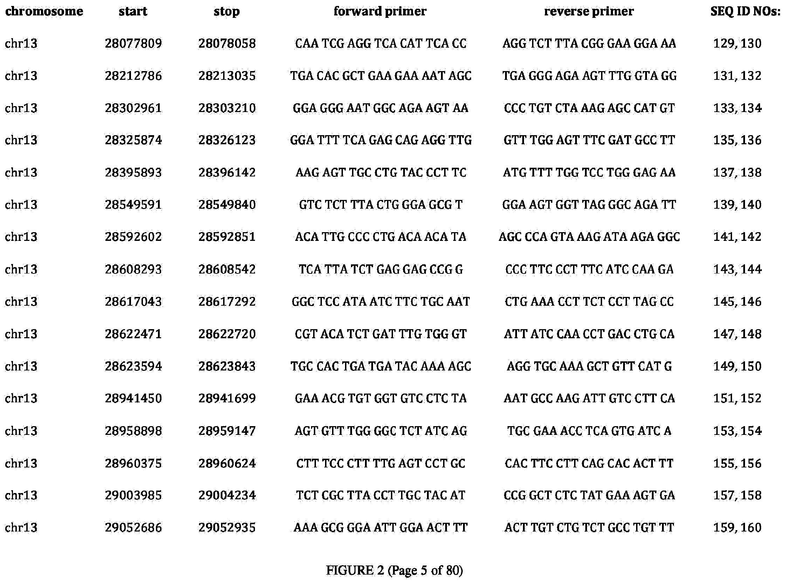

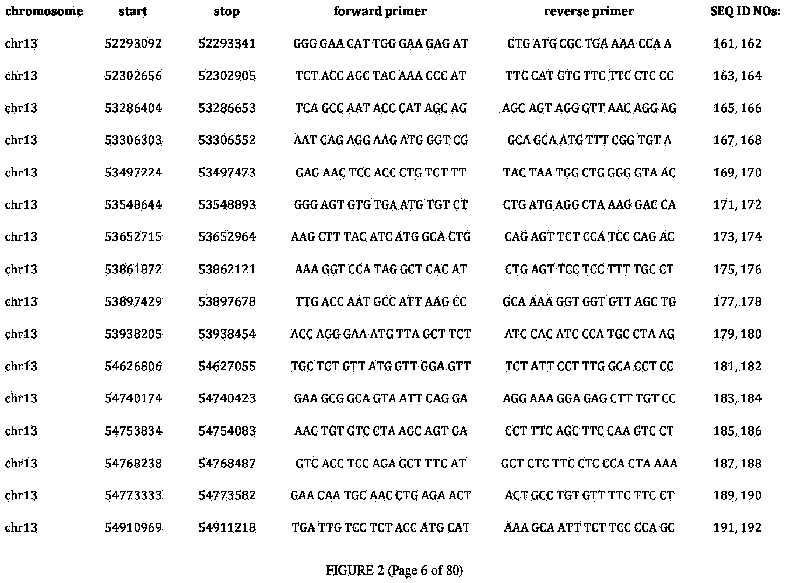

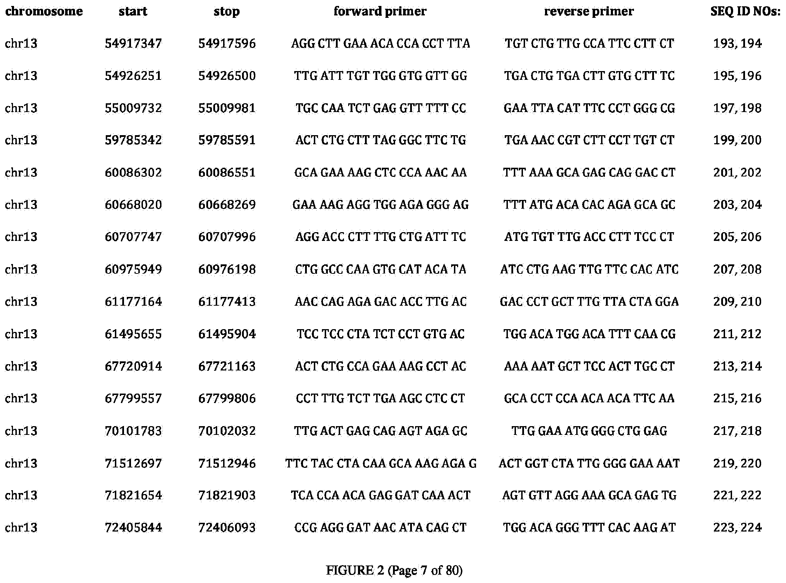

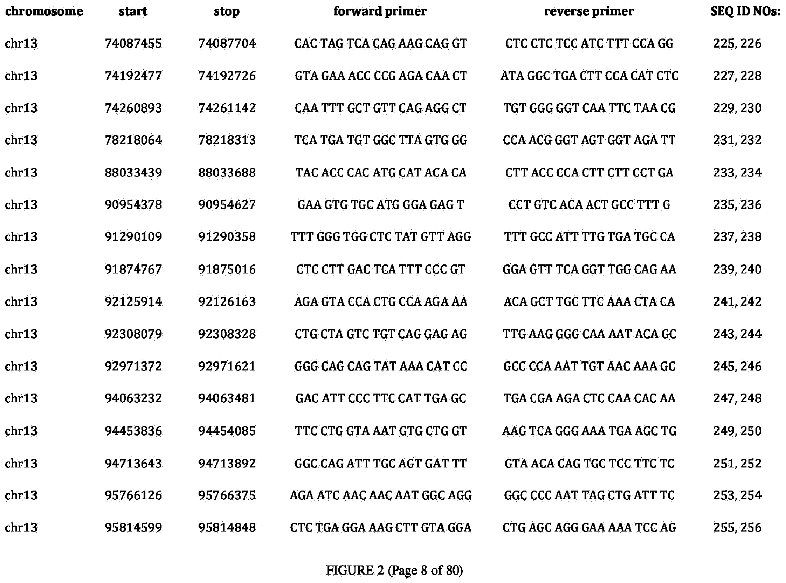

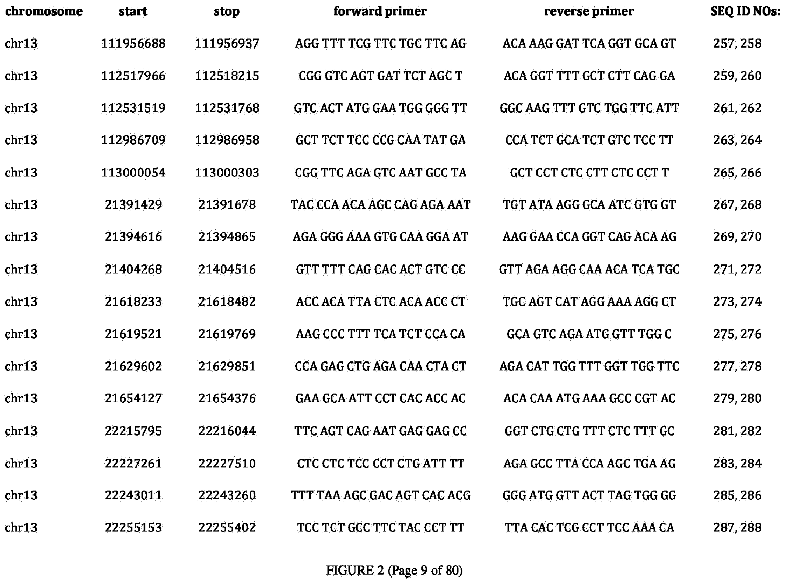

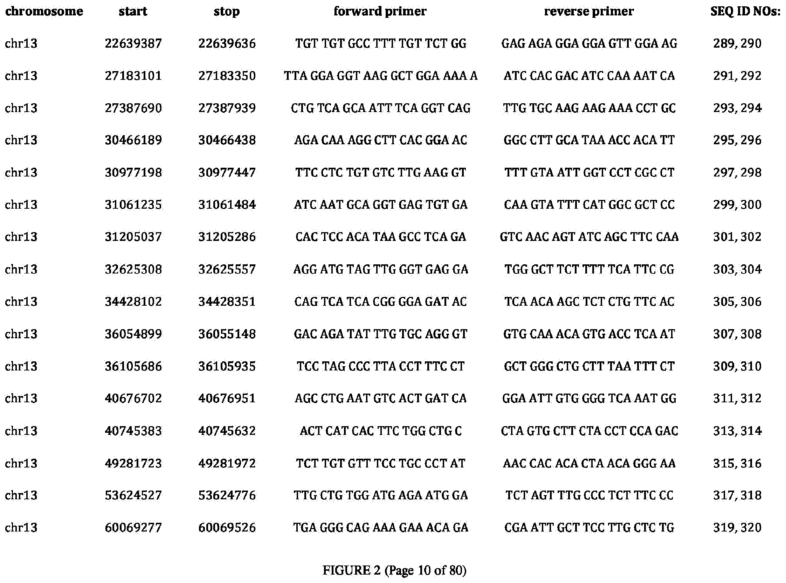

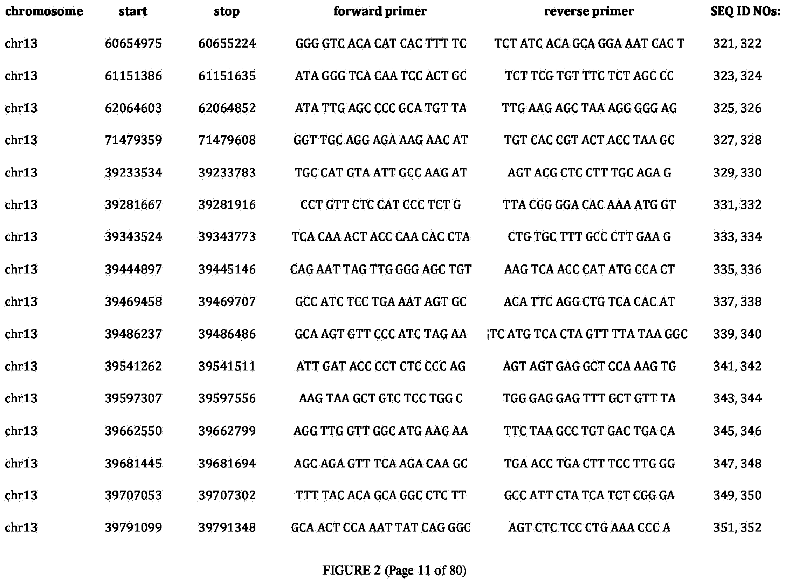

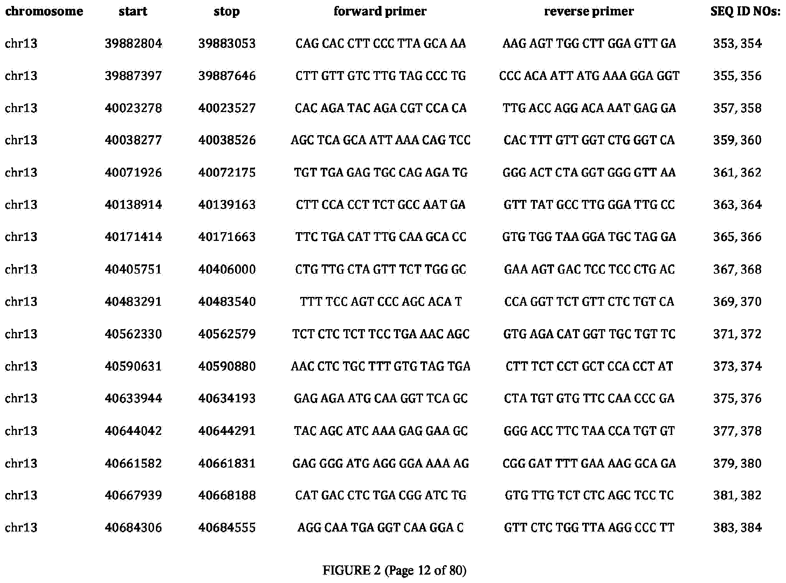

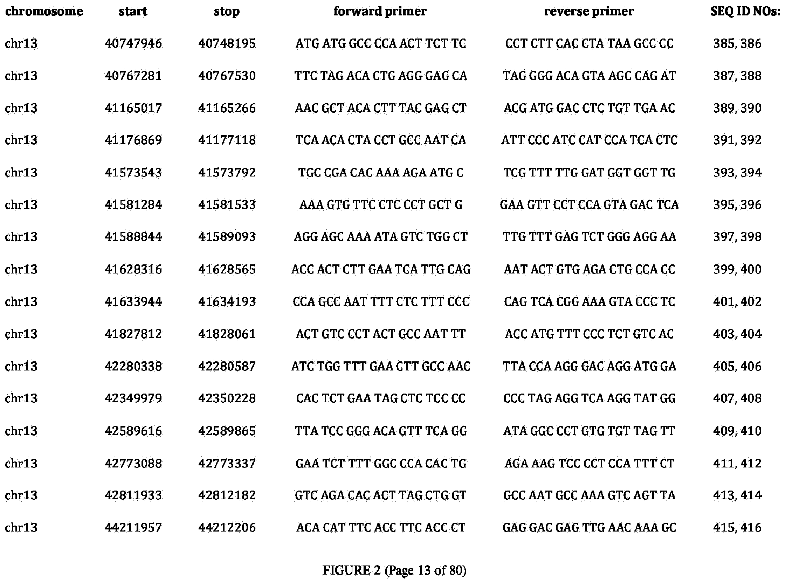

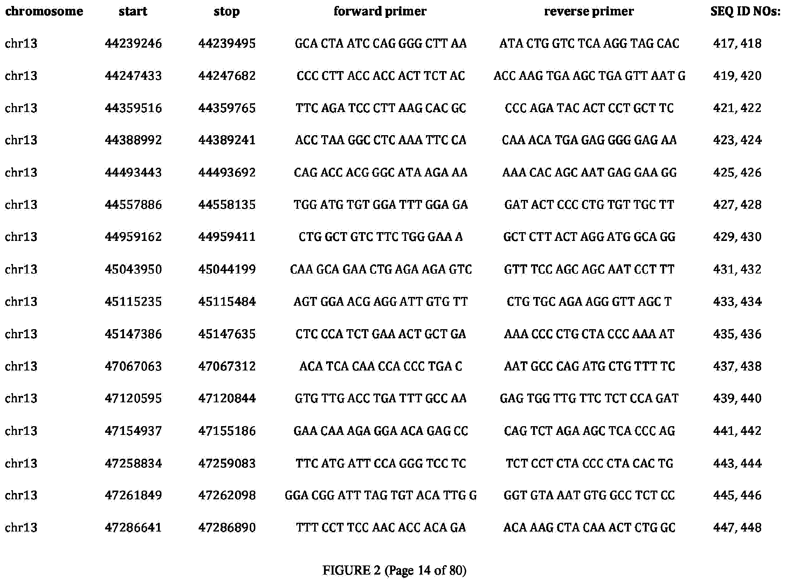

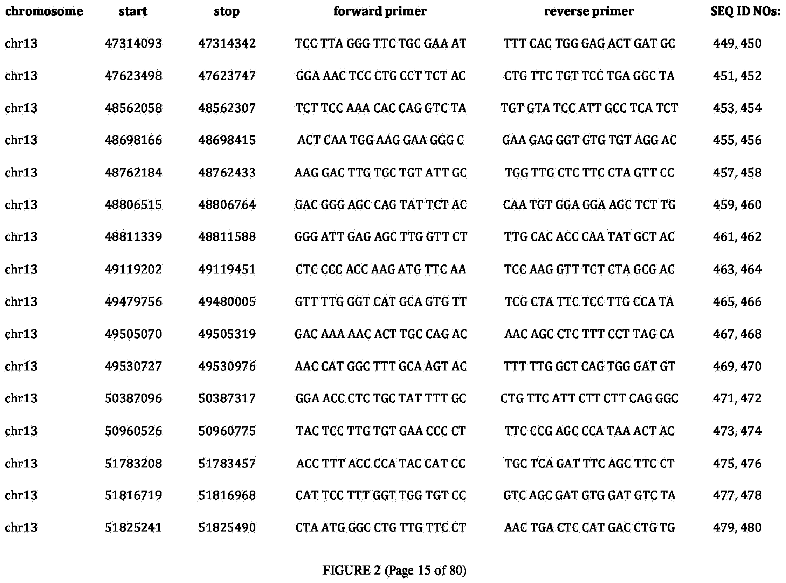

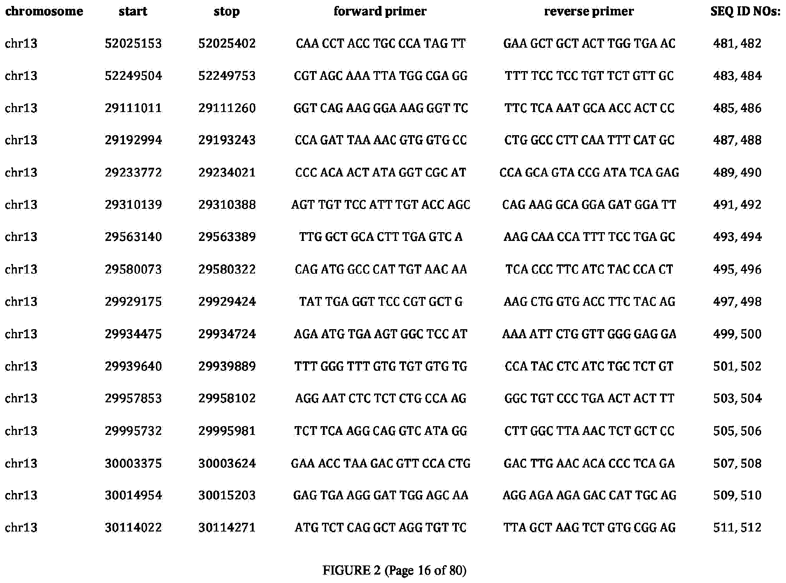

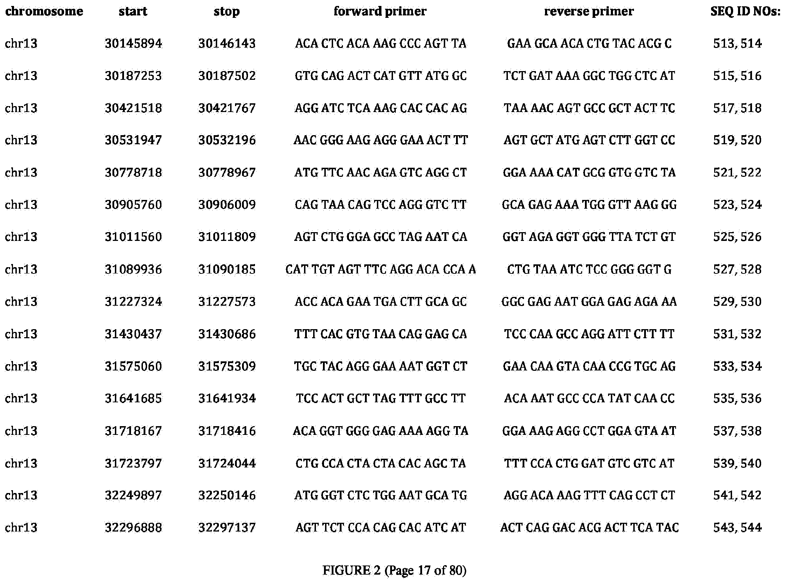

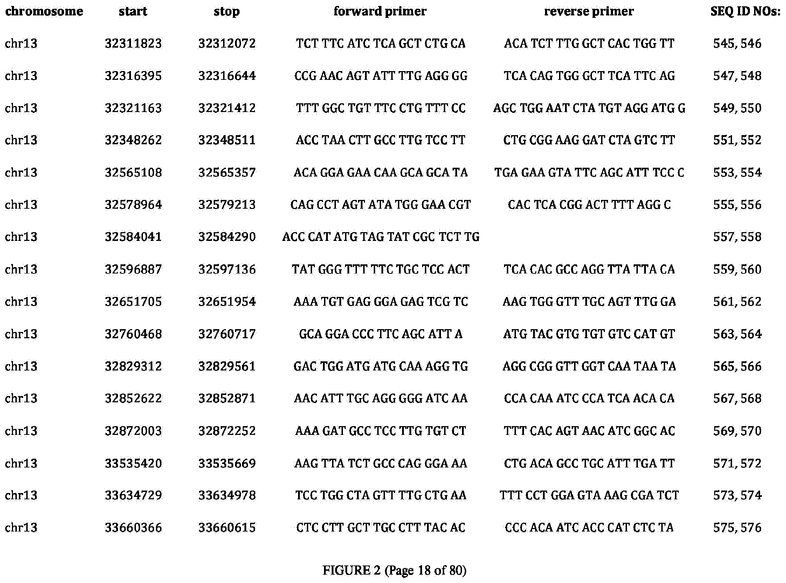

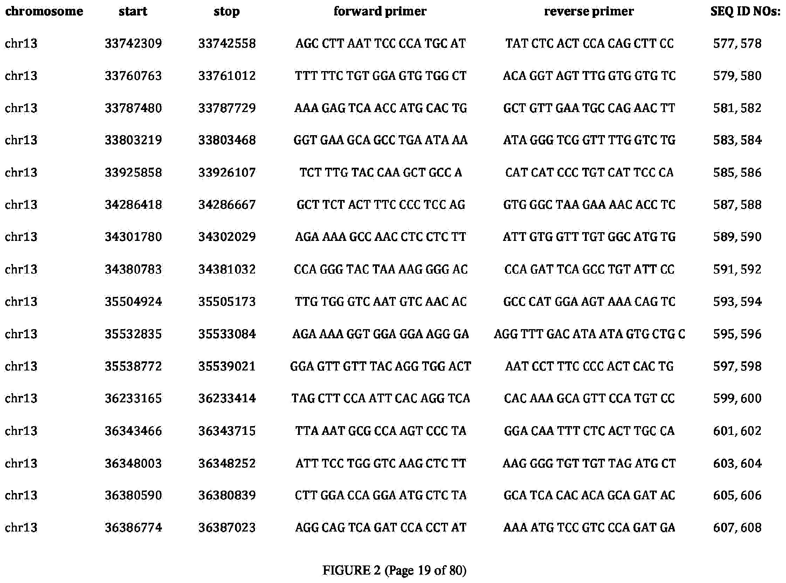

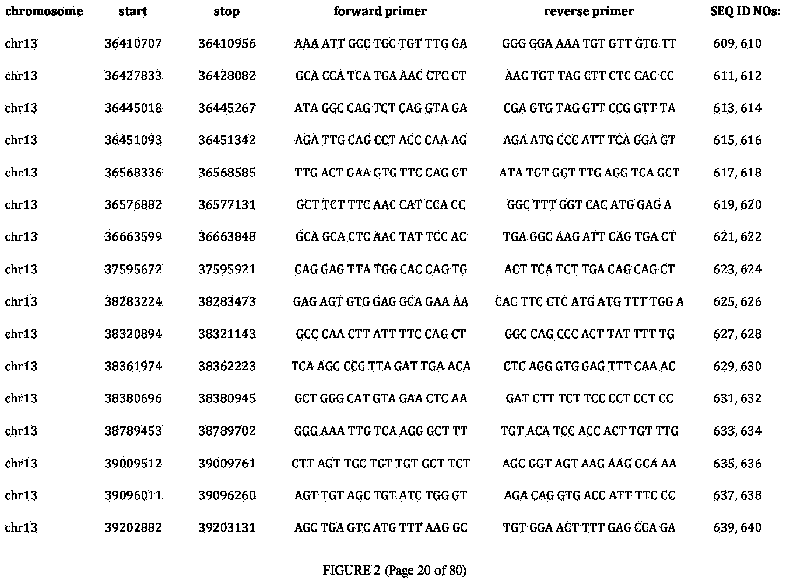

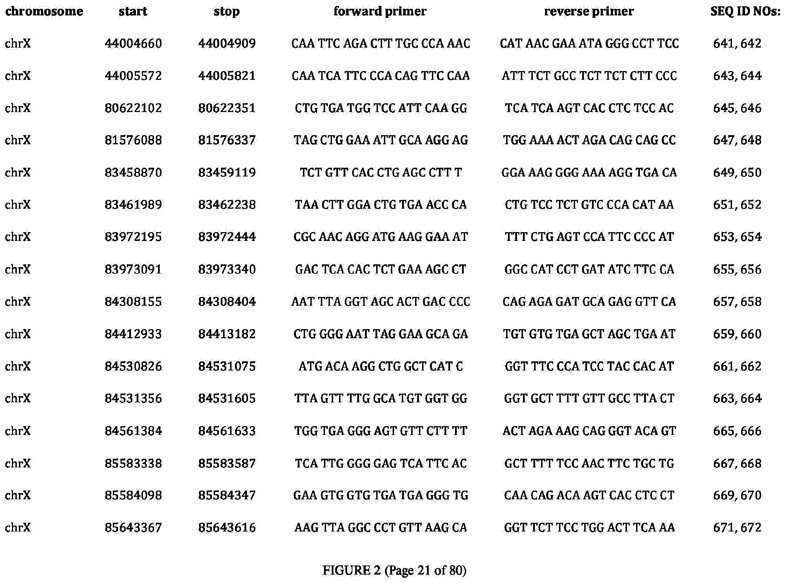

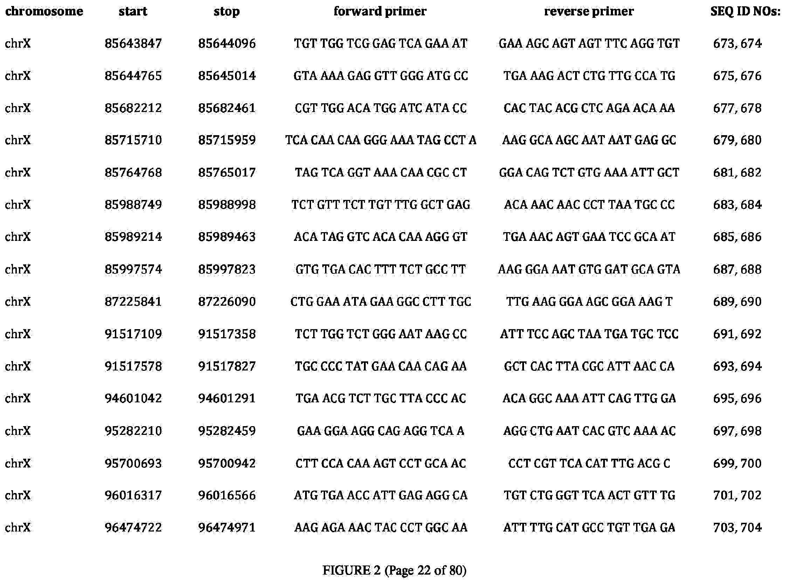

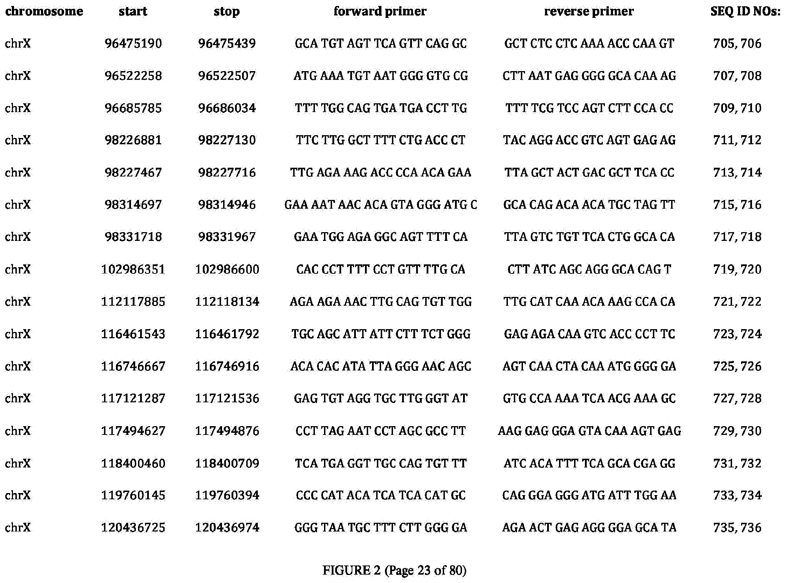

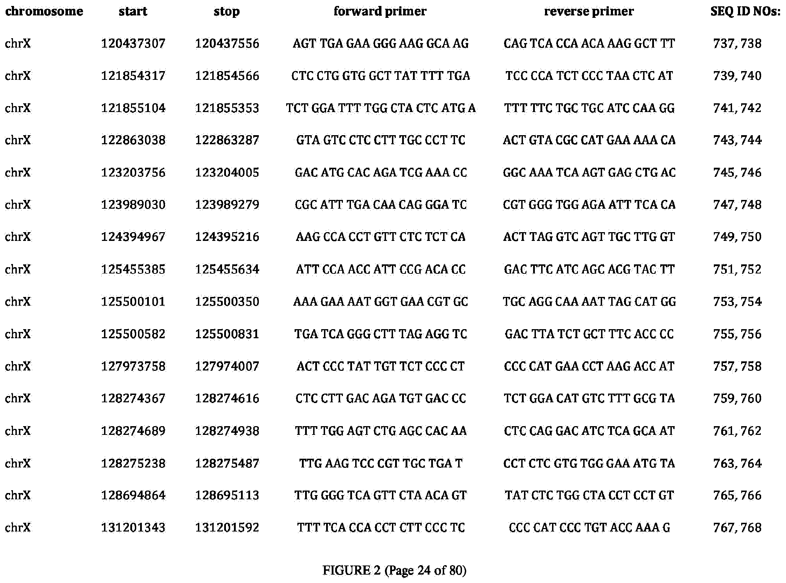

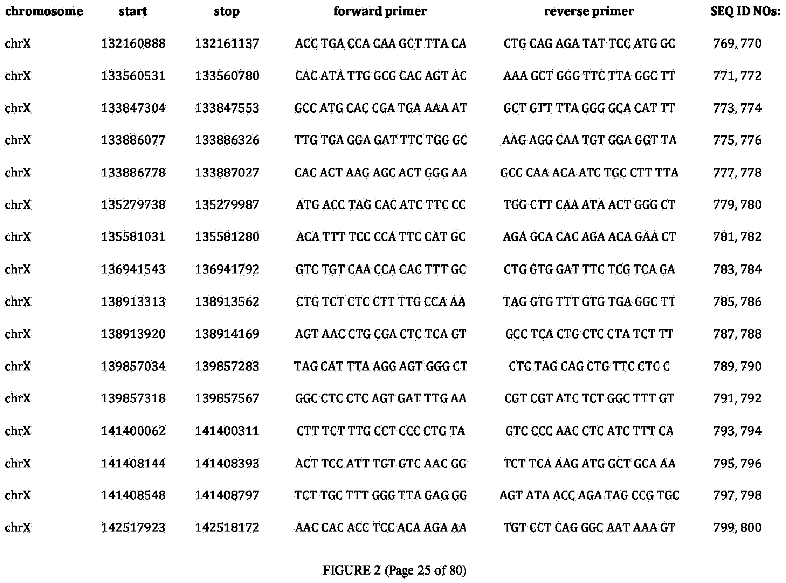

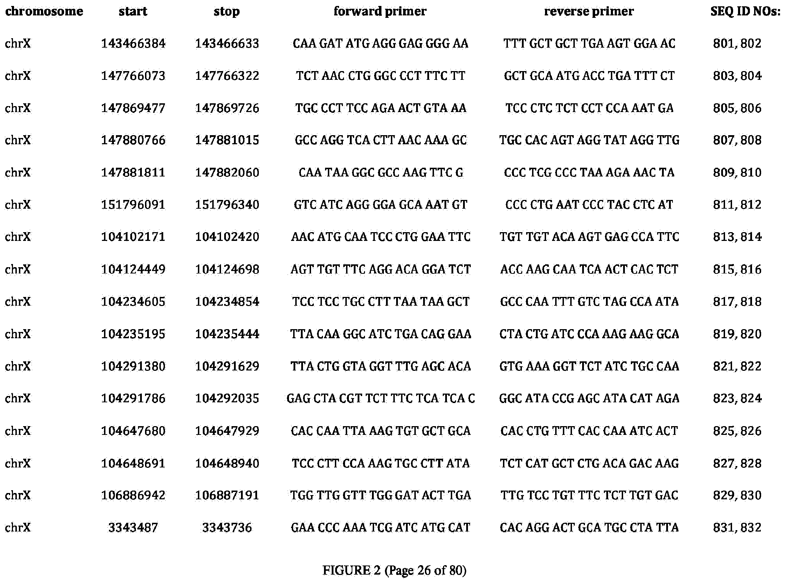

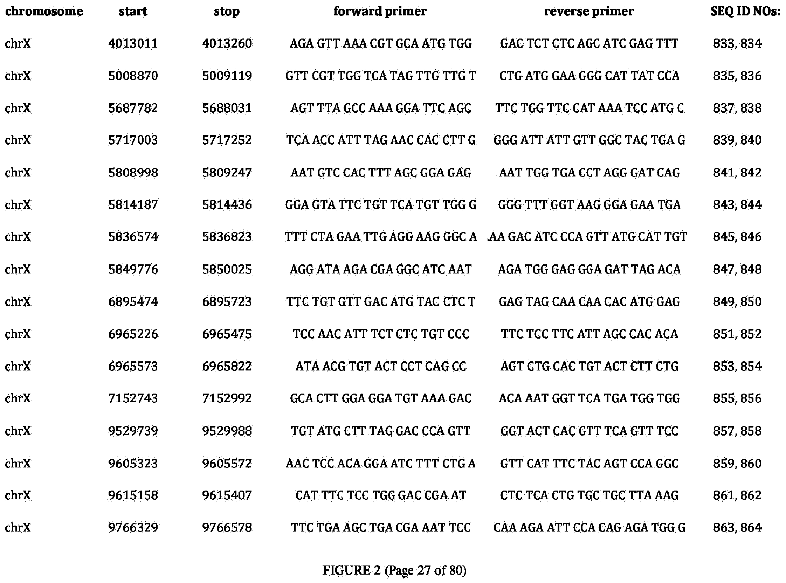

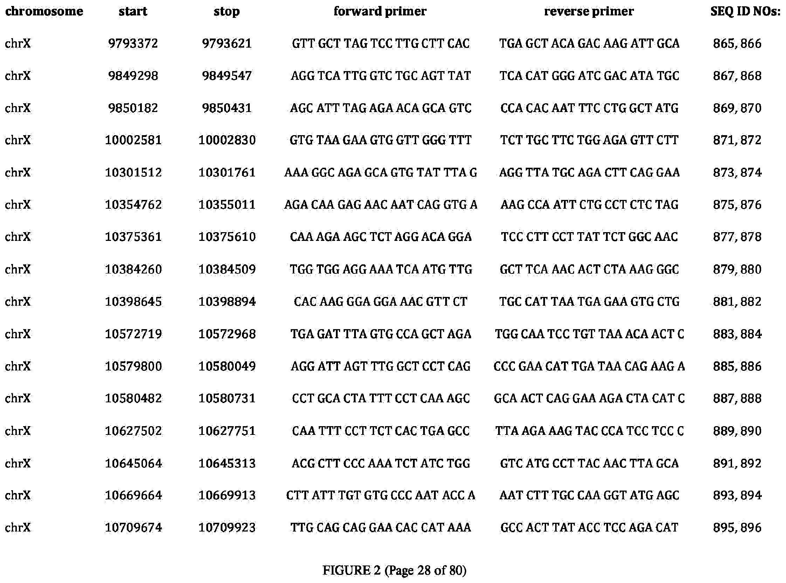

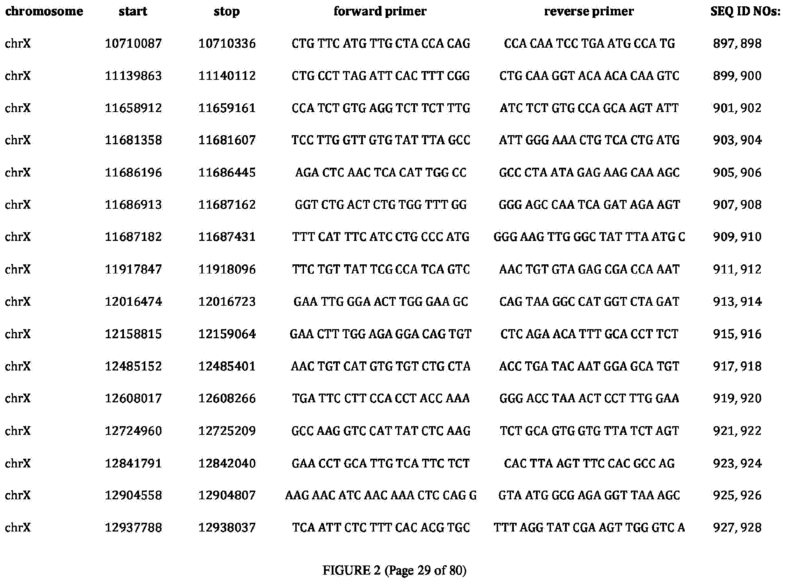

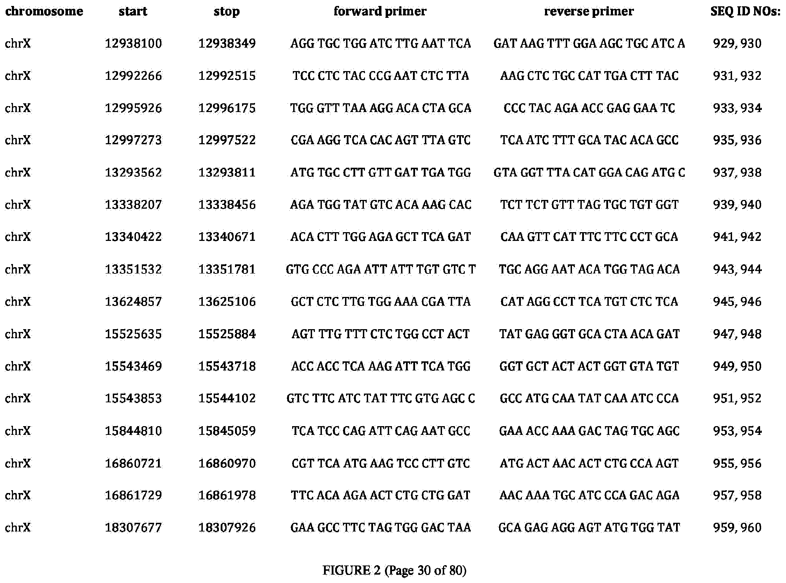

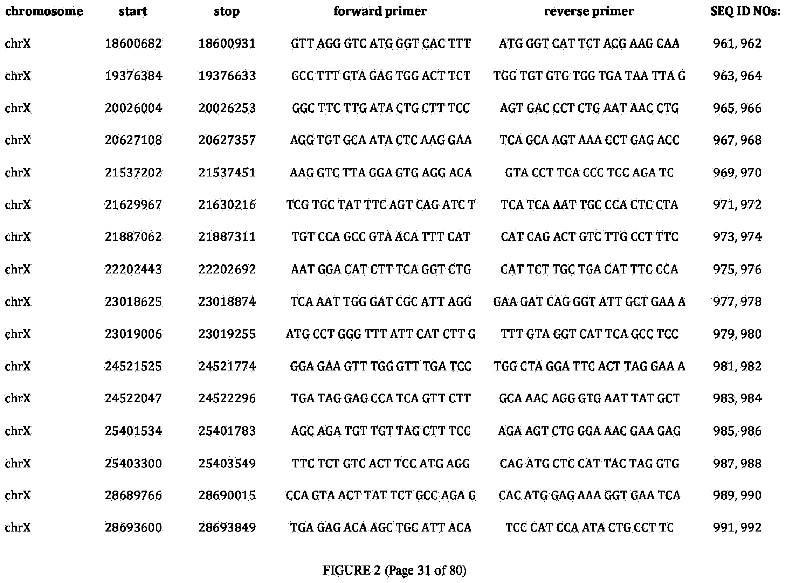

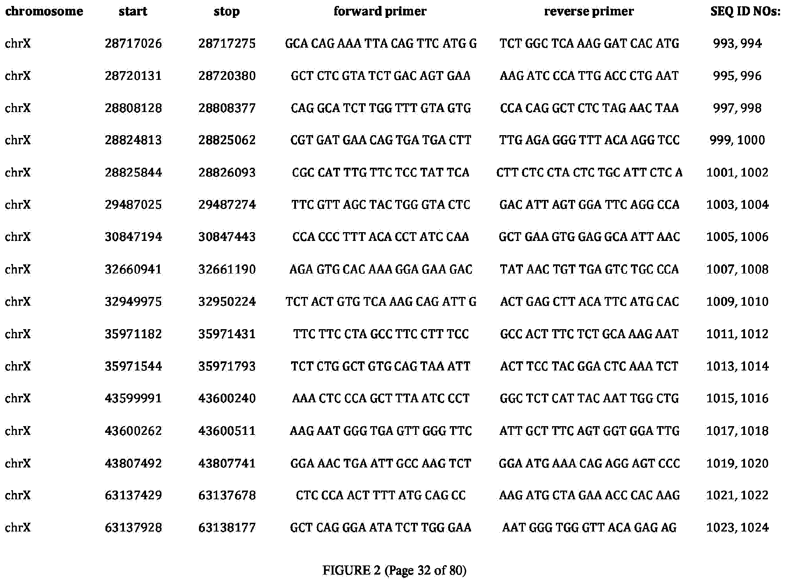

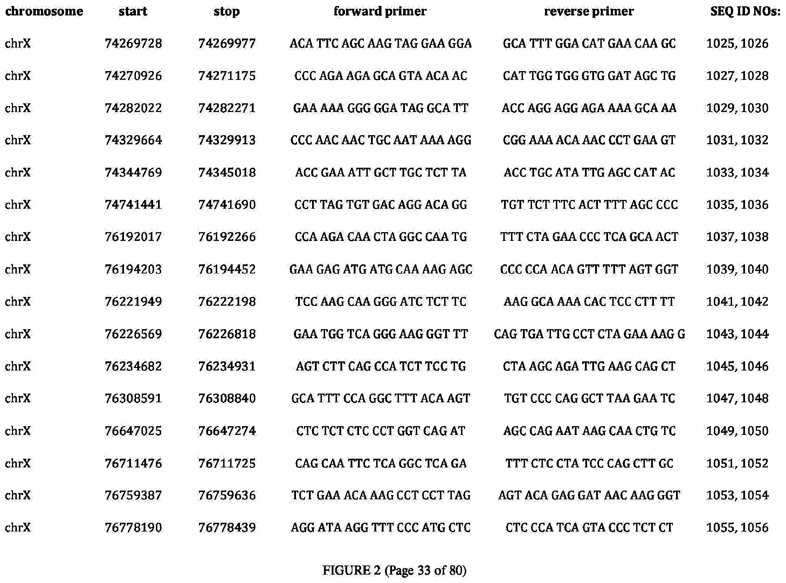

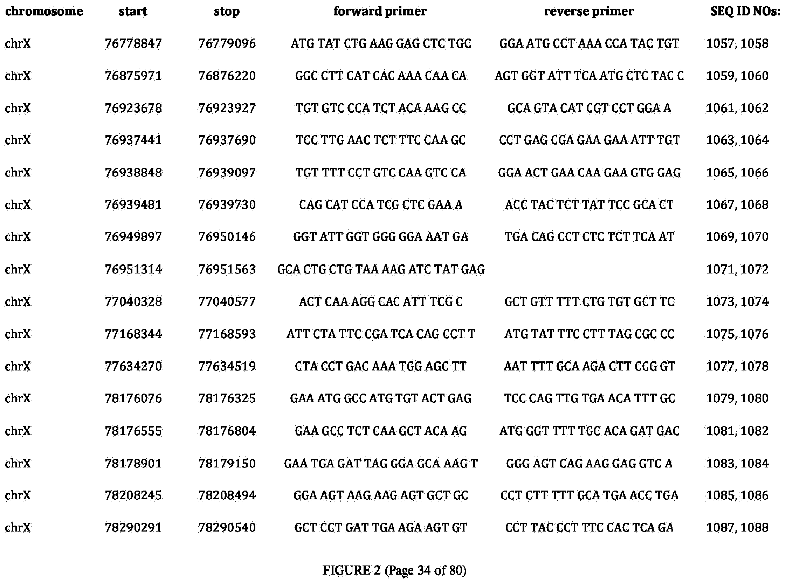

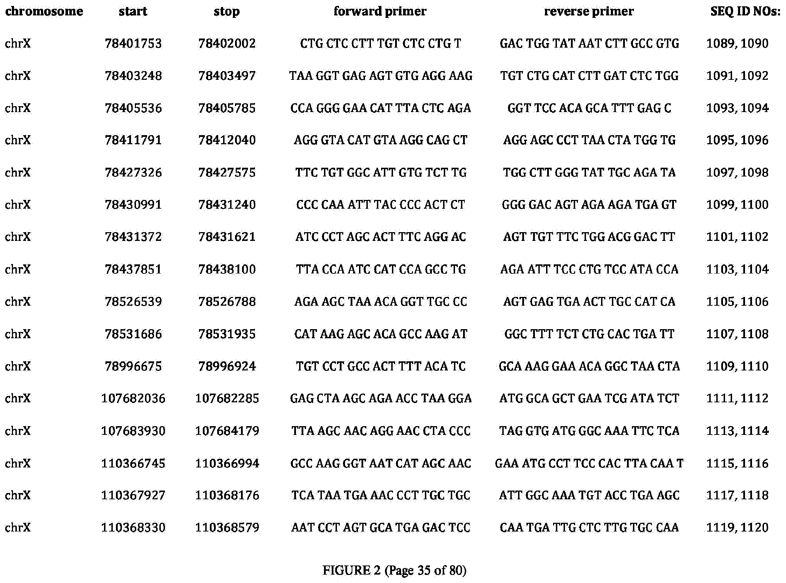

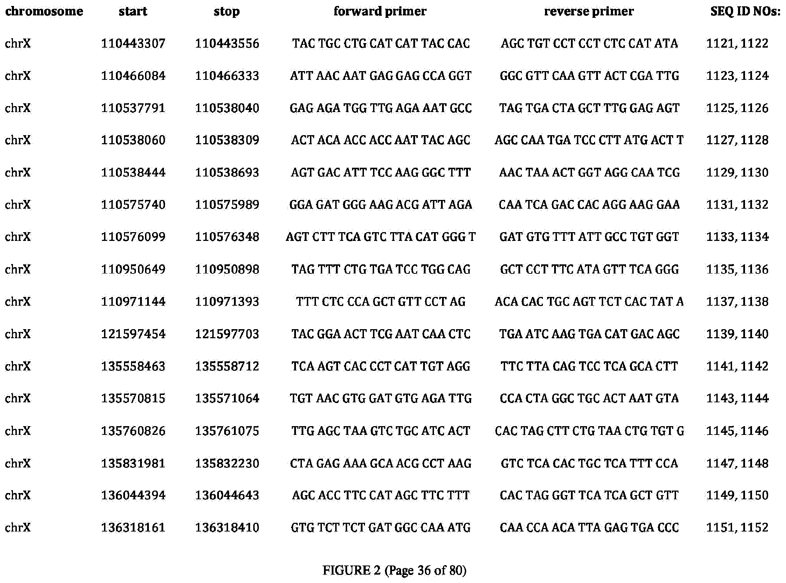

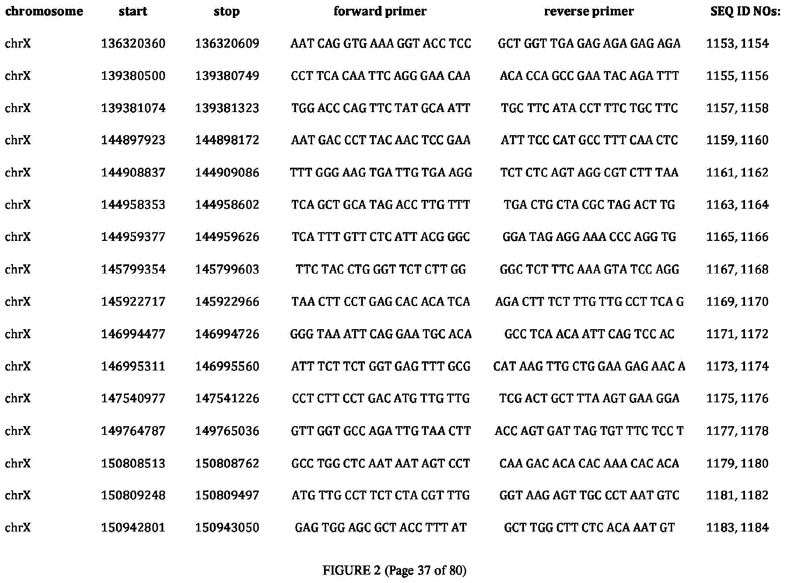

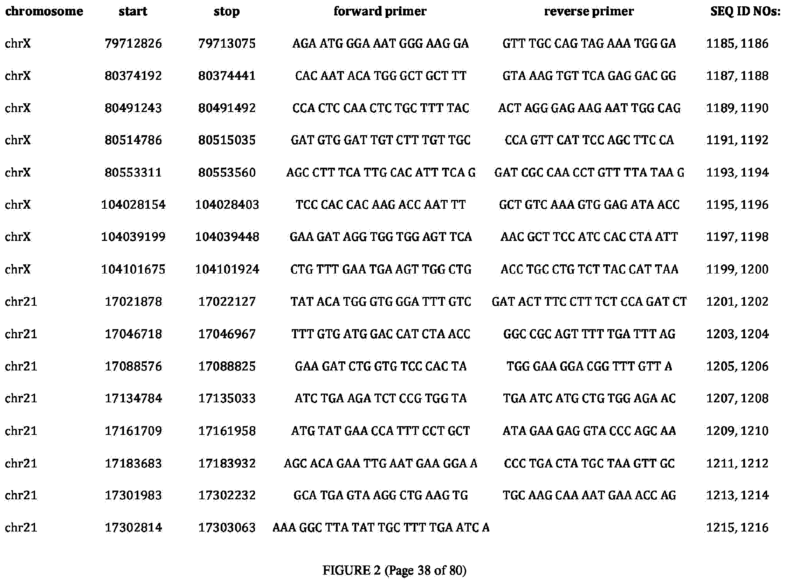

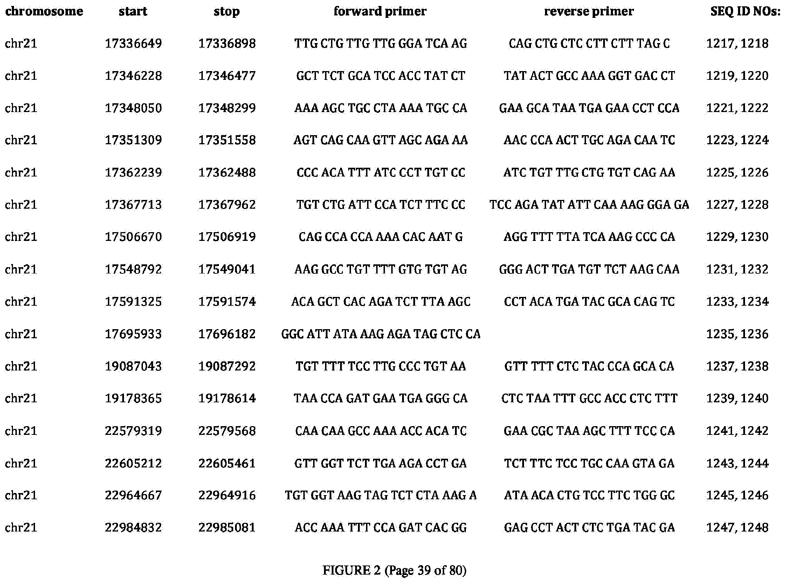

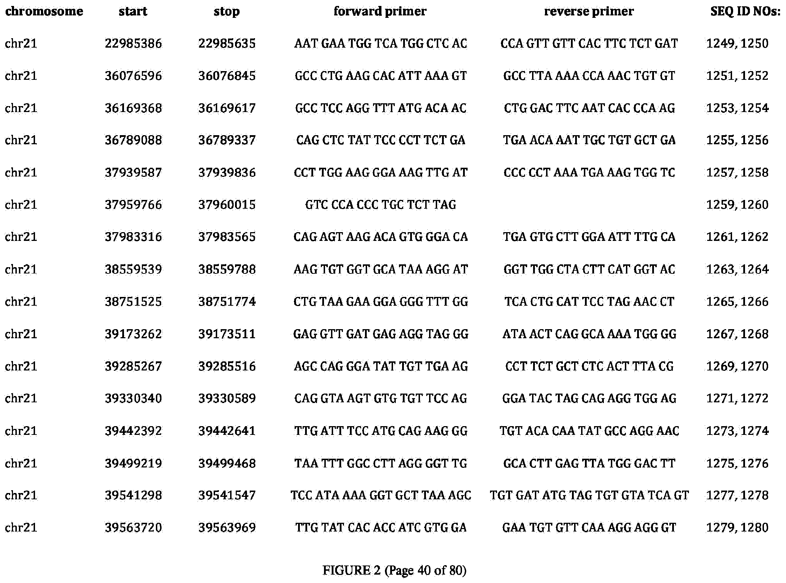

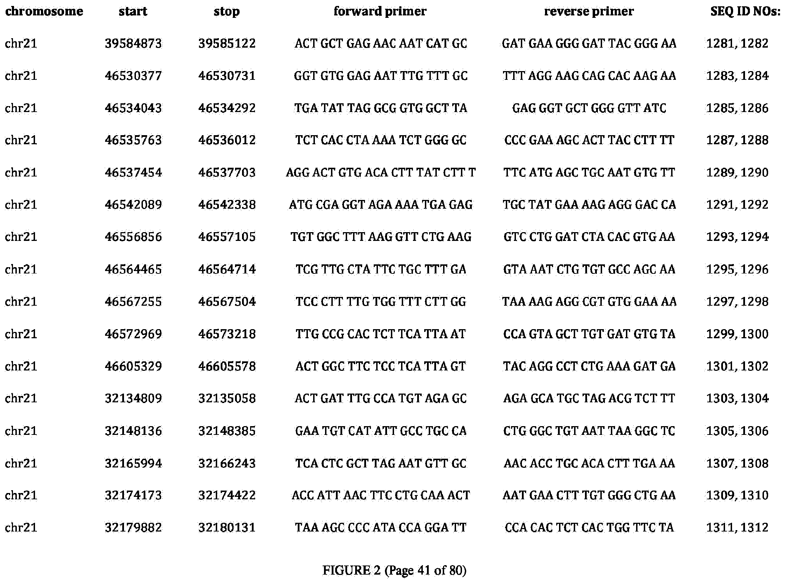

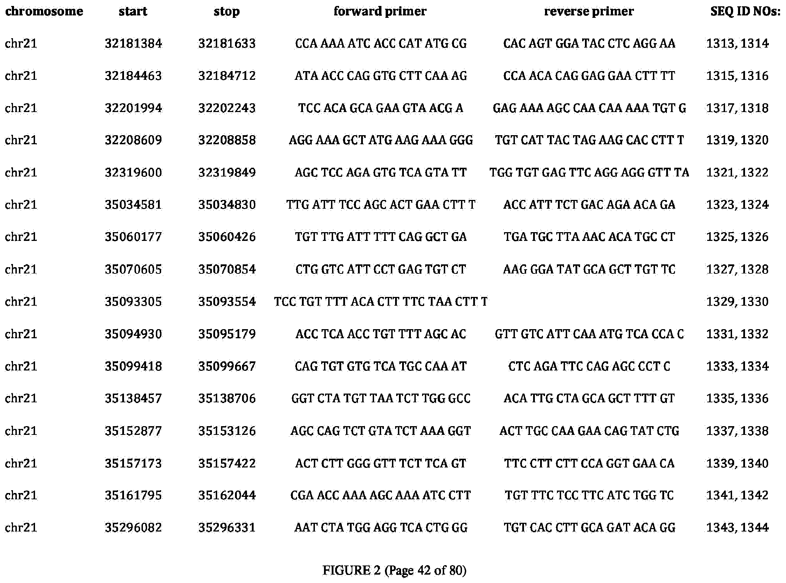

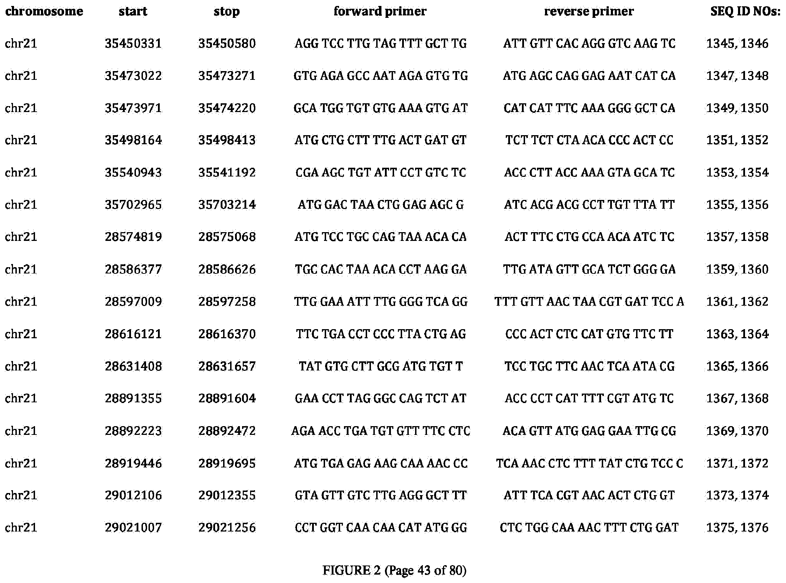

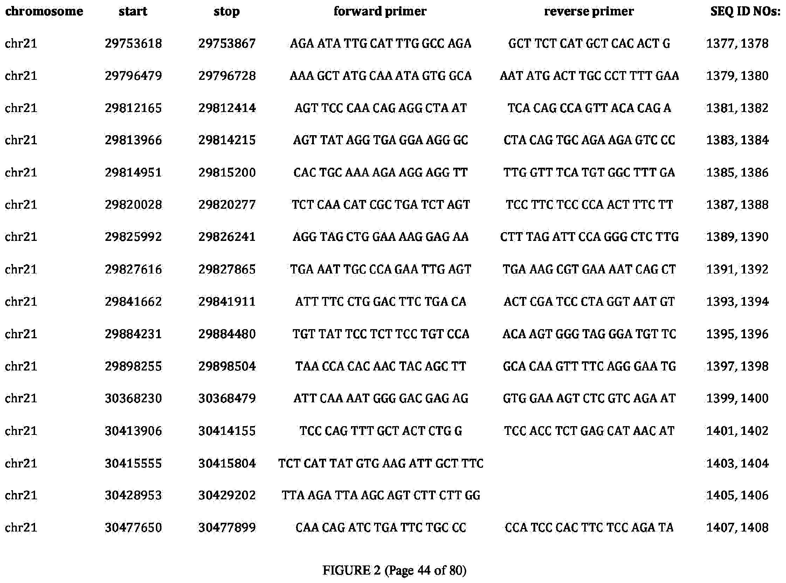

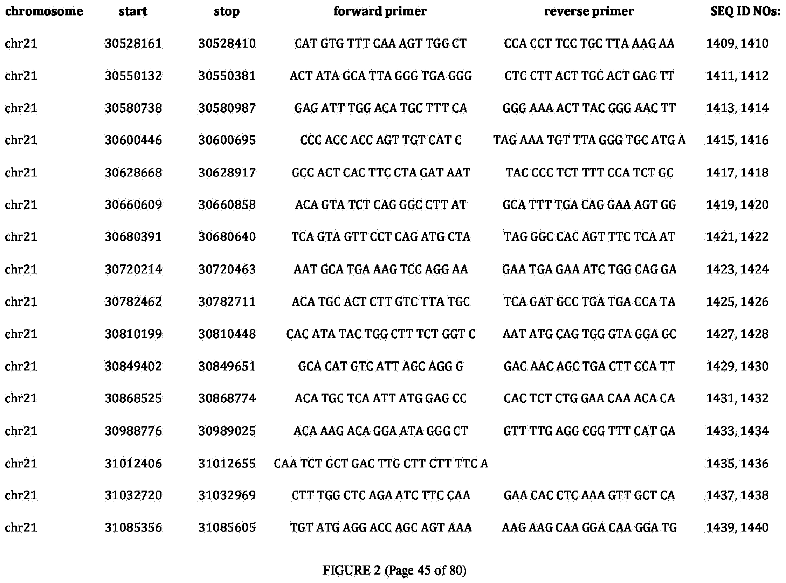

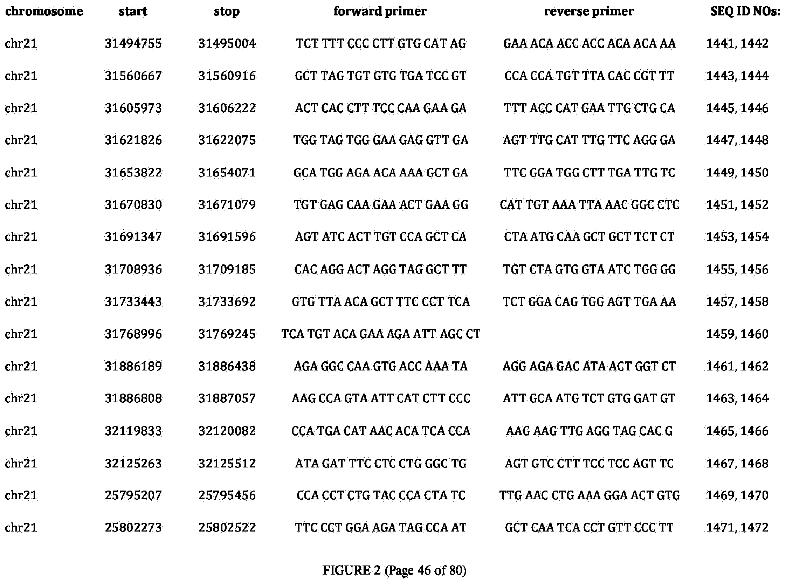

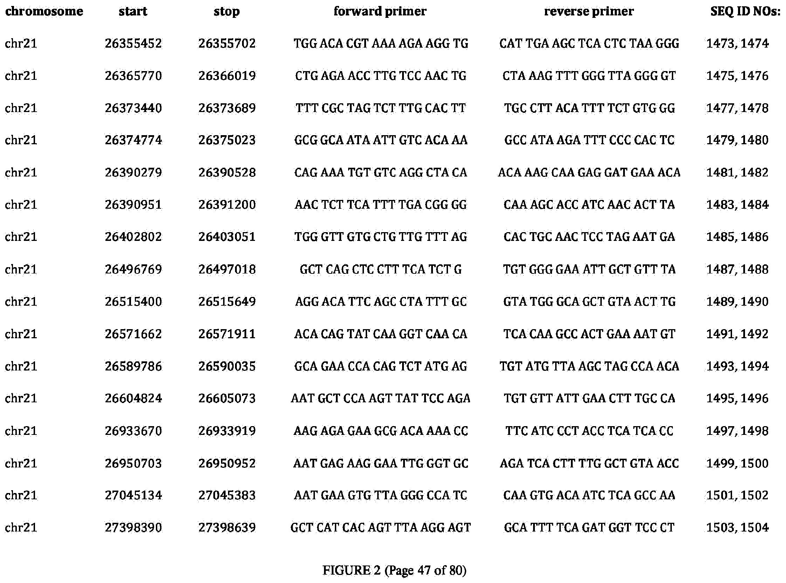

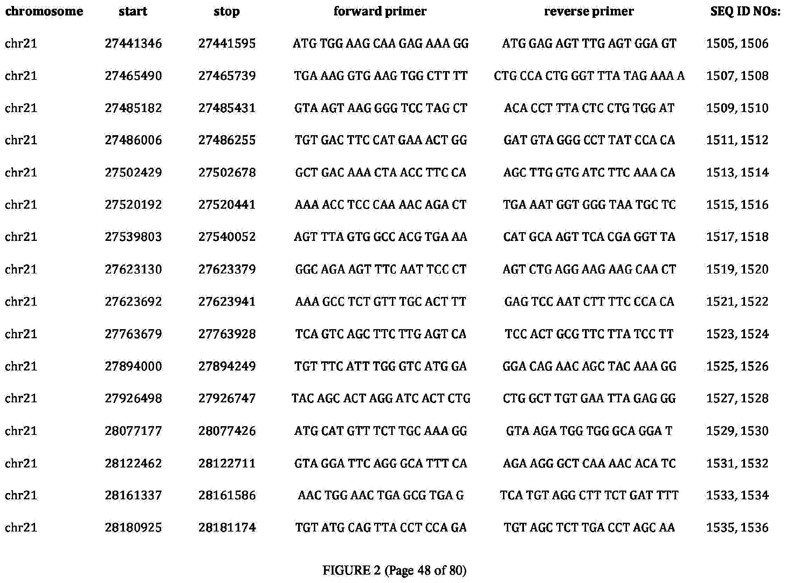

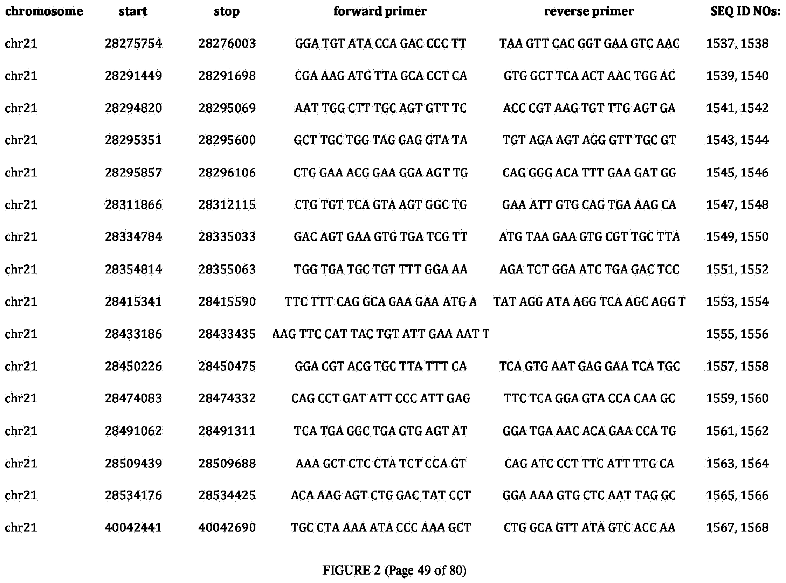

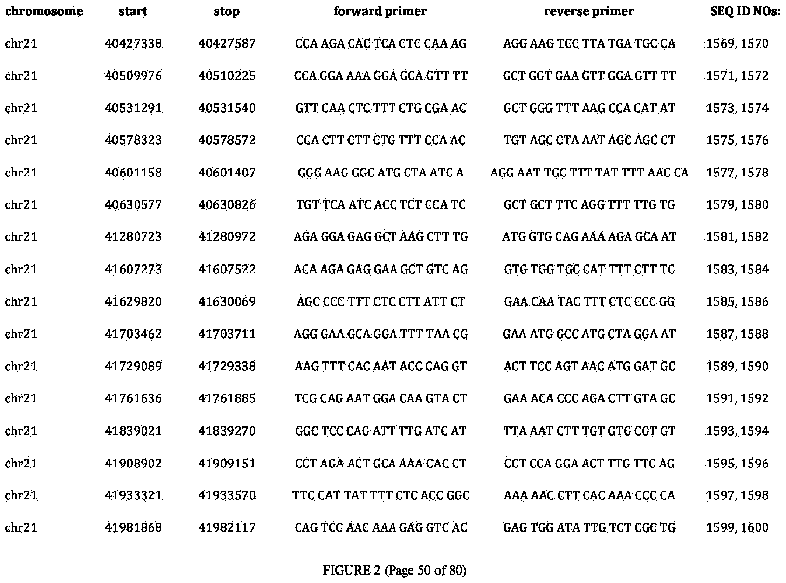

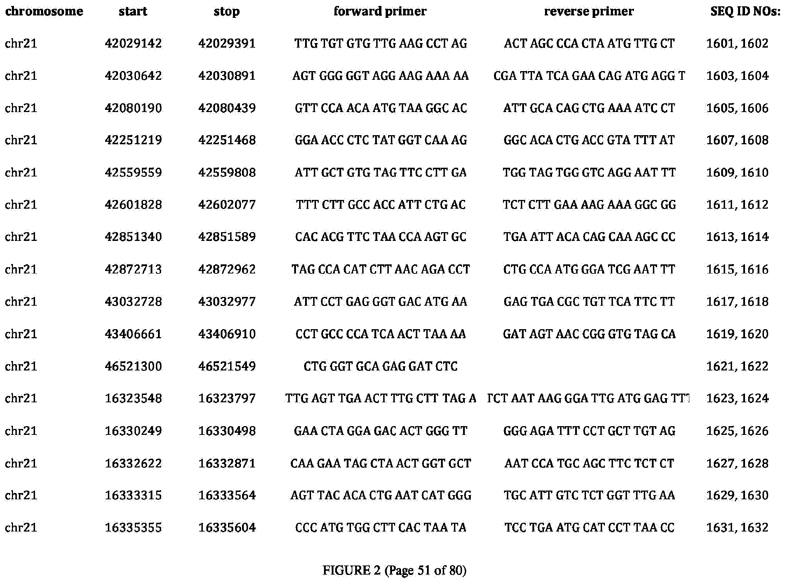

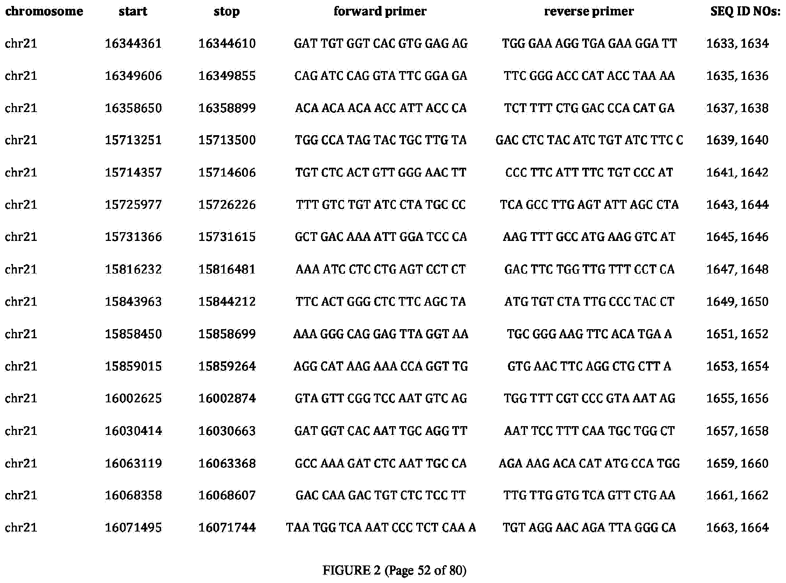

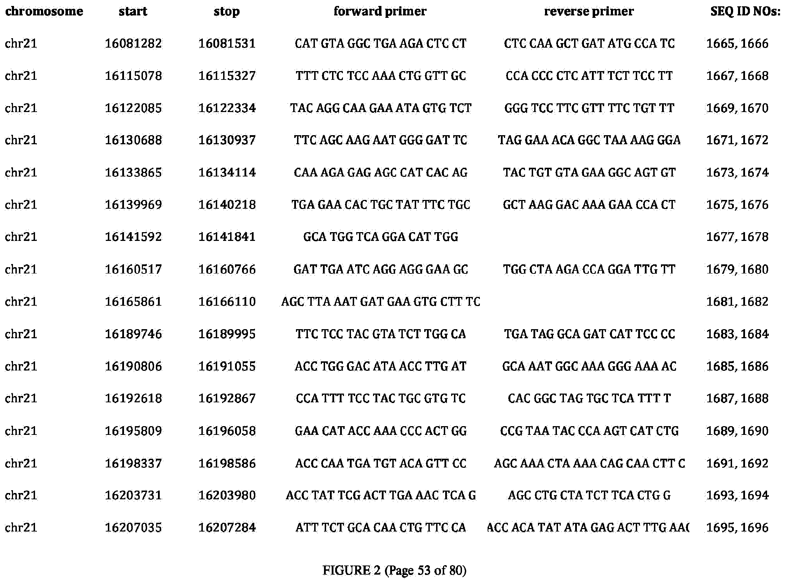

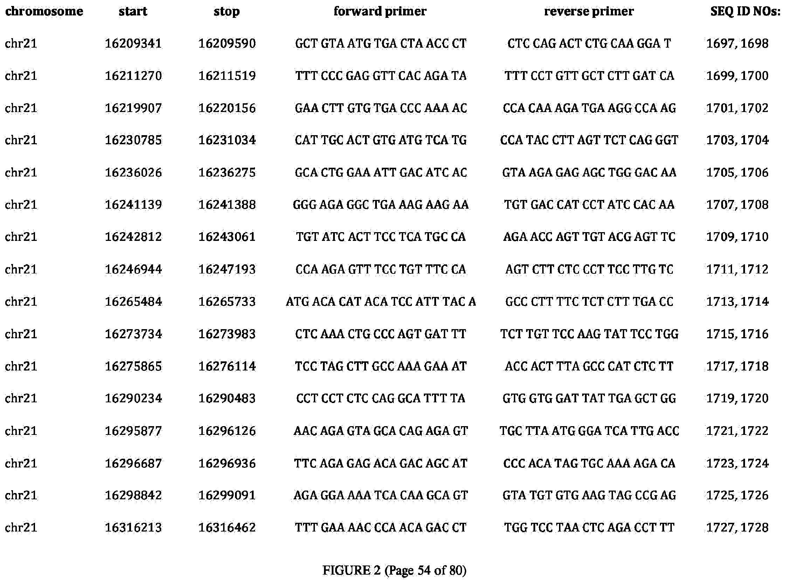

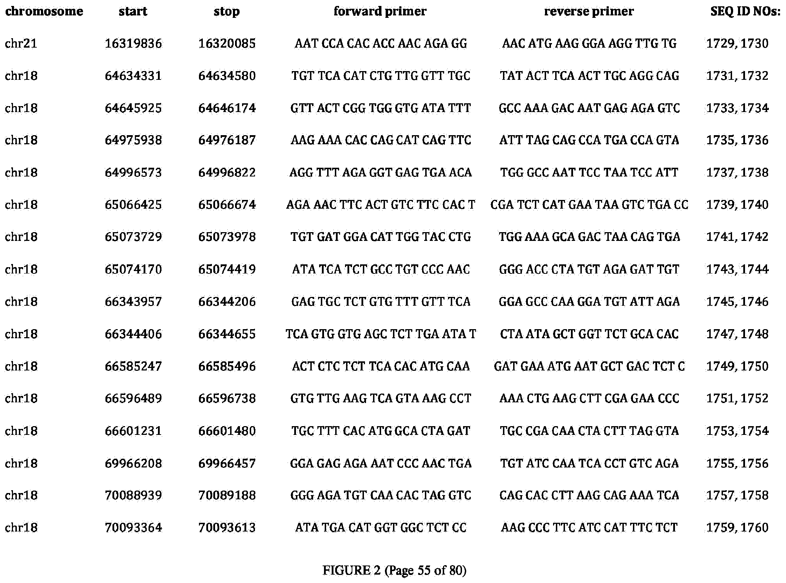

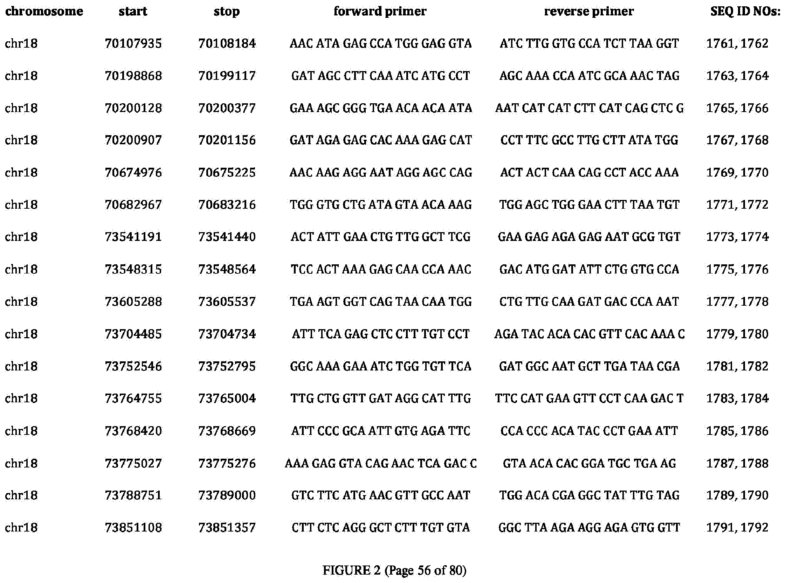

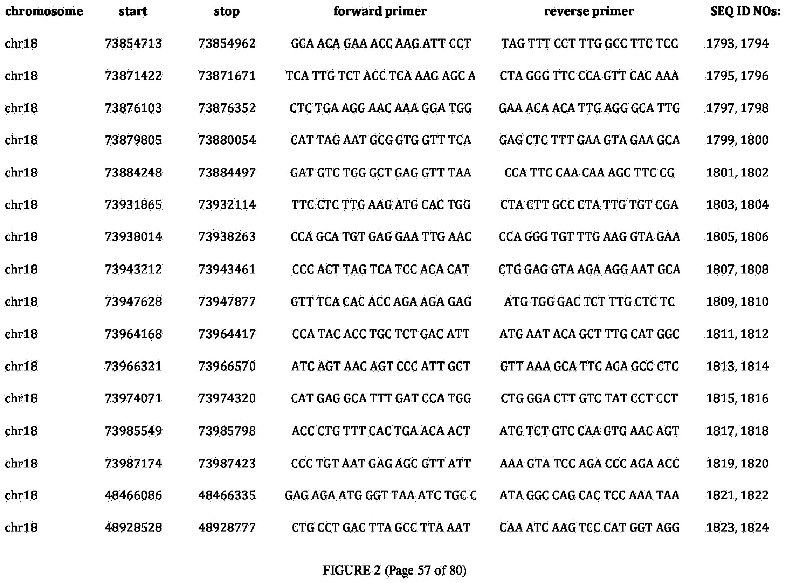

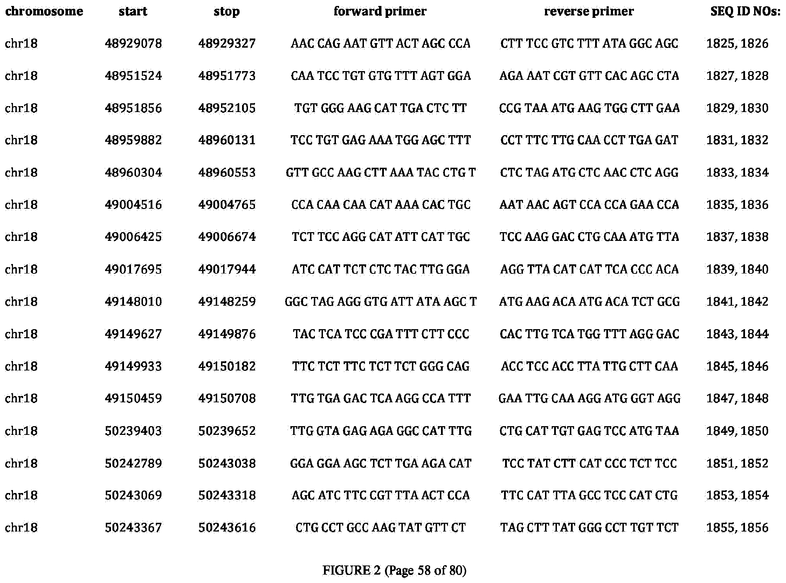

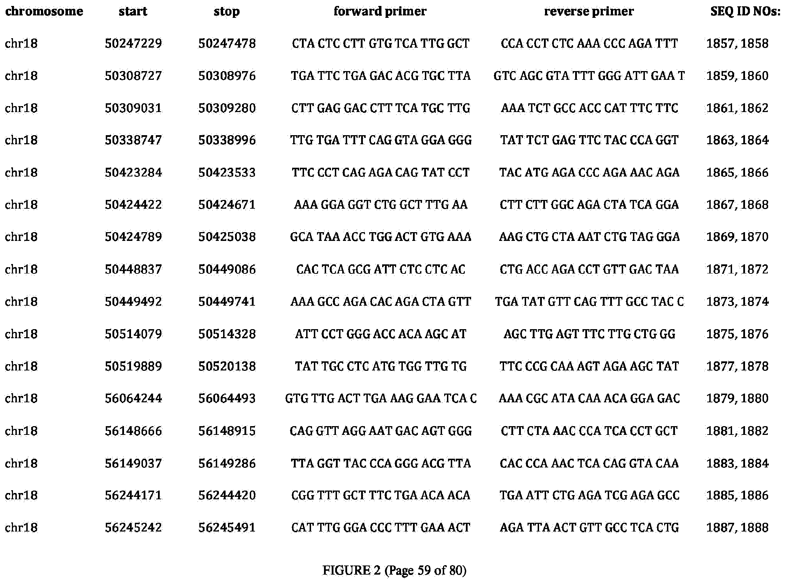

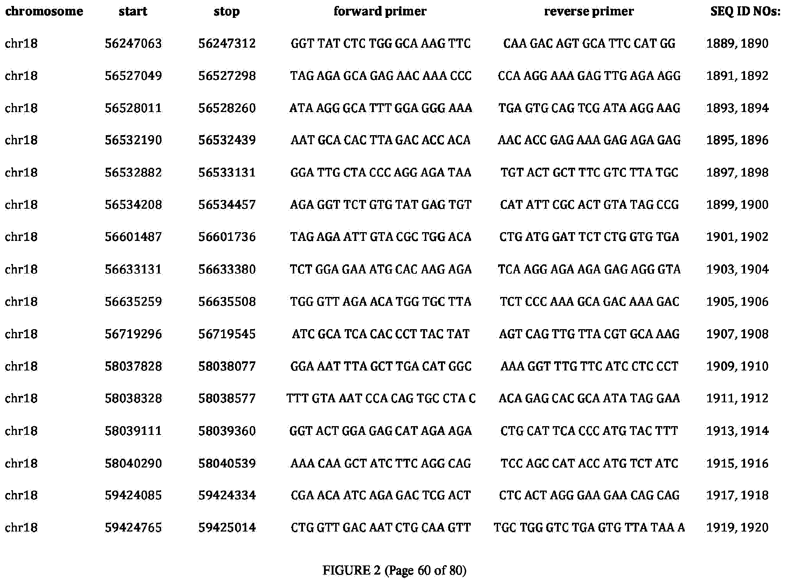

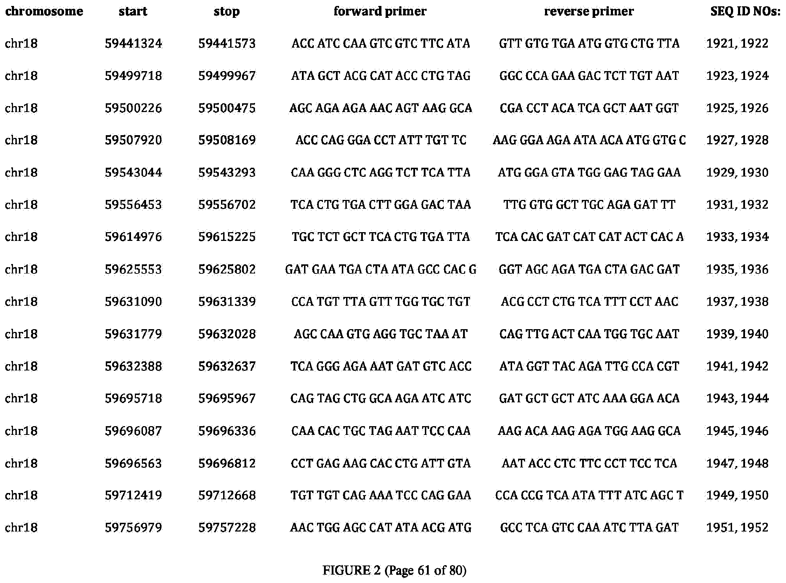

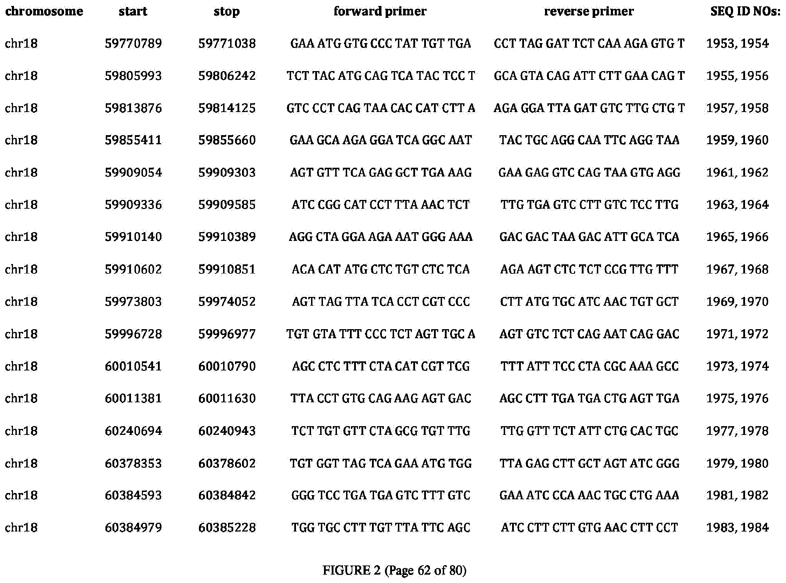

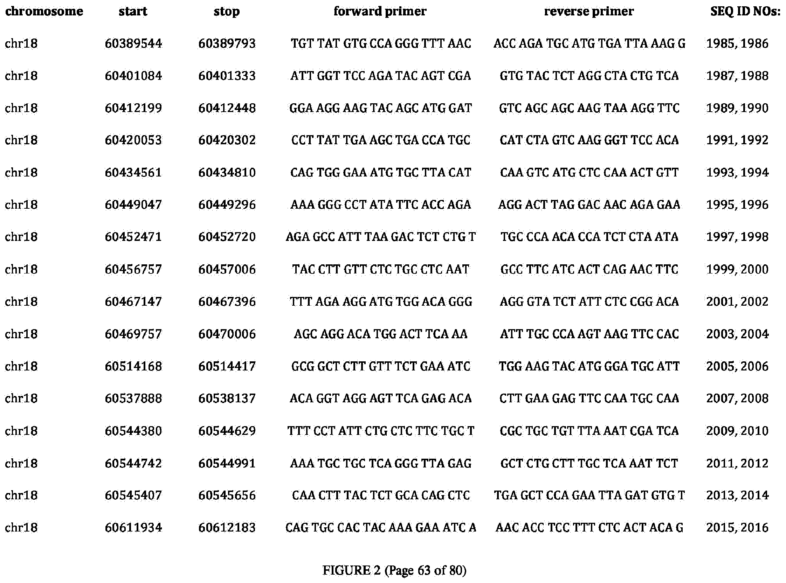

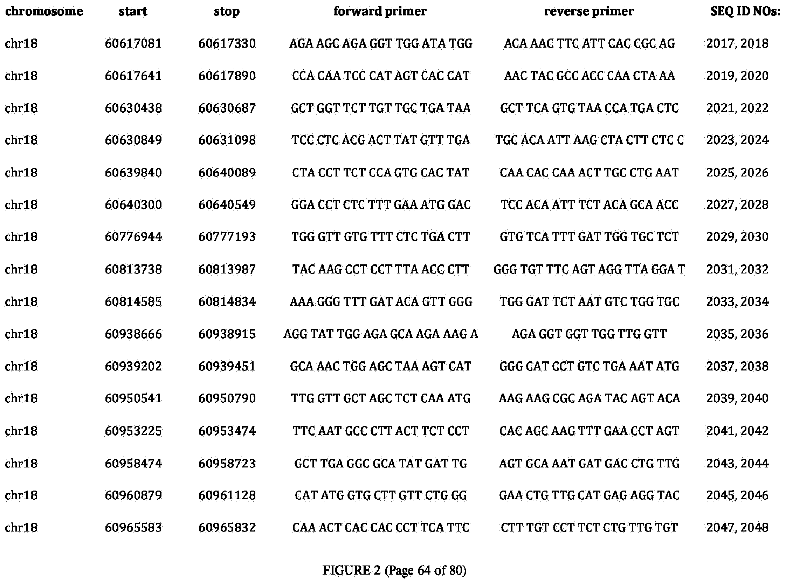

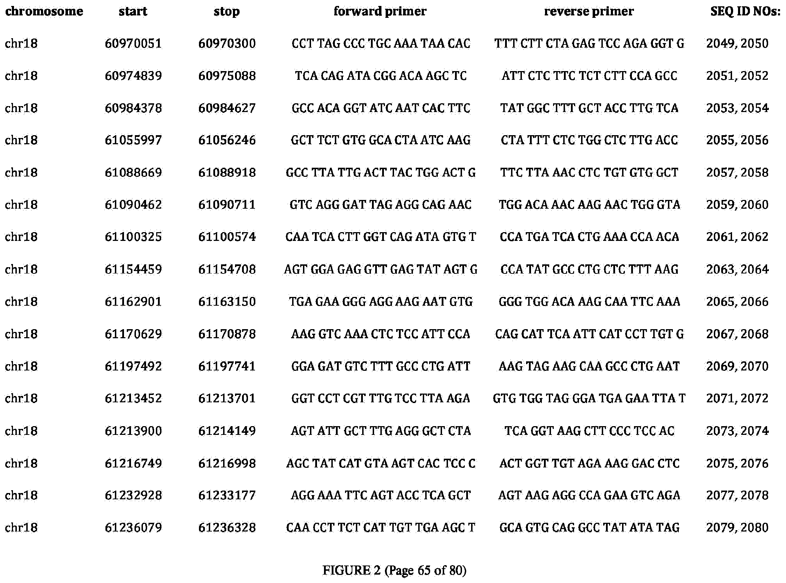

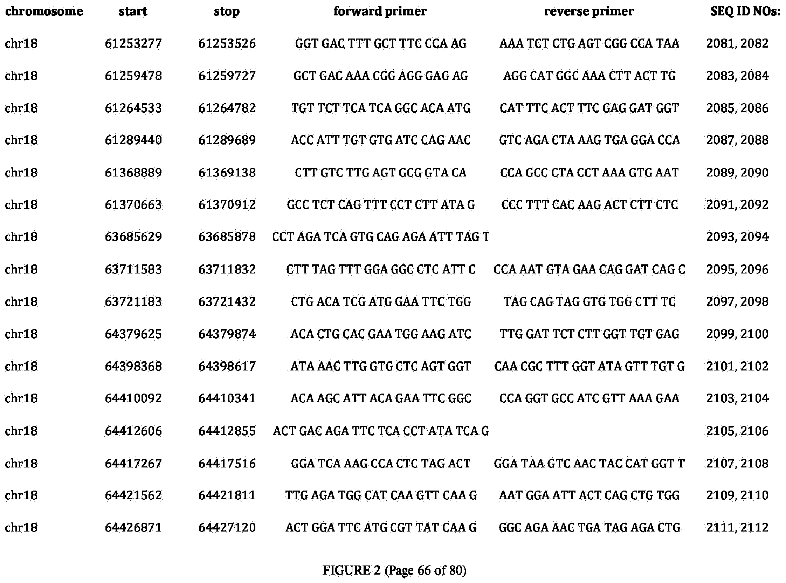

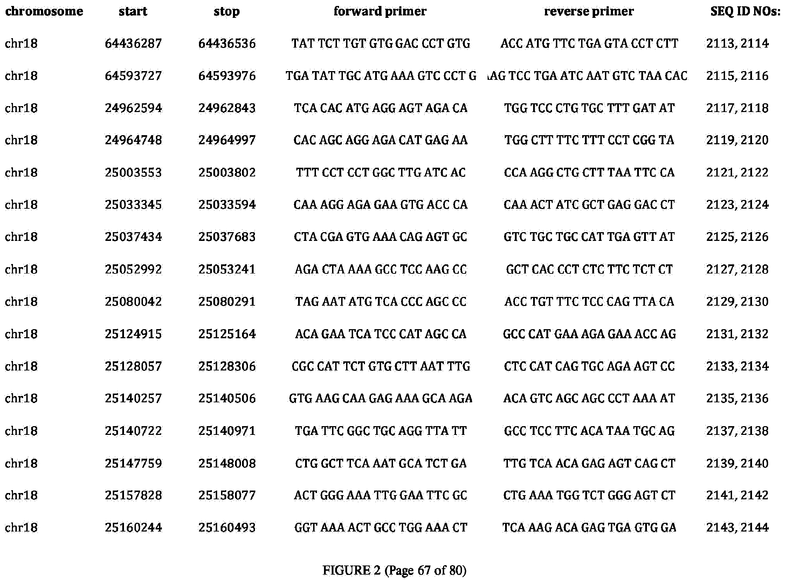

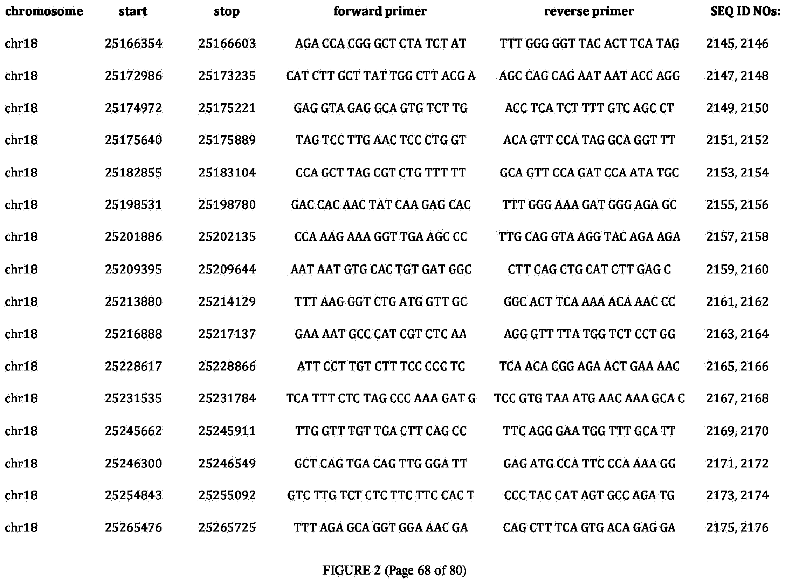

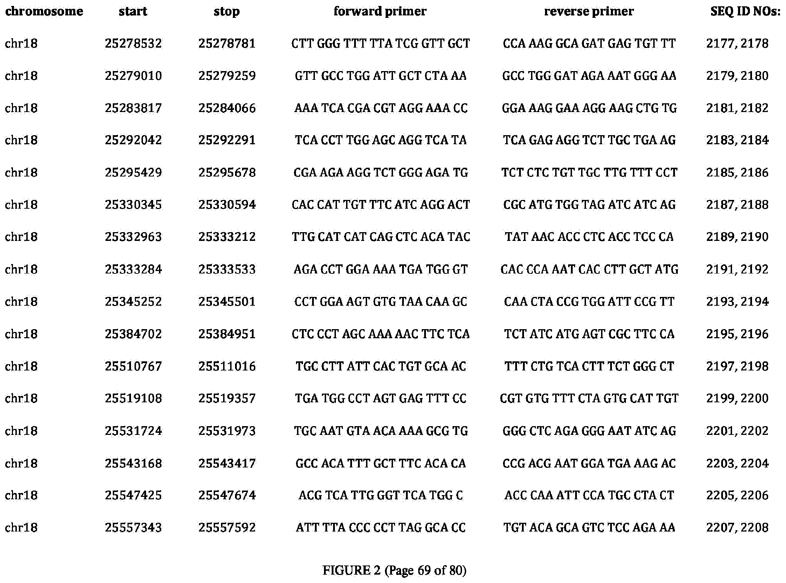

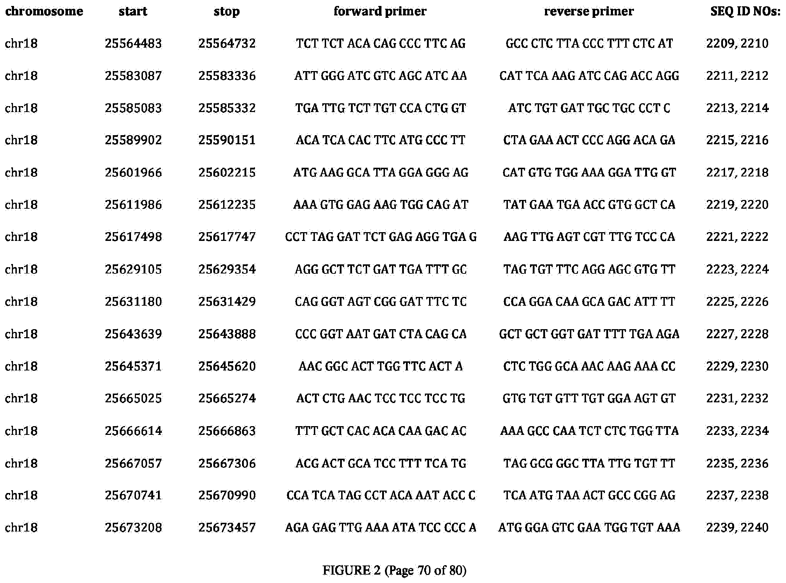

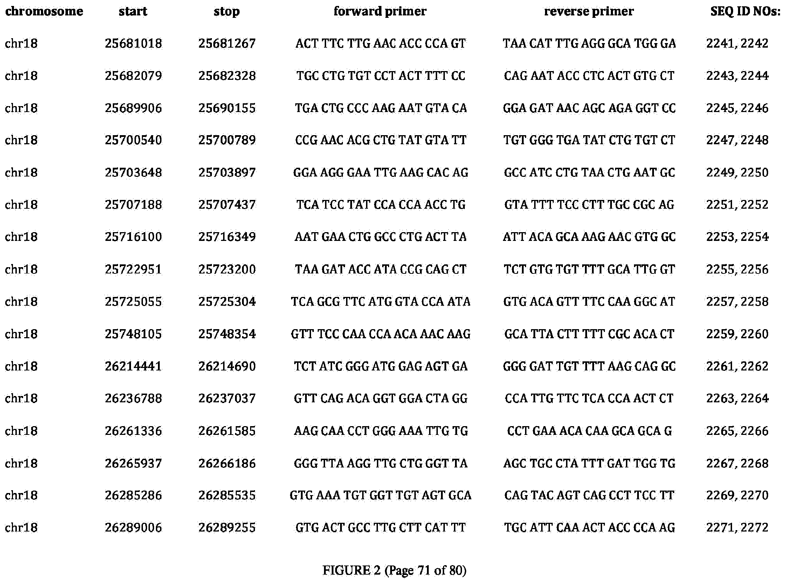

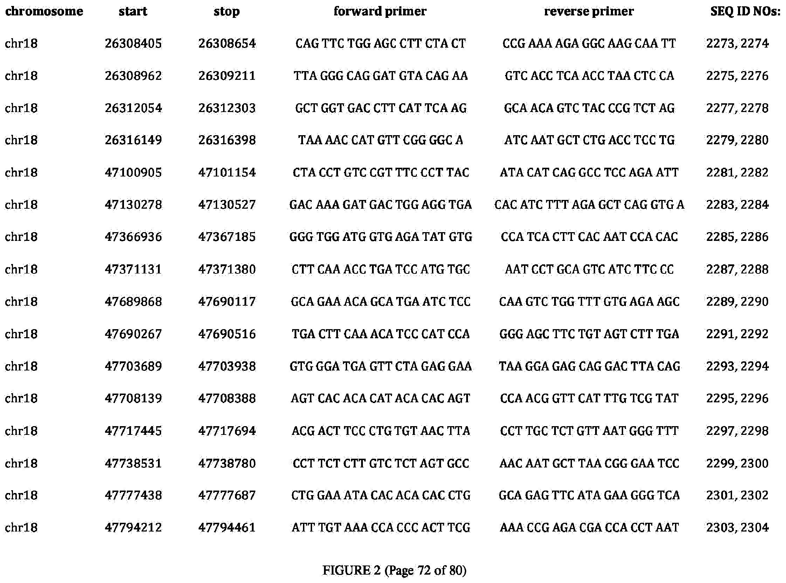

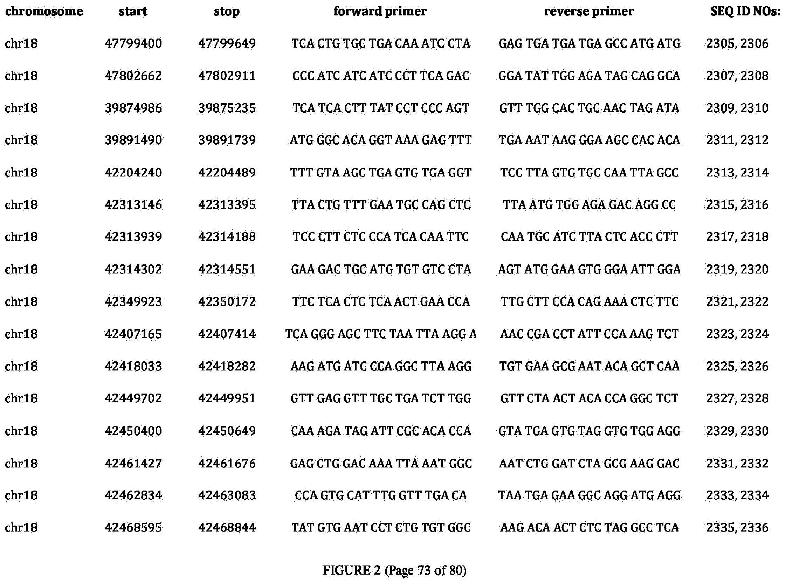

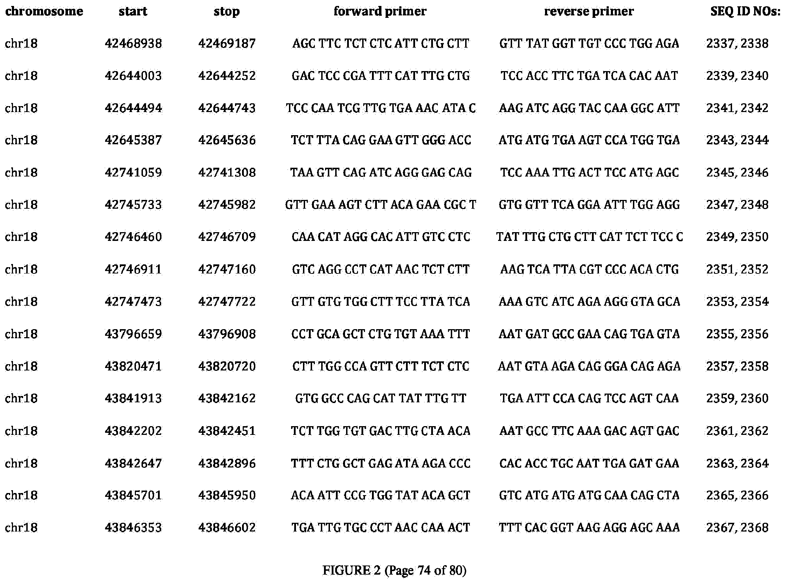

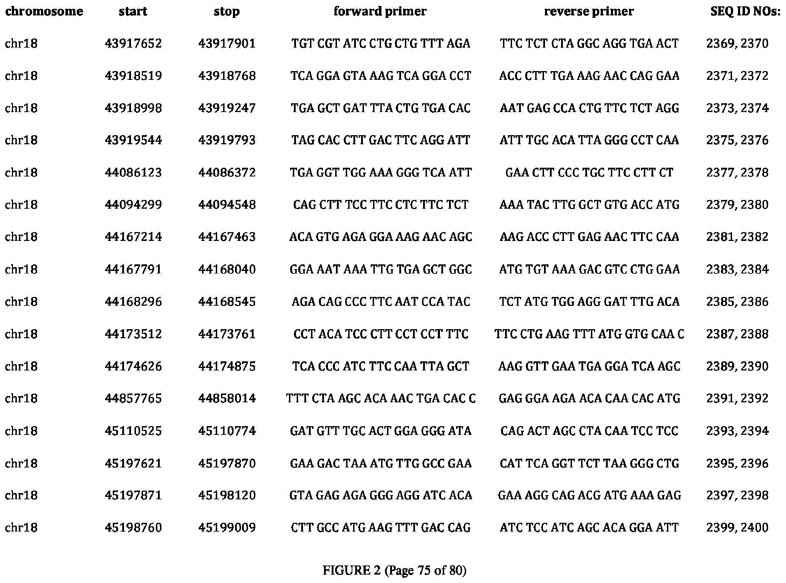

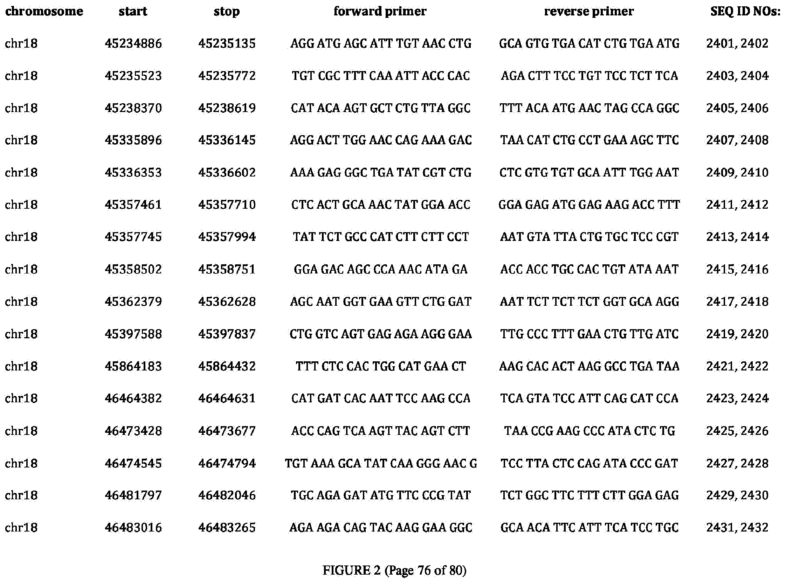

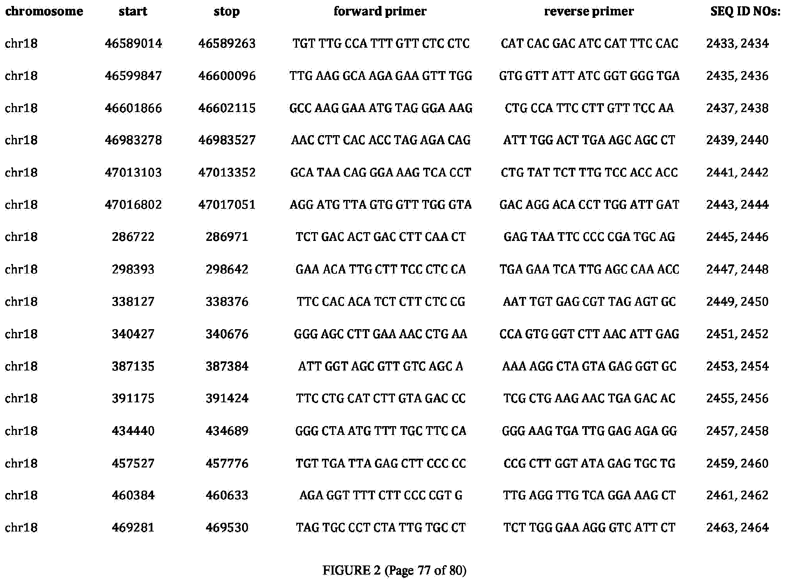

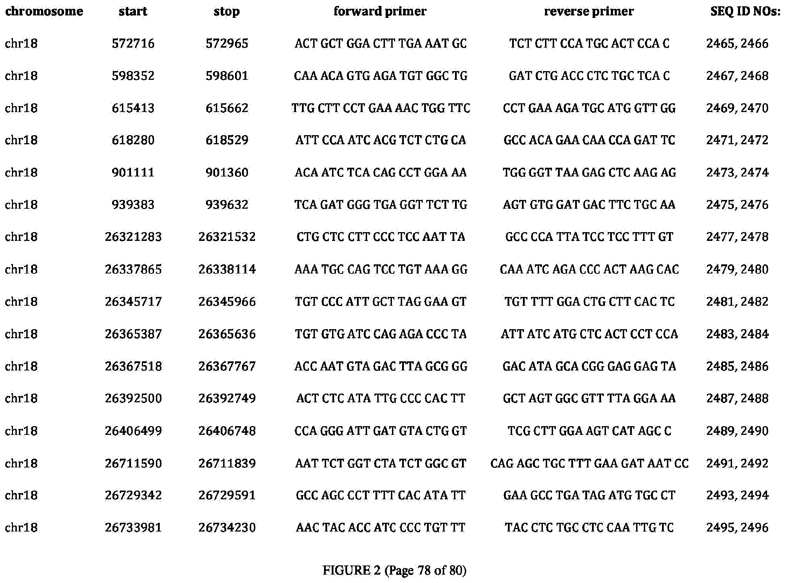

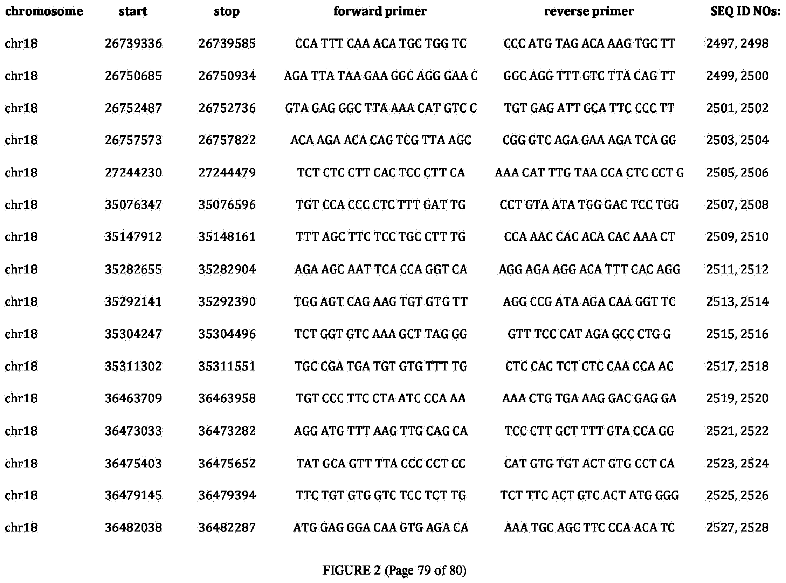

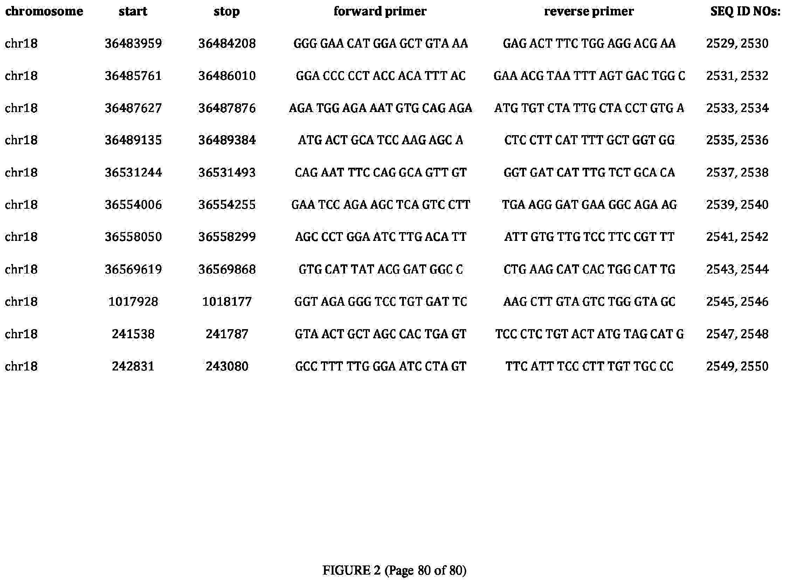

Figure 2 is a listing of exemplary chromosomal regions for amplifying TACS that bind to for example chromosomes 13, 18, 21 or X. A more extensive list is shown in Table 1 below.

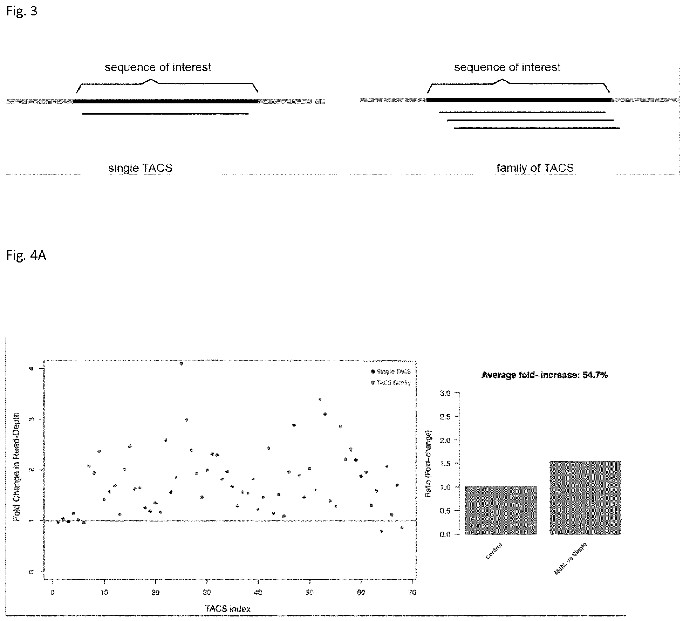

Figure 3 is a schematic diagram of TACS-based enrichment of a sequence of interest (bold line) using a single TACS (left) versus TACS-based enrichment using a family of TACS (right).

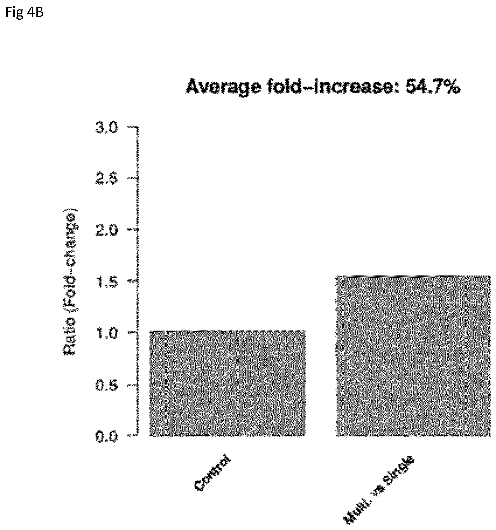

Figures 4A-4B are graphs showing enrichment using families of TACS versus a single TACS, as illustrated by increase in the average read-depth. Figure 4A shows loci enriched using a family of TACS (red dots) as compared to loci enriched using a single TACS (blue dots), with different target sequences shown on the x-axis and the fold change in read-depth shown on the y-axis. Figure 4B is a bar graph illustrating the average fold-increase in read-depth (54.7%) using a family of TACS (right) versus a single TACS (left).

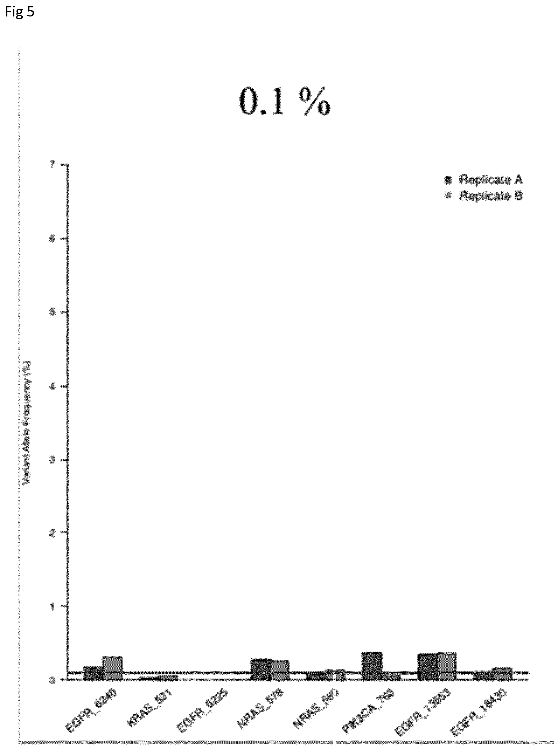

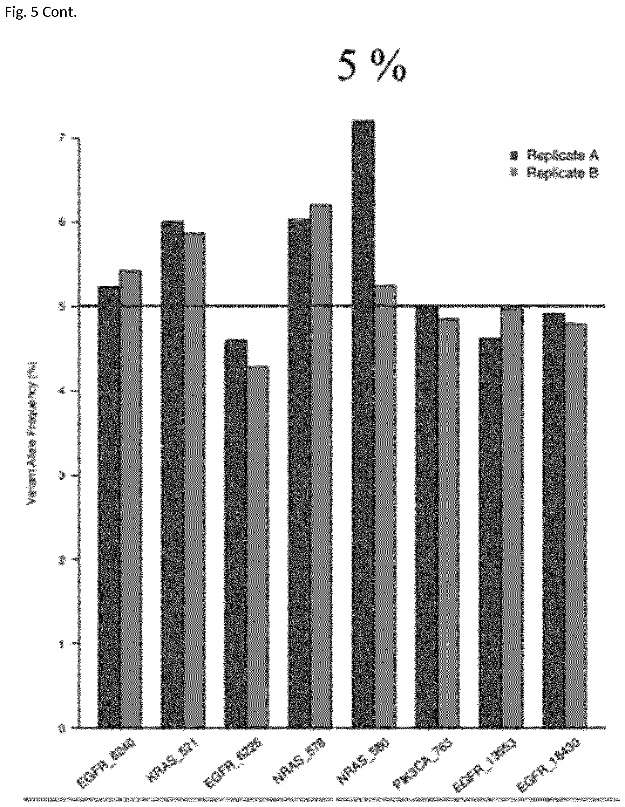

Figure 5 shows bar graphs illustrating detection of known genetic mutations that are tumor biomarkers in certified reference material harboring the mutations. Two replicates of the reference material are shown. The line illustrates the expected minor allele frequency (MAF) for each of the assessed tumor loads. The bars (x-axis) illustrate the detected MAF (y-axis) for the indicated genetic mutations in the certified reference material.

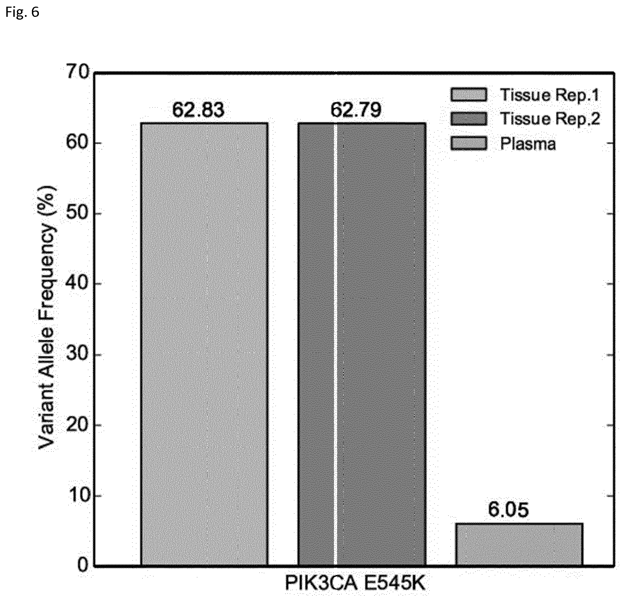

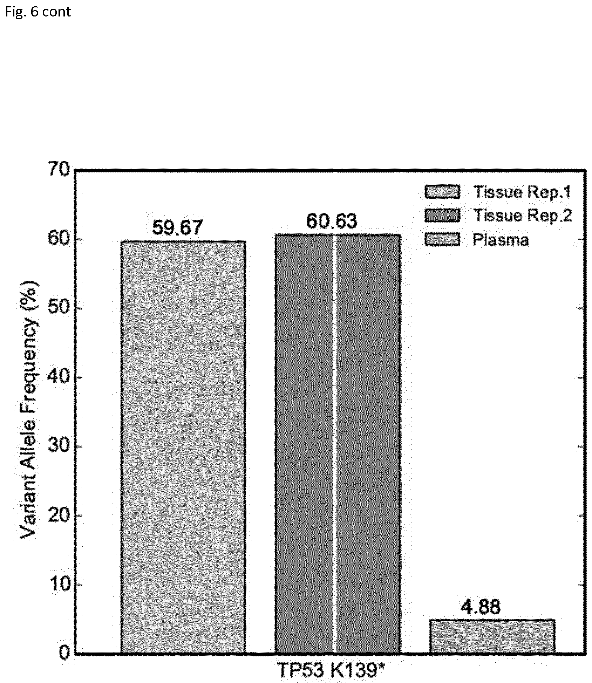

Figure 6 shows bar graphs illustrating detection of tumor biomarkers in cancer patient samples. Results are shown for two patients, one harboring mutation PIK3CA E545K (top bars) and one harboring mutation TP53 K139 (bottom bars). Both tumor tissue samples ("Tissue Rep. 1" and "Tissue Rep. 2") and plasma samples ("Plasma") are shown. The y-axis shows % variant allele frequency (VAF) detected in the samples.

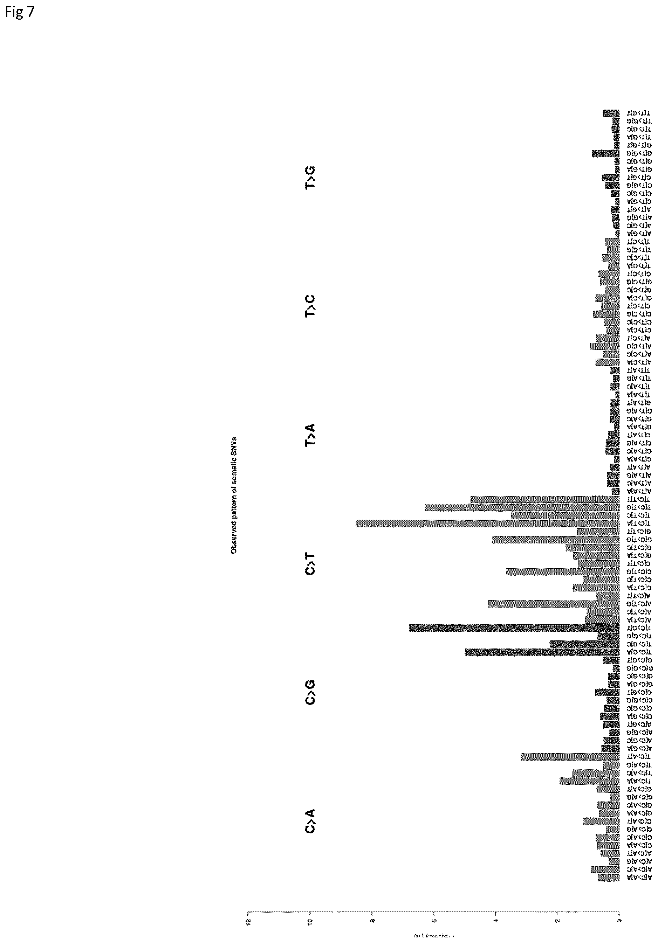

Figure 7 is a bar graph showing the observed pattern of somatic SNVs in breast cancer, as found in the COSMIC database. The x-axis shows a single base mutation observed in cancer in the context of its neighboring sequences. For example A[C>A]T describes the mutation of Cytosine (C ) to Adenine (A) where the upstream sequence is Adenine and the downstream sequence is Thymine. The y-axis shows the frequency of occurrence of this mutation in breast cancer.

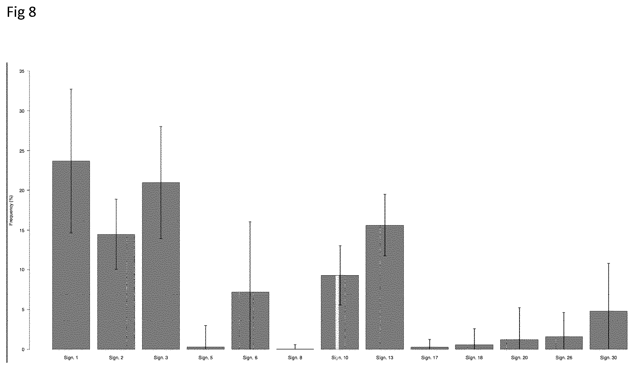

Figure 8 is a bar graph showing results of a simulations study where simulated sequencing data includes mutational motifs. The data were subjected to mutational motif detection. The bars indicate the average estimated frequency of the known mutational breast cancer motifs computed from a data set of 10000 simulations. Results illustrate that detection of mutational motifs is possible using the developed algorithm.

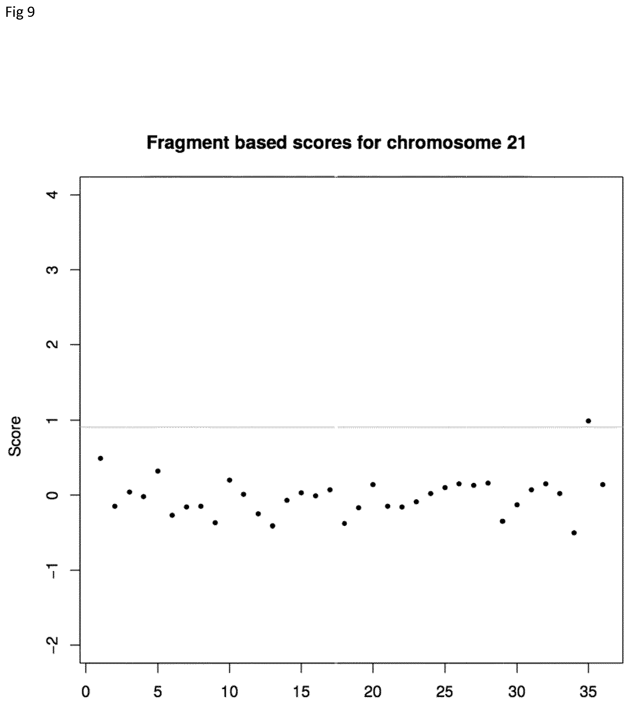

Figure 9 is a dot plot graph showing results of a fragments-based test for detecting increased numbers of smaller-size fragments in a mixed sample. An abnormal, aneuploid sample, with an estimated fetal fraction of 2.8%, was correctly detected using this method. The black dots are individual samples. The x-axis shows the sample index. The y-axis shows the score result of the fragments-size based method. A score result greater than the threshold shown by the grey line indicates a deviation from the expected size of fragments illustrating the presence of aneuploidy.

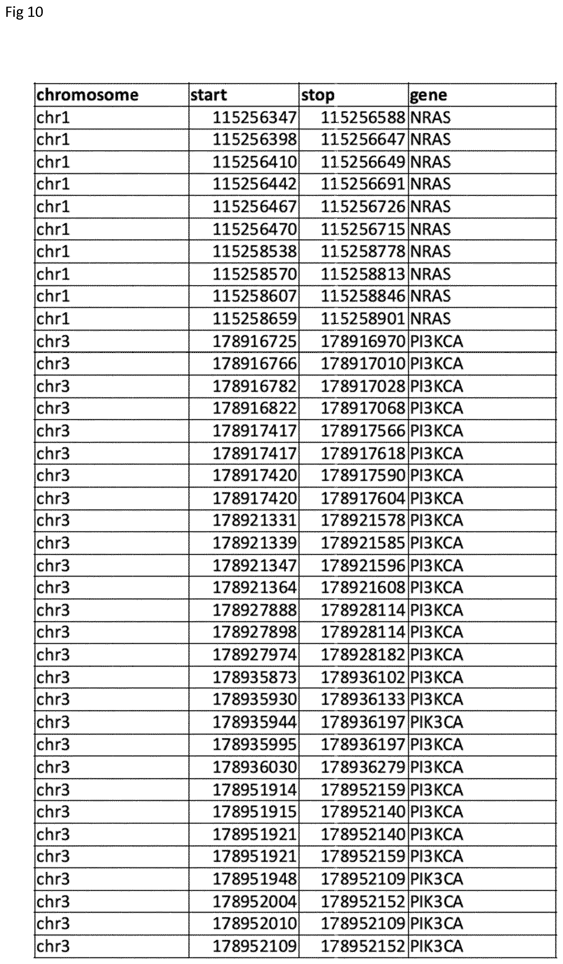

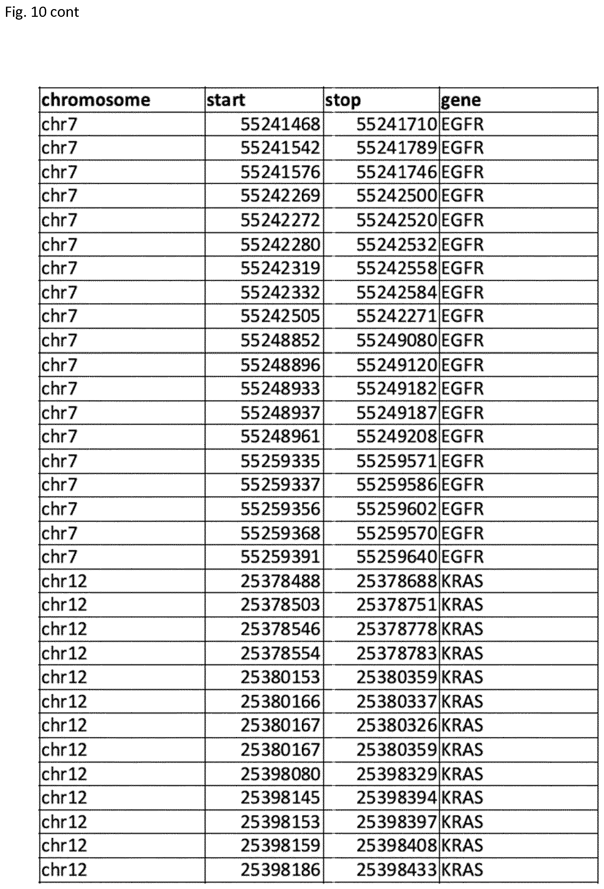

Figure 10 is a listing of exemplary chromosomal regions for amplifying TACS that bind to exemplary, non-limiting tumor biomarker genes.

Table 1 shows exemplary and preferred TACS positions.

| Chr. | Start | Stop | GC | Gene | Chr. | Start | Stop | GC | Gene |
|------|-------|------|-----|------|------|-------|------|-----|------|
| chr1 | 11169250 | 11169491 | 0.434 | MTOR | chr17 | 37880017 | 37880264 | 0.528 | ERBB2 |
| chr1 | 11169262 | 11169509 | 0.419 | MTOR | chr17 | 37880031 | 37880255 | 0.524 | ERBB2 |
| chr1 | 11169280 | 11169519 | 0.400 | MTOR | chr17 | 37880061 | 37880274 | 0.505 | ERBB2 |
| chr1 | 11169299 | 11169548 | 0.392 | MTOR | chr17 | 37880069 | 37880274 | 0.500 | ERBB2 |
| chr1 | 11174376 | 11174632 | 0.541 | MTOR | chr17 | 37880955 | 37881196 | 0.595 | ERBB2 |
| chr1 | 11174392 | 11174632 | 0.535 | MTOR | chr17 | 37880969 | 37881216 | 0.589 | ERBB2 |
| chr1 | 11174392 | 11174691 | 0.527 | MTOR | chr17 | 37880974 | 37881216 | 0.593 | ERBB2 |
| chr1 | 11174468 | 11174698 | 0.515 | MTOR | chr17 | 37880983 | 37881227 | 0.592 | ERBB2 |
| chr1 | 11184541 | 11184796 | 0.504 | MTOR | chr17 | 37881166 | 37881380 | 0.609 | ERBB2 |
| chr1 | 11184563 | 11184812 | 0.504 | MTOR | chr17 | 37881201 | 37881450 | 0.584 | ERBB2 |
| chr1 | 11184564 | 11184816 | 0.502 | MTOR | chr17 | 37881273 | 37881520 | 0.573 | ERBB2 |
| chr1 | 11187992 | 11188236 | 0.535 | MTOR | chr17 | 37881304 | 37881521 | 0.573 | ERBB2 |
| chr1 | 11188010 | 11188249 | 0.521 | MTOR | chr17 | 37881453 | 37881652 | 0.595 | ERBB2 |
| chr1 | 11188018 | 11188257 | 0.513 | MTOR | chr17 | 37881465 | 37881668 | 0.598 | ERBB2 |
| chr1 | 11188029 | 11188274 | 0.492 | MTOR | chr17 | 37881510 | 37881737 | 0.601 | ERBB2 |
| chr1 | 17345194 | 17345459 | 0.316 | SDHB | chr17 | 37881598 | 37881798 | 0.632 | ERBB2 |
| chr1 | 17349096 | 17349342 | 0.543 | SDHB | chr17 | 41196311 | 41196511 | 0.393 | BRCA1 |
| chr1 | 17350413 | 17350563 | 0.497 | SDHB | chr17 | 41197220 | 41197464 | 0.429 | BRCA1 |
| chr1 | 17350566 | 17350779 | 0.430 | SDHB | chr17 | 41197314 | 41197566 | 0.419 | BRCA1 |
| chr1 | 17354089 | 17354304 | 0.463 | SDHB | chr17 | 41197571 | 41197819 | 0.550 | BRCA1 |
| chr1 | 17355058 | 17355208 | 0.417 | SDHB | chr17 | 41199637 | 41199729 | 0.559 | BRCA1 |
| chr1 | 17359477 | 17359689 | 0.427 | SDHB | chr17 | 41201055 | 41201304 | 0.476 | BRCA1 |
| chr1 | 17371214 | 17371394 | 0.470 | SDHB | chr17 | 41202997 | 41203243 | 0.449 | BRCA1 |
| chr1 | 17380408 | 17380619 | 0.670 | SDHB | chr17 | 41209041 | 41209195 | 0.445 | BRCA1 |

(continued)

| Chr. | Start | Stop | GC | Gene | Chr. | Start | Stop | GC | Gene |
|------|-------|------|-----|------|------|-------|------|-----|------|
| chr1 | 43814917 | 43815186 | 0.633 | MPL | chr17 | 41215338 | 41215577 | 0.433 | BRCA1 |
| chr1 | 45795001 | 45795250 | 0.536 | MUTYH | chr17 | 41215806 | 41216045 | 0.375 | BRCA1 |
| chr1 | 45796024 | 45796223 | 0.530 | MUTYH | chr17 | 41219623 | 41219726 | 0.337 | BRCA1 |
| chr1 | 45796871 | 45797092 | 0.536 | MUTYH | chr17 | 41222828 | 41223088 | 0.402 | BRCA1 |
| chr1 | 45797060 | 45797289 | 0.648 | MUTYH | chr17 | 41222835 | 41223088 | 0.406 | BRCA1 |
| chr1 | 45797289 | 45797529 | 0.635 | MUTYH | chr17 | 41222885 | 41223141 | 0.444 | BRCA1 |
| chr1 | 45797602 | 45797802 | 0.577 | MUTYH | chr17 | 41223090 | 41223269 | 0.494 | BRCA1 |
| chr1 | 45797819 | 45798019 | 0.622 | MUTYH | chr17 | 41256181 | 41256358 | 0.315 | BRCA1 |
| chr1 | 45797986 | 45798270 | 0.593 | MUTYH | chr17 | 41256830 | 41257034 | 0.376 | BRCA1 |
| chr1 | 45798204 | 45798404 | 0.602 | MUTYH | chr17 | 41256884 | 41257140 | 0.339 | BRCA1 |
| chr1 | 45798364 | 45798564 | 0.547 | MUTYH | chr17 | 41258410 | 41258681 | 0.324 | BRCA1 |
| chr1 | 45798532 | 45798732 | 0.557 | MUTYH | chr17 | 41267598 | 41267807 | 0.371 | BRCA1 |
| chr1 | 45798672 | 45798872 | 0.567 | MUTYH | chr17 | 41267602 | 41267807 | 0.369 | BRCA1 |
| chr1 | 45798867 | 45799097 | 0.628 | MUTYH | chr17 | 41267603 | 41267794 | 0.354 | BRCA1 |
| chr1 | 45799150 | 45799357 | 0.582 | MUTYH | chr17 | 41267603 | 41267834 | 0.371 | BRCA1 |
| chr1 | 45800062 | 45800304 | 0.539 | MUTYH | chr17 | 41267630 | 41267779 | 0.353 | BRCA1 |
| chr1 | 115252133 | 115252352 | 0.445 | NRAS | chr17 | 41267645 | 41267836 | 0.391 | BRCA1 |
| chr1 | 115252133 | 115252354 | 0.441 | NRAS | chr17 | 41275961 | 41276197 | 0.312 | BRCA1 |
| chr1 | 115252138 | 115252350 | 0.446 | NRAS | chr17 | 46805588 | 46805837 | 0.672 | EOXB13 |
| chr1 | 115252142 | 115252350 | 0.450 | NRAS | chr17 | 47696239 | 47696485 | 0.453 | SPOP |
| chr1 | 115256347 | 115256588 | 0.409 | NRAS | chr17 | 47696300 | 47696545 | 0.455 | SPOP |
| chr1 | 115256398 | 115256647 | 0.448 | NRAS | chr17 | 47696324 | 47696565 | 0.442 | SPOP |
| chr1 | 115256410 | 115256649 | 0.458 | NRAS | chr17 | 47696359 | 47696607 | 0.422 | SPOP |
| chr1 | 115256442 | 115256691 | 0.440 | NRAS | chr17 | 47696424 | 47696669 | 0.415 | SPOP |
| chr1 | 115256467 | 115256726 | 0.442 | NRAS | chr17 | 47696450 | 47696669 | 0.405 | SPOP |
| chr1 | 115256470 | 115256715 | 0.451 | NRAS | chr17 | 47696450 | 47696689 | 0.413 | SPOP |
| chr1 | 115258538 | 115258778 | 0.440 | NRAS | chr17 | 47696486 | 47696715 | 0.387 | SPOP |
| chr1 | 115258570 | 115258813 | 0.463 | NRAS | chr17 | 56769976 | 56770175 | 0.590 | RAD51C |
| chr1 | 115258607 | 115258846 | 0.492 | NRAS | chr17 | 56772289 | 56772542 | 0.421 | RAD51C |
| chr1 | 115258659 | 115258901 | 0.444 | NRAS | chr17 | 56773994 | 56774243 | 0.404 | RAD51C |
| chr1 | 156268500 | 156268651 | 0.454 | VHLL | chr17 | 56780486 | 56780723 | 0.345 | RAD51C |
| chr1 | 156269007 | 156269221 | 0.493 | VHLL | chr17 | 56787247 | 56787446 | 0.365 | RAD51C |
| chr1 | 156846129 | 156846362 | 0.641 | NTRK1 | chr17 | 56787285 | 56787460 | 0.341 | RAD51C |
| chr1 | 156846129 | 156846377 | 0.643 | NTRK1 | chr17 | 56798102 | 56798172 | 0.352 | RAD51C |
| chr1 | 156846130 | 156846362 | 0.639 | NTRK1 | chr17 | 56801331 | 56801553 | 0.390 | RAD51C |
| chr1 | 156848889 | 156849132 | 0.611 | NTRK1 | chr17 | 56809830 | 56810049 | 0.382 | RAD51C |
| chr1 | 156848911 | 156849153 | 0.617 | NTRK1 | chr17 | 56811478 | 56811716 | 0.385 | RAD51C |
| chr1 | 156848921 | 156849168 | 0.613 | NTRK1 | chr17 | 59756779 | 59756979 | 0.313 | BRIP1 |
| chr1 | 156848925 | 156849168 | 0.611 | NTRK1 | chr17 | 59759726 | 59759955 | 0.252 | BRIP1 |
| chr1 | 161284168 | 161284376 | 0.598 | SDHC | chr17 | 59760615 | 59760854 | 0.308 | BRIP1 |
| chr1 | 161293229 | 161293429 | 0.343 | SDHC | chr17 | 59760809 | 59761044 | 0.335 | BRIP1 |
| chr1 | 161298181 | 161298414 | 0.436 | SDHC | chr17 | 59761001 | 59761201 | 0.353 | BRIP1 |
| chr1 | 161333007 | 161333261 | 0.447 | SDHC | chr17 | 59761265 | 59761494 | 0.413 | BRIP1 |
| chr1 | 161333275 | 161333550 | 0.391 | SDHC | chr17 | 59761395 | 59761636 | 0.343 | BRIP1 |
| chr1 | 161333589 | 161333868 | 0.439 | SDHC | chr17 | 59763229 | 59763449 | 0.357 | BRIP1 |
| chr1 | 161333890 | 161334157 | 0.392 | SDHC | chr17 | 59763460 | 59763660 | 0.338 | BRIP1 |
| chr1 | 161334206 | 161334492 | 0.394 | SDHC | chr17 | 59770816 | 59771046 | 0.307 | BRIP1 |
| chr1 | 162748235 | 162748450 | 0.440 | DDR2 | chr17 | 59793106 | 59793349 | 0.316 | BRIP1 |
| chr1 | 162748273 | 162748512 | 0.442 | DDR2 | chr17 | 59793137 | 59793367 | 0.338 | BRIP1 |
| chr1 | 162748316 | 162748557 | 0.450 | DDR2 | chr17 | 59820379 | 59820551 | 0.393 | BRIP1 |

(continued)

| Chr. | Start | Stop | GC | Gene | Chr. | Start | Stop | GC | Gene |
|---|---|---|---|---|---|---|---|---|---|
| chr1 | 162748370 | 162748580 | 0.483 | DDR2 | chr17 | 59821796 | 59821945 | 0.373 | BRIP1 |
| chr10 | 8111358 | 8111588 | 0.506 | GATA3 | chr17 | 59853687 | 59853887 | 0.353 | BRIP1 |
| chr10 | 8111468 | 8111707 | 0.529 | GATA3 | chr1 7 | 59857537 | 59857737 | 0.333 | BRIP1 |
| chr10 | 8115737 | 8115986 | 0.572 | GATA3 | chr1 7 | 59858201 | 59858428 | 0.355 | BRIP1 |
| chr10 | 8115741 | 8115988 | 0.569 | GATA3 | chr1 7 | 59861553 | 59861773 | 0.326 | BRIP1 |
| chr10 | 8115783 | 8115988 | 0.612 | GATA3 | chr1 7 | 59870956 | 59871190 | 0.345 | BRIP1 |
| chr10 | 8115789 | 8115988 | 0.620 | GATA3 | chr1 7 | 59876405 | 59876605 | 0.388 | BRIP1 |
| chr10 | 43595933 | 43596179 | 0.623 | RET | chr1 7 | 59876622 | 59876822 | 0.284 | BRIP1 |
| chr10 | 43597765 | 43598054 | 0.614 | RET | chr1 7 | 59878569 | 59878769 | 0.413 | BRIP1 |
| chr10 | 43600476 | 43600682 | 0.686 | RET | chr1 7 | 59878611 | 59878844 | 0.397 | BRIP1 |
| chr10 | 43601846 | 43602088 | 0.638 | RET | chr1 7 | 59885818 | 59886038 | 0.439 | BRIP1 |
| chr10 | 43604444 | 43604706 | 0.620 | RET | chr1 7 | 59886058 | 59886268 | 0.346 | BRIP1 |
| chr10 | 43606653 | 43606911 | 0.595 | RET | chr1 7 | 59924485 | 59924714 | 0.348 | BRIP1 |
| chr10 | 43607465 | 43607728 | 0.659 | RET | chr1 7 | 59926478 | 59926707 | 0.348 | BRIP1 |
| chr10 | 43608226 | 43608455 | 0.596 | RET | chr1 7 | 59934381 | 59934581 | 0.383 | BRIP1 |
| chr10 | 43608980 | 43609217 | 0.626 | RET | chr1 7 | 59937155 | 59937392 | 0.374 | BRIP1 |
| chr10 | 43609917 | 43610183 | 0.625 | RET | chr1 7 | 59938714 | 59938914 | 0.333 | BRIP1 |
| chr10 | 43611943 | 43612187 | 0.522 | RET | chr1 7 | 59940627 | 59940827 | 0.577 | BRIP1 |
| chr10 | 43613689 | 43613933 | 0.555 | RET | chr1 7 | 59940844 | 59941054 | 0.592 | BRIP1 |
| chr10 | 43614921 | 43615150 | 0.687 | RET | chr1 8 | 48556368 | 48556604 | 0.692 | SMAD4 |
| chr10 | 43615517 | 43615759 | 0.568 | RET | chr1 8 | 48556368 | 48556608 | 0.689 | SMAD4 |
| chr10 | 43617287 | 43617531 | 0.457 | RET | chr1 8 | 48556413 | 48556612 | 0.680 | SMAD4 |
| chr10 | 43619092 | 43619322 | 0.584 | RET | chr1 8 | 48556414 | 48556616 | 0.675 | SMAD4 |
| chr10 | 43620303 | 43620509 | 0.551 | RET | chr1 8 | 48573249 | 48573492 | 0.328 | SMAD4 |
| chr10 | 43623746 | 43624029 | 0.493 | RET | chr1 8 | 48573500 | 48573738 | 0.364 | SMAD4 |
| chr10 | 43624152 | 43624439 | 0.490 | RET | chr1 8 | 48575147 | 48575346 | 0.340 | SMAD4 |
| chr10 | 43624946 | 43625222 | 0.433 | RET | chr1 8 | 48575524 | 48575724 | 0.294 | SMAD4 |
| chr10 | 50686414 | 50686655 | 0.368 | ERCC6 | chr1 8 | 48581203 | 48581433 | 0.455 | SMAD4 |
| chr10 | 88635669 | 88635923 | 0.333 | BMPR1A | chr1 8 | 48581249 | 48581498 | 0.428 | SMAD4 |
| chr10 | 88649829 | 88650073 | 0.384 | BMPR1A | chr1 8 | 48584399 | 48584599 | 0.443 | SMAD4 |
| chr10 | 88659444 | 88659644 | 0.368 | BMPR1A | chr1 8 | 48584673 | 48584893 | 0.439 | SMAD4 |
| chr10 | 88677042 | 88677242 | 0.388 | BMPR1A | chr1 8 | 48586106 | 48586306 | 0.308 | SMAD4 |
| chr10 | 89622844 | 89623045 | 0.594 | KLLN | chr1 8 | 48591791 | 48592035 | 0.408 | SMAD4 |
| chr10 | 89624131 | 89624370 | 0.492 | PTEN | chr1 8 | 48593378 | 48593608 | 0.407 | SMAD4 |
| chr10 | 89624178 | 89624422 | 0.465 | PTEN | chr1 8 | 48602902 | 48603166 | 0.430 | SMAD4 |
| chr10 | 89624198 | 89624444 | 0.445 | PTEN | chr1 8 | 48602922 | 48603132 | 0.445 | SMAD4 |
| chr10 | 89624214 | 89624463 | 0.436 | PTEN | chr1 8 | 48603000 | 48603249 | 0.424 | SMAD4 |
| chr10 | 89685273 | 89685522 | 0.304 | PTEN | chr1 8 | 48603132 | 48603332 | 0.289 | SMAD4 |
| chr10 | 89692746 | 89692946 | 0.398 | PTEN | chr1 8 | 48604566 | 48604815 | 0.488 | SMAD4 |
| chr10 | 89692746 | 89692977 | 0.392 | PTEN | chr1 8 | 48604617 | 48604861 | 0.490 | SMAD4 |
| chr10 | 89692746 | 89692999 | 0.402 | PTEN | chr1 8 | 48604640 | 48604881 | 0.492 | SMAD4 |
| chr10 | 89692763 | 89693015 | 0.403 | PTEN | chr1 8 | 48604711 | 48604957 | 0.421 | SMAD4 |
| chr10 | 89692787 | 89692999 | 0.413 | PTEN | chr1 8 | 48605303 | 48605503 | 0.294 | SMAD4 |
| chr10 | 89711788 | 89712024 | 0.380 | PTEN | chr1 8 | 48605551 | 48605798 | 0.351 | SMAD4 |
| chr10 | 89711798 | 89712045 | 0.367 | PTEN | chr1 8 | 48605981 | 48606181 | 0.318 | SMAD4 |
| chr10 | 89711867 | 89712069 | 0.399 | PTEN | chr1 8 | 48606203 | 48606477 | 0.349 | SMAD4 |
| chr10 | 89711880 | 89712129 | 0.348 | PTEN | chr1 8 | 48606469 | 48606712 | 0.357 | SMAD4 |
| chr10 | 89717531 | 89717770 | 0.404 | PTEN | chr1 8 | 48607311 | 48607586 | 0.326 | SMAD4 |
| chr10 | 89717558 | 89717802 | 0.408 | PTEN | chr18 | 48607638 | 48607926 | 0.412 | SMAD4 |
| chr10 | 89717558 | 89717831 | 0.394 | PTEN | chr18 | 48608009 | 48608208 | 0.375 | SMAD4 |

(continued)

| Chr. | Start | Stop | GC | Gene | Chr. | Start | Stop | GC | Gene |
|------|-------|------|-----|------|------|-------|------|-----|------|
| chr10 | 89717571 | 89717820 | 0.400 | PTEN | chr18 | 48608225 | 48608485 | 0.368 | SMAD4 |
| chr10 | 89717602 | 89717831 | 0.413 | PTEN | chr18 | 48608754 | 48608904 | 0.450 | SMAD4 |
| chr10 | 89717603 | 89717769 | 0.455 | PTEN | chr18 | 48609535 | 48609685 | 0.470 | SMAD4 |
| chr10 | 89717627 | 89717872 | 0.362 | PTEN | chr18 | 48609670 | 48609869 | 0.355 | SMAD4 |
| chr10 | 89720757 | 89720967 | 0.336 | PTEN | chr18 | 48610653 | 48610803 | 0.358 | SMAD4 |
| chr10 | 89720757 | 89721005 | 0.329 | PTEN | chr18 | 48610833 | 48611078 | 0.488 | SMAD4 |
| chr10 | 89720767 | 89720968 | 0.337 | PTEN | chr18 | 48611097 | 48611339 | 0.490 | SMAD4 |
| chr10 | 89720775 | 89721018 | 0.324 | PTEN | chr18 | 48611405 | 48611605 | 0.353 | SMAD4 |
| chr10 | 89726371 | 89726571 | 0.289 | PTEN | chr19 | 1206543 | 1206797 | 0.643 | STK11 |
| chr10 | 89726794 | 89727038 | 0.310 | PTEN | chr19 | 1206613 | 1206843 | 0.558 | STK11 |
| chr10 | 89727021 | 89727240 | 0.282 | PTEN | chr19 | 1206770 | 1207017 | 0.560 | STK11 |
| chr10 | 89727261 | 89727519 | 0.317 | PTEN | chr19 | 1206962 | 1207206 | 0.596 | STK11 |
| chr10 | 89727756 | 89727916 | 0.404 | PTEN | chr19 | 1218302 | 1218502 | 0.532 | STK11 |
| chr10 | 89727978 | 89728203 | 0.389 | PTEN | chr19 | 1219161 | 1219399 | 0.653 | STK11 |
| chr10 | 89728310 | 89728512 | 0.389 | PTEN | chr19 | 1220251 | 1220492 | 0.632 | STK11 |
| chr10 | 89729027 | 89729257 | 0.251 | PTEN | chr19 | 1220276 | 1220492 | 0.636 | STK11 |
| chr10 | 89729816 | 89729967 | 0.336 | PTEN | chr19 | 1220475 | 1220701 | 0.705 | STK11 |
| chr10 | 89730284 | 89730433 | 0.307 | PTEN | chr19 | 1220502 | 1220701 | 0.715 | STK11 |
| chr10 | 89731453 | 89731610 | 0.329 | PTEN | chr19 | 1220681 | 1220938 | 0.647 | STK11 |
| chr11 | 22644366 | 22644570 | 0.273 | FANCF | chr19 | 1220681 | 1220941 | 0.648 | STK11 |
| chr11 | 22644511 | 22644731 | 0.344 | FANCF | chr19 | 1221151 | 1221351 | 0.582 | STK11 |
| chr11 | 22644738 | 22644938 | 0.318 | FANCF | chr19 | 1221821 | 1222071 | 0.685 | STK11 |
| chr11 | 22645645 | 22645808 | 0.348 | FANCF | chr19 | 1222984 | 1223224 | 0.643 | STK11 |
| chr11 | 22645808 | 22646060 | 0.352 | FANCF | chr19 | 1226453 | 1226708 | 0.715 | STK11 |
| chr11 | 22646058 | 22646268 | 0.322 | FANCF | chr19 | 1226464 | 1226754 | 0.704 | STK11 |
| chr11 | 22646388 | 22646588 | 0.532 | FANCF | chr19 | 1226465 | 1226707 | 0.716 | STK11 |
| chr11 | 22646657 | 22646927 | 0.598 | FANCF | chr19 | 1228261 | 1228461 | 0.547 | STK11 |
| chr11 | 22646959 | 22647229 | 0.657 | FANCF | chr19 | 3114798 | 3115046 | 0.683 | GNA11 |
| chr11 | 22647378 | 22647578 | 0.478 | FANCF | chr19 | 3114798 | 3115049 | 0.683 | GNA11 |
| chr11 | 47236728 | 47236949 | 0.635 | DDB2 | chr19 | 3114839 | 3115040 | 0.688 | GNA11 |
| chr11 | 47237804 | 47238058 | 0.537 | DDB2 | chr19 | 3114841 | 3115040 | 0.685 | GNA11 |
| chr11 | 47238291 | 47238491 | 0.483 | DDB2 | chr19 | 3118772 | 3118996 | 0.622 | GNA11 |
| chr11 | 47254333 | 47254547 | 0.470 | DDB2 | chr19 | 3118795 | 3119044 | 0.620 | GNA11 |
| chr11 | 47256241 | 47256494 | 0.547 | DDB2 | chr19 | 3118818 | 3119047 | 0.613 | GNA11 |
| chr11 | 47256782 | 47257010 | 0.594 | DDB2 | chr19 | 3118863 | 3119109 | 0.636 | GNA11 |
| chr11 | 47259397 | 47259552 | 0.468 | DDB2 | chr19 | 10600276 | 10600491 | 0.588 | KEAP1 |
| chr11 | 47259555 | 47259796 | 0.492 | DDB2 | chr19 | 10600284 | 10600528 | 0.592 | KEAP1 |
| chr11 | 47260567 | 47260808 | 0.483 | DDB2 | chr19 | 10600284 | 10600532 | 0.594 | KEAP1 |
| chr11 | 61197603 | 61197830 | 0.588 | SDHAF2 | chr19 | 10602242 | 10602441 | 0.645 | KEAP1 |
| chr11 | 61205114 | 61205296 | 0.481 | SDHAF2 | chr19 | 10602423 | 10602671 | 0.691 | KEAP1 |
| chr11 | 61205433 | 61205588 | 0.404 | SDHAF2 | chr19 | 10602539 | 10602747 | 0.632 | KEAP1 |
| chr11 | 61213390 | 61213639 | 0.488 | SDHAF2 | chr19 | 10602539 | 10602753 | 0.628 | KEAP1 |
| chr11 | 61213676 | 61213931 | 0.508 | SDHAF2 | chr19 | 10602653 | 10602854 | 0.609 | KEAP1 |
| chr11 | 61213967 | 61214232 | 0.462 | SDHAF2 | chr19 | 10610056 | 10610289 | 0.585 | KEAP1 |
| chr11 | 64570946 | 64571196 | 0.478 | MEN1 | chr19 | 10610069 | 10610306 | 0.576 | KE EAP1 |
| chr11 | 64571178 | 64571436 | 0.544 | MEN1 | chr19 | 10610081 | 10610306 | 0.580 | KE EAP1 |
| chr11 | 64571465 | 64571704 | 0.546 | MEN1 | chr19 | 10610083 | 10610306 | 0.580 | KEAP1 |
| chr11 | 64571732 | 64571978 | 0.567 | MEN1 | chr19 | 11094768 | 11095038 | 0.697 | S MARCA4 |
| chr11 | 64572483 | 64572713 | 0.619 | MEN1 | chr19 | 11095950 | 11096150 | 0.592 | S MARCA4 |
| chr11 | 64573013 | 64573278 | 0.602 | MEN1 | chr19 | 11096820 | 11097078 | 0.625 | S MARCA4 |

(continued)

| Chr. | Start | Stop | GC | Gene | Chr. | Start | Stop | GC | Gene |
|------|-------|------|-----|------|------|-------|------|-----|------|
| chr11 | 64573641 | 64573871 | 0.593 | MEN1 | chr19 | 11097475 | 11097685 | 0.645 | S MARCA4 |
| chr11 | 64574483 | 64574728 | 0.602 | MEN1 | chr19 | 11098265 | 11098553 | 0.706 | S MARCA4 |
| chr11 | 64575094 | 64575345 | 0.544 | MEN1 | chr19 | 11098298 | 11098538 | 0.726 | S MARCA4 |
| chr11 | 64575352 | 64575623 | 0.614 | MEN1 | chr19 | 11098376 | 11098606 | 0.732 | S MARCA4 |
| chr11 | 64577138 | 64577410 | 0.634 | MEN1 | chr19 | 11099901 | 11100111 | 0.607 | S MARCA4 |
| chr11 | 64577437 | 64577683 | 0.700 | MEN1 | chr19 | 11101757 | 11101957 | 0.612 | S MARCA4 |
| chr11 | 64577902 | 64578171 | 0.681 | MEN1 | chr19 | 11105466 | 11105666 | 0.522 | S MARCA4 |
| chr11 | 94150558 | 94150800 | 0.362 | MRE11A | chr19 | 11106694 | 11106939 | 0.557 | S MARCA4 |
| chr11 | 94151042 | 94151242 | 0.373 | NRE11A | chr19 | 11107143 | 11107352 | 0.476 | S MARCA4 |
| chr11 | 94151616 | 94151816 | 0.537 | MRE11A | chr19 | 11113645 | 11113845 | 0.592 | S MARCA4 |
| chr11 | 94151903 | 94152103 | 0.343 | NRE11A | chr19 | 11113855 | 11114055 | 0.493 | S MARCA4 |
| chr11 | 94152190 | 94152390 | 0.333 | NRE11A | chr19 | 11118483 | 11118683 | 0.587 | S MARCA4 |
| chr11 | 94153182 | 94153395 | 0.308 | MRE11A | chr19 | 11120977 | 11121177 | 0.557 | S MARCA4 |
| chr11 | 94168979 | 94169178 | 0.335 | MRE11A | chr19 | 11123551 | 11123751 | 0.592 | S MARCA4 |
| chr11 | 94170337 | 94170576 | 0.300 | MRE11A | chr19 | 11129571 | 11129781 | 0.555 | S MARCA4 |
| chr11 | 94178876 | 94179116 | 0.386 | NRE11A | chr19 | 11130134 | 11130372 | 0.619 | S MARCA4 |
| chr11 | 94180384 | 94180615 | 0.500 | MRE11A | chr19 | 11130167 | 11130367 | 0.612 | S MARCA4 |
| chr11 | 94189360 | 94189588 | 0.323 | NRE11A | chr19 | 11130245 | 11130468 | 0.585 | S MARCA4 |
| chr11 | 94192639 | 94192838 | 0.370 | MRE11A | chr19 | 11130248 | 11130473 | 0.584 | S MARCA4 |
| chr11 | 94193992 | 94194257 | 0.301 | MRE11A | chr19 | 11132367 | 11132587 | 0.611 | S MARCA4 |
| chr11 | 94197223 | 94197451 | 0.349 | MRE11A | chr19 | 11132607 | 11132807 | 0.622 | S MARCA4 |
| chr11 | 94200864 | 94201064 | 0.363 | NRE11A | chr19 | 11134126 | 11134326 | 0.557 | S MARCA4 |
| chr11 | 94203635 | 94203874 | 0.404 | MRE11A | chr19 | 11134905 | 11135105 | 0.617 | S MARCA4 |
| chr11 | 94204708 | 94204908 | 0.368 | NRE11A | chr19 | 11135986 | 11136186 | 0.602 | S MARCA4 |
| chr11 | 94209357 | 94209557 | 0.348 | MRE11A | chr19 | 11136874 | 11137104 | 0.610 | S MARCA4 |
| chr11 | 94211862 | 94212106 | 0.359 | MRE11A | chr19 | 11138402 | 11138674 | 0.502 | S MARCA4 |
| chr11 | 94212728 | 94212928 | 0.378 | MRE11A | chr19 | 11138598 | 11138818 | 0.597 | S MARCA4 |
| chr11 | 94219015 | 94219215 | 0.348 | NRE11A | chr19 | 11141332 | 11141532 | 0.637 | S MARCA4 |
| chr11 | 94219225 | 94219425 | 0.264 | MRE11A | chr19 | 11141549 | 11141749 | 0.627 | S MARCA4 |
| chr11 | 94223880 | 94224120 | 0.344 | MRE11A | chr19 | 11143924 | 11144134 | 0.621 | S MARCA4 |
| chr11 | 94223898 | 94224142 | 0.327 | MRE11A | chr19 | 11144041 | 11144262 | 0.617 | S MARCA4 |
| chr11 | 94225885 | 94226125 | 0.394 | MRE11A | chr19 | 11144868 | 11145108 | 0.618 | S MARCA4 |
| chr11 | 108093593 | 108093813 | 0.615 | ATM | chr19 | 11145552 | 11145823 | 0.654 | S MARCA4 |
| chr11 | 108093873 | 108094073 | 0.617 | ATM | chr19 | 11151919 | 11152129 | 0.616 | S MARCA4 |
| chr11 | 108098331 | 108098581 | 0.335 | ATM | chr19 | 11151919 | 11152189 | 0.627 | S MARCA4 |
| chr11 | 108098372 | 108098572 | 0.333 | ATM | chr19 | 11152171 | 11152371 | 0.572 | S MARCA4 |
| chr11 | 108098382 | 108098631 | 0.316 | ATM | chr19 | 11168890 | 11169140 | 0.625 | S MARCA4 |
| chr11 | 108098397 | 108098626 | 0.309 | ATM | chr19 | 11169359 | 11169619 | 0.644 | S MARCA4 |
| chr11 | 108098399 | 108098628 | 0.304 | ATM | chr19 | 11170610 | 11170850 | 0.668 | S MARCA4 |
| chr11 | 108099818 | 108100062 | 0.327 | ATM | chr19 | 11172452 | 11172706 | 0.498 | S MARCA4 |
| chr11 | 108106395 | 108106596 | 0.356 | ATM | chr19 | 11172557 | 11172757 | 0.423 | SMARCA4 |
| chr11 | 108114723 | 108115004 | 0.333 | ATM | chr19 | 11172753 | 11172953 | 0.493 | SMARCA4 |
| chr11 | 108114777 | 108115004 | 0.329 | ATM | chr19 | 45854611 | 45854870 | 0.581 | ERCC2 |
| chr11 | 108115492 | 108115736 | 0.351 | ATM | chr19 | 45854917 | 45855123 | 0.589 | ERCC2 |
| chr11 | 108117690 | 108117930 | 0.324 | ATM | chr19 | 45855406 | 45855649 | 0.656 | ERCC2 |
| chr11 | 108119692 | 108119907 | 0.343 | ATM | chr19 | 45855711 | 45855931 | 0.611 | ERCC2 |
| chr11 | 108119737 | 108119952 | 0.361 | ATM | chr19 | 45855908 | 45856123 | 0.611 | ERCC2 |
| chr11 | 108121367 | 108121602 | 0.377 | ATM | chr19 | 45855910 | 45856123 | 0.612 | ERCC2 |
| chr11 | 108121609 | 108121764 | 0.359 | ATM | chr19 | 45855914 | 45856123 | 0.610 | ERCC2 |
| chr11 | 108122506 | 108122706 | 0.363 | ATM | chr19 | 45855948 | 45856167 | 0.609 | ERCC2 |

(continued)

| Chr. | Start | Stop | GC | Gene | Chr. | Start | Stop | GC | Gene |
|---|---|---|---|---|---|---|---|---|---|
| chr11 | 108122716 | 108122936 | 0.317 | ATM | chr19 | 45855959 | 45856169 | 0.607 | ERCC2 |
| chr11 | 108123498 | 108123718 | 0.321 | ATM | chr19 | 45856222 | 45856442 | 0.652 | ERCC2 |
| chr11 | 108124529 | 108124729 | 0.393 | ATM | chr19 | 45857893 | 45858113 | 0.597 | ERCC2 |
| chr11 | 108126954 | 108127154 | 0.363 | ATM | chr19 | 45860528 | 45860730 | 0.635 | ERCC2 |
| chr11 | 108128041 | 108128241 | 0.294 | ATM | chr19 | 45860797 | 45861007 | 0.635 | ERCC2 |
| chr11 | 108129523 | 108129756 | 0.316 | ATM | chr19 | 45864772 | 45864905 | 0.575 | ERCC2 |
| chr11 | 108137908 | 108138102 | 0.374 | ATM | chr19 | 45866937 | 45867137 | 0.706 | ERCC2 |
| chr11 | 108139112 | 108139322 | 0.374 | ATM | chr19 | 45867119 | 45867343 | 0.724 | ERCC2 |
| chr11 | 108139329 | 108139529 | 0.338 | ATM | chr19 | 45867124 | 45867373 | 0.716 | ERCC2 |
| chr11 | 108141794 | 108142040 | 0.324 | ATM | chr19 | 45867329 | 45867584 | 0.684 | ERCC2 |
| chr11 | 108142019 | 108142263 | 0.359 | ATM | chr19 | 45867491 | 45867711 | 0.656 | ERCC2 |
| chr11 | 108143141 | 108143341 | 0.323 | ATM | chr19 | 45867506 | 45867746 | 0.660 | ERCC2 |
| chr11 | 108143328 | 108143577 | 0.336 | ATM | chr19 | 45867566 | 45867806 | 0.618 | ERCC2 |
| chr11 | 108150264 | 108150498 | 0.328 | ATM | chr19 | 45868096 | 45868344 | 0.635 | ERCC2 |
| chr11 | 108151721 | 108151951 | 0.364 | ATM | chr19 | 45868149 | 45868349 | 0.637 | ERCC2 |
| chr11 | 108153427 | 108153675 | 0.285 | ATM | chr19 | 45868154 | 45868396 | 0.626 | ERCC2 |
| chr11 | 108153471 | 108153632 | 0.284 | ATM | chr19 | 45868287 | 45868486 | 0.600 | ERCC2 |
| chr11 | 108153471 | 108153677 | 0.275 | ATM | chr19 | 45871786 | 45871991 | 0.524 | ERCC2 |
| chr11 | 108153505 | 108153680 | 0.267 | ATM | chr19 | 45872064 | 45872264 | 0.582 | ERCC2 |
| chr11 | 108153510 | 108153680 | 0.269 | ATM | chr19 | 45872211 | 45872411 | 0.562 | ERCC2 |
| chr11 | 108154843 | 108155094 | 0.282 | ATM | chr19 | 45873397 | 45873585 | 0.661 | ERCC2 |
| chr11 | 108154858 | 108155070 | 0.291 | ATM | chr19 | 45873421 | 45873651 | 0.675 | ERCC2 |
| chr11 | 108154954 | 108155155 | 0.396 | ATM | chr19 | 45873436 | 45873665 | 0.687 | ERCC2 |
| chr11 | 108154962 | 108155211 | 0.388 | ATM | chr19 | 45873632 | 45873861 | 0.700 | ERCC2 |
| chr11 | 108157899 | 108158161 | 0.354 | ATM | chr19 | 45873636 | 45873901 | 0.684 | ERCC2 |
| chr11 | 108158376 | 108158580 | 0.337 | ATM | chr19 | 45873726 | 45873936 | 0.645 | ERCC2 |
| chr11 | 108159642 | 108159842 | 0.343 | ATM | chr19 | 45916987 | 45917191 | 0.571 | ERCC1 |
| chr11 | 108160273 | 108160473 | 0.299 | ATM | chr19 | 45918047 | 45918240 | 0.619 | ERCC1 |
| chr11 | 108163344 | 108163589 | 0.378 | ATM | chr19 | 45918047 | 45918243 | 0.619 | ERCC1 |
| chr11 | 108164044 | 108164225 | 0.324 | ATM | chr19 | 45918048 | 45918243 | 0.622 | ERCC1 |
| chr11 | 108164078 | 108164281 | 0.314 | ATM | chr19 | 45918053 | 45918243 | 0.628 | ERCC1 |
| chr11 | 108165595 | 108165795 | 0.358 | ATM | chr19 | 45918060 | 45918235 | 0.625 | ERCC1 |
| chr11 | 108167798 | 108168039 | 0.269 | ATM | chr19 | 45922222 | 45922436 | 0.567 | ERCC1 |
| chr11 | 108167811 | 108168065 | 0.267 | ATM | chr19 | 45923506 | 45923678 | 0.607 | ERCC1 |
| chr11 | 108170423 | 108170622 | 0.380 | ATM | chr19 | 45924445 | 45924632 | 0.638 | ERCC1 |
| chr11 | 108172321 | 108172561 | 0.340 | ATM | chr19 | 45926611 | 45926815 | 0.615 | ERCC1 |
| chr11 | 108173513 | 108173723 | 0.351 | ATM | chr19 | 50902097 | 50902336 | 0.650 | POLD1 |
| chr11 | 108173723 | 108173923 | 0.313 | ATM | chr19 | 50902458 | 50902658 | 0.582 | POLD1 |
| chr11 | 108175366 | 108175566 | 0.398 | ATM | chr19 | 50904960 | 50905171 | 0.656 | POLD1 |
| chr11 | 108178629 | 108178814 | 0.360 | ATM | chr19 | 50905153 | 50905392 | 0.671 | POLD1 |
| chr11 | 108179595 | 108179834 | 0.375 | ATM | chr19 | 50905436 | 50905665 | 0.683 | POLD1 |
| chr11 | 108180801 | 108181050 | 0.320 | ATM | chr19 | 50905615 | 50905913 | 0.659 | POLD1 |
| chr11 | 108180821 | 108181050 | 0.322 | ATM | chr19 | 50905899 | 50906137 | 0.665 | POLD1 |
| chr11 | 108180821 | 108181066 | 0.313 | ATM | chr19 | 50906253 | 50906464 | 0.679 | POLD1 |
| chr11 | 108180871 | 108181071 | 0.333 | ATM | chr19 | 50906723 | 50906971 | 0.622 | POLD1 |
| chr11 | 108183026 | 108183226 | 0.333 | ATM | chr19 | 50906755 | 50906971 | 0.613 | POLD1 |
| chr11 | 108186520 | 108186768 | 0.357 | ATM | chr19 | 50909485 | 50909773 | 0.640 | POLD1 |
| chr11 | 108186672 | 108186911 | 0.404 | ATM | chr19 | 50910320 | 50910533 | 0.631 | POLD1 |
| chr11 | 108188080 | 108188262 | 0.399 | ATM | chr19 | 50910334 | 50910578 | 0.629 | POLD1 |
| chr11 | 108190668 | 108190878 | 0.313 | ATM | chr19 | 50910376 | 50910627 | 0.631 | POLD1 |

(continued)

| Chr. | Start | Stop | GC | Gene | Chr. | Start | Stop | GC | Gene |
|---|---|---|---|---|---|---|---|---|---|
| chr11 | 108191937 | 108192186 | 0.392 | ATM | chr19 | 50912042 | 50912281 | 0.617 | POLD1 |
| chr11 | 108195999 | 108196199 | 0.413 | ATM | chr19 | 50912288 | 50912520 | 0.635 | POLD1 |
| chr11 | 108196116 | 108196316 | 0.388 | ATM | chr19 | 50912795 | 50913010 | 0.667 | POLD1 |
| chr11 | 108196772 | 108196944 | 0.422 | ATM | chr19 | 50916709 | 50916950 | 0.640 | POLD1 |
| chr11 | 108198304 | 108198504 | 0.398 | ATM | chr19 | 50916970 | 50917221 | 0.623 | POLD1 |
| chr11 | 108199732 | 108199932 | 0.373 | ATM | chr19 | 50917955 | 50918169 | 0.628 | POLD1 |
| chr11 | 108200863 | 108201105 | 0.374 | ATM | chr19 | 50917960 | 50918169 | 0.624 | POLD1 |
| chr11 | 108201993 | 108202237 | 0.318 | ATM | chr19 | 50918653 | 50918824 | 0.640 | POLD1 |
| chr11 | 108202461 | 108202678 | 0.330 | ATM | chr19 | 50919033 | 50919269 | 0.692 | POLD1 |
| chr11 | 108202483 | 108202676 | 0.309 | ATM | chr19 | 50919496 | 50919745 | 0.684 | POLD1 |
| chr11 | 108202496 | 108202676 | 0.298 | ATM | chr19 | 50919683 | 50919957 | 0.691 | POLD1 |
| chr11 | 108202530 | 108202730 | 0.308 | ATM | chr19 | 50919685 | 50919957 | 0.689 | POLD1 |
| chr11 | 108203403 | 108203613 | 0.336 | ATM | chr19 | 50919770 | 50920048 | 0.699 | POLD1 |
| chr11 | 108203427 | 108203679 | 0.344 | ATM | chr19 | 50919821 | 50920050 | 0.691 | POLD1 |
| chr11 | 108203480 | 108203724 | 0.339 | ATM | chr19 | 50921104 | 50921313 | 0.576 | POLD1 |
| chr11 | 108203524 | 108203768 | 0.339 | ATM | chr2 | 29443543 | 29443787 | 0.539 | ALK |
| chr11 | 108203540 | 108203784 | 0.347 | ATM | chr2 | 29443549 | 29443783 | 0.545 | ALK |
| chr11 | 108204436 | 108204676 | 0.407 | ATM | chr2 | 29443555 | 29443789 | 0.540 | ALK |
| chr11 | 108205641 | 108205841 | 0.368 | ATM | chr2 | 29443585 | 29443816 | 0.500 | ALK |
| chr11 | 108206452 | 108206651 | 0.400 | ATM | chr2 | 29445100 | 29445340 | 0.552 | ALK |
| chr11 | 108213868 | 108214068 | 0.393 | ATM | chr2 | 29445107 | 29445340 | 0.556 | ALK |
| chr11 | 108216481 | 108216700 | 0.355 | ATM | chr2 | 29445121 | 29445350 | 0.570 | ALK |
| chr11 | 108217894 | 108218094 | 0.328 | ATM | chr2 | 29445121 | 29445367 | 0.571 | ALK |
| chr11 | 108224395 | 108224595 | 0.403 | ATM | chr2 | 47600500 | 47600700 | 0.323 | EPCAM |
| chr11 | 108225445 | 108225655 | 0.346 | ATM | chr2 | 47600912 | 47601112 | 0.473 | EPCAM |
| chr11 | 108235722 | 108235932 | 0.365 | ATM | chr2 | 47601122 | 47601322 | 0.383 | EPCAM |
| chr11 | 108235932 | 108236132 | 0.383 | ATM | chr2 | 47602199 | 47602445 | 0.328 | EPCAM |
| chr11 | 108235986 | 108236232 | 0.433 | ATM | chr2 | 47604131 | 47604282 | 0.342 | EPCAM |
| chr11 | 108236004 | 108236249 | 0.431 | ATM | chr2 | 47605987 | 47606187 | 0.249 | EPCAM |
| chr11 | 108236050 | 108236290 | 0.415 | ATM | chr2 | 47606812 | 47606991 | 0.361 | EPCAM |
| chr11 | 108236051 | 108236251 | 0.438 | ATM | chr2 | 47612268 | 47612488 | 0.398 | EPCAM |
| chr11 | 108236071 | 108236273 | 0.438 | ATM | chr2 | 47613710 | 47613910 | 0.343 | EPCAM |
| chr11 | 108238313 | 108238513 | 0.373 | **ATM** | chr2 | 47630105 | 47630305 | 0.652 | MSH2 |
| chr11 | 111957513 | 111957759 | 0.591 | SDHD | chr2 | 47630152 | 47630400 | 0.643 | MSH2 |
| chr11 | 111959529 | 111959746 | 0.486 | SDHD | chr2 | 47630268 | 47630467 | 0.615 | MSH2 |
| chr11 | 111965449 | 111965740 | 0.414 | SDHD | chr2 | 47630315 | 47630515 | 0.667 | MSH2 |
| chr11 | 111965464 | 111965754 | 0.416 | SDHD | chr2 | 47630316 | 47630530 | 0.647 | MSH2 |
| chr12 | 25378488 | 25378688 | 0.353 | KRAS | chr2 | 47630384 | 47630615 | 0.690 | MSH2 |
| chr12 | 25378503 | 25378751 | 0.341 | KRAS | chr2 | 47630425 | 47630625 | 0.706 | MSH2 |
| chr12 | 25378546 | 25378778 | 0.352 | KRAS | chr2 | 47635539 | 47635709 | 0.339 | MSH2 |
| chr12 | 25378554 | 25378783 | 0.348 | KRAS | chr2 | 47637342 | 47637582 | 0.456 | MSH2 |
| chr12 | 25380153 | 25380359 | 0.411 | KRAS | chr2 | 47637389 | 47637594 | 0.442 | MSH2 |
| chr12 | 25380166 | 25380337 | 0.407 | KRAS | chr2 | 47639447 | 47639662 | 0.310 | MSH2 |
| chr12 | 25380167 | 25380326 | 0.406 | KRAS | chr2 | 47641283 | 47641487 | 0.327 | MSH2 |
| chr12 | 25380167 | 25380359 | 0.420 | KRAS | chr2 | 47643346 | 47643546 | 0.398 | MSH2 |
| chr12 | 25398080 | 25398329 | 0.360 | KRAS | chr2 | 47656945 | 47657096 | 0.388 | MSH2 |
| chr12 | 25398145 | 25398394 | 0.348 | KRAS | chr2 | 47672586 | 47672786 | 0.299 | MSH2 |
| chr12 | 25398153 | 25398397 | 0.351 | KRAS | chr2 | 47690076 | 47690276 | 0.303 | MSH2 |
| chr12 | 25398159 | 25398408 | 0.356 | KRAS | chr2 | 47693747 | 47693947 | 0.348 | MSH2 |
| chr12 | 25398186 | 25398433 | 0.347 | KRAS | chr2 | 47698159 | 47698397 | 0.326 | MSH2 |

(continued)

| Chr. | Start | Stop | GC | Gene | Chr. | Start | Stop | GC | Gene |
|------|-------|------|-----|------|------|-------|------|-----|------|
| chr12 | 56478765 | 56478994 | 0.552 | ERBB3 | chr2 | 47702121 | 47702341 | 0.394 | MSH2 |
| chr12 | 56478781 | 56479029 | 0.558 | ERBB3 | chr2 | 47702174 | 47702413 | 0.388 | MSH2 |
| chr12 | 56478784 | 56479029 | 0.561 | ERBB3 | chr2 | 47703453 | 47703653 | 0.428 | MSH2 |
| chr12 | 56478807 | 56479047 | 0.560 | ERBB3 | chr2 | 47703663 | 47703863 | 0.363 | MSH2 |
| chr12 | 56481533 | 56481774 | 0.541 | ERBB3 | chr2 | 47705399 | 47705630 | 0.397 | MSH2 |
| chr12 | 56481559 | 56481798 | 0.521 | ERBB3 | chr2 | 47707788 | 47708018 | 0.424 | MSH2 |
| chr12 | 56481594 | 56481833 | 0.546 | ERBB3 | chr2 | 47709903 | 47710113 | 0.327 | MSH2 |
| chr12 | 56481628 | 56481942 | 0.530 | ERBB3 | chr2 | 47710107 | 47710336 | 0.287 | MSH2 |
| chr12 | 56481740 | 56481979 | 0.517 | ERBB3 | chr2 | 48010393 | 48010592 | 0.725 | MSH6 |
| chr12 | 56481773 | 56482020 | 0.476 | ERBB3 | chr2 | 48010420 | 48010619 | 0.740 | MSH6 |
| chr12 | 56481807 | 56482048 | 0.467 | ERBB3 | chr2 | 48010573 | 48010824 | 0.690 | MSH6 |
| chr12 | 56482218 | 56482457 | 0.513 | ERBB3 | chr2 | 48010575 | 48010781 | 0.725 | MSH6 |
| chr12 | 56482252 | 56482491 | 0.496 | ERBB3 | chr2 | 48017953 | 48018200 | 0.419 | MSH6 |
| chr12 | 56482278 | 56482521 | 0.504 | ERBB3 | chr2 | 48018205 | 48018415 | 0.431 | MSH6 |
| chr12 | 56482331 | 56482580 | 0.528 | ERBB3 | chr2 | 48023040 | 48023272 | 0.455 | MSH6 |
| chr12 | 56486559 | 56486791 | 0.502 | ERBB3 | chr2 | 48023105 | 48023304 | 0.430 | MSH6 |
| chr12 | 56486561 | 56486791 | 0.502 | ERBB3 | chr2 | 48025749 | 48025949 | 0.428 | MSH6 |
| chr12 | 56486566 | 56486813 | 0.508 | ERBB3 | chr2 | 48025933 | 48026201 | 0.476 | MSH6 |
| chr12 | 56486569 | 56486818 | 0.508 | ERBB3 | chr2 | 48026177 | 48026376 | 0.460 | MSH6 |
| chr12 | 56490766 | 56491006 | 0.523 | ERBB3 | chr2 | 48026340 | 48026558 | 0.457 | MSH6 |
| chr12 | 56490766 | 56491013 | 0.520 | ERBB3 | chr2 | 48026511 | 48026711 | 0.458 | MSH6 |
| chr12 | 56490773 | 56491013 | 0.519 | ERBB3 | chr2 | 48026765 | 48026965 | 0.383 | MSH6 |
| chr12 | 56490777 | 56491011 | 0.523 | ERBB3 | chr2 | 48027019 | 48027219 | 0.438 | MSH6 |
| chr12 | 56491580 | 56491801 | 0.554 | ERBB3 | chr2 | 48027273 | 48027473 | 0.438 | MSH6 |
| chr12 | 56491592 | 56491801 | 0.557 | ERBB3 | chr2 | 48027527 | 48027727 | 0.398 | MSH6 |
| chr12 | 56491596 | 56491799 | 0.559 | ERBB3 | chr2 | 48027746 | 48028017 | 0.426 | MSH6 |
| chr12 | 56491596 | 56491801 | 0.558 | ERBB3 | chr2 | 48028124 | 48028364 | 0.415 | MSH6 |
| chr12 | 58141882 | 58142151 | 0.396 | CDK4 | chr2 | 48030536 | 48030736 | 0.433 | MSH6 |
| chr12 | 58142160 | 58142391 | 0.474 | CDK4 | chr2 | 48030729 | 48031008 | 0.371 | MSH6 |
| chr12 | 58142983 | 58143287 | 0.570 | CDK4 | chr2 | 48031952 | 48032152 | 0.443 | MSH6 |
| chr12 | 58142985 | 58143287 | 0.568 | CDK4 | chr2 | 48032026 | 48032237 | 0.420 | MSH6 |
| chr12 | 58144413 | 58144686 | 0.478 | CDK4 | chr2 | 48032772 | 48032971 | 0.315 | MSH6 |
| chr12 | 58144452 | 58144687 | 0.479 | CDK4 | chr2 | 48033358 | 48033554 | 0.365 | MSH6 |
| chr12 | 58144692 | 58144932 | 0.490 | CDK4 | chr2 | 48033416 | 48033655 | 0.367 | MSH6 |
| chr12 | 58144696 | 58144939 | 0.492 | CDK4 | chr2 | 48033581 | 48033782 | 0.401 | MSH6 |
| chr12 | 58144957 | 58145214 | 0.523 | CDK4 | chr2 | 48034024 | 48034264 | 0.307 | MSH6 |
| chr12 | 58145023 | 58145294 | 0.515 | CDK4 | chr2 | 58386432 | 58386632 | 0.333 | FANCL |
| chr12 | 58145309 | 58145580 | 0.544 | CDK4 | chr2 | 58386777 | 58386929 | 0.307 | FANCL |
| chr12 | 58145326 | 58145580 | 0.541 | CDK4 | chr2 | 58387189 | 58387389 | 0.338 | FANCL |
| chr12 | 58145924 | 58146140 | 0.673 | CDK4 | chr2 | 58388496 | 58388732 | 0.346 | FANCL |
| chr12 | 133200375 | 133200607 | 0.541 | POLE | chr2 | 58389905 | 58390169 | 0.328 | FANCL |
| chr12 | 133200794 | 133201005 | 0.604 | POLE | chr2 | 58390005 | 58390214 | 0.352 | FANCL |
| chr12 | 133200978 | 133201217 | 0.608 | POLE | chr2 | 58390571 | 58390773 | 0.379 | FANCL |
| chr12 | 133201185 | 133201425 | 0.610 | POLE | chr2 | 58392779 | 58392979 | 0.398 | FANCL |
| chr12 | 133201428 | 133201670 | 0.654 | POLE | chr2 | 58431158 | 58431358 | 0.363 | FANCL |
| chr12 | 133202187 | 133202429 | 0.621 | POLE | chr2 | 58448972 | 58449172 | 0.323 | FANCL |
| chr12 | 133202217 | 133202436 | 0.605 | POLE | chr2 | 58453736 | 58453936 | 0.308 | FANCL |
| chr12 | 133202217 | 133202437 | 0.606 | POLE | chr2 | 58456824 | 58457044 | 0.285 | FANCL |
| chr12 | 133202218 | 133202437 | 0.605 | POLE | chr2 | 58459138 | 58459342 | 0.351 | FANCL |
| chr12 | 133202355 | 133202555 | 0.622 | POLE | chr2 | 58468279 | 58468479 | 0.592 | FANCL |

(continued)

| Chr. | Start | Stop | GC | Gene | Chr. | Start | Stop | GC | Gene |
|---|---|---|---|---|---|---|---|---|---|
| chr12 | 133202648 | 133202868 | 0.606 | POLE | chr2 | 128014784 | 128015047 | 0.394 | ERCC3 |
| chr12 | 133208842 | 133209042 | 0.517 | POLE | chr2 | 128015087 | 128015289 | 0.522 | ERCC3 |
| chr12 | 133209059 | 133209259 | 0.577 | POLE | chr2 | 128016793 | 128017023 | 0.541 | ERCC3 |
| chr12 | 133209191 | 133209391 | 0.647 | POLE | chr2 | 128018729 | 128018934 | 0.476 | ERCC3 |
| chr12 | 133210529 | 133210806 | 0.590 | POLE | chr2 | 128028846 | 128029046 | 0.493 | ERCC3 |
| chr12 | 133212413 | 133212654 | 0.488 | POLE | chr2 | 128030334 | 128030534 | 0.488 | ERCC3 |
| chr12 | 133214538 | 133214738 | 0.557 | POLE | chr2 | 128036695 | 128036895 | 0.413 | ERCC3 |
| chr12 | 133215637 | 133215885 | 0.582 | POLE | chr2 | 128037952 | 128038168 | 0.535 | ERCC3 |
| chr12 | 133218204 | 133218451 | 0.597 | POLE | chr2 | 128044271 | 128044501 | 0.580 | ERCC3 |
| chr12 | 133218707 | 133218953 | 0.579 | POLE | chr2 | 128044494 | 128044693 | 0.500 | ERCC3 |
| chr12 | 133219069 | 133219298 | 0.574 | POLE | chr2 | 128046183 | 128046383 | 0.502 | ERCC3 |
| chr12 | 133219399 | 133219599 | 0.617 | POLE | chr2 | 128046840 | 128047040 | 0.468 | ERCC3 |
| chr12 | 133219778 | 133220012 | 0.591 | POLE | chr2 | 128047177 | 128047377 | 0.522 | ERCC3 |
| chr12 | 133219996 | 133220226 | 0.606 | POLE | chr2 | 128047669 | 128047869 | 0.448 | ERCC3 |
| chr12 | 133220308 | 133220545 | 0.571 | POLE | chr2 | 128050206 | 128050419 | 0.514 | ERCC3 |
| chr12 | 133225529 | 133225773 | 0.612 | POLE | chr2 | 128051102 | 128051343 | 0.566 | ERCC3 |
| chr12 | 133225887 | 133226117 | 0.645 | POLE | chr2 | 128051633 | 128051852 | 0.655 | ERCC3 |
| chr12 | 133226161 | 133226408 | 0.613 | POLE | chr2 | 128051746 | 128051946 | 0.577 | ERCC3 |
| chr12 | 133233672 | 133233909 | 0.550 | POLE | chr2 | 209112977 | 209113230 | 0.394 | IDH1 |
| chr12 | 133233774 | 133234003 | 0.557 | POLE | chr2 | 209113091 | 209113340 | 0.444 | IDH1 |
| chr12 | 133234350 | 133234550 | 0.458 | POLE | chr2 | 212288833 | 212289075 | 0.366 | ERBB4 |
| chr12 | 133235953 | 133236173 | 0.534 | POLE | chr2 | 212288836 | 212289075 | 0.367 | ERBB4 |
| chr12 | 133237539 | 133237827 | 0.543 | POLE | chr2 | 212288849 | 212289089 | 0.378 | ERBB4 |
| chr12 | 133237547 | 133237781 | 0.519 | POLE | chr2 | 212288867 | 212289111 | 0.376 | ERBB4 |
| chr12 | 133238036 | 133238236 | 0.468 | POLE | chr2 | 212483732 | 212483976 | 0.327 | ERBB4 |
| chr12 | 133240495 | 133240681 | 0.545 | POLE | chr2 | 212483745 | 212483975 | 0.338 | ERBB4 |
| chr12 | 133241011 | 133241211 | 0.587 | POLE | chr2 | 212483745 | 212483989 | 0.343 | ERBB4 |
| chr12 | 133241805 | 133242034 | 0.600 | POLE | chr2 | 212483745 | 212483992 | 0.347 | ERBB4 |
| chr12 | 133244016 | 133244246 | 0.563 | POLE | chr2 | 212529983 | 212530215 | 0.468 | ERBB4 |
| chr12 | 133244860 | 133245070 | 0.578 | POLE | chr2 | 212529983 | 212530219 | 0.468 | ERBB4 |
| chr12 | 133245148 | 133245397 | 0.616 | POLE | chr2 | 212530006 | 212530255 | 0.444 | ERBB4 |
| chr12 | 133245378 | 133245623 | 0.537 | POLE | chr2 | 212530049 | 212530293 | 0.420 | ERBB4 |
| chr12 | 133248734 | 133248981 | 0.556 | POLE | chr2 | 212587102 | 212587341 | 0.379 | ERBB4 |
| chr12 | 133249169 | 133249404 | 0.568 | POLE | chr2 | 212587102 | 212587342 | 0.378 | ERBB4 |
| chr12 | 133249662 | 133249902 | 0.539 | POLE | chr2 | 212587104 | 212587343 | 0.379 | ERBB4 |
| chr12 | 133250240 | 133250445 | 0.549 | POLE | chr2 | 215591713 | 215591913 | 0.348 | BARD1 |
| chr12 | 133250282 | 133250482 | 0.517 | POLE | chr2 | 215592049 | 215592249 | 0.313 | BARD1 |
| chr12 | 133251897 | 133252126 | 0.600 | POLE | chr2 | 215592385 | 215592585 | 0.318 | BARD1 |
| chr12 | 133252231 | 133252480 | 0.512 | POLE | chr2 | 215592721 | 215592921 | 0.284 | BARD1 |
| chr12 | 133252591 | 133252826 | 0.470 | POLE | chr2 | 215593393 | 215593593 | 0.458 | BARD1 |
| chr12 | 133253058 | 133253290 | 0.502 | POLE | chr2 | 215593464 | 215593712 | 0.482 | BARD1 |
| chr12 | 133253869 | 133254099 | 0.429 | POLE | chr2 | 215595070 | 215595319 | 0.348 | BARD1 |
| chr12 | 133254080 | 133254312 | 0.502 | POLE | chr2 | 215609774 | 215610018 | 0.331 | BARD1 |
| chr12 | 133256057 | 133256299 | 0.494 | POLE | chr2 | 215610357 | 215610557 | 0.368 | BARD1 |
| chr12 | 133256546 | 133256806 | 0.441 | POLE | chr2 | 215617201 | 215617402 | 0.322 | BARD1 |
| chr12 | 133256751 | 133257006 | 0.469 | POLE | chr2 | 215617244 | 215617478 | 0.315 | BARD1 |
| chr12 | 133257134 | 133257364 | 0.429 | POLE | chr2 | 215632137 | 215632347 | 0.398 | BARD1 |
| chr12 | 133257609 | 133257851 | 0.523 | POLE | chr2 | 215632347 | 215632547 | 0.299 | BARD1 |
| chr13 | 28592527 | 28592791 | 0.434 | FLT3 | chr2 | 215633841 | 215634041 | 0.348 | BARD1 |
| chr13 | 32889625 | 32889857 | 0.631 | BRCA2 | chr2 | 215645283 | 215645483 | 0.418 | BARD1 |

(continued)

| Chr. | Start | Stop | GC | Gene | Chr. | Start | Stop | GC | Gene |
|---|---|---|---|---|---|---|---|---|---|
| chr13 | 32889901 | 32890111 | 0.583 | BRCA2 | chr2 | 215645568 | 215645768 | 0.398 | BARD1 |
| chr13 | 32890514 | 32890741 | 0.346 | BRCA2 | chr2 | 215645789 | 215646022 | 0.385 | BARD1 |
| chr13 | 32893139 | 32893383 | 0.339 | BRCA2 | chr2 | 215645997 | 215646196 | 0.405 | BARD1 |
| chr13 | 32900135 | 32900368 | 0.252 | BRCA2 | chr2 | 215646018 | 215646167 | 0.413 | BARD1 |
| chr13 | 32900239 | 32900484 | 0.305 | BRCA2 | chr2 | 215656994 | 215657164 | 0.421 | BARD1 |
| chr13 | 32900514 | 32900762 | 0.373 | BRCA2 | chr2 | 215661815 | 215662059 | 0.355 | BARD1 |
| chr13 | 32903445 | 32903674 | 0.278 | BRCA2 | chr2 | 215674040 | 215674299 | 0.662 | BARD1 |
| chr13 | 32904938 | 32905182 | 0.343 | BRCA2 | chr2 | 215674057 | 215674299 | 0.671 | BARD1 |
| chr13 | 32905002 | 32905201 | 0.315 | BRCA2 | chr2 | 215674060 | 215674299 | 0.675 | BARD1 |
| chr13 | 32905048 | 32905165 | 0.339 | BRCA2 | chr2 | 215674115 | 215674321 | 0.681 | BARD1 |
| chr13 | 32905048 | 32905168 | 0.347 | BRCA2 | chr20 | 57484302 | 57484538 | 0.439 | GNAS |
| chr13 | 32905048 | 32905170 | 0.341 | BRCA2 | chr21 | 36252718 | 36252963 | 0.427 | RUNX1 |
| chr13 | 32905049 | 32905165 | 0.342 | BRCA2 | chr21 | 36252753 | 36253001 | 0.454 | RUNX1 |
| chr13 | 32906224 | 32906468 | 0.302 | BRCA2 | chr21 | 36252796 | 36253037 | 0.471 | RUNX1 |
| chr13 | 32906406 | 32906650 | 0.310 | BRCA2 | chr21 | 36252819 | 36253063 | 0.469 | RUNX1 |
| chr13 | 32906408 | 32906628 | 0.317 | BRCA2 | chr22 | 29092793 | 29093014 | 0.383 | CHEK2 |
| chr13 | 32906426 | 32906673 | 0.306 | BRCA2 | chr22 | 29095766 | 29095985 | 0.468 | CHEK2 |
| chr13 | 32906464 | 32906663 | 0.305 | BRCA2 | chr22 | 29099378 | 29099614 | 0.354 | CHEK2 |
| chr13 | 32906520 | 32906768 | 0.317 | BRCA2 | chr22 | 29105946 | 29106126 | 0.243 | CHEK2 |
| chr13 | 32906575 | 32906818 | 0.361 | BRCA2 | chr22 | 29105988 | 29106140 | 0.288 | CHEK2 |
| chr13 | 32906606 | 32906846 | 0.378 | BRCA2 | chr22 | 29107796 | 29107996 | 0.373 | CHEK2 |
| chr13 | 32906668 | 32906912 | 0.388 | BRCA2 | chr22 | 29115374 | 29115613 | 0.292 | CHEK2 |
| chr13 | 32906748 | 32906987 | 0.388 | BRCA2 | chr22 | 29120968 | 29121207 | 0.358 | CHEK2 |
| chr13 | 32906815 | 32907062 | 0.363 | BRCA2 | chr22 | 29121185 | 29121429 | 0.376 | CHEK2 |
| chr13 | 32906856 | 32907103 | 0.367 | BRCA2 | chr22 | 29130538 | 29130762 | 0.556 | CHEK2 |
| chr13 | 32906893 | 32907106 | 0.383 | BRCA2 | chr22 | 29130552 | 29130805 | 0.508 | CHEK2 |
| chr13 | 32906938 | 32907183 | 0.378 | BRCA2 | chr22 | 29137634 | 29137834 | 0.547 | CHEK2 |
| chr13 | 32907059 | 32907264 | 0.374 | BRCA2 | chr3 | 10070202 | 10070402 | 0.383 | FANCD2 |
| chr13 | 32907059 | 32907307 | 0.378 | BRCA2 | chr3 | 10074431 | 10074641 | 0.303 | FANCD2 |
| chr13 | 32907288 | 32907533 | 0.350 | BRCA2 | chr3 | 10076444 | 10076673 | 0.430 | FANCD2 |
| chr13 | 32910416 | 32910655 | 0.354 | BRCA2 | chr3 | 10076732 | 10076932 | 0.323 | FANCD2 |
| chr13 | 32910596 | 32910835 | 0.388 | BRCA2 | chr3 | 10077981 | 10078130 | 0.313 | FANCD2 |
| chr13 | 32910778 | 32911027 | 0.340 | BRCA2 | chr3 | 10080961 | 10081110 | 0.367 | FANCD2 |
| chr13 | 32910967 | 32911215 | 0.317 | BRCA2 | chr3 | 10081391 | 10081592 | 0.510 | FANCD2 |
| chr13 | 32910988 | 32911187 | 0.335 | BRCA2 | chr3 | 10083255 | 10083471 | 0.433 | FANCD2 |
| chr13 | 32911008 | 32911252 | 0.331 | BRCA2 | chr3 | 10116194 | 10116415 | 0.405 | FANCD2 |
| chr13 | 32911035 | 32911252 | 0.321 | BRCA2 | chr3 | 10119764 | 10119916 | 0.523 | FANCD2 |
| chr13 | 32911045 | 32911295 | 0.331 | BRCA2 | chr3 | 10122693 | 10122903 | 0.422 | FANCD2 |
| chr13 | 32911167 | 32911415 | 0.341 | BRCA2 | chr3 | 10122903 | 10123103 | 0.378 | FANCD2 |
| chr13 | 32911340 | 32911588 | 0.333 | BRCA2 | chr3 | 10123144 | 10123344 | 0.358 | FANCD2 |
| chr13 | 32911594 | 32911838 | 0.322 | BRCA2 | chr3 | 10127494 | 10127703 | 0.433 | FANCD2 |
| chr13 | 32911841 | 32912085 | 0.384 | BRCA2 | chr3 | 10128659 | 10128873 | 0.414 | FANCD2 |
| chr13 | 32912080 | 32912319 | 0.342 | BRCA2 | chr3 | 10130055 | 10130255 | 0.448 | FANCD2 |
| chr13 | 32912267 | 32912511 | 0.265 | BRCA2 | chr3 | 10130418 | 10130618 | 0.398 | FANCD2 |
| chr13 | 32912502 | 32912746 | 0.331 | BRCA2 | chr3 | 10131860 | 10132052 | 0.461 | FANCD2 |
| chr13 | 32912749 | 32912986 | 0.307 | BRCA2 | chr3 | 10133776 | 10133976 | 0.418 | FANCD2 |
| chr13 | 32912979 | 32913218 | 0.404 | BRCA2 | chr3 | 10134833 | 10135033 | 0.448 | FANCD2 |
| chr13 | 32913217 | 32913460 | 0.336 | BRCA2 | chr3 | 10135971 | 10136220 | 0.484 | FANCD2 |
| chr13 | 32913444 | 32913691 | 0.323 | BRCA2 | chr3 | 10136796 | 10137016 | 0.425 | FANCD2 |
| chr13 | 32913682 | 32913927 | 0.321 | BRCA2 | chr3 | 10137928 | 10138128 | 0.398 | FANCD2 |

(continued)

| Chr. | Start | Stop | GC | Gene | Chr. | Start | Stop | GC | Gene |
|---|---|---|---|---|---|---|---|---|---|
| chr13 | 32913944 | 32914192 | 0.329 | BRCA2 | chr3 | 10140403 | 10140603 | 0.448 | FANCD2 |
| chr13 | 32914208 | 32914455 | 0.347 | BRCA2 | chr3 | 10140685 | 10140895 | 0.322 | FANCD2 |
| chr13 | 32914462 | 32914709 | 0.343 | BRCA2 | chr3 | 10183302 | 10183451 | 0.653 | VHL |
| chr13 | 32914691 | 32914936 | 0.329 | BRCA2 | chr3 | 10183681 | 10183874 | 0.706 | VHL |
| chr13 | 32914776 | 32915021 | 0.333 | BRCA2 | chr3 | 10188234 | 10188438 | 0.390 | VHL |
| chr13 | 32914895 | 32915115 | 0.326 | BRCA2 | chr3 | 10191445 | 10191700 | 0.484 | VHL |
| chr13 | 32914896 | 32915115 | 0.327 | BRCA2 | chr3 | 10191721 | 10191932 | 0.392 | VHL |
| chr13 | 32914906 | 32915155 | 0.328 | BRCA2 | chr3 | 10192245 | 10192450 | 0.350 | VHL |
| chr13 | 32915087 | 32915334 | 0.355 | BRCA2 | chr3 | 12645599 | 12645838 | 0.504 | RAF1 |
| chr13 | 32915144 | 32915384 | 0.357 | BRCA2 | chr3 | 12645599 | 12645843 | 0.502 | RAF1 |
| chr13 | 32918540 | 32918787 | 0.258 | BRCA2 | chr3 | 12645599 | 12645844 | 0.500 | RAF1 |
| chr13 | 32920834 | 32921033 | 0.295 | BRCA2 | chr3 | 12645603 | 12645844 | 0.504 | RAF1 |
| chr13 | 32928970 | 32929189 | 0.345 | BRCA2 | chr3 | 14186692 | 14186898 | 0.319 | XPC |
| chr13 | 32928972 | 32929201 | 0.357 | BRCA2 | chr3 | 14186863 | 14187104 | 0.521 | XPC |
| chr13 | 32928992 | 32929236 | 0.351 | BRCA2 | chr3 | 14187085 | 14187334 | 0.532 | XPC |
| chr13 | 32928996 | 32929196 | 0.358 | BRCA2 | chr3 | 14187312 | 14187552 | 0.589 | XPC |
| chr13 | 32928996 | 32929208 | 0.357 | BRCA2 | chr3 | 14187523 | 14187722 | 0.570 | XPC |
| chr13 | 32929176 | 32929423 | 0.339 | BRCA2 | chr3 | 14188679 | 14188879 | 0.552 | XPC |
| chr13 | 32929177 | 32929426 | 0.344 | BRCA2 | chr3 | 14189299 | 14189509 | 0.583 | XPC |
| chr13 | 32929220 | 32929467 | 0.323 | BRCA2 | chr3 | 14189991 | 14190191 | 0.597 | XPC |
| chr13 | 32929274 | 32929479 | 0.335 | BRCA2 | chr3 | 14190286 | 14190496 | 0.578 | XPC |
| chr13 | 32929297 | 32929498 | 0.322 | BRCA2 | chr3 | 14193741 | 14194025 | 0.596 | XPC |
| chr13 | 32930589 | 32930789 | 0.448 | BRCA2 | chr3 | 14197850 | 14198050 | 0.483 | XPC |
| chr13 | 32931650 | 32931879 | 0.257 | BRCA2 | chr3 | 14199573 | 14199852 | 0.543 | XPC |
| chr13 | 32931817 | 32932017 | 0.318 | BRCA2 | chr3 | 14199862 | 14200062 | 0.582 | XPC |
| chr13 | 32932034 | 32932234 | 0.313 | BRCA2 | chr3 | 14200146 | 14200394 | 0.522 | XPC |
| chr13 | 32936641 | 32936885 | 0.384 | BRCA2 | chr3 | 14206322 | 14206487 | 0.434 | XPC |
| chr13 | 32937319 | 32937563 | 0.384 | BRCA2 | chr3 | 14206933 | 14207089 | 0.522 | XPC |
| chr13 | 32937529 | 32937773 | 0.376 | BRCA2 | chr3 | 14208699 | 14208911 | 0.474 | XPC |
| chr13 | 32944444 | 32944688 | 0.359 | BRCA2 | chr3 | 14209663 | 14209863 | 0.517 | XPC |
| chr13 | 32945080 | 32945249 | 0.359 | BRCA2 | chr3 | 14211839 | 14212049 | 0.384 | XPC |
| chr13 | 32950820 | 32951019 | 0.440 | BRCA2 | chr3 | 14214355 | 14214600 | 0.476 | XPC |
| chr13 | 32953333 | 32953533 | 0.348 | BRCA2 | chr3 | 14220004 | 14220204 | 0.682 | XPC |
| chr13 | 32953442 | 32953686 | 0.363 | BRCA2 | chr3 | 37034589 | 37034809 | 0.570 | EPM2AIP1 |
| chr13 | 32953840 | 32954084 | 0.327 | BRCA2 | chr3 | 37034790 | 37035063 | 0.544 | EPM2AIP1 |
| chr13 | 32954054 | 32954299 | 0.346 | BRCA2 | chr3 | 37035069 | 37035306 | 0.622 | MLH1 |
| chr13 | 32954054 | 32954300 | 0.344 | BRCA2 | chr3 | 37038000 | 37038200 | 0.368 | MLH1 |
| chr13 | 32968741 | 32968971 | 0.359 | BRCA2 | chr3 | 37042434 | 37042645 | 0.354 | MLH1 |
| chr13 | 32968820 | 32969069 | 0.384 | BRCA2 | chr3 | 37045773 | 37045973 | 0.418 | MLH1 |
| chr13 | 32970989 | 32971236 | 0.379 | BRCA2 | chr3 | 37048411 | 37048645 | 0.353 | MLH1 |
| chr13 | 32971106 | 32971335 | 0.348 | BRCA2 | chr3 | 37050230 | 37050436 | 0.377 | MLH1 |
| chr13 | 32972257 | 32972489 | 0.373 | BRCA2 | chr3 | 37053207 | 37053427 | 0.335 | MLH1 |
| chr13 | 32972463 | 32972703 | 0.390 | BRCA2 | chr3 | 37053528 | 37053730 | 0.340 | MLH1 |
| chr13 | 32972600 | 32972845 | 0.378 | BRCA2 | chr3 | 37055893 | 37056093 | 0.373 | MLH1 |
| chr13 | 32972664 | 32972864 | 0.418 | BRCA2 | chr3 | 37058865 | 37059114 | 0.424 | MLH1 |
| chr13 | 32972671 | 32972922 | 0.397 | BRCA2 | chr3 | 37061804 | 37062039 | 0.542 | MLH1 |
| chr13 | 32972708 | 32972954 | 0.389 | BRCA2 | chr3 | 37067171 | 37067392 | 0.500 | MLH1 |
| chr13 | 32973392 | 32973641 | 0.336 | BRCA2 | chr3 | 37067236 | 37067492 | 0.514 | MLH1 |
| chr13 | 32973613 | 32973807 | 0.267 | BRCA2 | chr3 | 37070194 | 37070394 | 0.408 | MLH1 |
| chr13 | 48916668 | 48916868 | 0.318 | RB1 | chr3 | 37070355 | 37070605 | 0.462 | MLH1 |

(continued)

| Chr. | Start | Stop | GC | Gene | Chr. | Start | Stop | GC | Gene |
|------|-------|------|-----|------|------|-------|------|-----|------|
| chr13 | 48919151 | 48919351 | 0.284 | RB1 | chr3 | 37081625 | 37081777 | 0.438 | MLH1 |
| chr13 | 48921945 | 48922145 | 0.308 | RB1 | chr3 | 37083681 | 37083889 | 0.354 | MLH1 |
| chr13 | 48923001 | 48923221 | 0.258 | RB1 | chr3 | 37088877 | 37089113 | 0.468 | MLH1 |
| chr13 | 48936897 | 48937117 | 0.330 | RB1 | chr3 | 37088933 | 37089152 | 0.473 | MLH1 |
| chr13 | 48939068 | 48939268 | 0.318 | RB1 | chr3 | 37089899 | 37090099 | 0.468 | MLH1 |
| chr13 | 48941560 | 48941760 | 0.318 | RB1 | chr3 | 37090327 | 37090527 | 0.428 | MLH1 |
| chr13 | 48947429 | 48947629 | 0.323 | RB1 | chr3 | 37091892 | 37092087 | 0.480 | MLH1 |
| chr13 | 48951017 | 48951237 | 0.348 | RB1 | chr3 | 37091894 | 37092142 | 0.466 | MLH1 |
| chr13 | 48954364 | 48954564 | 0.328 | RB1 | chr3 | 41265974 | 41266223 | 0.464 | CTNNB1 |
| chr13 | 48955326 | 48955526 | 0.303 | RB1 | chr3 | 41266013 | 41266252 | 0.488 | CTNNB1 |
| chr13 | 48955536 | 48955736 | 0.338 | RB1 | chr3 | 41266021 | 41266268 | 0.476 | CTNNB1 |
| chr13 | 49027033 | 49027233 | 0.333 | RB1 | chr3 | 41266036 | 41266265 | 0.474 | CTNNB1 |
| chr13 | 49030212 | 49030472 | 0.364 | RB1 | chr3 | 52435062 | 52435320 | 0.583 | BAP1 |
| chr13 | 49033792 | 49033992 | 0.428 | RB1 | chr3 | 52435350 | 52435631 | 0.543 | BAP1 |
| chr13 | 49037854 | 49038084 | 0.299 | RB1 | chr3 | 52435934 | 52436211 | 0.561 | BAP1 |
| chr13 | 49039190 | 49039390 | 0.378 | RB1 | chr3 | 52436217 | 52436465 | 0.647 | BAP1 |
| chr13 | 49039267 | 49039467 | 0.383 | RB1 | chr3 | 52436552 | 52436790 | 0.552 | BAP1 |
| chr13 | 49047356 | 49047616 | 0.287 | RB1 | chr3 | 52436807 | 52437063 | 0.572 | BAP1 |
| chr13 | 49050783 | 49050983 | 0.373 | RB1 | chr3 | 52437127 | 52437370 | 0.574 | BAP1 |
| chr13 | 49051484 | 49051724 | 0.299 | RB1 | chr3 | 52437447 | 52437693 | 0.599 | BAP1 |
| chr13 | 49054133 | 49054333 | 0.413 | RB1 | chr3 | 52437754 | 52437978 | 0.556 | BAP1 |
| chr13 | 49054700 | 49054910 | 0.251 | RB1 | chr3 | 52438443 | 52438682 | 0.575 | BAP1 |
| chr13 | 49055078 | 49055278 | 0.323 | RB1 | chr3 | 52439130 | 52439357 | 0.583 | BAP1 |
| chr13 | 49055456 | 49055656 | 0.343 | RB1 | chr3 | 52439752 | 52440014 | 0.544 | BAP1 |
| chr13 | 49055834 | 49056034 | 0.254 | RB1 | chr3 | 52440273 | 52440505 | 0.618 | BAP1 |
| chr13 | 103498123 | 103498440 | 0.629 | ERCC5 | chr3 | 52440695 | 52440946 | 0.552 | BAP1 |
| chr13 | 103498155 | 103498432 | 0.640 | ERCC5 | chr3 | 52441181 | 52441475 | 0.576 | BAP1 |
| chr13 | 103498190 | 103498390 | 0.642 | ERCC5 | chr3 | 52441983 | 52442230 | 0.532 | BAP1 |
| chr13 | 103498192 | 103498440 | 0.631 | ERCC5 | chr3 | 52442460 | 52442693 | 0.500 | BAP1 |
| chr13 | 103498192 | 103498453 | 0.626 | ERCC5 | chr3 | 52443505 | 52443740 | 0.602 | BAP1 |
| chr13 | 103498470 | 103498701 | 0.608 | ERCC5 | chr3 | 138374171 | 138374400 | 0.400 | PIK3CB |
| chr13 | 103498494 | 103498713 | 0.618 | ERCC5 | chr3 | 138374183 | 138374428 | 0.386 | PIK3CB |
| chr13 | 103498494 | 103498717 | 0.621 | ERCC5 | chr3 | 138374197 | 138374427 | 0.398 | PIK3CB |
| chr13 | 103498574 | 103498757 | 0.641 | ERCC5 | chr3 | 138374204 | 138374443 | 0.408 | PIK3CB |
| chr13 | 103504312 | 103504529 | 0.349 | ERCC5 | chr3 | 138409848 | 138410087 | 0.375 | PIK3CB |
| chr13 | 103504364 | 103504578 | 0.372 | ERCC5 | chr3 | 138409848 | 138410097 | 0.380 | PIK3CB |
| chr13 | 103506028 | 103506230 | 0.409 | ERCC5 | chr3 | 138409872 | 138410116 | 0.380 | PIK3CB |
| chr13 | 103506526 | 103506726 | 0.463 | ERCC5 | chr3 | 138409872 | 138410118 | 0.385 | PIK3CB |
| chr13 | 103508374 | 103508544 | 0.275 | ERCC5 | chr3 | 138417690 | 138417920 | 0.390 | PIK3CB |
| chr13 | 103510603 | 103510803 | 0.393 | ERCC5 | chr3 | 138417690 | 138417924 | 0.383 | PIK3CB |
| chr13 | 103513836 | 103514056 | 0.407 | ERCC5 | chr3 | 138417696 | 138417915 | 0.391 | PIK3CB |
| chr13 | 103514321 | 103514572 | 0.433 | ERCC5 | chr3 | 138417697 | 138417916 | 0.395 | PIK3CB |
| chr13 | 103514574 | 103514792 | 0.534 | ERCC5 | chr3 | 138665132 | 138665361 | 0.600 | FOXL2 |
| chr13 | 103514801 | 103515020 | 0.482 | ERCC5 | chr3 | 138665147 | 138665396 | 0.600 | FOXL2 |
| chr13 | 103515021 | 103515221 | 0.428 | ERCC5 | chr3 | 138665151 | 138665396 | 0.602 | FOXL2 |
| chr13 | 103515235 | 103515435 | 0.433 | ERCC5 | chr3 | 138665170 | 138665409 | 0.613 | FOXL2 |
| chr13 | 103517984 | 103518184 | 0.478 | ERCC5 | chr3 | 178916725 | 178916970 | 0.362 | PIK3CA |
| chr13 | 103518194 | 103518404 | 0.412 | ERCC5 | chr3 | 178916766 | 178917010 | 0.351 | PIK3CA |
| chr13 | 103518516 | 103518726 | 0.370 | ERCC5 | chr3 | 178916782 | 178917028 | 0.356 | PIK3CA |
| chr13 | 103519057 | 103519257 | 0.368 | ERCC5 | chr3 | 178916822 | 178917068 | 0.336 | PIK3CA |

(continued)

| Chr. | Start | Stop | GC | Gene | Chr. | Start | Stop | GC | Gene |
|------|-------|------|-----|------|------|-------|------|-----|------|
| chr13 | 103520541 | 103520748 | 0.370 | ERCC5 | chr3 | 178917417 | 178917566 | 0.320 | PIK3CA |
| chr13 | 103524493 | 103524693 | 0.413 | ERCC5 | chr3 | 178917417 | 178917618 | 0.347 | PIK3CA |
| chr13 | 103524585 | 103524802 | 0.445 | ERCC5 | chr3 | 178917420 | 178917590 | 0.345 | PIK3CA |
| chr13 | 103525496 | 103525696 | 0.368 | ERCC5 | chr3 | 178917420 | 178917604 | 0.351 | PIK3CA |
| chr13 | 103527656 | 103527856 | 0.408 | ERCC5 | chr3 | 178921331 | 178921578 | 0.367 | PIK3CA |
| chr13 | 103527863 | 103528063 | 0.408 | ERCC5 | chr3 | 178921339 | 178921585 | 0.372 | PIK3CA |
| chr13 | 103528070 | 103528300 | 0.437 | ERCC5 | chr3 | 178921347 | 178921596 | 0.368 | PIK3CA |
| chr14 | 38060515 | 38060750 | 0.589 | FOXA1 | chr3 | 178921364 | 178921608 | 0.351 | PIK3CA |
| chr14 | 38060564 | 38060810 | 0.591 | FOXA1 | chr3 | 178927888 | 178928114 | 0.339 | PIK3CA |
| chr14 | 38060574 | 38060818 | 0.592 | FOXA1 | chr3 | 178927898 | 178928114 | 0.346 | PIK3CA |
| chr14 | 38060586 | 38060833 | 0.601 | FOXA1 | chr3 | 178927910 | 178928144 | 0.323 | PIK3CA |
| chr14 | 45605103 | 45605333 | 0.558 | FANCM | chr3 | 178927974 | 178928182 | 0.354 | PIK3CA |
| chr14 | 45605305 | 45605553 | 0.610 | FANCM | chr3 | 178935873 | 178936102 | 0.313 | PIK3CA |
| chr14 | 45605573 | 45605799 | 0.511 | FANCM | chr3 | 178935930 | 178936133 | 0.353 | PIK3CA |
| chr14 | 45606233 | 45606433 | 0.398 | FANCM | chr3 | 178935944 | 178936197 | 0.335 | PIK3CA |
| chr14 | 45618024 | 45618224 | 0.363 | FANCM | chr3 | 178935995 | 178936197 | 0.365 | PIK3CA |
| chr14 | 45620541 | 45620741 | 0.333 | FANCM | chr3 | 178936030 | 178936279 | 0.320 | PIK3CA |
| chr14 | 45623034 | 45623234 | 0.348 | FANCM | chr3 | 178951914 | 178952159 | 0.382 | PIK3CA |
| chr14 | 45628297 | 45628497 | 0.408 | FANCM | chr3 | 178951915 | 178952140 | 0.385 | PIK3CA |
| chr14 | 45633510 | 45633710 | 0.373 | FANCM | chr3 | 178951921 | 178952140 | 0.386 | PIK3CA |
| chr14 | 45633720 | 45633920 | 0.383 | FANCM | chr3 | 178951921 | 178952159 | 0.381 | PIK3CA |
| chr14 | 45636166 | 45636386 | 0.376 | FANCM | chr3 | 178951948 | 178952109 | 0.389 | PIK3CA |
| chr14 | 45639788 | 45640013 | 0.341 | FANCM | chr3 | 178952004 | 178952152 | 0.389 | PIK3CA |
| chr14 | 45642242 | 45642442 | 0.403 | FANCM | chr3 | 178952010 | 178952109 | 0.390 | PIK3CA |
| chr14 | 45644281 | 45644486 | 0.340 | FANCM | chr3 | 178952109 | 178952152 | 0.386 | PIK3CA |
| chr14 | 45644653 | 45644863 | 0.313 | FANCM | chr4 | 55151865 | 55152127 | 0.513 | PDGFRA |
| chr14 | 45644862 | 45645099 | 0.382 | FANCM | chr4 | 55593487 | 55593733 | 0.389 | KIT |
| chr14 | 45645143 | 45645342 | 0.350 | FANCM | chr4 | 55593505 | 55593749 | 0.400 | KIT |
| chr14 | 45645413 | 45645613 | 0.378 | FANCM | chr4 | 55593514 | 55593765 | 0.397 | KIT |
| chr14 | 45645666 | 45645955 | 0.331 | FANCM | chr4 | 55593530 | 55593778 | 0.390 | KIT |
| chr14 | 45645983 | 45646183 | 0.303 | FANCM | chr4 | 55594116 | 55594365 | 0.416 | KIT |
| chr14 | 45650718 | 45650970 | 0.332 | FANCM | chr4 | 55594125 | 55594365 | 0.411 | KIT |
| chr14 | 45650772 | 45650971 | 0.320 | FANCM | chr4 | 55594139 | 55594380 | 0.401 | KIT |
| chr14 | 45652886 | 45653086 | 0.338 | FANCM | chr4 | 55594139 | 55594385 | 0.401 | KIT |
| chr14 | 45654389 | 45654589 | 0.303 | FANCM | chr4 | 55599168 | 55599408 | 0.365 | KIT |
| chr14 | 45656912 | 45657134 | 0.269 | FANCM | chr4 | 55599173 | 55599417 | 0.367 | KIT |
| chr14 | 45658004 | 45658204 | 0.333 | FANCM | chr4 | 55599207 | 55599435 | 0.384 | KIT |
| chr14 | 45658183 | 45658427 | 0.371 | FANCM | chr4 | 55602645 | 55602892 | 0.423 | KIT |
| chr14 | 45658417 | 45658644 | 0.368 | FANCM | chr4 | 55602657 | 55602888 | 0.422 | KIT |
| chr14 | 45665608 | 45665811 | 0.348 | FANCM | chr4 | 55602657 | 55602892 | 0.424 | KIT |
| chr14 | 45667934 | 45668145 | 0.358 | FANCM | chr4 | 153244069 | 153244316 | 0.464 | FBXW7 |
| chr14 | 45668983 | 45669204 | 0.320 | FANCM | chr4 | 153244075 | 153244315 | 0.465 | FBXW7 |
| chr14 | 45669474 | 45669678 | 0.346 | FANCM | chr4 | 153244085 | 153244324 | 0.454 | FBXW7 |
| chr14 | 95557315 | 95557515 | 0.313 | DICER1 | chr4 | 153244106 | 153244353 | 0.427 | FBXW7 |
| chr14 | 95557526 | 95557726 | 0.463 | DICER1 | chr4 | 153245291 | 153245531 | 0.398 | FBXW7 |
| chr14 | 95559982 | 95560182 | 0.542 | DICER1 | chr4 | 153245297 | 153245541 | 0.400 | FBXW7 |
| chr14 | 95560204 | 95560434 | 0.494 | DICER1 | chr4 | 153245312 | 153245560 | 0.406 | FBXW7 |
| chr14 | 95560444 | 95560644 | 0.398 | DICER1 | chr4 | 153245328 | 153245572 | 0.396 | FBXW7 |
| chr14 | 95562161 | 95562361 | 0.368 | DICER1 | chr4 | 153247109 | 153247353 | 0.433 | FBXW7 |
| chr14 | 95562361 | 95562592 | 0.530 | DICER1 | chr4 | 153247122 | 153247369 | 0.448 | FBXW7 |

(continued)

| Chr. | Start | Stop | GC | Gene | Chr. | Start | Stop | GC | Gene |
|------|-------|------|-----|------|------|-------|------|-----|------|
| chr14 | 95562601 | 95562801 | 0.438 | DICER1 | chr4 | 153247150 | 153247399 | 0.456 | FBXW7 |
| chr14 | 95562666 | 95562891 | 0.398 | DICER1 | chr4 | 153249264 | 153249512 | 0.414 | FBXW7 |
| chr14 | 95566129 | 95566335 | 0.406 | DICER1 | chr4 | 153249285 | 153249525 | 0.419 | FBXW7 |
| chr14 | 95569682 | 95569882 | 0.468 | DICER1 | chr4 | 153249290 | 153249529 | 0.417 | FBXW7 |
| chr14 | 95569994 | 95570194 | 0.403 | DICER1 | chr4 | 153249303 | 153249551 | 0.414 | FBXW7 |
| chr14 | 95570228 | 95570428 | 0.418 | DICER1 | chr4 | 153250763 | 153251006 | 0.340 | FBXW7 |
| chr14 | 95571340 | 95571540 | 0.488 | DICER1 | chr4 | 153250791 | 153251017 | 0.348 | FBXW7 |
| chr14 | 95571974 | 95572174 | 0.348 | DICER1 | chr4 | 153250791 | 153251031 | 0.349 | FBXW7 |
| chr14 | 95572314 | 95572514 | 0.428 | DICER1 | chr4 | 153250808 | 153251047 | 0.342 | FBXW7 |
| chr14 | 95572524 | 95572764 | 0.249 | DICER1 | chr4 | 153251774 | 153252018 | 0.380 | FBXW7 |
| chr14 | 95573959 | 95574159 | 0.338 | DICER1 | chr4 | 153251789 | 153252028 | 0.388 | FBXW7 |
| chr14 | 95574168 | 95574368 | 0.348 | DICER1 | chr4 | 153251792 | 153252040 | 0.382 | FBXW7 |
| chr14 | 95574657 | 95574857 | 0.443 | DICER1 | chr4 | 153251832 | 153252071 | 0.379 | FBXW7 |
| chr14 | 95577599 | 95577799 | 0.433 | DICER1 | chr4 | 153258843 | 153259092 | 0.356 | FBXW7 |
| chr14 | 95578391 | 95578591 | 0.358 | DICER1 | chr4 | 153258850 | 153259097 | 0.355 | FBXW7 |
| chr14 | 95579340 | 95579540 | 0.358 | DICER1 | chr4 | 153258888 | 153259137 | 0.344 | FBXW7 |
| chr14 | 95581926 | 95582126 | 0.433 | DICER1 | chr4 | 153258953 | 153259199 | 0.368 | FBXW7 |
| chr14 | 95582756 | 95582956 | 0.358 | DICER1 | chr4 | 153268077 | 153268219 | 0.517 | FBXW7 |
| chr14 | 95582966 | 95583166 | 0.348 | DICER1 | chr4 | 153268078 | 153268219 | 0.514 | FBXW7 |
| chr14 | 95583900 | 95584100 | 0.378 | DICER1 | chr4 | 153268079 | 153268219 | 0.518 | FBXW7 |
| chr14 | 95590532 | 95590732 | 0.358 | DICER1 | chr5 | 56161075 | 56161306 | 0.328 | MAP3K1 |
| chr14 | 95590767 | 95590967 | 0.393 | DICER1 | chr5 | 56161096 | 56161325 | 0.330 | MAP3K1 |
| chr14 | 95592822 | 95593064 | 0.305 | DICER1 | chr5 | 56161113 | 56161321 | 0.349 | MAP3K1 |
| chr14 | 95595734 | 95595934 | 0.358 | DICER1 | chr5 | 56161178 | 56161386 | 0.321 | MAP3K1 |
| chr14 | 95596337 | 95596537 | 0.343 | DICER1 | chr5 | 56161545 | 56161787 | 0.346 | MAP3K1 |
| chr14 | 95598778 | 95598978 | 0.383 | DICER1 | chr5 | 56161548 | 56161797 | 0.348 | MAP3K1 |
| chr14 | 95598988 | 95599188 | 0.308 | DICER1 | chr5 | 56161563 | 56161807 | 0.355 | MAP3K1 |
| chr14 | 95599591 | 95599801 | 0.436 | DICER1 | chr5 | 56161577 | 56161806 | 0.365 | MAP3K1 |
| chr14 | 95623722 | 95623973 | 0.750 | DICER1 | chr5 | 56180437 | 56180676 | 0.363 | MAP3K1 |
| chr14 | 105246388 | 105246637 | 0.596 | AKT1 | chr5 | 56180457 | 56180659 | 0.389 | MAP3K1 |
| chr14 | 105246483 | 105246730 | 0.617 | AKT1 | chr5 | 56180502 | 56180707 | 0.369 | MAP3K1 |
| chr14 | 105246501 | 105246707 | 0.633 | AKT1 | chr5 | 56181614 | 56181861 | 0.335 | MAP3K1 |
| chr14 | 105246501 | 105246745 | 0.633 | AKT1 | chr5 | 56181627 | 56181876 | 0.332 | MAP3K1 |
| chr15 | 32968921 | 32969121 | 0.303 | GREM1 | chr5 | 56181632 | 56181871 | 0.338 | MAP3K1 |
| chr15 | 32976883 | 32977083 | 0.393 | GREM1 | chr5 | 56181648 | 56181887 | 0.346 | MAP3K1 |
| chr15 | 32984845 | 32985055 | 0.469 | GREM1 | chr5 | 56183135 | 56183374 | 0.429 | MAP3K1 |
| chr15 | 32988826 | 32989036 | 0.370 | SCG5 | chr5 | 56183138 | 56183347 | 0.429 | MAP3K1 |
| chr15 | 33000769 | 33000969 | 0.512 | GREM1 | chr5 | 56183138 | 56183361 | 0.429 | MAP3K1 |
| chr15 | 33022952 | 33023162 | 0.654 | GREM1 | chr5 | 56183176 | 56183375 | 0.455 | MAP3K1 |
| chr15 | 33022952 | 33023205 | 0.638 | GREM1 | chr5 | 112043137 | 112043364 | 0.649 | APC |
| chr15 | 33023018 | 33023279 | 0.626 | GREM1 | chr5 | 112043137 | 112043365 | 0.651 | APC |
| chr15 | 33023148 | 33023435 | 0.521 | GREM1 | chr5 | 112043186 | 112043431 | 0.663 | APC |
| chr15 | 33023151 | 33023435 | 0.519 | GREM1 | chr5 | 112043190 | 112043431 | 0.661 | APC |
| chr15 | 33023205 | 33023450 | 0.504 | GREM1 | chr5 | 112043201 | 112043471 | 0.672 | APC |
| chr15 | 33023686 | 33023886 | 0.517 | GREM1 | chr5 | 112043206 | 112043428 | 0.668 | APC |
| chr15 | 33024084 | 33024294 | 0.464 | GREM1 | chr5 | 112073451 | 112073699 | 0.639 | APC |
| chr15 | 33024482 | 33024682 | 0.398 | GREM1 | chr5 | 112073948 | 112074148 | 0.488 | APC |
| chr15 | 33026472 | 33026672 | 0.299 | GREM1 | chr5 | 112090491 | 112090691 | 0.373 | APC |
| chr15 | 66727327 | 66727566 | 0.546 | MAP2K1 | chr5 | 112102006 | 112102208 | 0.330 | APC |
| chr15 | 66727339 | 66727587 | 0.550 | MAP2K1 | chr5 | 112102893 | 112103093 | 0.433 | APC |

(continued)

| Chr. | Start | Stop | GC | Gene | Chr. | Start | Stop | GC | Gene |
|---|---|---|---|---|---|---|---|---|---|
| chr15 | 66727339 | 66727588 | 0.548 | MAP2K1 | chr5 | 112111368 | 112111578 | 0.289 | APC |
| chr15 | 66727359 | 66727598 | 0.542 | MAP2K1 | chr5 | 112116375 | 112116639 | 0.340 | APC |
| chr15 | 66729024 | 66729277 | 0.496 | MAP2K1 | chr5 | 112128134 | 112128341 | 0.332 | APC |
| chr15 | 66729065 | 66729264 | 0.515 | MAP2K1 | chr5 | 112128152 | 112128420 | 0.309 | APC |
| chr15 | 66774016 | 66774220 | 0.527 | MAP2K1 | chr5 | 112136780 | 112137014 | 0.404 | APC |
| chr15 | 66774016 | 66774260 | 0.510 | MAP2K1 | chr5 | 112151086 | 112151286 | 0.368 | APC |
| chr15 | 66774048 | 66774260 | 0.516 | MAP2K1 | chr5 | 112154610 | 112154859 | 0.424 | APC |
| chr15 | 66774052 | 66774260 | 0.517 | MAP2K1 | chr5 | 112154826 | 112155071 | 0.504 | APC |
| chr15 | 66777305 | 66777541 | 0.591 | MAP2K1 | chr5 | 112157485 | 112157695 | 0.341 | APC |
| chr15 | 66777336 | 66777568 | 0.597 | MAP2K1 | chr5 | 112162781 | 112162991 | 0.379 | APC |
| chr15 | 66777336 | 66777570 | 0.600 | MAP2K1 | chr5 | 112163441 | 112163655 | 0.288 | APC |
| chr15 | 66777338 | 66777568 | 0.597 | MAP2K1 | chr5 | 112163452 | 112163681 | 0.309 | APC |
| chr15 | 89787224 | 89787484 | 0.651 | FANCI | chr5 | 112164471 | 112164655 | 0.319 | APC |
| chr15 | 89790756 | 89790956 | 0.368 | FANCI | chr5 | 112270600 | 112170800 | 0.393 | APC |
| chr15 | 89801818 | 89802048 | 0.403 | FANCI | chr5 | 112170810 | 112171010 | 0.289 | APC |
| chr15 | 89803854 | 89804054 | 0.403 | FANCI | chr5 | 112173249 | 112173449 | 0.383 | APC |
| chr15 | 89804662 | 89804884 | 0.372 | FANCI | chr5 | 112173471 | 112173712 | 0.405 | APC |
| chr15 | 89804949 | 89805159 | 0.365 | FANCI | chr5 | 112173688 | 112173932 | 0.404 | APC |
| chr15 | 89806635 | 89806874 | 0.371 | FANCI | chr5 | 112173840 | 112174071 | 0.466 | APC |
| chr15 | 89807040 | 89807240 | 0.403 | FANCI | chr5 | 112174070 | 112174313 | 0.361 | APC |
| chr15 | 89807671 | 89807891 | 0.344 | FANCI | chr5 | 112174297 | 112174537 | 0.353 | APC |
| chr15 | 89811673 | 89811922 | 0.364 | FANCI | chr5 | 112174544 | 112174783 | 0.392 | APC |
| chr15 | 89817473 | 89817702 | 0.370 | FANCI | chr5 | 112174788 | 112175027 | 0.400 | APC |
| chr15 | 89819937 | 89820137 | 0.418 | FANCI | chr5 | 112174985 | 112175185 | 0.378 | APC |
| chr15 | 89821824 | 89822080 | 0.358 | FANCI | chr5 | 112175164 | 112175405 | 0.459 | APC |
| chr15 | 89824311 | 89824511 | 0.333 | FANCI | chr5 | 112175413 | 112175652 | 0.471 | APC |
| chr15 | 89824830 | 89825066 | 0.359 | FANCI | chr5 | 112175519 | 112175768 | 0.460 | APC |
| chr15 | 89828191 | 89828429 | 0.439 | FANCI | chr5 | 112175523 | 112175780 | 0.461 | APC |
| chr15 | 89833373 | 89833592 | 0.368 | FANCI | chr5 | 112175853 | 112176053 | 0.333 | APC |
| chr15 | 89834739 | 89834949 | 0.365 | FANCI | chr5 | 112175867 | 112176116 | 0.380 | APC |
| chr15 | 89835684 | 89835936 | 0.336 | FANCI | chr5 | 112175900 | 112176040 | 0.326 | APC |
| chr15 | 89836099 | 89836321 | 0.327 | FANCI | chr5 | 112176145 | 112176391 | 0.449 | APC |
| chr15 | 89837021 | 89837221 | 0.358 | FANCI | chr5 | 112176384 | 112176633 | 0.412 | APC |
| chr15 | 89838207 | 89838356 | 0.480 | FANCI | chr5 | 112176631 | 112176875 | 0.322 | APC |
| chr15 | 89843116 | 89843345 | 0.417 | FANCI | chr5 | 112176860 | 112177104 | 0.396 | APC |
| chr15 | 89843449 | 89843649 | 0.403 | FANCI | chr5 | 112176995 | 112177234 | 0.400 | APC |
| chr15 | 89844490 | 89844700 | 0.455 | FANCI | chr5 | 112177404 | 112177650 | 0.385 | APC |
| chr15 | 89846965 | 89847165 | 0.388 | FANCI | chr5 | 112177589 | 112177789 | 0.408 | APC |
| chr15 | 89848390 | 89848629 | 0.479 | FANCI | chr5 | 112177796 | 112178080 | 0.389 | APC |
| chr15 | 89848727 | 89848927 | 0.478 | FANCI | chr5 | 112178130 | 112178379 | 0.456 | APC |
| chr15 | 89849176 | 89849396 | 0.439 | FANCI | chr5 | 112178293 | 112178573 | 0.391 | APC |
| chr15 | 89850578 | 89850778 | 0.403 | FANCI | chr5 | 112178510 | 112178755 | 0.407 | APC |
| chr15 | 89850795 | 89850995 | 0.438 | FANCI | chr5 | 112178763 | 112179036 | 0.449 | APC |
| chr15 | 89857735 | 89857935 | 0.398 | FANCI | chr5 | 112179030 | 112179324 | 0.380 | APC |
| chr15 | 89858460 | 89858694 | 0.498 | FANCI | chr5 | 112179325 | 112179525 | 0.423 | APC |
| chr15 | 89859420 | 89859663 | 0.430 | FANCI | chr5 | 112179582 | 112179827 | 0.472 | APC |
| chr15 | 89859773 | 89859973 | 0.517 | FANCI | chr5 | 112180193 | 112180393 | 0.338 | APC |
| chr15 | 89859992 | 89860192 | 0.443 | FANCI | chr5 | 112181061 | 112181261 | 0.398 | APC |
| chr15 | 89860258 | 89860477 | 0.359 | FANCI | chr5 | 112181929 | 112182129 | 0.313 | APC |
| chr15 | 90631785 | 90632007 | 0.570 | IDH2 | chr5 | 131892606 | 131892857 | 0.718 | RAD50 |

(continued)

| Chr. | Start | Stop | GC | Gene | Chr. | Start | Stop | GC | Gene |
|------|-------|------|-----|------|------|-------|------|-----|------|
| chr15 | 90631814 | 90632043 | 0.548 | IDH2 | chr5 | 131892983 | 131893197 | 0.474 | RAD50 |
| chr15 | 90631843 | 90632054 | 0.519 | IDH2 | chr5 | 131892984 | 131893223 | 0.463 | RAD50 |
| chr16 | 3631168 | 3631388 | 0.443 | SLX4 | chr5 | 131893020 | 131893223 | 0.466 | RAD50 |
| chr16 | 3632424 | 3632665 | 0.645 | SLX4 | chr5 | 131893049 | 131893292 | 0.492 | RAD50 |
| chr16 | 3632693 | 3632893 | 0.597 | SLX4 | chr5 | 131894808 | 131895037 | 0.252 | RAD50 |
| chr16 | 3633083 | 3633283 | 0.582 | SLX4 | chr5 | 131911382 | 131911618 | 0.376 | RAD50 |
| chr16 | 3633339 | 3633565 | 0.590 | SLX4 | chr5 | 131915004 | 131915223 | 0.382 | RAD50 |
| chr16 | 3634696 | 3634896 | 0.502 | SLX4 | chr5 | 131915567 | 131915717 | 0.364 | RAD50 |
| chr16 | 3639002 | 3639202 | 0.617 | SLX4 | chr5 | 131923251 | 131923500 | 0.312 | RAD50 |
| chr16 | 3639197 | 3639423 | 0.652 | SLX4 | chr5 | 131923517 | 131923757 | 0.328 | RAD50 |
| chr16 | 3639505 | 3639754 | 0.632 | SLX4 | chr5 | 131924402 | 131924651 | 0.364 | RAD50 |
| chr16 | 3639692 | 3639932 | 0.639 | SLX4 | chr5 | 131925323 | 131925552 | 0.343 | RAD50 |
| chr16 | 3639911 | 3640159 | 0.530 | SLX4 | chr5 | 131926850 | 131927094 | 0.363 | RAD50 |
| chr16 | 3640185 | 3640409 | 0.498 | SLX4 | chr5 | 131927570 | 131927750 | 0.354 | RAD50 |
| chr16 | 3640478 | 3640721 | 0.607 | SLX4 | chr5 | 131930491 | 131930738 | 0.323 | RAD50 |
| chr16 | 3640760 | 3641047 | 0.660 | SLX4 | chr5 | 131931278 | 131931508 | 0.442 | RAD50 |
| chr16 | 3641072 | 3641272 | 0.468 | SLX4 | chr5 | 131939061 | 131939291 | 0.377 | RAD50 |
| chr16 | 3642628 | 3642858 | 0.597 | SLX4 | chr5 | 131939553 | 131939789 | 0.350 | RAD50 |
| chr16 | 3644537 | 3644773 | 0.608 | SLX4 | chr5 | 131940409 | 131940654 | 0.346 | RAD50 |
| chr16 | 3645495 | 3645705 | 0.673 | SLX4 | chr5 | 131944351 | 131944557 | 0.261 | RAD50 |
| chr16 | 3646003 | 3646244 | 0.698 | SLX4 | chr5 | 131944387 | 131944597 | 0.251 | RAD50 |
| chr16 | 3646031 | 3646244 | 0.720 | SLX4 | chr5 | 131944873 | 131945090 | 0.271 | RAD50 |
| chr16 | 3646124 | 3646405 | 0.699 | SLX4 | chr5 | 131951603 | 131951803 | 0.323 | RAD50 |
| chr16 | 3646149 | 3646391 | 0.712 | SLX4 | chr5 | 131951820 | 131952020 | 0.294 | RAD50 |
| chr16 | 3646157 | 3646403 | 0.704 | SLX4 | chr5 | 131953730 | 131953966 | 0.333 | RAD50 |
| chr16 | 3647312 | 3647583 | 0.610 | SLX4 | chr5 | 131972864 | 131973090 | 0.454 | RAD50 |
| chr16 | 3647601 | 3647800 | 0.540 | SLX4 | chr5 | 131973728 | 131973957 | 0.443 | RAD50 |
| chr16 | 3647864 | 3648074 | 0.578 | SLX4 | chr5 | 131976168 | 131976417 | 0.520 | RAD50 |
| chr16 | 3650971 | 3651251 | 0.530 | SLX4 | chr5 | 131977815 | 131978052 | 0.378 | RAD50 |
| chr16 | 3652058 | 3652258 | 0.557 | SLX4 | chr5 | 131978075 | 131978308 | 0.372 | RAD50 |
| chr16 | 3656390 | 3656622 | 0.506 | SLX4 | chr5 | 131978265 | 131978518 | 0.417 | RAD50 |
| chr16 | 3656642 | 3656842 | 0.443 | SLX4 | chr5 | 131978882 | 131979101 | 0.486 | RAD50 |
| chr16 | 3658480 | 3658725 | 0.528 | SLX4 | chr5 | 170837389 | 170837646 | 0.318 | NPM1 |
| chr16 | 3658510 | 3658671 | 0.562 | SLX4 | chr5 | 170837423 | 170837672 | 0.316 | NPM1 |
| chr16 | 3658784 | 3659014 | 0.455 | SLX4 | chr6 | 35419950 | 35420204 | 0.710 | FANCE |
| chr16 | 3659560 | 3659760 | 0.438 | SLX4 | chr6 | 35420484 | 35420712 | 0.677 | FANCE |
| chr16 | 14013935 | 14014175 | 0.651 | ERCC4 | chr6 | 35423523 | 35423753 | 0.610 | FANCE |
| chr16 | 14013966 | 14014166 | 0.662 | ERCC4 | chr6 | 35423747 | 35423980 | 0.568 | FANCE |
| chr16 | 14013970 | 14014170 | 0.662 | ERCC4 | chr6 | 35423937 | 35424160 | 0.500 | FANCE |
| chr16 | 14013971 | 14014170 | 0.665 | ERCC4 | chr6 | 35425325 | 35425579 | 0.541 | FANCE |
| chr16 | 14015823 | 14016023 | 0.373 | ERCC4 | chr6 | 35425576 | 35425862 | 0.516 | FANCE |
| chr16 | 14016033 | 14016243 | 0.355 | ERCC4 | chr6 | 35425990 | 35426190 | 0.602 | FANCE |
| chr16 | 14020466 | 14020710 | 0.351 | ERCC4 | chr6 | 35427014 | 35427214 | 0.443 | FANCE |
| chr16 | 14021912 | 14022151 | 0.338 | ERCC4 | chr6 | 35427343 | 35427543 | 0.552 | FANCE |
| chr16 | 14024532 | 14024732 | 0.373 | ERCC4 | chr6 | 35428293 | 35428492 | 0.560 | FANCE |
| chr16 | 14025949 | 14026157 | 0.344 | ERCC4 | chr6 | 35433986 | 35434203 | 0.550 | FANCE |
| chr16 | 14027949 | 14028149 | 0.348 | ERCC4 | chr6 | 35434173 | 35434385 | 0.493 | FANCE |
| chr16 | 14028984 | 14029224 | 0.415 | ERCC4 | chr6 | 43544117 | 43544321 | 0.459 | POLH |
| chr16 | 14029445 | 14029685 | 0.452 | ERCC4 | chr6 | 43550014 | 43550163 | 0.420 | POLH |
| chr16 | 14031514 | 14031714 | 0.373 | ERCC4 | chr6 | 43550754 | 43550953 | 0.415 | POLH |

(continued)

| Chr. | Start | Stop | GC | Gene | Chr. | Start | Stop | GC | Gene |
|------|-------|------|-----|------|------|-------|------|-----|------|
| chr16 | 14038575 | 14038734 | 0.494 | ERCC4 | chr6 | 43554978 | 43555223 | 0.476 | POLH |
| chr16 | 14041470 | 14041670 | 0.468 | ERCC4 | chr6 | 43565476 | 43565625 | 0.467 | POLH |
| chr16 | 14041655 | 14041919 | 0.509 | ERCC4 | chr6 | 43568621 | 43568821 | 0.448 | POLH |
| chr16 | 14041900 | 14042126 | 0.498 | ERCC4 | chr6 | 43571533 | 43571733 | 0.423 | POLH |
| chr16 | 14042120 | 14042369 | 0.404 | ERCC4 | chr6 | 43572382 | 43572582 | 0.433 | POLH |
| chr16 | 14042584 | 14042854 | 0.424 | ERCC4 | chr6 | 43572829 | 43573058 | 0.374 | POLH |
| chr16 | 14042844 | 14043078 | 0.336 | ERCC4 | chr6 | 43578220 | 43578420 | 0.507 | POLH |
| chr16 | 14042902 | 14043146 | 0.347 | ERCC4 | chr6 | 43581333 | 43581514 | 0.407 | POLH |
| chr16 | 14043430 | 14043679 | 0.284 | ERCC4 | chr6 | 43581613 | 43581843 | 0.433 | POLH |
| chr16 | 14044214 | 14044436 | 0.332 | ERCC4 | chr6 | 43581852 | 43582085 | 0.517 | POLH |
| chr16 | 14045149 | 14045380 | 0.276 | ERCC4 | chr6 | 43582072 | 43582231 | 0.475 | POLH |
| chr16 | 14045389 | 14045634 | 0.427 | ERCC4 | chr6 | 43587356 | 43587593 | 0.361 | POLH |
| chr16 | 14045727 | 14045927 | 0.328 | ERCC4 | chr6 | 43587578 | 43587778 | 0.393 | POLH |
| chr16 | 14046154 | 14046384 | 0.303 | ERCC4 | chr6 | 43587842 | 43588084 | 0.444 | POLH |
| chr16 | 23614570 | 23614827 | 0.310 | PALB2 | chr6 | 152265305 | 152265551 | 0.555 | ESR1 |
| chr16 | 23614836 | 23615076 | 0.407 | PALB2 | chr6 | 152265322 | 152265569 | 0.556 | ESR1 |
| chr16 | 23619110 | 23619349 | 0.479 | PALB2 | chr6 | 152265335 | 152265582 | 0.548 | ESR1 |
| chr16 | 23625213 | 23625413 | 0.398 | PALB2 | chr6 | 152265349 | 152265597 | 0.562 | ESR1 |
| chr16 | 23632741 | 23632891 | 0.391 | PALB2 | chr6 | 152332663 | 152332910 | 0.444 | ESR1 |
| chr16 | 23634215 | 23634415 | 0.393 | PALB2 | chr6 | 152332707 | 152332937 | 0.463 | ESR1 |
| chr16 | 23635260 | 23635465 | 0.374 | PALB2 | chr6 | 152332732 | 152332961 | 0.483 | ESR1 |
| chr16 | 23637513 | 23637713 | 0.458 | PALB2 | chr6 | 152415449 | 152415694 | 0.553 | ESR1 |
| chr16 | 23640440 | 23640682 | 0.370 | PALB2 | chr6 | 152415449 | 152415729 | 0.555 | ESR1 |
| chr16 | 23640927 | 23641175 | 0.470 | PALB2 | chr6 | 152415452 | 152415694 | 0.556 | ESR1 |
| chr16 | 23641292 | 23641502 | 0.431 | PALB2 | chr6 | 152415469 | 152415763 | 0.542 | ESR1 |
| chr16 | 23641570 | 23641813 | 0.385 | PALB2 | chr6 | 152419792 | 152420036 | 0.576 | ESR1 |
| chr16 | 23646217 | 23646416 | 0.470 | PALB2 | chr6 | 152419822 | 152420047 | 0.575 | ESR1 |
| chr16 | 23646250 | 23646449 | 0.460 | PALB2 | chr6 | 152419877 | 152420111 | 0.591 | ESR1 |
| chr16 | 23646427 | 23646636 | 0.390 | PALB2 | chr7 | 6029374 | 6029583 | 0.367 | PMS2 |
| chr16 | 23646623 | 23646823 | 0.403 | PALB2 | chr7 | 6035119 | 6035348 | 0.387 | PMS2 |
| chr16 | 23646780 | 23647008 | 0.345 | PALB2 | chr7 | 6036912 | 6037112 | 0.378 | PMS2 |
| chr16 | 23646984 | 23647228 | 0.400 | PALB2 | chr7 | 6038718 | 6039008 | 0.430 | PMS2 |
| chr16 | 23647144 | 23647434 | 0.385 | PALB2 | chr7 | 55241468 | 55241710 | 0.564 | EGFR |
| chr16 | 23647358 | 23647558 | 0.483 | PALB2 | chr7 | 55241542 | 55241789 | 0.565 | EGFR |
| chr16 | 23647369 | 23647610 | 0.467 | PALB2 | chr7 | 55241576 | 55241746 | 0.526 | EGFR |
| chr16 | 23649127 | 23649357 | 0.355 | PALB2 | chr7 | 55241613 | 55241853 | 0.531 | EGFR |
| chr16 | 23649265 | 23649465 | 0.358 | PALB2 | chr7 | 55242269 | 55242500 | 0.509 | EGFR |
| chr16 | 23652430 | 23652650 | 0.692 | PALB2 | chr7 | 55242271 | 55242505 | 0.506 | EGFR |
| chr16 | 23652647 | 23652887 | 0.647 | PALB2 | chr7 | 55242272 | 55242520 | 0.506 | EGFR |
| chr16 | 68772169 | 68772389 | 0.679 | CDH1 | chr7 | 55242280 | 55242532 | 0.506 | EGFR |
| chr16 | 68835529 | 68835729 | 0.463 | CDH1 | chr7 | 55242319 | 55242558 | 0.496 | EGFR |
| chr16 | 68835564 | 68835767 | 0.485 | CDH1 | chr7 | 55242332 | 55242584 | 0.490 | EGFR |
| chr16 | 68842243 | 68842453 | 0.469 | CDH1 | chr7 | 55248852 | 55249080 | 0.585 | EGFR |
| chr16 | 68842518 | 68842718 | 0.423 | CDH1 | chr7 | 55248896 | 55249120 | 0.622 | EGFR |
| chr16 | 68844017 | 68844227 | 0.512 | CDH1 | chr7 | 55248933 | 55249182 | 0.600 | EGFR |
| chr16 | 68845519 | 68845719 | 0.483 | CDH1 | chr7 | 55248937 | 55249187 | 0.598 | EGFR |
| chr16 | 68845706 | 68845945 | 0.517 | CDH1 | chr7 | 55248961 | 55249208 | 0.597 | EGFR |
| chr16 | 68845947 | 68846147 | 0.502 | CDH1 | chr7 | 55259335 | 55259571 | 0.523 | EGFR |
| chr16 | 68846157 | 68846377 | 0.416 | CDH1 | chr7 | 55259337 | 55259586 | 0.532 | EGFR |
| chr16 | 68847216 | 68847450 | 0.477 | CDH1 | chr7 | 55259356 | 55259602 | 0.534 | EGFR |

(continued)

| Chr. | Start | Stop | GC | Gene | Chr. | Start | Stop | GC | Gene |
|------|-------|------|-----|------|------|-------|------|-----|------|
| chr16 | 68849385 | 68849585 | 0.488 | CDH1 | chr7 | 55259368 | 55259570 | 0.537 | EGFR |
| chr16 | 68849423 | 68849649 | 0.529 | CDH1 | chr7 | 55259391 | 55259640 | 0.544 | EGFR |
| chr16 | 68853169 | 68853348 | 0.522 | CDH1 | chr7 | 116411722 | 116411963 | 0.417 | MET |
| chr16 | 68855861 | 68856061 | 0.453 | CDH1 | chr7 | 116411832 | 116412076 | 0.424 | MET |
| chr16 | 68856071 | 68856271 | 0.488 | CDH1 | chr7 | 116411854 | 116412102 | 0.410 | MET |
| chr16 | 68856802 | 68857067 | 0.560 | CDH1 | chr7 | 116411942 | 116412183 | 0.347 | MET |
| chr16 | 68857287 | 68857494 | 0.481 | CDH1 | chr7 | 140453006 | 140453257 | 0.381 | BRAF |
| chr16 | 68862023 | 68862255 | 0.511 | CDH1 | chr7 | 140453042 | 140453283 | 0.364 | BRAF |
| chr16 | 68863473 | 68863683 | 0.488 | CDH1 | chr7 | 140453060 | 140453259 | 0.385 | BRAF |
| chr16 | 68867121 | 68867366 | 0.488 | CDH1 | chr7 | 140453085 | 140453319 | 0.336 | BRAF |
| chr16 | 68867369 | 68867599 | 0.476 | CDH1 | chr7 | 140453086 | 140453335 | 0.340 | BRAF |
| chr16 | 89804046 | 89804215 | 0.553 | FANCA | chr7 | 140453105 | 140453353 | 0.329 | BRAF |
| chr16 | 89804303 | 89804513 | 0.592 | FANCA | chr7 | 140481224 | 140481471 | 0.355 | BRAF |
| chr16 | 89804648 | 89804858 | 0.635 | FANCA | chr7 | 140481234 | 140481475 | 0.360 | BRAF |
| chr16 | 89804928 | 89805172 | 0.596 | FANCA | chr7 | 140481251 | 140481496 | 0.358 | BRAF |
| chr16 | 89805241 | 89805441 | 0.498 | FANCA | chr7 | 140481263 | 140481507 | 0.363 | BRAF |
| chr16 | 89805462 | 89805662 | 0.612 | FANCA | chr8 | 90947700 | 90947947 | 0.319 | NBN |
| chr16 | 89805672 | 89805892 | 0.548 | FANCA | chr8 | 90949123 | 90949343 | 0.326 | NBN |
| chr16 | 89806299 | 89806509 | 0.502 | FANCA | chr8 | 90955446 | 90955690 | 0.351 | NBN |
| chr16 | 89807188 | 89807437 | 0.364 | FANCA | chr8 | 90958290 | 90958510 | 0.321 | NBN |
| chr16 | 89809197 | 89809432 | 0.542 | FANCA | chr8 | 90959971 | 90960119 | 0.329 | NBN |
| chr16 | 89811314 | 89811494 | 0.646 | FANCA | chr8 | 90965407 | 90965703 | 0.347 | NBN |
| chr16 | 89812889 | 89813109 | 0.557 | FANCA | chr8 | 90965709 | 90965960 | 0.333 | NBN |
| chr16 | 89813161 | 89813385 | 0.551 | FANCA | chr8 | 90967522 | 90967722 | 0.388 | NBN |
| chr16 | 89814966 | 89815166 | 0.587 | FANCA | chr8 | 90967762 | 90967962 | 0.299 | NBN |
| chr16 | 89816227 | 89816392 | 0.608 | FANCA | chr8 | 90970951 | 90971177 | 0.414 | NBN |
| chr16 | 89818546 | 89818699 | 0.416 | FANCA | chr8 | 90976592 | 90976816 | 0.324 | NBN |
| chr16 | 89825033 | 89825256 | 0.563 | FANCA | chr8 | 90976592 | 90976817 | 0.323 | NBN |
| chr16 | 89825068 | 89825276 | 0.545 | FANCA | chr8 | 90982598 | 90982805 | 0.385 | NBN |
| chr16 | 89828413 | 89828604 | 0.396 | FANCA | chr8 | 90983304 | 90983514 | 0.308 | NBN |
| chr16 | 89831297 | 89831493 | 0.533 | FANCA | chr8 | 90990344 | 90990544 | 0.318 | NBN |
| chr16 | 89833531 | 89833695 | 0.358 | FANCA | chr8 | 90992886 | 90993086 | 0.323 | NBN |
| chr16 | 89836321 | 89836521 | 0.617 | FANCA | chr8 | 90993103 | 90993303 | 0.284 | NBN |
| chr16 | 89836550 | 89836790 | 0.598 | FANC A | chr8 | 90993522 | 90993742 | 0.321 | NBN |
| chr16 | 89836823 | 89837062 | 0.604 | FANCA | chr8 | 90994780 | 90995004 | 0.347 | NBN |
| chr16 | 89836842 | 89837075 | 0.615 | FANCA | chr8 | 90996593 | 90996859 | 0.678 | NBN |
| chr16 | 89836876 | 89837138 | 0.635 | FANCA | chr9 | 5073651 | 5073873 | 0.354 | JAK2 |
| chr16 | 89836882 | 89837082 | 0.627 | FANCA | chr9 | 21968200 | 21968432 | 0.545 | CDKN2A |
| chr16 | 89836894 | 89837156 | 0.627 | FANCA | chr9 | 21970919 | 21971216 | 0.721 | CDKN2A |
| chr16 | 89838040 | 89838261 | 0.514 | FANCA | chr9 | 21970920 | 21971216 | 0.721 | CDKN2A |
| chr16 | 89839593 | 89839838 | 0.573 | FANCA | chr9 | 21973486 | 21973718 | 0.365 | CDKN2A |
| chr16 | 89842062 | 89842262 | 0.498 | FANCA | chr9 | 21974363 | 21974602 | 0.500 | CDKN2A |
| chr16 | 89845140 | 89845350 | 0.483 | FANCA | chr9 | 21994083 | 21994336 | 0.654 | CDKN2A |
| chr16 | 89845256 | 89845456 | 0.527 | FANCA | chr9 | 21994085 | 21994334 | 0.656 | CDKN2A |
| chr16 | 89846317 | 89846557 | 0.498 | FANCA | chr9 | 35073773 | 35074045 | 0.432 | FANCG |
| chr16 | 89849141 | 89849341 | 0.502 | FANCA | chr9 | 35074052 | 35074258 | 0.517 | FANCG |
| chr16 | 89849323 | 89849591 | 0.554 | FANCA | chr9 | 35074248 | 35074484 | 0.565 | FANCG |
| chr16 | 89857774 | 89858016 | 0.560 | FANCA | chr9 | 35074847 | 35075072 | 0.535 | FANCG |
| chr16 | 89858250 | 89858450 | 0.567 | FANCA | chr9 | 35075125 | 35075335 | 0.493 | FANCG |
| chr16 | 89858752 | 89858951 | 0.555 | FANCA | chr9 | 35075354 | 35075564 | 0.531 | FANCG |

(continued)

| Chr. | Start | Stop | GC | Gene | Chr. | Start | Stop | GC | Gene |
|---|---|---|---|---|---|---|---|---|---|
| chr16 | 89862215 | 89862425 | 0.512 | FANCA | chr9 | 35075451 | 35075651 | 0.562 | FANCG |
| chr16 | 89865452 | 89865652 | 0.532 | FANCA | chr9 | 35075668 | 35075868 | 0.587 | FANCG |
| chr16 | 89865648 | 89865886 | 0.498 | FANCA | chr9 | 35075897 | 35076097 | 0.557 | FANCG |
| chr16 | 89865874 | 89866074 | 0.408 | FANCA | chr9 | 35076388 | 35076632 | 0.494 | FANCG |
| chr16 | 89869678 | 89869909 | 0.461 | FANCA | chr9 | 35076699 | 35076959 | 0.529 | FANCG |
| chr16 | 89871619 | 89871819 | 0.522 | FANCA | chr9 | 35076908 | 35077108 | 0.537 | FANCG |
| chr16 | 89874567 | 89874718 | 0.375 | FANCA | chr9 | 35077204 | 35077404 | 0.517 | FANCG |
| chr16 | 89877021 | 89877274 | 0.382 | FANCA | chr9 | 35078145 | 35078385 | 0.593 | FANCG |
| chr16 | 89877278 | 89877525 | 0.520 | FANCA | chr9 | 35078512 | 35078712 | 0.522 | FANCG |
| chr16 | 89880876 | 89881036 | 0.342 | FANCA | chr9 | 35079068 | 35079268 | 0.587 | FANCG |
| chr16 | 89882268 | 89882508 | 0.564 | FANCA | chr9 | 35079437 | 35079667 | 0.671 | FANCG |
| chr17 | 7572710 | 7572941 | 0.547 | TP53 | chr9 | 35079767 | 35079970 | 0.667 | FANCG |
| chr17 | 7572841 | 7573094 | 0.524 | TP53 | chr9 | 35079828 | 35080069 | 0.702 | FANCG |
| chr17 | 7573785 | 7574015 | 0.571 | TP53 | chr9 | 35079839 | 35080069 | 0.706 | FANCG |
| chr17 | 7573785 | 7574050 | 0.583 | TP53 | chr9 | 35079923 | 35080159 | 0.667 | FANCG |
| chr17 | 7573803 | 7574050 | 0.593 | TP53 | chr9 | 80336120 | 80336372 | 0.478 | GNAQ |
| chr17 | 7573811 | 7574017 | 0.580 | TP53 | chr9 | 80336121 | 80336372 | 0.476 | GNAQ |
| chr17 | 7576734 | 7576934 | 0.468 | TP53 | chr9 | 80336223 | 80336472 | 0.500 | GNAQ |
| chr17 | 7576933 | 7577155 | 0.552 | TP53 | chr9 | 80336253 | 80336500 | 0.484 | GNAQ |
| chr17 | 7576951 | 7577151 | 0.557 | TP53 | chr9 | 80336259 | 80336500 | 0.483 | GNAQ |
| chr17 | 7576970 | 7577197 | 0.548 | TP53 | chr9 | 80409367 | 80409598 | 0.353 | GNAQ |
| chr17 | 7576998 | 7577191 | 0.552 | TP53 | chr9 | 80409379 | 80409628 | 0.352 | GNAQ |
| chr17 | 7576998 | 7577241 | 0.529 | TP53 | chr9 | 97863312 | 97863512 | 0.572 | FANCC |
| chr17 | 7576998 | 7577242 | 0.531 | TP53 | chr9 | 97863840 | 97864111 | 0.548 | FANCC |
| chr17 | 7577014 | 7577263 | 0.532 | TP53 | chr9 | 97869338 | 97869594 | 0.595 | FANCC |
| chr17 | 7577304 | 7577572 | 0.565 | TP53 | chr9 | 97872619 | 97872829 | 0.308 | FANCC |
| chr17 | 7577329 | 7577570 | 0.574 | TP53 | chr9 | 97872955 | 97873165 | 0.398 | FANCC |
| chr17 | 7577329 | 7577576 | 0.569 | TP53 | chr9 | 97873179 | 97873409 | 0.494 | FANCC |
| chr17 | 7577346 | 7577570 | 0.596 | TP53 | chr9 | 97873711 | 97873957 | 0.587 | FANCC |
| chr17 | 7577346 | 7577576 | 0.589 | TP53 | chr9 | 97876827 | 97877027 | 0.517 | FANCC |
| chr17 | 7577371 | 7577620 | 0.564 | TP53 | chr9 | 97879613 | 97879852 | 0.404 | FANCC |
| chr17 | 7577398 | 7577598 | 0.572 | TP53 | chr9 | 97887262 | 97887462 | 0.403 | FANCC |
| chr17 | 7578196 | 7578426 | 0.563 | TP53 | chr9 | 97888682 | 97888882 | 0.423 | FANCC |
| chr17 | 7578263 | 7578502 | 0.629 | TP53 | chr9 | 97897551 | 97897761 | 0.417 | FANCC |
| chr17 | 7578283 | 7578494 | 0.637 | TP53 | chr9 | 97897761 | 97897961 | 0.313 | FANCC |
| chr17 | 7578298 | 7578566 | 0.617 | TP53 | chr9 | 97912208 | 97912455 | 0.456 | FANCC |
| chr17 | 7578299 | 7578502 | 0.642 | TP53 | chr9 | 97933298 | 97933498 | 0.363 | FANCC |
| chr17 | 7578363 | 7578562 | 0.615 | TP53 | chr9 | 97934219 | 97934419 | 0.333 | FANCC |
| chr17 | 7578363 | 7578598 | 0.597 | TP53 | chr9 | 98002823 | 98003043 | 0.321 | FANCC |
| chr17 | 7579266 | 7579515 | 0.624 | TP53 | chr9 | 98009694 | 98009940 | 0.328 | FANCC |
| chr17 | 7579311 | 7579460 | 0.620 | TP53 | chr9 | 98011375 | 98011575 | 0.418 | FANCC |
| chr17 | 7579326 | 7579526 | 0.627 | TP53 | chr9 | 98011585 | 98011785 | 0.433 | FANCC |
| chr17 | 7579332 | 7579550 | 0.616 | TP53 | chr9 | 98079868 | 98080148 | 0.669 | FANCC |
| chr17 | 7579817 | 7579987 | 0.556 | TP53 | chr9 | 100437190 | 100437390 | 0.428 | **XPA** |
| chr17 | 7590681 | 7590919 | 0.573 | TP53 | chr9 | 100437359 | 100437558 | 0.475 | **XPA** |
| chr17 | 33426833 | 33427064 | 0.474 | RAD51D | chr9 | 100437525 | 100437765 | 0.299 | **XPA** |
| chr17 | 33427044 | 33427283 | 0.500 | RAD51D | chr9 | 100437793 | 100437993 | 0.383 | **XPA** |
| chr17 | 33427269 | 33427513 | 0.437 | RAD51D | chr9 | 100444474 | 100444677 | 0.397 | **XPA** |
| chr17 | 33427477 | 33427725 | 0.518 | RAD51D | chr9 | 100447108 | 100447308 | 0.348 | **XPA** |
| chr17 | 33427706 | 33427935 | 0.504 | RAD51D | chr9 | 100449321 | 100449540 | 0.309 | **XPA** |

(continued)

| Chr. | Start | Stop | GC | Gene | Chr. | Start | Stop | GC | Gene |
|------|-------|------|-----|------|------|-------|------|-----|------|
| chr17 | 33427916 | 33428162 | 0.498 | RAD51D | chr9 | 100451744 | 100451944 | 0.323 | **XPA** |
| chr17 | 33428100 | 33428345 | 0.577 | RAD51D | chr9 | 100455831 | 100456031 | 0.328 | **XPA** |
| chr17 | 33430196 | 33430419 | 0.567 | RAD51D | chr9 | 100459275 | 100459499 | 0.720 | **XPA** |
| chr17 | 33430401 | 33430620 | 0.573 | RAD51D | chr9 | 100459396 | 100459601 | 0.728 | **XPA** |
| chr17 | 33433344 | 33433506 | 0.564 | RAD51D | chr9 | 100459482 | 100459691 | 0.724 | **XPA** |
| chr17 | 33433897 | 33434133 | 0.489 | RAD51D | chr9 | 100459482 | 100459695 | 0.720 | **XPA** |
| chr17 | 33434295 | 33434532 | 0.500 | RAD51D | chr9 | 100459482 | 100459721 | 0.721 | **XPA** |
| chr17 | 33445499 | 33445672 | 0.586 | RAD51D | chrX | 14861796 | 14861980 | 0.416 | FANCB |
| chr17 | 33446550 | 33446780 | 0.662 | RAD51D | chrX | 14861961 | 14862165 | 0.322 | FANCB |
| chr17 | 37868175 | 37868423 | 0.606 | ERBB2 | chrX | 14862588 | 14862788 | 0.368 | FANCB |
| chr17 | 37868177 | 37868424 | 0.605 | ERBB2 | chrX | 14862805 | 14863005 | 0.294 | FANCB |
| chr17 | 37868184 | 37868432 | 0.614 | ERBB2 | chrX | 14862977 | 14863177 | 0.358 | FANCB |
| chr17 | 37868192 | 37868432 | 0.622 | ERBB2 | chrX | 14863227 | 14863447 | 0.403 | FANCB |
| chr17 | 41226314 | 41226567 | 0.433 | BRCA1 | chrX | 14868691 | 14868896 | 0.306 | FANCB |
| chr17 | 41228463 | 41228682 | 0.368 | BRCA1 | chrX | 14871130 | 14871330 | 0.313 | FANCB |
| chr17 | 41231290 | 41231560 | 0.432 | BRCA1 | chrX | 14875743 | 14875993 | 0.307 | FANCB |
| chr17 | 41234185 | 41234455 | 0.435 | BRCA1 | chrX | 14877225 | 14877425 | 0.313 | FANCB |
| chr17 | 41234392 | 41234631 | 0.438 | BRCA1 | chrX | 14877435 | 14877635 | 0.274 | FANCB |
| chr17 | 41242906 | 41243146 | 0.444 | BRCA1 | chrX | 14882681 | 14882881 | 0.393 | FANCB |
| chr17 | 41243450 | 41243690 | 0.411 | BRCA1 | chrX | 14882885 | 14883085 | 0.328 | FANCB |
| chr17 | 41243675 | 41243921 | 0.397 | BRCA1 | chrX | 14883089 | 14883289 | 0.383 | FANCB |
| chr17 | 41243914 | 41244153 | 0.433 | BRCA1 | chrX | 14883275 | 14883501 | 0.291 | FANCB |
| chr17 | 41244048 | 41244300 | 0.368 | BRCA1 | chrX | 14883493 | 14883735 | 0.350 | FANCB |
| chr17 | 41244075 | 41244265 | 0.366 | BRCA1 | chrX | 14887070 | 14887270 | 0.323 | FANCB |
| chr17 | 41244473 | 41244712 | 0.371 | BRCA1 | chrX | 14890941 | 14891170 | 0.578 | FANCB |
| chr17 | 41244534 | 41244778 | 0.376 | BRCA1 | chrX | 47426013 | 47426262 | 0.636 | ARAF |
| chr17 | 41244766 | 41245013 | 0.387 | BRCA1 | chrX | 47426015 | 47426263 | 0.635 | ARAF |
| chr17 | 41245011 | 41245240 | 0.409 | BRCA1 | chrX | 47426016 | 47426262 | 0.640 | ARAF |
| chr17 | 41245209 | 41245462 | 0.374 | BRCA1 | chrX | 47426063 | 47426266 | 0.627 | ARAF |
| chr17 | 41245347 | 41245586 | 0.404 | BRCA1 | chrX | 66765925 | 66766173 | 0.635 | AR |
| chr17 | 41245598 | 41245827 | 0.370 | BRCA1 | chrX | 66765934 | 66766173 | 0.638 | AR |
| chr17 | 41245825 | 41246064 | 0.375 | BRCA1 | chrX | 66766002 | 66766226 | 0.671 | AR |
| chr17 | 41246061 | 41246310 | 0.376 | BRCA1 | chrX | 66766013 | 66766226 | 0.682 | AR |
| chr17 | 41246304 | 41246553 | 0.416 | BRCA1 | chrX | 66931238 | 66931486 | 0.522 | AR |
| chr17 | 41246534 | 41246786 | 0.423 | BRCA1 | chrX | 66931245 | 66931461 | 0.525 | AR |
| chr17 | 41246546 | 41246786 | 0.427 | BRCA1 | chrX | 66931245 | 66931492 | 0.524 | AR |
| chr17 | 41246595 | 41246842 | 0.419 | BRCA1 | chrX | 66931246 | 66931484 | 0.523 | AR |
| chr17 | 41246643 | 41246890 | 0.403 | BRCA1 | chrX | 66937264 | 66937498 | 0.536 | AR |
| chr17 | 41246709 | 41246956 | 0.379 | BRCA1 | chrX | 66937327 | 66937559 | 0.532 | AR |
| chr17 | 41246794 | 41247027 | 0.338 | BRCA1 | chrX | 66937327 | 66937576 | 0.524 | AR |
| chr17 | 41247801 | 41248006 | 0.403 | BRCA1 | chrX | 66943491 | 66943695 | 0.468 | AR |
| chr17 | 41249211 | 41249311 | 0.356 | BRCA1 | chrX | 66943494 | 66943696 | 0.473 | AR |
| chr17 | 41251673 | 41251893 | 0.380 | BRCA1 | chrX | 66943515 | 66943684 | 0.476 | AR |
| chr17 | 41251732 | 41251945 | 0.397 | BRCA1 | chrX | 66943534 | 66943689 | 0.474 | AR |

## Detailed Description

The invention pertains to a method for analyzing tumor biomarker sequences that involves hybridization-based enrichment of selected target regions across the human genome in a multiplexed panel assay, followed by quantification, coupled with a novel bioinformatics and mathematical analysis pipeline. An overview of the method is shown schematically in Figure 1.

In-solution hybridization enrichment has been used in the past to enrich specific regions of interest prior to sequencing (see e.g., Meyer, M and Kirchner, M. (2010) Cold Spring Harb. Protoc. 2010(6):pdbprot5448; Liao, G.J. et al. (2012) PLoS One 7:e38154; Maricic, T. et al. (2010) PLoS One 5:e14004; Tewhey, R. et al.(2009) Genome Biol. 10:R116; Tsangaras, K. et al. (2014) PLoS One 9:e109101; PCT Publication WO 2016/189388; US Patent Publication 2016/0340733; Koumbaris, G. et al. (2016) Clinical chemistry, 62(6), pp.848-855). However, for the methods of the invention, the target sequences (referred to as TArget Capture Sequences, or TACS) used to enrich for specific regions of interest have been optimized for maximum efficiency, specificity and accuracy and, furthermore, in certain embodiments are used in families of TACS, comprising a plurality of members that bind to the same tumor biomarker sequence but with differing start and/or stop positions, such that enrichment of the tumor biomarker sequences of interest is significantly improved compared to use of a single TACS binding to the genomic sequence. An example of a configuration of such families of TACS is illustrated schematically in Figure 3, showing that the different start and/or stop positions of the members of the TACS family when bound to the genomic sequence of interest results in a staggered binding pattern for the family members.

The use of families of TACS with the TACS pool that bind to each target sequence of interest, as compared to use of a single TACS within the TACS pool that binds to each target sequence of interest, significantly increases enrichment for the target sequences of interest, as evidenced by a greater than 50% average increase in read-depth for the family of TACS versus a single TACS. Comparison of use of a family of TACS versus a single TACS, and the significantly improved read-depth that was observed, is described in detail in Example 5.

Tumor Biomarker Detection

The methods and kits of the disclosure are used in the analysis of tumor biomarkers in biological samples. As described in detail in Examples 6-9, the methods of the invention can used for the detection of large panels of tumor biomarkers at tumor loads as low as 0.1% and can detect tumor biomarkers in both tumor tissue and in liquid biopsy samples from tumor patients. Accordingly, in one aspect, the invention pertains to a method of detecting one or more tumor biomarkers in a DNA sample from a subject having or suspected of having a tumor, the method comprising:

(a) preparing a sequencing library from the DNA sample;

(b) hybridizing the sequencing library to a pool of double-stranded TArget Capture Sequences (TACS) that bind to one or more tumor biomarker sequences of interest, wherein:

(i) each member sequence within the pool of TACS is between 100-500 base pairs in length, each member sequence having a 5' end and a 3' end;

(ii) preferably each member sequence binds to the tumor biomarker sequence of interest at least 50 base pairs away, on both the 5' end and the 3' end, from regions harboring Copy Number Variations (CNVs), Segmental duplications or repetitive DNA elements; and

(iii) the GC content of the pool of TACS is between 19% and 80%, as determined by calculating the GC content of each member within the pool of TACS;

(c) isolating members of the sequencing library that bind to the pool of TACS to obtain an enriched library;

(d) amplifying and sequencing the enriched library; and

(e) performing statistical analysis on the enriched library sequences, optionally utilizing only fragments of a specific size range, to thereby detect the tumor biomarker(s) in the DNA sample.

In one embodiment, the pool of TACS comprises a plurality of TACS families, wherein each member of a TACS family binds to the same tumor biomarker sequence of interest but with different start and/or stop positions on the sequence with respect to a reference coordinate system (i.e., binding of TACS family members to the target sequence is staggered) to thereby enrich for target sequences of interest, followed by massive parallel sequencing and statistical analysis of the enriched population. Typically, the reference coordinate system that is used for analyzing human genomic DNA is the human reference genome built hg19, which is publically available in the art, although other versions may be used. Alternatively, the reference coordinate system can be an artificially created genome based on built hg19 that contains only the genomic sequences of interest. Exemplary non-limiting examples of start/stop positions for TACS that bind to chromosome 13, 18, 21, X or Y are shown in Figure 2. Exemplary non-limiting examples of start/stop positions for TACS that bind to NRAS on chromosome 1, PI3KCA on chromosome 3, EGFR on chromosome 7 or KRAS on chromosome 12 (as non-limiting examples of tumor biomarkers) are shown in Figure 10.

Accordingly, in another aspect, the invention pertains to a method of detecting one or more tumor biomarkers in a DNA sample from a subject having or suspected of having a tumor, the method comprising:

(a) preparing a sequencing library from the DNA sample;

(b) hybridizing the sequencing library to a pool of double-stranded TArget Capture Sequences (TACS) that bind to one or more tumor biomarker sequences of interest, wherein the pool of TACS comprises a plurality of TACS families, wherein each member of a TACS family binds to the same tumor biomarker sequence of interest but with different start and/or stop positions on the sequence with respect to a reference coordinate system, and further wherein:

(i) each member sequence within the pool of TACS is between 100-500 base pairs in length, each member sequence having a 5' end and a 3' end;

(ii) preferably each member sequence binds to the tumor biomarker sequence of interest at least 50 base pairs away, on both the 5' end and the 3' end, from regions harboring Copy Number Variations (CNVs), Segmental duplications or repetitive DNA elements; and

(iii) the GC content of the pool of TACS is between 19% and 80%, as determined by calculating the GC content of each member within the pool of TACS;

(c) isolating members of the sequencing library that bind to the pool of TACS to obtain an enriched library;

(d) amplifying and sequencing the enriched library; and

(e) performing statistical analysis on the enriched library sequences, optionally utilizing only fragments of a specific size range, to thereby detect the tumor biomarker(s) in the DNA sample.

The TACS-enrichment based method of the disclosure can be used in the detection of a wide variety of genetic abnormalities. In one embodiment, the genetic abnormality is a chromosomal aneuploidy (such as a trisomy, a partial trisomy or a monosomy). In other embodiments, the genomic abnormality is a structural abnormality, including but not limited to copy number changes including microdeletions and microduplications, insertions, translocations, inversions and small-size mutations including point mutations and mutational signatures. In another embodiment, the genetic abnormality is a chromosomal mosaicism.

Further aspects and features of the methods of the disclosure are described in the subsections below.

TArget Capture Sequence Design

As used herein, the term "TArget Capture Sequences" or "TACS" refers to short DNA sequences that are complementary to the region(s) of interest on a genomic sequence(s) of interest (e.g., chromosome(s) of interest) and which are used as "bait" to capture and enrich the region of interest from a large library of sequences, such as a whole genomic sequencing library prepared from a biological sample. In addition to the features of the families of TACS described above (e.g., staggered binding to the genomic sequence of interest), a pool of TACS is used for enrichment wherein the sequences within the pool have been optimized with regard to: (i) the length of the sequences; (ii) the distribution of the TACS across the region(s) of interest; and (iii) the GC content of the TACS. The number of sequences within the TACS pool (pool size) has also been optimized.

It has been discovered that TACS having a length of 100-500 base pairs are optimal to maximize enrichment efficiency. In various other embodiments, each sequence within the pool of TACS is between 150-260 base pairs, 100-200 base pairs, 200-260 base pairs, 100-350 bp in length, or 100-500 bp in length. In preferred embodiments, the length of the TACS within the pool is at least 250 base pairs, or is 250 base pairs or is 260 base pairs or is 280 base pairs. It will be appreciated by the ordinarily skilled artisan that a slight variation in TACS size typically can be used without altering the results (e.g., the addition or deletion of a few base pairs on either end of the TACS); accordingly, the base pair lengths given herein are to be considered "about" or "approximate", allowing for some slight variation (e.g., 1-5%) in length. Thus, for example, a length of "250 base pairs" is intended to refer to "about 250 base pairs" or "approximately 250 base pairs", such that, for example, 248 or 252 base pairs is also encompassed.

The distribution of the TACS across each region or chromosome of interest has been optimized to avoid, if applicable, high copy repeats, low copy repeats and copy number variants, while at the same time also being able to target informative single nucleotide polymorphisms (SNPs) in order to enable both aneuploidy, or structural copy number change detection, and fraction of interest estimation. Accordingly, each sequence within the TACS pool is designed such that the 5' end and the 3' end are each at least 50 base pairs away from regions in the genome that are known to harbor one or more of the following genomic elements: Copy Number Variations (CNVs), Segmental duplications and/or repetitive DNA elements (such as transposable elements or tandem repeat areas). In various other embodiments, each sequence within the TACS pool is designed such that the 5' end and the 3' end are each at least 50, 100, 150, 200, 250, 300, 400 or 500 base pairs away from regions in the genome that are known to harbor one or more of the aforementioned elements.

The term "Copy Number Variations" is a term of art that refers to a form of structural variation in the human genome in which there can be alterations in the DNA of the genome in different individuals that can result in a fewer or greater than normal number of a section(s) of the genome in certain individuals. CNVs correspond to relatively large regions of the genome that may be deleted (e.g., a section that normally is A-B-C-D can be A-B-D) or may be duplicated (e.g., a section that normally is A-B-C-D can be A-B-C-C-D). CNVs account for roughly 13% of the human genome, with each variation ranging in size from about 1 kilobase to several megabases in size.

The term "Segmental duplications" (also known as "low-copy repeats") is also a term of art that refers to blocks of DNA that range from about 1 to 400 kilobases in length that occur at more than one site within the genome and typically share a high level (greater than 90%) of sequence identity. Segmental duplications are reviewed in, for example, Eichler. E.E. (2001) Trends Genet. 17:661-669.

The term "repetitive DNA elements" (also known as "repeat DNA" or "repeated DNA") is also a term of art that refers to patterns of DNA that occur in multiple copies throughout the genome. The term "repetitive DNA element" encompasses terminal repeats, tandem repeats and interspersed repeats, including transposable elements. Repetitive DNA elements in NGS is discussed further in, for example, Todd, J. et al. (2012) Nature Reviews Genet. 13:36-46.

The TACS are designed with specific GC content characteristics in order to minimize data GC bias and to allow a custom and innovative data analysis pipeline. It has been determined that TACS with a GC content of 19-80% achieve optimal enrichment and perform best with cell free DNA. Within the pool of TACS, different sequences can have different % GC content, although to be selected for inclusion with the pool, the % GC content of each sequence is chosen as between 19-80%, as determined by calculating the GC content of each member within each family of TACS. That is, every member within each family of TACS has a % GC content within the given percentage range (e.g., between 19-80% GC content).

In some instances, the pool of TACS (i.e., each member within each family of TACS) may be chosen so as to define a different % GC content range, deemed to be more suitable for the assessment of specific genetic abnormalities. Non-limiting examples of various % GC content ranges, can be between 19% and 80%, or between 19% and 79%, or between 19% and 78%, or between 19% and 77%, or between 19% and 76%, or between 19% and 75%, or between 19% and 74%, or between 19% and 73%, or between 19% and 72%, or between 19% and 71%, or between 19% and 70%, or between 19% and 69%, or between 19% and 68%, or between 19% and 67%, or between 19% and 66%, or between 19% and 65%, or between 19% and 64%, or between 19% and 63%, or between 19% and 62%, or between 19% and 61%, or between 19% and 60%, or between 19% and 59%, or between 19% and 58%, or between 19% and 57%, or between 19% and 56%, or between 19% and 55%, or between 19% and 54%, or between 19% and 53%, or between 19% and 52%, or between 19% and 51%, or between 19% and 50%, or between 19% and 49%, or between 19% and 48%, or between 19% and 47%, or between 19% and 46%, or between 19% and 45%, or between 19% and 44%, or between 19% and 43%, or between 19% and 42%, or between 19% and 41%, or between 19% and 40%.

As described in further detail below with respect to one embodiment of the data analysis, following amplification and sequencing of the enriched sequences, the test loci and reference loci can then be "matched" or grouped together according to their % GC content (e.g., test loci with a % GC content of 40% is matched with reference loci with a % GC content of 40%). It is appreciated that the % GC content matching procedure may allow slight variation in the allowed matched % GC range. A non-limiting instance, and with reference to the previously described example in text, a test locus with % GC content of 40% could be matched with reference loci of % GC ranging from 39-41%, thereby encompassing the test locus % GC within a suitable range.

To prepare a pool of TACS having the optimized criteria set forth above with respect to size, placement within the human genome and % GC content, both manual and computerized analysis methods known in the art can be applied to the analysis of the human reference genome. In one embodiment, a semi-automatic method is implemented where regions are firstly manually designed based on the human reference genome build 19 (hg19) ensuring that, if applicable, the aforementioned repetitive regions are avoided and subsequently are curated for GC-content using software that computes the % GC-content of each region based on its coordinates on the human reference genome build 19 (hg19). In another embodiment, custom-built software is used to analyses the human reference genome in order to identify suitable TACS regions that fulfill certain criteria, such as but not limited to, % GC content, proximity to repetitive regions and/or proximity to other TACS.

The number of TACS in the pool has been carefully examined and adjusted to achieve the best balance between result robustness and assay cost/throughput. The pool typically contains at least 800 or more TACS, but can include more, such as 1500 or more TACS, 2000 or more TACS or 2500 or more TACS or 3500 or more TACS or 5000 or more TACS. It has been found that an optimal number of TACS in the pool is 5000. It will be appreciated by the ordinarily skilled artisan that a slight variation in pool size typically can be used without altering the results (e.g., the addition or removal of a small number of TACS); accordingly, the number sizes of the pool given herein are to be considered "about" or "approximate", allowing for some slight variation (e.g., 1-5%) in size. Thus, for example, a pool size of "1600 sequences" is intended to refer to "about 1600 sequences" or "approximately 1600 sequences", such that, for example, 1590 or 1610 sequences is also encompassed.

In view of the foregoing, in another aspect, the invention provides a method for preparing a pool of TACS for use in the method of the invention for detecting risk of a chromosomal and/or other genetic abnormality, wherein the method for preparing the pool of TACS comprises: selecting regions in one or more chromosomes of interest having the criteria set forth above (e.g., at least 50 base pairs away on either end from the aforementioned repetitive sequences and a GC content of between 19% and 80%, as determined by calculating the GC content of each member within each family of TACS), preparing primers that amplify sequences that hybridize to the selected regions, and amplifying the sequences, wherein each sequence is 100-500 base pairs in length.

For use in the methods of the disclosure, the pool of TACS typically is fixed to a solid support, such as beads (such as magnetic beads) or a column. In one embodiment, the pool of TACS are labeled with biotin and are bound to magnetic beads coated with a biotin-binding substance, such as streptavidin or avidin, to thereby fix the pool of TACS to a solid support. Other suitable binding systems for fixing the pool of TACS to a solid support (such as beads or column) are known to the skilled artisan and readily available in the art. When magnetic beads are used as the solid support, sequences that bind to the TACS affixed to the beads can be separated magnetically from those sequences that do not bind to the TACS.

Families of TACS

In one embodiment, the pool of TACS comprises a plurality of TACS families directed to different tumor biomarker sequences of interest. Each TACS family comprises a plurality of members that bind to the same tumor biomarker sequence of interest but having different start and/or stop positions with respect to a reference coordinate system for the genomic sequence of interest. Typically, the reference coordinate system that is used for analyzing human genomic DNA is the human reference genome built hg19, which is publically available in the art, but other coordinate systems may also be used. Alternatively, the reference coordinate system can be an artificially created genome based on publically available coordinate systems, such as for example built hg19 of the human genome, that contains only the genomic sequences of interest. Exemplary non-limiting examples of start/stop positions for TACS that bind to chromosome 13, 18, 21, X or Y are shown in Figure 2.

Each TACS family comprises at least 2 members that bind to the same genomic sequence of interest. In various embodiments, each TACS family comprises at least 2 member sequences, or at least 3 member sequences, or at least 4 member sequences, or at least 5 member sequences, or at least 6 member sequences, or at least 7 member sequences, or at least 8 member sequence, or at least 9 member sequences, or at least 10 member sequences. In various embodiments, each TACS family comprises 2 member sequences, or 3 member sequences, or 4 member sequences, or 5 member sequences, or 6 member sequences, or 7 member sequences, or 8 member sequences, or 9 member sequences, or 10 member sequences. In various embodiments, the plurality of TACS families comprises different families having different numbers of member sequences. For example, a pool of TACS can comprise one TACS family that comprises 3 member sequences, another TACS family that comprises 4 member sequences, and yet another TACS family that comprises 5 member sequences, and the like. In one embodiment, a TACS family comprises 3-5 member sequences. In another embodiment, the TACS family comprises 4 member sequences.

The pool of TACS comprises a plurality of TACS families. Thus, a pool of TACS comprises at least 2 TACS families. In various embodiments, a pool of TACS comprises at least 3 different TACS families, or at least 5 different TACS families, or at least 10 different TACS families, or at least 50 different TACS families, or at least 100 different TACS families, or at least 500 different TACS families, or at least 1000 different TACS families, or at least 2000 TACS families, or at least 4000 TACS families, or at least 5000 TACS families.

Each member within a family of TACS binds to the same genomic region of interest but with different start and/or stop positions, with respect to a reference coordinate system for the genomic sequence of interest, such that the binding pattern of the members of the TACS family is staggered (for example see Figure 3). In various embodiments, the start and/or stop positions are staggered by at least 3 base pairs, or at least 4 base pairs, or at least 5 base pairs, or at least 6 base pairs, or at least 7 base pairs, or at least 8 base pairs, or at least 9 base pairs, or at least 10 base pairs, or at least 15 base pairs, or at least 20 base pairs, or at least 25 base pairs. Typically, the start and/or stop positions are staggered by 5-10 base pairs. In one embodiment, the start and/or stop positions are staggered by 5 base pairs. In another embodiment, the start and/or stop positions are staggered by 10 base pairs.

Sample Collection and Preparation

The methods of the invention can be used with a variety of biological samples. Essentially any biological sample containing DNA, and in particular cell-free DNA (cfDNA), can be used as the sample in the methods, allowing for genetic analysis of the DNA therein. For example, a peripheral whole blood sample can be obtained from a subject and plasma can be obtained from the whole blood sample by standard methods. Total cell free DNA can then be extracted from the sample using standard techniques, non-limiting examples of which include a Qiasymphony protocol (Qiagen) suitable for cell free DNA isolation or any other manual or automated extraction method suitable for cell free DNA isolation.

For tumor biomarker detection, the sample is a biological sample obtained from a patient having or suspected of having a tumor. In one embodiment, the DNA sample comprises cell free tumor DNA (cftDNA). In one embodiment, the oncology sample is a sample of tissue (e.g., from a tumor biopsy). In another embodiment the sample is a patient's urine, sputum, ascites, cerebrospinal fluid or pleural effusion. In another embodiment, the oncology sample is a patient plasma sample, prepared from patient peripheral blood. Thus, the sample can be a liquid biopsy sample that is obtained non-invasively from a patient's blood sample, thereby potentially allowing for early detection of cancer prior to development of a detectable or palpable tumor, or can be from a tissue that has or is suspected of having cancer. In another embodiment, the oncology sample is a patient's healthy tissue such as buffy coat, prepared from patient peripheral blood, or buccal swab or healthy tissue adjacent to the tumor or another source of healthy cells. Thus, the healthy cells can provide a source of DNA that allows for detection of germline mutations and comparison with tumor DNA.

For the biological sample preparation, typically cells are lysed and DNA is extracted using standard techniques known in the art, a non-limiting example of which is the Qiagen DNeasy Blood and Tissue protocol. In another embodiment, cell free DNA is isolated from plasma using standard techniques, a non-limiting example of which is the Qiasymphony (Qiagen) protocol.

Following isolation, the cell free DNA of the sample is used for sequencing library construction to make the sample compatible with a downstream sequencing technology, such as Next Generation Sequencing. Typically this involves ligation of adapters onto the ends of the cell free DNA fragments, followed by amplification. Sequencing library preparation kits are commercially available. A non-limiting exemplary protocol for sequencing library preparation is described in detail in Example 1. In another embodiment, nuclear DNA (a non-limiting example of which is DNA extracted from tissue of buffy coat) is fragmented using standard techniques. A non-limiting example of DNA fragmentation is sonication. Fragmented nuclear DNA is then subjected to the same downstream procedures for cell free DNA described in this paragraph.

Enrichment by TACS Hybridization

The region(s) of interest on the chromosome(s) of interest (e.g., tumor biomarker sequences) is enriched by hybridizing the pool of TACS to the sequencing library, followed by isolation of those sequences within the sequencing library that bind to the TACS. To facilitate isolation of the desired, enriched sequences, typically the TACS sequences are modified in such a way that sequences that hybridize to the TACS can be separated from sequences that do not hybridize to the TACS. Typically, this is achieved by fixing the TACS to a solid support. This allows for physical separation of those sequences that bind the TACS from those sequences that do not bind the TACS. For example, each sequence within the pool of TACS can be labeled with biotin and the pool can then be bound to beads coated with a biotin-binding substance, such as streptavidin or avidin. In a preferred embodiment, the TACS are labeled with biotin and bound to streptavidin-coated magnetic beads. The ordinarily skilled artisan will appreciate, however, that other affinity binding systems are known in the art and can be used instead of biotin-streptavidin/avidin. For example, an antibody-based system can be used in which the TACS are labeled with an antigen and then bound to antibody-coated beads. Moreover, the TACS can incorporate on one end a sequence tag and can be bound to a solid support via a complementary sequence on the solid support that hybridizes to the sequence tag. Furthermore in addition to magnetic beads, other types of solid supports can be used, such as polymer beads and the like.

In certain embodiments, the members of the sequencing library that bind to the pool of TACS are fully complementary to the TACS. In other embodiments, the members of the sequencing library that bind to the pool of TACS are partially complementary to the TACS. For example, in certain circumstances it may be desirable to utilize and analyze data that are from DNA fragments that are products of the enrichment process but that do not necessarily belong to the genomic regions of interest (i.e. such DNA fragments could bind to the TACS because of part homologies (partial complementarity) with the TACS and when sequenced would produce very low coverage throughout the genome in non-TACS coordinates).

Following enrichment of the sequence(s) of interest using the TACS, thereby forming an enriched library, the members of the enriched library are eluted from the solid support and are amplified and sequenced using standard methods known in the art. Next Generation Sequencing is typically used, although other sequencing technologies can also be employed, which provides very accurate counting in addition to sequence information. To detect genetic abnormalities, such as but not limited to, aneuploidies or structural copy number changes requires very accurate counting and NGS is a type of technology that enables very accurate counting. Accordingly, for the detection of genetic abnormalities, such as but not limited to, aneuploidies or structural copy number changes, other accurate counting methods, such as digital PCR and microarrays can also be used instead of NGS. Non-limiting exemplary protocols for amplification and sequencing of the enriched library are described in detail in Example 3.

Data Analysis

The information obtained from the sequencing of the enriched library can be analyzed using an innovative biomathematical/biostatistical data analysis pipeline. Details of an exemplary analysis using this pipeline are described in depth in Example 4, and in further detail below. Alternative data analysis approaches for different purposes are also provided herein. For example, data analysis approaches for analyzing oncology samples are described in detail in Example 6-9 and in the oncology section below.

The analysis pipeline described in Example 4 exploits the characteristics of the TACS, and the high-efficiency of the target capture enables efficient detection of aneuploidies or structural copy number changes, as well as other types of genetic abnormalities. In the analysis, first the sample's sequenced DNA fragments are aligned to the human reference genome. QC metrics are used to inspect the aligned sample's properties and decide whether the sample is suitable to undergo classification. These QC metrics can include, but are not limited to, analysis of the enrichment patterns of the loci of interest, such as for example the overall sequencing depth of the sample, the on-target sequencing output of the sample, TACS performance, GC bias expectation, fraction of interest quantification. For determining the risk of a chromosomal abnormality in the DNA of the sample, an innovative algorithm is applied. The steps of the algorithm include, but are not limited to, removal of inadequately sequenced loci, read-depth and fragment-size information extraction at TACS-specific coordinates, genetic (GC-content) bias alleviation and ploidy status classification.

Ploidy status determination can be achieved using one or more statistical methods, non-limiting examples of which include a t-test method, a bootstrap method, a permutation test and/or a binomial test of proportions and/or segmentation-based methods and/or combinations thereof. It will be appreciated by the ordinarily skilled artisan that the selection and application of tests to be included in ploidy status determination is based on the number of data points available. As such, the suitability of each test is determined by various factors such as, but not limited to, the number of TACS utilized and the respective application for GC bias alleviation, if applicable. Thus, the aforementioned methods are to be taken as examples of the types of statistical analysis that may be employed and are not the only methods suitable for the determination of ploidy status. Typically, the statistical method results in a score value for the mixed sample and risk of the chromosomal abnormality in the DNA is detected when the score value for the mixed sample is above a reference threshold value.

In particular, one aspect of the statistical analysis involves quantifying and alleviating GC-content bias. In addition to the challenge of detecting small signal changes in DNA in the mixed sample, and/or other components of DNA of interest part of a mixed sample (for example, but not limited to, additional or less genetic material from certain chromosomal regions), the sequencing process itself introduces certain biases that can obscure signal detection. One such bias is the preferential sequencing/amplification of genetic regions based on their GC-content. As such, certain detection methods, such as but not limited to, read-depth based methods, need to account for such bias when examining sequencing data. Thus, the bias in the data needs to be quantified and, subsequently, suitable methods are applied to account for it such that genetic context dependencies cannot affect any statistical methods that may be used to quantify genetic abnormality risk.

For example, one method of quantifying the GC-content bias is to use a locally weighted scatterplot smoothing (LOESS) technique on the sequencing data. Each targeted locus may be defined by its sequencing read-depth output and its' GC-content. A line of best fit through these two variables, for a large set of loci, provides an estimate of the expected sequencing read-depth given the GC-content. Once this GC-bias quantification step is completed, the next step is to use this information to account for possible biases in the data. One method is to normalize the read-depth of all loci by their expected read-depth (based on each locus' GC-content). In principle, this unlinks the read-depth data from their genetic context and makes all data comparable. As such, data that are retrieved from different GC-content regions, such as for example, but not limited, to different chromosomes, can now be used in subsequent statistical tests for detection of any abnormalities. Thus, using the LOESS procedure, the GC bias is unlinked from the data prior to statistical testing. In one embodiment, the statistical analysis of the enriched library sequences comprises alleviating GC bias using a LOESS procedure.

In an alternative embodiment, the GC-content bias is quantified and alleviated by grouping together loci of similar (matching) GC-content. Thus, conceptually this method for alleviating GC-content bias comprises of three steps, as follows:

    1) identification and calculation of GC-content in the TACS;

2) alleviation/accounting of GC-content bias using various matching/grouping procedures of the TACS; and

3) calculation of risk of any genetic abnormalities that may be present in the fetus utilizing statistical and mathematical methods on datasets produced from step 2.

For the t-test method, the dataset is split into two groups; the test loci and the reference loci. For each group, subsets of groups are created where loci are categorized according to their GC-content as illustrated in a non-limiting example in the sample Table 1 below:

Table 1

| GC | Reference loci read-depth | Test loci read-depth |
|---|---|---|
| 40% | $x_1^{40}, x_2^{40}, \ldots, x_{nx40}^{40}$ | $y_1^{40}, y_2^{40}, \ldots, y_{ny40}^{40}$ |
| 41% | $x_1^{41}, x_2^{41}, \ldots, x_{nx41}^{41}$ | $y_1^{41}, y_2^{41}, \ldots, y_{ny41}^{41}$ |
| 42% | $x_1^{42}, x_2^{42}, \ldots, x_{nx42}^{42}$ | $y_1^{42}, y_2^{42}, \ldots, y_{ny42}^{42}$ |
| ... | ... | |

It is appreciated by the ordinarily skilled artisan that subgroup creation may involve encompassing a range of appropriate GC-content and/or a subset of loci that are defined by a given GC-content and/or GC-content range. Accordingly, the % GC content given in the non-limiting example of Table 1 are to be considered "about" or "approximate", allowing for some slight variation (e.g., 1-2%). Thus, for example, a % GC content of "40%" is intended to refer to "about 40%" or "approximately 40%", such that, for example, "39%-41%" GC-content loci may also be encompassed if deemed appropriate.

Hence, when referring to a particular GC-content it is understood that the reference and test loci subgroups may comprise of any number of loci related to a particular % GC content and/or range.

Subsequently, for each GC-content subgroup, a representative read-depth is calculated. A number of methods may be utilized to choose this such as, but not limited to, the mean, median or mode of each set. Thus, two vectors of representative read-depth are created where one corresponds to the reference loci and the other to the test loci (e.g., Xm, Ym). In one embodiment, the two vectors may be tested against each other to identify significant differences in read-depth. In another embodiment, the difference of the two vectors may be used to assess if there are significant discrepancies between the test and reference loci. The sample is attributed the score of the test.

For statistical analysis using a bootstrap approach, the dataset is split into two groups, the test loci and the reference loci. The GC-content of each locus is then calculated. Then the following procedure is performed:

A random locus is selected from the reference loci; its read-depth and GC-content are recorded. Subsequently, a random locus from the test loci is selected, with the only condition being that its' GC-content is similar to that of the reference locus. Its read-depth is recorded. It is appreciated by the ordinarily skilled artisan that GC-content similarity may encompass a range of suitable GC-content. As such, referral to a specific % GC content may be considered as "approximate" or "proximal" or "within a suitable range" (e.g., 1%-2%) encompassing the specific % GC content under investigation. Thus, a reference-test locus pair of similar GC-content is created. The difference of the reference-test pair is recorded, say E1. The loci are then replaced to their respective groups. This process is repeated until a bootstrap sample of the same size as the number of test TACS present is created. A representative read-depth of the bootstrap sample is estimated, say E_mu, and recorded. A number of methods may be utilized to do so, such as but not limited to, the mean, mode or median value of the vector, and/or multiples thereof.

The process described above is repeated as many times as necessary and a distribution of E_mu is created. The sample is then attributed a score that corresponds to a percentile of this distribution.

For statistical analysis using a permutation test, the dataset is sorted firstly into two groups, the test-loci and the reference loci. For each group, subsets of groups are created, where loci are categorized according to their GC-content similarity (see columns 2 and 3 of the non-limiting sample Table 2 below). The number of loci present in each test subgroup is also recorded. The loci of the test group are utilized to calculate an estimate of the test-group's read-depth, say Yobs. A representative number from each GC-content subgroup may be selected to do so. Any number of methods may be used to provide a read-depth estimate, such as but not limited to, the mean, median or mode of the chosen loci.

Table 2

| GC | Reference loci read- | Test loci read-depth | test loci | Merging of loci |
|---|---|---|---|---|
| 40% | $x_1^{40}, x_2^{40}, \ldots, x_{nx40}^{40}$ | $x_1^{40}, \ldots, x_{nx40}^{40}, y_1^{40}, \ldots, y_{ny40}^{40}$ | ny40 | $y_1^{40}, y_2^{40}, \ldots, y_{ny40}^{40}$ |
| 41% | $x_1^{41}, x_2^{41}, \ldots, x_{nx41}^{41}$ | $y_1^{41}, y_2^{41}, \ldots, y_{ny41}^{41}$ | ny41 | $x_1^{41}, \ldots, x_{nx41}^{41}, y_1^{41}, \ldots, y_{ny41}^{41}$ |
| 42% | $x_1^{42}, x_2^{42}, \ldots, x_{nx42}^{42}$ | $y_1^{42}, y_2^{42}, \ldots, y_{ny42}^{42}$ | ny42 | $x_1^{42}, \ldots, x_{nx42}^{42}, y_1^{42}, \ldots, y_{ny42}^{42}$ |
| ... | ... | ... | ... | .. |

A distribution to test Yobs is then built utilizing loci irrespective of their test or reference status as follows. The test and reference loci of each GC-content subgroup (see last column of sample Table 2) are combined to allow for calculation of a new read-depth estimate. From each merged subgroup a number of loci are chosen at random, where this number is upper-bounded by the number of test-loci utilized in the original calculation of Yobs (e.g., for GC content 40%, and in the context of the non-limiting sample Table 2, this number of loci may be in the range [1,ny40]). The new read-depth estimate is calculated from all the chosen loci. The procedure is iterated as many times as necessary in order to build a distribution of observed means. A sample is then attributed a score that corresponds to the position of Yobs in this distribution using a suitable transformation that accounts for the moments of the built distribution. As with the already described methods, it is appreciated that slight variation in % GC content is allowed (e.g., 1%-2%), if deemed appropriate. Hence, reference to a specific GC-content could be taken as "about" or "approximate", so that for example when referring to a 40% GC-content, loci that are "approximately" or "about" 40% (e.g., 39%-41%) may be utilized in the method.

For statistical analysis using a binomial test of proportions, fragment-sizes aligned to TACS-specific genomic coordinates are used. There is evidence from the literature that specific types of cancer can be characterized by and/or associated with fragments in the plasma having a smaller size than the expected size of fragments originating from healthy tissues (Jiang et al, (2015), Proceedings of the National Academy of Sciences, 112(11), ppE1317-E1325). The same hypothesis holds true for fragments originating from the placenta/fetus. Specifically, it has been shown that fragments of cell free genetic material originating from the placenta tend to be smaller in length when compared to other cell free genetic material (Chan, K.C. (2004) Clin. Chem. 50:88-92). Hence, the statistic of interest is whether the proportion of small-size fragments aligned to a TACS-specific test-region deviates significantly from what is expected when comparing it to the respective proportion of other TACS-specific reference-regions, as this would indicate fetal genetic abnormalities.

Thus, fragment-sizes are assigned into two groups. Sizes related to the test loci are assigned to one group and fragment-sizes related to the reference loci are assigned to the other group. Subsequently, in each group, fragment sizes are distributed into two subgroups, whereby small-size fragments are assigned into one subgroup and all remaining fragments are designated to the remaining subgroup. The last step computes the proportion of small-sized fragments in each group and uses these quantities in a binomial test of proportions. The score of the test is attributed to the sample under investigation.

The final result of a sample may be given by combining one or more scores derived from the different statistical methods, non-limiting examples of which are given in Example 4.

For statistical analysis using segmentation methods, the read-depth and sequence composition of non-overlapping genomic regions of interest of fixed-size is obtained. On the obtained dataset, GC-content read-depth bias alleviation may be performed, but is not limited to, using a local polynomial fitting method in order to estimate the expected read-depth of regions based on their GC content. The expected value, dependent on GC-content, is then used to normalize regions using suitable methods known to those skilled in the art. The normalized dataset is subsequently processed using one or more segmentation-based classification routines. To do so the algorithms process consecutive data points to detect the presence of read-depth deviations which manifest in the form of a "jump/drop" from their surrounding data points. Depending on the segmentation routine used, data points are given a score which is used towards assigning membership into segments of similar performing read-depths. For example, consecutive data points with score values within a suitable range may be classified as one segment, whereas consecutive data points with score values which exceed the set thresholds may be assigned to a different segment.

Kits of the Invention

In another aspect, the invention provides kits for carrying out the methods of the disclosure. In one embodiment, the kit comprises a container consisting of the pool of TACS and instructions for performing the method. In one embodiment, the TACS are provided in a form that allows them to be bound to a solid support, such as biotinylated TACS. In another embodiment, the TACS are provided together with a solid support, such as biotinylated TACS provided together with streptavidin-coated magnetic beads.

In one embodiment, the kit comprises a container comprising the pool of TACS and instructions for performing the method, wherein the pool of TACS comprises a plurality of TACS families, wherein each TACS family comprises a plurality of member sequences, wherein each member sequence binds to the same genomic sequence of interest (e.g., tumor biomarker sequence of interest) but has different start and/or stop positions with respect to a reference coordinate system for the genomic sequence of interest, and further wherein:

(i) each member sequence within each TACS family is between 100-500 base pairs in length, each member sequence having a 5' end and a 3' end;

(ii) preferably each member sequence binds to the same genomic sequence of interest, and if applicable at least 50 base pairs away, on both the 5' end and the 3' end, from regions harboring Copy Number Variations (CNVs), Segmental duplications or repetitive DNA elements; and

(iii) the GC content of the pool of TACS is between 19% and 80%, as determined by calculating the GC content of each member within each family of TACS.

Furthermore, any of the various features described herein with respect to the design and structure of the TACS can be incorporated into the TACS that are included in the kit.

In various other embodiments, the kit can comprise additional components for carrying out other aspects of the method. For example, in addition to the pool of TACS, the kit can comprise one or more of the following (i) one or more components for isolating cell free DNA or nucleated DNA from a biological sample (e.g., as described in Example 1); (ii) one or more components for preparing the sequencing library (e.g., primers, adapters, buffers, linkers, restriction enzymes, ligation enzymes, polymerase enzymes and the like as described in detail in Example 1); (iii) one or more components for amplifying and/or sequencing the enriched library (e.g., as described in Example 3); and/or (iv) software for performing statistical analysis (e.g., as described in Examples 4 and 6-11).

Oncology Uses

In various embodiments, the TACS-based enrichment method of the disclosure can be used for a variety of purposes in the oncology field. As described in detail in Examples 6-9, the method allows for detection of tumor biomarkers (including cancer related-germline mutations) in biological samples. The method can be applied to the analysis of essentially any known tumor biomarker. An extensive catalogue of known cancer-associated mutations is known in the art, referred to as COSMIC (Catalogue of Somatic Mutations in Cancer), described in, for example, Forbes, S.A. et al. (2016) Curr. Protocol Hum. Genetic 91:10.11.1-10.11.37; Forbes, S.A. et al. (2017) Nucl. Acids Res. 45:D777-D783; and Prior et al. (2012) Cancer Res. 72:2457-2467. The COSMIC database is publically available at **www.cancer.sanger.ac.uk.** The database includes oncogenes that have been associated with cancers, any of which can be analyzed using the method of the disclosure. In addition to the COSMIC catalogue, other compilations of tumor biomarker mutations have been described in the art, non limiting examples of which include the ENCODE Project, which describes mutations in the regulatory sites of oncogenes (see e.g., Shar, N.A. et al. (2016) Mol. Canc. 15:76) and ClinVar, a National Center for Biotechnology Information (NCBI) database for genomic variations associated with human health. The ClinVar database is publicly available at www.ncbi.nlm.nih.gov/clinvar.

The methods of the invention can be used to simultaneously analyze a large panel of tumor biomarkers in a single biological sample. For example, in various embodiments, the pool of TACS used in the method detects at least 5, or at least 10, or at least 15, or at least 20, or at least 25, or at least 30, or at least 35, or at least 40, or at least 45 or at least 50 different tumor biomarkers.

For detection of tumor biomarkers, TACS are designed based on the design criteria described herein and the known sequences of tumor biomarker genes and genetic mutations therein associated with cancer. In one embodiment, a plurality of TACS families used in the method bind to a plurality of tumor biomarker sequences of interest selected from the group comprising of ABL, AKT, AKT1, ALK, APC, AR, ARAF, ATM, BAP1, BARD1, BCL, BMPR1A, BRAF, BRCA, BRCA1, BRCA2, BRIP1, CDH1, CDKN, CHEK2, CTNNB1, DDB2, DDR2, DICER1, EGFR, EPCAM, ErbB, ErcC, ESR1, FANCA, FANCB, FANCC, FANCD2, FANCE, FANCF, FANCG, FANCI, FANCL, FANCM, FBXW7, FGFR, FLT, FLT3, FOXA1, FOXL2, GATA3, GNA11, GNAQ, GNAS, GREM1, HOX, HOXB13, HRAS, IDH1, JAK, JAK2, KEAP1, KIT, KRAS, MAP2Ks, MAP3Ks, MET, MLH1, MPL, MRE11A, MSH2, MSH6, MTOR, MUTYH, NBN, NPM1, NRAS, NTRK1, PALB2, PDGFRs, PI3KCs, PMS2, POLD1, POLE, POLH, PTEN, RAD50, RAD51C, RAD51D, RAF1, RB1, RET, RUNX1, SLX4, SMAD, SMAD4, SMARCA4, SPOP, STAT, STK11, TP53, VHL, XPA and XPC, and combinations thereof.

In one embodiment, the plurality of TACS families used in the method bind to a plurality of tumor biomarker sequences of interest selected from the group consisting of, but not limited to, EGFR_6240, KRAS_521, EGFR_6225, NRAS_578, NRAS_580, PIK3CA_763, EGFR_13553, EGFR_18430, BRAF_476, KIT_1314, NRAS_584, EGFR_12378, and combinations thereof.

Representative, exemplary and non-limiting examples of chromosomal start and stop positions for amplifying TACS that bind to exemplary, non-limiting tumor biomarker genes are shown in Figure 10, for NRAS on chromosome 1, for PI3KCA on chromosome 3, for EGFR on chromosome 7 and for KRAS on chromosome 12. Alternative suitable chromosomal start and stop positions, for these oncogenes and/or for other oncogenes, for amplifying TACS are readily identifiable by one of ordinary skill in the art based on the teachings herein.

In one embodiment of the method, following sequencing of the library preparation and enrichment for the sequences of interest through TACS hybridization, the subsequent step of amplifying the enriched library is performed in the presence of blocking sequences that inhibit amplification of wild-type sequences. Thus, amplification is biased toward amplification of the mutant tumor biomarker sequences.

The pool of TACS and families of TACS used in the method of detecting tumor biomarkers can include any of the design features described herein with respect to the design of the TACS. For example, in various embodiments, each TACS family comprises at least 2, at least 3, at least 4 or at least 5 different member sequences. In one embodiment, each TACS family comprises 4 different member sequences. In various embodiments, the start and/or stop positions for the member sequences within a TACS family, with respect to a reference coordinate system for the genomic sequence of interest, are staggered by at least 5 base pairs, or at least 10 base pairs, or by 5-10 base pairs. In various embodiments, the pool of TACS comprises at least 5, or at least 10 or at least 50 or at least 100 different TACS families, or more.

Suitable statistical analysis approaches for use with oncology samples and detection of tumor biomarkers are described further in Examples 6-9.

The method for detecting tumor biomarkers can be used in a variety of different clinical circumstances in the oncology field. For example, the method can be used for making an initial cancer diagnosis in a subject suspected of having cancer. Accordingly in one embodiment, the method further comprises making a diagnosis of the subject based on detection of at least one tumor biomarker sequence.

Additionally, the method can be used to select an appropriate treatment regimen for a patient diagnosed with cancer, wherein the treatment regimen is designed to be effective against a tumor having the tumor biomarkers detected in the patient's tumor (i.e., known in the art as personalized medicine). Accordingly, in another embodiment, the method further comprises selecting a therapeutic regimen for the subject based on detection of at least one tumor biomarker sequence.

Still further, the method can be used to monitor the efficacy of a therapeutic regiment, wherein changes in tumor biomarker detection are used as an indicator of treatment efficacy. Accordingly, in another embodiment, the method further comprises monitoring treatment efficacy of a therapeutic regimen in the subject based on detection of at least one tumor biomarker sequence.

Moreover, the method can be used to detect relapse and minimal residual disease (MRD), wherein detection of at least one tumor biomarker are used as an indicator of remaining tumor cells in a patient after treatment or tumor recurrence. Accordingly in another embodiment, the method further informs of MRD and disease relapse.

Also, the method can be used to detect cancer-related germline (hereditary) mutations in patients with cancer or individuals suspected of a cancer pre-disposing syndrome wherein detection of at least one germline mutation is used as an indicator for having a cancer pre-disposing syndrome. Accordingly, in another embodiment, the method further comprises diagnosing a patient or an individual with a hereditary cancer pre-disposing syndrome that can inform the clinician to allow for early medical intervention, treatment selection and close monitoring.

Fragment-Based Analysis

In another aspect, the invention pertains to fragment based analysis of samples, described further in Example 9. There is evidence from the literature that specific types of cancer can be characterized by and/or associated with fragments in the plasma having a smaller size than the expected size of fragments originating from healthy tissues (Jiang et al, (2015), Proceedings of the National Academy of Sciences, 112(11), ppE1317-E1325). The same hypothesis holds true for fragments originating from the placenta/fetus. Specifically, placenta derived fragments are generally of smaller size when compared to fragments originating from maternal tissues/cells. Accordingly, a fragment size-based test was developed and assessed, demonstrating its ability to identify samples harboring chromosomal abnormalities.

Thus, the fragments-based detection may be used to detect abnormalities in mixed samples with low signal-to-noise ratio (e.g., as is the case in detection of cancer).

Accordingly, in one embodiment, a fragments-based test is utilized to detect the presence of somatic copy number aberrations in a sample from a patient suspected of having cancer. For example, a binomial test of proportions, as described Example 4 and Example 9, can be used for the detection of increased presence of nucleic acid material originating from non-healthy tissue (e.g., tumor tissue) based on fragment size. In particular, under the null hypothesis that the distribution of fragment sizes originating from both healthy and cancerous cells is the same, a binomial test for proportions (as described in Example 4 and Example 9) using continuity correction can be utilized to quantify any evidence against it.

## EXAMPLES

The present invention is further illustrated by the following examples, which should not be construed as further limiting.

### Example 1: Sample Collection and Library Preparation

The general methodology for the TACS-based multiplexed parallel analysis approach for genetic assessment is shown schematically in Figure 1. In this example, methods for collecting and processing a maternal plasma sample (containing maternal and fetal DNA), followed by sequencing library preparation for use in the methodology of Figure 1 are described. The DNA sample and library preparation described herein can similarly be used with DNA samples from tumors for tumor biomarker detection (see Example 6-9).

### Sample collection

Plasma samples were obtained anonymously from pregnant women after the 10th week of gestation. Protocols used for collecting samples for our study were approved by the Cyprus National Bioethics Committee, and informed consent was obtained from all participants.

### Sample extraction

Cell Free DNA was extracted from 2-4ml plasma from each individual using a manual or automated extraction method suitable for cell free DNA isolation such as for example, but not limited to, Qiasymphony protocol suitable for cell free fetal DNA isolation (Qiagen) (Koumbaris, G. et al. (2016) Clinical chemistry, 62(6), pp.848-855).

### Sequencing library preparation

Extracted DNA from maternal plasma samples was used for sequencing library construction. Standard library preparation methods were used with the following modifications. A negative control extraction library was prepared separately to monitor any contamination introduced during the experiment. During this step, 5' and 3' overhangs were filled-in, by adding 12 units of T4 polymerase (NEB) while 5' phosphates were attached using 40 units of T4 polynucleotide kinase (NEB) in a 100µl reaction and subsequent incubation at 25°C for 15 minutes and then 12° C for 15 minutes. Reaction products were purified using the MinElute kit (Qiagen). Subsequently, adaptors P5 and P7 (see adaptor preparation) were ligated at 1:10 dilution to both ends of the DNA using 5 units of T4 DNA ligase (NEB) in a 40µl reaction for 20 minutes at room temperature, followed by purification using the MinElute kit (Qiagen). Nicks were removed in a fill-in reaction with 16 units of Bst polymerase (NEB) in a 40 µl reaction with subsequent incubation at 65°C for 25 minutes and then 12°C for 20 minutes. Products were purified using the MinElute kit (Qiagen). Library amplification was performed using a Fusion polymerase (Herculase II Fusion DNA polymerase (Agilent Technologies) or Pfusion High Fidelity Polymerase (NEB)) in 50 µl reactions and with the following cycling conditions, 95°C for 3 minutes; followed by 10 cycles at 95°C for 30 seconds, 60°C for 30 seconds, 72°C for 30 seconds and finally 72°C for 3 minutes (Koumbaris, G. et al. (2016) Clinical chemistry, 62(6), pp.848-855). The final library products were purified using the MinElute Purification Kit (Qiagen) and measured by spectrophotometry.

### Adaptor preparation

Hybridization mixtures for adapter P5 and P7 were prepared separately and incubated for 10 seconds at 95° C followed by a ramp from 95° C to 12° C at a rate of 0.1° C /second. P5 and P7 reactions were combined to obtain a ready-to-use adapter mix (100 µM of each adapter). Hybridization mixtures were prepared as follows: P5 reaction mixture contained adaptor P5_F (500 µM) at a final concentration of 200 µM, adaptor P5+P7_R (500 µM) at a final concentration of 200 µM with 1X oligo hybridization buffer. In addition, P7 reaction mixture contained adaptor P7_F (500 µM) at a final concentration of 200 µM, adapter P5+P7_R(500 µM) at a final concentration of 200 µM with 1X oligo hybridization buffer (Koumbaris, G. et al. (2016) Clinical chemistry, 62(6), pp.848-855.). Sequences were as follows, wherein * = a phosphorothioate bond (PTO) (Integrated DNA Technologies):

adaptor P5_F:
A*C*A*C*TCTTTCCCTACACGACGCTCTTCCG*A*T*C*T (SEQ ID NO: 2551)

adaptor P7_F:
G*T*G*A*CTGGAGTTCAGACGTGTGCTCTTCCG*A*T*C*T (SEQ ID NO: 2552),

adaptor_P5+P7_R:
A*G*A*T*CGGAA*G*A*G*C (SEQ ID NO: 2553).

## Example 2: TArget Capture Sequences (TACS) Design and Preparation

This example describes preparation of custom TACS for the detection of whole or partial chromosomal abnormalities for chromosomes 13, 18, 21, X, Y or any other chromosome, as well as other genetic abnormalities, such as but not limited to, microdeletion/microduplication syndromes, translocations, inversions, insertions, and other point or small size mutations. The genomic target-loci used for TACS design were selected based on their GC content and their distance from repetitive elements (minimum 50 bp away). TACS size can be variable. In one embodiment of the method the TACS range from 100-500 bp in size and are generated through a PCR-based approach as described below. The TACS were prepared by simplex polymerase chain reaction using standard Taq polymerase, primers designed to amplify the target-loci, and normal DNA used as template. The chromosomal regions used to design primers to amplify suitable loci on chromosomes 13, 18, 21 and X, to thereby prepare the pool of TACS for analysis of chromosomes 13, 18, 21 and X, are shown in Figure 2.

All custom TACS were generated using the following cycling conditions: 95°C for 3 minutes; 40 cycles at 95°C for 15 seconds, 60°C for 15 seconds, 72°C for 12 seconds; and 72°C for 12 seconds, followed by verification via agarose gel electrophoresis and purification using standard PCR clean up kits such as the Qiaquick PCR Purification Kit (Qiagen) or the NucleoSpin 96 PCR clean-up (Mackerey Nagel) or the Agencourt AMPure XP for PCR Purification (Beckman Coulter). Concentration was measured by Nanodrop (Thermo Scientific).

## Example 3: TACS Hybridization and Amplification

This example describes the steps schematically illustrated in Figure 1 of target capture by hybridization using TACS, followed by quantitation of captured sequences by Next Generation Sequencing (NGS).

## TACS Biotinylation

TACS were prepared for hybridization, as previously described (Koumbaris, G. et al. (2016) Clinical chemistry, 62(6), pp.848-855), starting with blunt ending with the Quick Blunting Kit (NEB) and incubation at room temperature for 30 minutes. Reaction products were subsequently purified using the MinElute kit (Qiagen) and were ligated with a biotin adaptor using the Quick Ligation Kit (NEB) in a 40μl reaction at RT for 15 minutes. The reaction products were purified with the MinElute kit (Qiagen) and were denatured into single stranded DNA prior to immobilization on streptavidin coated magnetic beads (Invitrogen).

## TACS Hybridization

Amplified libraries were mixed with blocking oligos (Koumbaris, G. et al. (2016) Clinical chemistry, 62(6), pp.848-855) (200 μM), 5μg of Cot-1 DNA (Invitrogen), 50 μg of Salmon Sperm DNA (Invitrogen), Agilent hybridization buffer 2x, Agilent blocking agent 10X, and were heated at 95°C for 3 minutes to denature the DNA strands. Denaturation was followed by 30 minute incubation at 37°C to block repetitive elements and adaptor sequences. The resulting mixture was then added to the biotinylated TACS. All samples were incubated in a rotating incubator for 12- 48 hours at 66°C. After incubation, the beads were washed as described previously and DNA was eluted by heating (Koumbaris, G. et al. (2016) Clinical chemistry, 62(6), pp.848-855). Eluted products were amplified using outer-bound adaptor primers. Enriched amplified products were pooled equimolarly and sequenced on a suitable platform.

If appropriate, amplification may be biased toward amplification of specific/desired sequences. In one embodiment of the method, this is performed when amplification is performed in the presence of sequences that hybridize to the undesired sequence of interest, and as such block the action of the polymerase enzyme during the process. Hence, the action of the amplification enzyme is directed toward the sequence of interest during the process.

### Example 4: Bioinformatics Sample Analysis

This example describes representative statistical analysis approaches for use in the methodology illustrated in Figure 1 ("analysis pipeline" in Figure 1).

Human Genome Alignment

For each sample, the bioinformatic pipeline routine described below was applied in order to align the sample's sequenced DNA fragments to the human reference genome. Targeted paired-end read fragments obtained from NGS results were processed to remove adaptor sequences and poor quality reads (Q-score<25) using the cutadapt software (Martin, M. et al. (2011) EMB.netJournal 17.1). The quality of the raw and/or processed reads as well as any descriptive statistics which aid in the assessment of quality check of the sample's sequencing output were obtained using the FastQC software (Babraham Institute (2015) FastQC) and/or other custom-built software. Processed reads which were at least 25 bases long were aligned to the human reference genome built hg19 (UCSC Genome Bioinformatics) using the Burrows-Wheel Alignment algorithm (Li, H. and Durbin, R. (2009) Bioinformatics 25:1754-1760) but other algorithms known to those skilled in the art may be used as well. If relevant, duplicate reads were removed post-alignment. Where applicable, sequencing output pertaining to the same sample but processed on separate sequencing lanes, was merged to a single sequencing output file. The removal of duplicates and merging procedures were performed using the Picard tools software suite (Broad Institute (2015) Picard) and/or the Sambamba tools software suite (Tarasov, Artem, et al. "Sambamba: fast processing of NGS alignment formats." Bioinformatics 31.12 (2015): 2032-2034.). A realignment procedure, using tools known to those in the art, may also be performed.

The above software analysis resulted in a final aligned version of a sequenced sample against the human reference genome and all subsequent steps were based on this aligned version. Information in terms of Short Nucleotide Polymorphisms (SNPs) at loci of interest was obtained using bcftools from the SAMtools software suite (Li, H. et al. (2009) Bioinformatics 25:2078-2079) and/or other software known to those skilled in the art. The read-depth per base, at loci of interest, was obtained using the mpileup option of the SAMtools software suite, from here on referred to as the mpileup file. Information pertaining to the size of the aligned fragments was obtained using the view option of the SAMtools software suite, from here on referred to as the fragment-sizes file and/or other software known to those skilled in the art.

The mpileup file and the fragment-sizes file were processed using custom-build application programming interfaces (APIs) written in the Python and R programming languages (Python Software Foundation (2015) Python; The R Foundation (2015) The R Project for Statistical Computing). The APIs were used to determine the ploidy state of chromosomes of interest, and/or other genetic abnormalities in regions of interest across the human genome, using a series of steps (collectively henceforth referred to as the "algorithm") and to also collect further descriptive statistics to be used as quality check metrics, such as but not limited to fetal fraction and/or fraction of interest quantification (collectively henceforth referred to as the "QC metrics").The APIs can also be used for the assessment of genetic abnormalities from data generated when applying the described method in cases of multiple gestation pregnancies, as well as other genetic abnormalities such as, but not limited to, microdeletions, microduplications, copy number variations, translocations, inversions, insertions, point mutations and mutational signatures.

QC Metrics

QC metrics were used to inspect an aligned sample's properties and decide whether the sample was suitable to undergo classification. These metrics were, but are not limited to:

(a) The enrichment of a sample. The patterns of enrichment are indicative of whether a sample has had adequate enrichment across loci of interest in a particular sequencing experiment (herein referred to as a "run"). To assess this, various metrics are assessed, non-limiting examples of which are:

(i) overall sample on-target read depth,

(ii) sample on-target sequencing output with respect to total mapped reads,

(iii) individual TACS performance in terms of achieved read-depth,

(iv) kurtosis and skewness of individual TACS enrichment,

(v) kurtosis and skewness moments that arise from all TACS,

(vi) fragment size distribution,

(vii) percentage of duplication,

(viii) percentage of paired reads and,

(ix) percentage of aligned reads,

if applicable.

The above checks are also taken into consideration with regards to GC-bias enrichment. Samples that fail to meet one or more of the criteria given above are flagged for further inspection, prior to classification.

(b) A sample's fetal fraction or fraction of interest. Samples with an estimated fetal fraction, or fraction of interest, that is below a specific threshold are not classified. Furthermore, if applicable the fraction of interest may be calculated using more than one method and concordance of results between estimation methods may be used as an additional QC prior to classification.

The Algorithm

The algorithm is a collection of data processing, mathematical and statistical model routines arranged as a series of steps. The algorithm's steps aim in deciding the relative ploidy state of a chromosome of interest with respect to all other chromosomes of the sequenced sample and is used for the detection of whole or partial chromosomal abnormalities for chromosomes 13, 18, 21, X, Y or any other chromosome, as well as other genetic abnormalities such as, but not limited to, microdeletion/microduplication syndromes and other point or small size mutations. As such the algorithm can be used, but is not limited to, the detection of whole or partial chromosomal abnormalities for chromosomes 13, 18, 21, X, Y or any other chromosome, as well as other genetic abnormalities such as, but not limited to, microdeletions, microduplications, copy number variations, translocations, inversions, insertions, point mutations and other mutational signatures. The algorithm carries out, but is not limited to, two types of assessments, one pertaining to the read-depth information of each sample and the other to the distribution of fragment-sizes, across TACS-specific regions. One or more statistical tests may be associated with each type of assessment, non-limiting examples of which are given in the statistical methods described herein.

In the case of read-depth associated tests, the algorithm compares sequentially the read-depth of loci from each chromosome of interest (herein referred to as the test chromosome) against the read-depth of all other loci (herein referred to as the reference loci) to classify its ploidy state. For each sample, these steps were, but are not limited to:

(a) Removal of inadequately sequenced loci. The read-depth of each locus was retrieved. Loci that have not achieved a minimum number of reads, were considered as inadequately enriched and were removed prior to subsequent steps.

(b) Genetic (GC-content) bias alleviation. The sequencing procedure may introduce discrepancies in read-depth across the loci of interest depending on their GC content. To account for such bias, a novel sequence-matching approach that increases both sensitivity and specificity to detect chromosomal aneuploidies was employed. The GC content of each locus on the test chromosome was identified and similar genetic loci were grouped together to form genetically matched groups. The procedure was repeated for the reference loci. Then, genetically matched groups from the test chromosome were conditionally paired with their genetically matched group counterparts on the reference chromosome(s). The groups may have any number of members. The conditionally matched groups were then used to assess the ploidy status of test chromosomes.

(c) Genetic abnormality determination. Ploidy status determination, or other genetic abnormalities of interest such as but not limited to microdeletions, microduplications, copy number variations, translocations, inversions, insertions, point mutations and other mutational signatures was achieved using a single statistical method and/or a weighted score approach on the result from the following, but not limited to, statistical methods:

Statistical Method 1: The differences in read-depth of the conditionally paired groups were tested for statistical significance using the t-test formula:

$$t = \frac{\hat{x} - \mu}{s/\sqrt{n}}$$

where t is the result of the t-test, $\hat{x}$ is the average of the differences of the conditionally paired groups, $\mu$ is the expected read-depth and is set to a value that represents insignificant read-depth differences between the two groups, s the standard deviation of the differences of the conditionally paired groups and n the length of the vector of the conditionally paired differences. The magnitude of the t-score was then used to identify evidence, if any, against the null hypothesis of same ploidy between reference and test chromosomes. Specifically, t>=c1 (where c1 is a predefined threshold belonging to the set of all positive numbers) shows evidence against the null hypothesis of no difference.

Statistical Method 2: Bivariate nonparametric bootstrap. The bootstrap method depends on the relationship between the random variables X (read-depth of reference loci) and Y (read-depth of test loci). Here, the read depth of baits on the reference group (random variable denoted by X) were treated as the independent covariate. The first step of the iterative procedure involved random sampling with replacement (bootstrapping) of the read-depths of loci on the reference chromosomes, i.e. (x1,g1),...,(xn,gn), where the parameter g is known and denotes the GC-content of the chosen bait. Then, for each randomly selected reference bait (xi,gi), a corresponding read depth was generated for a genetically matched locus i.e., (y1,g1),...,(yn,gn). Thus, the bivariate data (x1,y1), (x2,y2),...,(xn,yn) was arrived at, which was conditionally matched on their GC-content (parameter gi). The differences between the read depths of the genetically matched bootstrapped values xi and yi were used to compute the statistic of interest in each iteration. In one embodiment this statistical measure can be, but is not limited to, the mode, mean or median of the recorded differences, and/or multiples thereof. The procedure was repeated as necessary to build up the distribution of the statistic of interest from these differences. The sample was assigned a score that corresponds to a specific percentile of the built distribution (e.g. 5th percentile). Under the null hypothesis the ploidy between chromosomes in the reference and test groups is not different. As such, samples whose score for a particular chromosome, was greater than a predefined threshold, say c2, were classified as statistically unlikely to have the same ploidy. Other statistical measures may be employed.

Statistical Method 3: Stratified permutation test. The statistic of interest is the read-depth estimate of the test chromosome, denoted by $\hat{Y}_{obs}$, which is calculated using all loci of the test chromosome's genetically matched groups as follows:

$$\hat{Y}_{obs} = \frac{\sum_{j=1}^{j=T}\sum_{i=1}^{i=Nj} y_{ij}}{\sum_{j=1}^{j=T} Nj}$$

where $y_{ij}$ is the read-depth of locus $i$ part of the genetically matched group $j$ (i.e., loci belonging to a specific group based on their GC-content), $Nj$ is the number of test loci part of the genetically matched group j and T the number of genetically matched groups.

Subsequently, a null distribution to test $\hat{Y}_{obs}$ was built. To do so, for each group j, the test and reference loci were combined (exchangeability under the null hypothesis), and each group j was sampled randomly up to $Nj$ times without replacement (stratified permutation). This created a vector of values, say yi, and from this the vector's average value, say $\acute{y}_i$, was calculated. The procedure was repeated as necessary to build the null distribution. Finally $\hat{Y}_{obs}$, was studentised against the null distribution using the formula:

$$Z_{Yobs} = \frac{\hat{Y}_{obs}-\acute{Y}}{\sigma_Y}$$

where $\hat{Y}$ and $\sigma_Y$ are the first and square root of the second moment of all permuted $\acute{y}_i$ statistic values. Samples whose $Z_{Yobs}$ was greater than a predefined threshold, say c3, were statistically less likely to have the same ploidy in the reference and test groups.

In the case of fragment-size associated tests, the algorithm computes the proportion of small-size fragments found in test-loci and compares it with the respective proportion in reference-loci as described in Statistical Method 4 below.

Statistical Method 4: Fragment Size Proportions. For each sample the number and size of fragments aligned onto the human reference genome at the corresponding TACS coordinates, is extracted. The data is subsequently filtered so as to remove fragment-sizes considered statistical outliers using the median outlier detection method. Specifically, outliers are defined as those fragments whose size is above or below the thresholds, $F_{thr}$, set by equation:

$$F_{thr} = F_{median} \pm (X \times IQR)$$

where $F_{median}$ is the median fragment-size of all fragments of a sample, X is a variable that can take values from the set of R +, and *IQR* is the interquartile range of fragment sizes. Thereafter, a binomial test of proportions is carried out to test for supporting evidence against the null hypothesis, H0, where this is defined as:

H0: The proportion of small fragments of the test-region is not different from the proportion of small-fragments of the reference region.

In various embodiments of the invention, small fragments are defined as those fragments whose size is less than or equal to a subset of $Z+$, that is upper-bounded by 160bp. If the set of all TACS are defined as T, then the test region can be any proper subset S which defines the region under investigation, and the reference region is the relative complement of S in T. For example, in one embodiment of the invention, the set S is defined by all TACS-captured sequences of chromosome 21 and thus the reference set is defined by all TACS-captured fragments on the reference chromosomes, and/or other reference loci

The alternative hypothesis, H1, is defined as:

H1: The proportion of small fragments of the test-region is not equal to the proportion of test fragments of the reference region.

As such, and taking into account continuity correction, the following score is computed (Brown et. al, Harrel):

$$W_{test} = (\acute{p} - p_{ref}) \Big/ \sqrt{\frac{\acute{p}(1 - \acute{p})}{N_{test}}}$$

where

$$\acute{p} = \frac{(\acute{F} + 0.5)}{(N_{test} + 1)}$$

$$p_{ref} = \frac{(F_{ref} + 0.5)}{(N_{ref} + 1)}$$

$\acute{F}$ is the number of small-size fragments on the test-region, $F_{ref}$ the number of small size fragments on the reference region, $N_{test}$ the number of all fragments on the test region and $N_{ref}$ the number of all fragments on the reference region.

For each sample, the algorithm tests sequentially the proportion of fragment sizes of regions under investigation (for example, but not limited to, chromosome 21, chromosome 18, chromosome 13 or other (sub)chromosomal regions of interest) against reference regions; those not under investigation at the time of testing. For each sample a score is assigned for each test. Scores above a set-threshold, say c4, provide evidence against the null hypothesis.

Weighted Score method 1: In one embodiment of the method, a weighted score was attributed to each sample s, computed as a weighted sum of all statistical methods using the formula:

$$V_s(R, F) = z_1 max\{R_s, F_s\} + (1 - z_1) min\{R_s, F_s\}$$

where $R_s$ is the run-specific corrected score arising from a weighted contribution of each read-depth related statistical method for sample s and is defined as:

$$R_s = \frac{(\sum_i w_i S_{is} - \acute{R}_r)}{\sigma_r}$$

and $\acute{R}_r$ is the run-specific median value calculated from the vector of all unadjusted read-depth related weighted scores that arise from a single sequencing run, and $\sigma_r$ is a multiple of the standard deviation of R scores calculated from a reference set of 100 euploid samples. The terms $max\{R_s, F_s\}$ and $min\{R_s, F_s\}$ denote the maximum and minimum values of the bracketed set, respectively.

$F_s$ is the run-specific corrected score arising from the fragment-size related statistical method and is defined as:

$$F_s = \frac{\left(W_{test} - \acute{R}_f\right)}{\sigma_f}$$

where $W_{test}$ is as defined earlier, $\acute{R}_f$ is the run specific median calculated from the vector of all unadjusted fragment-related statistical scores that arise from a single sequencing run, and $\sigma_f$ is a multiple of the standard deviation of $F$ scores calculated from a reference set of 100 euploid samples.

A unique classification score of less than a predefined value indicates that there is no evidence from the observed data that a sample has a significant risk of aneuploidy.

Weighted Score method 2: In another embodiment of the method, the weighted score arising from the statistical methods described above was used to assign each sample a unique genetic abnormality risk score using the formula:

$$R(t, c) = \sum_{j=0}^{j=N} w_j \frac{t_j}{c_j}$$

where R is the weighted score result, $w_j$ the weight assigned to method j, $t_j$ the observed score resulting from method $j$, and $c_j$ the threshold of method $j$.

A unique classification score of less than a predefined value indicates that there is no evidence from the observed data that a sample has a significant risk of aneuploidy.

Since all read depths from baits in the reference group were assumed to be generated from the same population, and in order to have a universal threshold, run-specific adjustments were also employed to alleviate run-specific biases.

The aforementioned method(s), are also suitable for the detection of other genetic abnormalities, such as but not limited to, subchromosomal abnormalities. A non-limiting example is the contiguous partial loss of chromosomal material leading to a state of microdeletion, or the contiguous partial gain of chromosomal material leading to a state of microduplication. A known genetic locus subject to both such abnormalities is 7q11.23. In one embodiment of statistical method 1, synthetic plasma samples of 5%, 10% and 20% fetal material were tested for increased risk of microdeletion and/or microduplication states for the genetic locus 7q11.23.

For point mutations various binomial tests are carried out that take into consideration the fetal fraction estimate of the sample, f, the read-depth of the minor allele, **r,** and the total read-depth of the sequenced base, n. Two frequent, yet non-limiting examples involve assessment of the risk when the genetic abnormality is a recessive point mutation or a dominant point mutation.

In the non-limiting example of a recessive point mutation the null hypothesis tested is that both the mother and the fetus are heterozygous (minor allele frequency is 0.5) against the alternative in which the fetus is homozygous (minor allele frequency is 0.5-f/2). A small p-value from the corresponding likelihood ratio test would indicate evidence against the null. In the non-limiting example of a dominant point mutation the null hypothesis tested is that the mother and fetus are homozygous at the given position against the alternative in which only the fetus is heterozygous for the given position. A small p-value from the corresponding likelihood ratio test would indicate evidence against the null.

In addition to the above, fetal sex determination methods were also developed, with non-limiting examples given below. In one embodiment of the invention, fetal sex was assigned to a sample using a Poisson test using the formula:

$$Pr\left(r_y \le k\right) = e^{-\lambda} \sum_{i=0}^{i=k} \frac{\lambda^i}{i!}$$

where

$$\lambda = \frac{fB\mu}{2}$$

and f is the fetal fraction estimate of the sample, B is the number of target sequences on chromosome Y, $\mu$ is the read-depth of the sample and k is the sum of reads obtained from all targets B. The null hypothesis of the Poisson test was that the sample is male. A value of $Pr(r_y)$ less than a threshold $c_y$ was considered as enough evidence to reject the null hypothesis, i.e. the sample is not male. If any of the terms for computing $Pr(r_y)$ were unavailable, then the sample's sex was classified as NA (not available).

In another embodiment of the invention, fetal sex was assigned using the average read-depth of target sequences on chromosome Y. If the average read-depth of the target-sequences was over a predefined threshold, where such threshold may be defined using other sample-specific characteristics such as read-depth and fetal-fraction estimate, the fetal sex was classified as male. If the average read-depth was below such threshold then the sample was classified as female.

Fetal Fraction Estimation/Fraction of Interest Estimation

Several methods have been developed to estimate fetal fraction that can be applied to singleton and/or to multiple gestation pregnancies. As such, and dependent on the type of pregnancy, the fetal fraction estimate can be obtained from either method or as a weighted estimate from a subset and/or all developed methods. Some non-limiting examples are given below.

In one embodiment, a machine learning technique has been developed based on Bayesian inference to compute the posterior distribution of fetal DNA fraction using allelic counts at heterozygous loci in maternal plasma of singleton pregnancies. Three possible informative combinations of maternal/fetal genotypes were utilized within the model to identify those fetal DNA fraction values that get most of the support from the observed data.

Let $f$ denote the fetal DNA fraction. If the mother is heterozygous at a given genomic locus, the fetal genotype can be either heterozygous or homozygous resulting in expected minor allele frequencies at *0.5* and *0.5-f/2*, respectively. If the mother is homozygous and the fetus is heterozygous then the expected minor allele frequency will be *f/2*. A Markov chain Monte Carlo method (a Metropolis-Hastings algorithm) (The R Foundation (2015) The R Project for Statistical Computing) was used with either a non-informative or an informative prior (i.e. incorporate additional information such as gestational age, maternal weight etc.) to obtain a sequence of random samples from the posterior probability distribution of fetal DNA fraction that is based on a finite mixture model.

In another embodiment, the fetal fraction estimate is computed only from the fetus-specific minor allele frequency (MAF) cluster, i.e. the cluster formed when the mother is homozygous and the fetus is heterozygous for a given genomic locus. It is assumed that the mean value of the fetal fraction estimate is normally distributed as $N(2\acute{x}, \sigma_{\acute{x}})$, where $\acute{x}$ is the mean of the fetus-specific MAF, and $\sigma_{\acute{x}}$ is the standard deviation of the fetus-specific MAF. The fetal fraction estimate is then obtained from percentiles of the computed distribution, $N(2\acute{x}, \sigma_{\acute{x}})$.

For multiple gestation pregnancies, non-limiting examples of which include monozygotic and dizygotic twin pregnancies, triplet pregnancies and various egg and/or sperm donor cases, the fetal fraction can be estimated using information obtained from heterozygous genetic loci whose MAF value is less than a threshold, say $M_{thresh}$, and derived from potential fetus-specific SNPs. The ordinarily skilled artisan will appreciate that fetus specific SNPs can originate from any fetus, or from any possible combination of the fetuses or from all the fetuses of the gestation. As such, an algorithm that estimates the fetal fraction of the fetus with the smallest contribution to the total fetal content, by taking into account the combinatorial contribution of each fetus to the MAF values that define fetus-specific SNPs, and also allows for inhomogeneous contribution of fetal material to the total fetal content of plasma derived material has been developed. To this effect, a two-step approach is employed by the algorithm.

In one embodiment of the algorithm, the multiple gestation pregnancy under consideration is a dizygotic twin pregnancy. As a first step, the algorithmic implementation of the model utilizes all informative SNPs and allows for inhomogeneous fetal contribution that can be explained with a fold-difference in fetal fraction estimates of a set threshold, say cf. Specifically, if f1 and f2 represent the fetal fractions of fetus one and fetus two, and f1 <= f2, then the assumption is that f2 <= cf f1, with cf being a positive real number greater than or equal to 1. Under this assumption, the observed data D, defined as counts of the alternate and reference alleles at informative SNP loci, are believed to be generated from a mixture distribution of three Binomials (defined by parameters, f1/2, f2/2 and (f1+f2)/2), with the posterior distribution p(f1,f2|D) being proportional to the observational model which can be written as p(f1|f2,D) p(f2|D). The posterior distribution p(f1,f2|D) is sampled with an MCMC Metropolis-Hastings algorithm using a uniform prior. The empirical quantile approach is performed on the generated data array to infer the fetal fractions.

As a second step, the algorithm runs a model-based clustering algorithm (Finite Gaussian mixture modeling fitted via EM algorithm; R-package: mclust) to identify whether there exists a separate outlier SNP cluster which is believed to be centered around f1/2. Existence of such a cluster with a mean invalidating the cf >= f2/f1 assumption, leads to estimation of f1 using only SNPs part of the identified cluster.

The methods described above are suited to the determination of the fraction of any component of interest part of a mixed sample. As such, the methods are not to be understood as applicable only to the application of fetal fraction estimation and can be applied to the estimation of any component of interest part of a mixed sample, as outlined in Example 6.

## Example 5: Target Enrichment Using Families of TACS

In this example, a family of TACS, containing a plurality of members that all bind to the same target sequence of interest, was used for enrichment, compared to use of a single TACS binding to a target sequence of interest. Each member of the family of TACS bound to the same target sequence of interest but had different start/stop coordinates with respect to a reference coordinate system for that target sequence (e.g., the human reference genome, built hg19). Thus, when aligned to the target sequence, the family of TACS exhibit a staggered binding pattern, as illustrated in Figure 3. Typically, the members of a TACS family were staggered approximately 5-10 base pairs.

A family of TACS containing four members (i.e., four sequences that bound to the same target sequence but having different start and/or stop positions such that the binding of the members to the target sequence was staggered) was prepared. Single TACS hybridization was also prepared as a control. The TACS were fixed to a solid support by labelling with biotin and binding to magnetic beads coated with a biotin-binding substance (e.g., streptavidin or avidin) as described in Example 3. The family of TACS and single TACS were then hybridized to a sequence library, bound sequences were eluted and amplified, and these enriched amplified products were then pooled equimolarly and sequenced on a suitable sequencing platform, as described in Example 3.

The enriched sequences from the family of TACS sample and the single TACS sample were analyzed for read-depth. The results are shown in Figures 4A and 4B. As shown in Figure 4A, target sequences of interest enriched using the family of four TACS (red dots) exhibited a fold-change in read-depth when compared to control sequences that were subjected to enrichment using only a single TACS (blue dots). Fold-change was assessed by normalizing the read-depth of each locus by the average read-depth of a sample, wherein the average read-depth was calculated from all loci enriched with a single TACS. As shown in Figure 4B, an overall 54.7% average increase in read-depth was observed using the family of four TACS.

This example demonstrates that use of a family of TACS, as compared to a single TACS, results in significantly improved enrichment of a target sequence of interest resulting in significantly improved read-depth of that sequence.

## Example 6: Tumor Biomarker Detection in Reference Material

In this example, the TACS methodology, illustrated in Figure 1, was used for the detection of tumor biomarkers in certified reference material known to harbor particular genetic mutations that are tumor biomarkers. For detection of the tumor biomarker sequences of interest, families of TACS, as described in Example 5, were used.

A sample of certified reference material harboring known tumor-associated genetic mutations was commercially obtained and samples were prepared to simulate tumor loads of 0.1%, 1.0% and 5.0%.

The samples were subjected to the TACS methodology illustrated in Figure 1 using families of TACS that bound to the following tumor-associated genetic mutations: EGFR_6240, KRAS_521, EGFR_6225, NRAS_578, NRAS_580, PIK3CA_763, EGFR_13553, EGFR_18430.

Following amplification and sequence of the TACS-enriched products, data analysis was performed as follows. Sequencing products were processed to remove adaptor sequences and poor quality reads. Reads whose length was at least 25 bases long post adaptor-removal were aligned to either:

(a) the human reference genome built hg19, or

(b) an artificially created genome based on built hg19 which contains only sequences of interest.

If relevant, duplicate reads were removed post-alignment. Where applicable, sequencing output pertaining to the same sample but processed on separate sequencing lanes was merged to a single sequencing output file. Local realignment of the data, using tools known in the art, may also be performed. The above software analysis provided a final aligned version of a sequenced sample against the reference genome, defined here as the final BAM file, where information can be extracted from it in terms of Short Nucleotide Polymorphisms (SNPs), Single Nucleotide Variants (SNVs) and other genetic variations with respect to a reference sequence at loci of interest, read-depth per base and the size of aligned fragments. Various available tools known to those skilled in the art, such as but not limited to bcftools, which is part of the samtools software suite, or varDict can be used to collect SNP information from the final BAM file. Such information concerns the sequence and number of times each variant is present in a sequenced sample was detected and was used to

(a) infer the presence of a genetic mutation, and

(b) to estimate the tumor load using the fetal-fraction estimation/fraction of interest estimation method described in Example 4.

In addition to the detection of the genetic mutation, statistical confidence was ascribed to a detected mutation using the estimated tumor load of the sample and the read-depth of each of the detected variants at a given position using binomial statistics. More than one test may be employed from which one can compute the probability of obtaining the sequenced information, or obtain a 95% confidence interval which describes a range of possible read-depths for the genetic mutation, or whether the obtained proportion of reads which can be ascribed to the genetic mutation is consistent with what would be expected at the given tumor load. A suitable binomial test of proportions is described in Example 4 (in the context of classification of chromosomal abnormalities).

The results are shown in Figure 5. The line illustrates the expected minor allele frequency (MAF) for each percent (%) tumor load. The bars (x-axis) illustrate the detected MAF (y-axis) for each sample for the indicated genetic mutations. Two technical replicates are shown for the reference material.

The data demonstrates that the TACS methodology successfully detected the tumor-associated genetic mutations EGFR_6240, KRAS_521, EGFR_6225, NRAS_578, NRAS_580, PIK3CA_763, EGFR_13553 and EGFR_18430 at the expected tumor loads of 1.0% and 5.0%. Mutations EGFR_6240, NRAS_578, PIK3CA_763, EGFR_13553 and EFGR_18430 were also successfully detected at 0.1% tumor load.

Accordingly, this example demonstrates the successful detection of a large panel of different tumor biomarkers using the TACS methodology at tumor loads as low as 0.1%.

## Example 7: Tumor Biomarker Detection in Patient Samples

In this example, the TACS methodology, illustrated in Figure 1, was used for the detection of tumor biomarkers in tumor tissue and blood plasma samples from untreated cancer patients with confirmed diagnosis. For detection of the tumor biomarker sequences of interest, families of TACS, as described in Example 5, were used.

Matched pairs of peripheral blood and tumor tissue samples from untreated cancer patients were used to further validate the performance of the TACS methodology for tumor biomarker detection for a patient harboring mutation PIK3CA E545K (Patient 1) and for a patient harboring mutation TP53 K139 (Patient 2). The results are shown in Figure 6.

As shown in Figure 6, application of the TACS methodology to a tissue sample obtained from Patient 1 harboring mutation PIK3CA E545K (top bars) provided a variant allele frequency (VAF) percentage (i.e., the percentage that the genetic mutation is present instead of the normal allele) of ~62%. Plasma obtained from peripheral blood of Patient 1 was processed according to the method described in Example 1 and provided a 6.05 % VAF. Similarly, application of the TACS methodology to samples obtained from Patient 2 harboring mutation TP53 K139 (bottom bars) provided a VAF of ~60% for tumor tissue and a VAF of 4.88 % for plasma obtained from a peripheral blood sample.

Accordingly, this example demonstrates the successful detection of tumor biomarkers in cancer patient samples, in both tumor tissue samples and plasma samples, thereby demonstrating the suitability of the TACS methodology for tissue biopsy and for non-invasive tumor biomarker detection using liquid biopsy.

## Example 8: Detection of Mutational Profiles

Given the ability of the TACS methodology illustrated in Figure 1 to detect a number of somatic single nucleotide variations (SNVs), these can be examined in the context of motifs, also referred to as mutational profiles. Most somatic mutations in tumors can be considered as passengers and may not be associated with pathogenesis if examined individually. Nonetheless, examining the profile of detected mutations as a whole can be useful in determining and/or detecting a pathogenesis-associated mutational profile. Various algorithms have been developed to decompose known mutational motifs operative in many cancer types. Alternatively, other metrics utilizing specific characteristics such as the type of mutations detected in the context of their neighboring bases can be utilized to this effect. The developed algorithms can infer the most likely scenario(s) that explain the observed data. Decomposition of the number and types of known mutational patterns/signatures that have, most likely, generated the observed mutational profile has been achieved using, but not limited to, the Lawson-Hanson non-negative least squares algorithm.

Figure 7 shows the observed pattern of somatic SNVs for breast cancer using data downloaded from the COSMIC database. The x-axis shows a single base mutation observed in cancer in the context of its neighboring sequences. For example A[C>A]T describes the mutation of Cytosine (C ) to Adenine (A) where the upstream sequence is Adenine and the downstream sequence is Thymine. The y-axis shows the frequency of occurrence of this mutation in breast cancer.

Figure 8 illustrates the results of a simulations study where mutational profiles were randomly generated by sampling a subset of SNVs each time, from data available in the COSMIC database, thereby simulating individuals. The simulated data were then subjected to the decomposition algorithms described above in order to detect the likely underlying mutational motifs. The bars indicate the average estimated frequency of the known mutational breast signatures computed from a data set of 10000 simulations. The developed algorithm shows evidence of detection of the mutational profiles, thereby demonstrating that detection of mutational profiles, or motifs, is possible using the developed algorithms.

## Example 9: Fragment Size Based Tests

There is evidence from the literature that specific types of cancer can be characterized by and/or associated with fragments in the plasma having a smaller size than the expected size of fragments originating from healthy tissues (Jiang et al, (2015), Proceedings of the National Academy of Sciences, 112(11), ppE1317-E1325). Thus, a fragments-size based test can be utilized to detect the presence of somatic copy number variations in individuals suspected of having cancer. To this effect, a binomial test of proportions, as described Example 4, can be used for the detection of increased presence of nucleic acid material originating from non-healthy tissue (e.g., tumor tissue) based on fragment size. In particular, under the null hypothesis that the distribution of fragment sizes originating from both healthy and non-healthy cells (for example, but not limited to cancerous cells) is the same, a binomial test for proportions (as described in Example 4) using continuity correction can be utilized to quantify any evidence against it.

The same hypothesis holds true for fragments originating from the placenta/fetus. Specifically, placenta derived fragments are generally of smaller size when compared to fragments originating from maternal tissues/cells. Accordingly, assessment of the fragment size-based test was performed using maternal plasma samples (i.e., mixed samples where cell free DNA is of maternal and fetal origin). The size of fragments that have aligned to TACS-enriched regions can be obtained from the aligned data. Subsequently, the proportion of fragments under a specific threshold from a test region is compared respective proportion of fragments from a reference region for evidence against the null hypothesis H0,

H0: The proportion of small fragments of the test-region is not different from the proportion of small-fragments of the reference region.

Figure 9 shows results when applying the fragment sizes method to the mixed sample containing maternal and fetal DNA. The black dots are individual samples. The x-axis shows the sample index. The y-axis shows the score result of the fragments-based method. A score result greater than the one indicated by the threshold, illustrated as a grey line, indicates a deviation from the expected size of fragments illustrating the presence of aneuploidy. The results demonstrate that an aneuploid sample, having an estimated fetal fraction equal to 2.8%, was correctly identified, illustrating that fragments-based detection may be used to detect abnormalities in mixed samples with low signal-to-noise ratio (e.g., as is the case in detection of cancer).

Accordingly, this example demonstrates the successful ability of the fragments-based detection method in detecting genetic abnormalities in mixed samples with low signal-to-noise ratios, thereby demonstrating the suitability of the fragments-based test for analysis of either cancer samples for oncology purposes or maternal samples for NIPT.

Since small-sized fragments are associated with fragments from non-healthy tissues (Jiang et al, (2015), Proceedings of the National Academy of Sciences, 112(11), ppE1317-E1325) they can also be leveraged for the detection of small-sized mutations, such as point mutations and mutational signatures. For example, one may only use small-sized fragments in Variant Allele Frequency estimation as described in examples 6-9, thereby increasing the signal-to-noise ratio.

## Example 10: Use of the method for tissue biopsies

Five FFPE samples from Breast carcinoma and 13 tissue samples (fresh/frozen and FFPE) from lung adenocarcinoma were subjected to the method and the mutational status was successfully detected. The data are presented below.

## Breast Carcinoma:

| Type of specimen | Patient ID | Gene | CDS mutation | AA change | COMSIC ID | MAF% |
|---|---|---|---|---|---|---|
| FFPE | BCa1 | TP53 | c.569delC | p.190fs*57 | COSM100030 | 17.39 |
| | | TP53 | c.559+25G>A | intronic | COSM45841 | 22.22 |
| FFPE | BCa2 | PIK3CA | c.1624G>A | p.E542K | COSM760 | 32.15 |
| | | FLT3 | c.2501G>A | p.R834Q | COSM28047 | 2.01 |
| FFPE | BCa3 | ALK | c.3515+18C>T | intronic | COSM28496 | 49.43 |
| FFPE | BCa4 | AKT1 | c.49G>A | p.E17K | COSM33765 | 42.86 |
| FFPE | BCa5 | PIK3CA | c.1633G>A | E545K | COSM763 | 62.79 |
| | | PTEN | c.1-9C>G | intronic | COSM5915 | 26.19 |

| | | TP53 | c.415A>T | p.K139* | COSM44678 | 60.63 |
|---|---|---|---|---|---|---|

**Lung adenocarcinoma:**

| Type of specimen | Sample ID | COSMIC ID | NIPD method | Independent method |
|---|---|---|---|---|
| Fresh/frozen | LCA1 | COSM459 | 0.0245 | 0.0261 |
| | | COSM527 | 0.036 | 0.0378 |
| Fresh/frozen | LCA2 | COSM763 | 0.1427 | 0.1558 |
| Fresh/frozen | LCA3 | COSM522 | 0.3815 | 0.3492 |
| Fresh/frozen | LCA4 | COSM521 | 0.106 | 0.0923 |
| Fresh/frozen | LCA6 | COSM3675521 | 0.1387 | 0.1026 |
| Fresh/frozen | LCA13 | MET c.3028+1G>T | 0.0126 | Not covered |
| Fresh/frozen | LCA15 | COSM27887 | 0.1352 | Not covered |
| | | COSM521 | 0.2871 | 0.3185 |
| Fresh/frozen | LCA21 | COSM763 | 0.2798 | 0.3128 |
| FFPE | LCA36 | COSM6224 | 0.2431 | 0.1847 |
| FFPE | LCA40 | COSM6225 | 0.08 | 0.1148 |
| FFPE | LCA45 | COSM6223 | 0.2098 | 0.2456 |
| FFPE | LCA47 | COSM12370 | 0.6295 | 0.5719 |
| FFPE | LCA48 | COSM522 | 0.2649 | 0.4032 |

For the lung cancer data, results were compared with data obtained for the same tissue samples with an independent method. For the genomic regions covered by both methods we observed 100% concordance.

## Claims

1. A method of diagnosing cancer in a subject suspected of having cancer based on the detection of at least one tumor biomarker sequence in a DNA sample from said subject, wherein the DNA sample is a blood sample or urine sample, and comprises cell free tumor DNA (cftDNA),
wherein the method comprises:

(a) preparing a sequencing library from the DNA sample;
(b) hybridizing the sequencing library to a pool of double-stranded TArget Capture Sequences (TACS) that bind to one or more tumor biomarker sequences of interest, wherein:

(i) the pool of TACS comprises a plurality of TACS families directed to different tumor biomarker sequences of interest, wherein each TACS family comprises a plurality of member sequences, wherein each member sequence binds to the same tumor biomarker sequence of interest but has different start and/or stop positions with respect to a reference coordinate system for the tumor biomarker sequence of interest, wherein the start and/or stop positions are staggered by 5-10 base pairs;

(ii) each member sequence within the pool of TACS is between 150-260 base pairs in length, each member sequence having a 5' end and a 3' end;

(iii) each member sequence binds to the tumor biomarker sequence of interest at least 50 base pairs away, on both the 5' end and the 3' end, from regions harboring Copy Number Variations (CNVs), Segmental duplications or repetitive DNA elements; and

(iv) the GC content of the pool of TACS is between 19% and 80%, as determined by calculating the GC content of each member within the pool of TACS;

(c) isolating members of the sequencing library that bind to the pool of TACS to obtain an enriched library;

(d) amplifying and sequencing the enriched library; and

(e) statistically analyzing the enriched library sequences for the presence or absence of the at least one tumor biomarker sequence, thereby diagnosing the patient.

2. The method according to claim 1, wherein the pool of TACS comprises at least 50 different TACS families.

3. The method according to any one of the preceding claims, wherein each TACS family comprises at least 3 member sequences.

4. The method according to any one of the preceding claims, wherein the TACS are biotinylated and are bound to streptavidin-coated magnetic beads.

5. The method according to any one of the preceding claims, wherein the pool of TACS binds to at least one tumor biomarker sequence selected from the genes listed in table 1.

6. The method according to any one of the preceding claims, wherein amplification of the enriched library is performed in the presence of blocking sequences that inhibit amplification of wild-type sequences.

7. The method according to any one of the preceding claims used to determine and/or detect mutational profiles.

8. A kit comprising a container comprising a pool of TArget Capture Sequences (TACS),

wherein the pool of TACS comprises a plurality of TACS families directed to different tumor biomarker sequences of interest,

wherein each TACS family comprises a plurality of member sequences,

wherein each member sequence binds to the same tumor biomarker sequence of interest but has different start and/or stop positions with respect to a reference coordinate system for the tumor biomarker sequence of interest, wherein the start and/or stop positions are staggered by 5-10 base pairs,

and further wherein:

(i) each member sequence within each TACS family is between 150-260 base pairs in length, each member sequence having a 5' end and a 3' end;

(ii) each member sequence binds to the same genomic sequence of interest at least 50 base pairs away, on both the 5' end and the 3' end, from regions harboring Copy Number Variations (CNVs), Segmental duplications or repetitive DNA elements; and

(iii) the GC content of the pool of TACS is between 19% and 80%, as determined by calculating the GC content of each member within each family of TACS.

**Patentansprüche**

1. Verfahren zur Diagnose von Krebs bei einem Patienten, bei dem der Verdacht auf Krebs besteht, basierend auf dem Nachweis mindestens einer Tumor-Biomarker-Sequenz in einer DNA-Probe des Patienten, wobei die DNA-Probe eine Blutprobe oder Urinprobe ist und zellfreie Tumor-DNA (cftDNA) umfasst, wobei das Verfahren folgendes umfasst:

(a) Herstellen einer Sequenzierungsbibliothek aus der DNA-Probe;

(b) Hybridisieren der Sequenzierungsbibliothek mit einem Pool von doppelsträngigen TArget Capture Sequences (TACS), die an eine oder mehrere Tumor-Biomarker-Sequenzen von Interesse binden, wobei:

(i) der Pool von TACS eine Vielzahl von TACS-Familien umfasst, die auf verschiedene Tumor-Biomarker-Sequenzen von Interesse gerichtet sind, wobei jede TACS-Familie eine Vielzahl von Mitgliedssequenzen umfasst, wobei jede Mitgliedssequenz an dieselbe Tumor-Biomarker-Sequenz von Interesse bindet, jedoch unterschiedliche Start- und/oder Stopp-Positionen in Bezug auf ein Referenzkoordinatensystem für die Tumor-Biomarker-Sequenz von Interesse aufweist, wobei die Start- und/oder Stopp-Positionen um 5-10 Basenpaare versetzt sind;

(ii) jede Mitgliedssequenz innerhalb des Pools von TACS eine Länge zwischen 150 und 260 Basenpaaren aufweist, wobei jede Mitgliedssequenz ein 5'-Ende und ein 3'-Ende aufweist;

(iii) jede Mitgliedssequenz an die Tumor-Biomarker-Sequenz von Interesse mindestens 50 Basenpaar entfernt sowohl am 5'-Ende als auch am 3'-Ende von Regionen bindet, die Kopienzahlvariationen (CNVs), segmentale Duplikationen oder repetitive DNA-Elemente aufweisen; und

(iv) der GC-Gehalt des TACS-Pools zwischen 19% und 80% liegt, bestimmt durch Berechnung des GC-Gehalts jedes Mitglieds innerhalb des TACS-Pools;

(c) Isolieren von Mitgliedern der Sequenzierungsbibliothek, die an den Pool von TACS binden, um eine angereicherte Bibliothek zu erhalten;

(d) Amplifizieren und Sequenzieren der angereicherten Bibliothek; und

(e) Statistisches Analysieren der Sequenzen der angereicherten Bibliothek auf das Vorhandensein oder Fehlen der mindestens einen Tumor-Biomarker-Sequenz, um so den Patienten zu diagnostizieren.

2. Verfahren nach Anspruch 1, wobei der Pool von TACS mindestens 50 verschiedene TACS-Familien umfasst.

3. Verfahren nach einem der vorherigen Ansprüche, wobei jede TACS-Familie mindestens 3 Mitgliedssequenzen umfasst.

4. Verfahren nach einem der vorherigen Ansprüche, wobei die TACS biotinyliert sind und an Streptavidin-beschichtete Magnetkügelchen gebunden sind.

5. Verfahren nach einem der vorherigen Ansprüche, wobei der Pool von TACS an mindestens eine Tumor-Biomarker-Sequenz bindet, die aus den in Tabelle 1 aufgeführten Genen ausgewählt ist.

6. Verfahren nach einem der vorherigen Ansprüche, wobei die Amplifikation der angereicherten Bibliothek in Gegenwart von Blockierungssequenzen durchgeführt wird, die die Amplifikation von Wildtyp-Sequenzen hemmen.

7. Verfahren nach einem der vorherigen Ansprüche, das zur Bestimmung und/oder zum Nachweis von Mutationsprofilen verwendet wird.

8. Kit, umfassend einen Behälter, der einen Pool von TArget Capture Sequences (TACS) enthält,

wobei der Pool von TACS eine Vielzahl von TACS-Familien umfasst, die an verschiedene Tumor-Biomarker-Sequenzen von Interesse gerichtet sind,
wobei jede TACS-Familie eine Vielzahl von Mitgliedssequenzen umfasst,
wobei jede Mitgliedssequenz an dieselbe Tumor-Biomarker-Sequenz von Interesse bindet, jedoch unterschiedliche Start- und/oder Stopp-Positionen in Bezug auf ein Referenzkoordinatensystem für die Tumor-Biomarker-Sequenz von Interesse aufweist, wobei die Start- und/oder Stopp-Positionen um 5-10 Basenpaare versetzt sind, und wobei ferner:

(i) jede Mitgliedssequenz innerhalb jeder TACS-Familie eine Länge zwische 150 und 260 Basenpaaren aufweist, wobei jede Mitgliedssequenz ein 5'-Ende und ein 3'-Ende aufweist;

(ii) jede Mitgliedssequenz an dieselbe genomische Sequenz von Interesse bindet, die mindestens 50 Basenpaare entfernt ist, sowohl am 5'-Ende als auch am 3'-Ende, von Regionen mit Kopienzahlvariationen (CNVs), segmentalen Duplikationen oder repetitiven DNA-Elementen; und

(iii) der GC-Gehalt des TACS-Pools zwischen 19% und 80% liegt, bestimmt durch Berechnung des GC-Gehalts jedes Mitglieds innnerhalb jeder TACS-Familie.

**Revendications**

1. Procédé de diagnostic du cancer chez un sujet suspecté d'être atteint d'un cancer, basé sur la détection d'au moins une séquence biomarqueur tumoral dans un échantillon d'ADN prélevé sur ledit sujet, dans lequel l'échantillon d'ADN est un échantillon de sang ou d'urine et comprend de l'ADN tumoral libre (cftDNA),
   dans lequel le procédé comprend :

   (a) la préparation d'une bibliothèque de séquençage à partir de l'échantillon d'ADN ;
   (b) l'hybridation de la bibliothèque de séquençage à un pool de séquences de capture de cibles (TACS) double brin qui se lient à une ou plusieurs séquences de biomarqueurs tumoraux d'intérêt, dans lequel :

   (i) le pool de TACS comprend une pluralité de familles de TACS dirigées vers différentes séquences de biomarqueurs tumoraux d'intérêt, dans lequel chaque famille de TACS comprend une pluralité de séquences membres, dans lequel chaque séquence membre se lie à la même séquence de biomarqueur tumoral d'intérêt mais a des positions de départ et/ou d'arrêt différentes par rapport à un système de coordonnées de référence pour la séquence de biomarqueur tumoral d'intérêt, dans lequel les positions de départ et/ou d'arrêt sont décalées de 5 à 10 paires de bases ;
   (ii) chaque séquence membre du pool de TACS a une longueur comprise entre 150 et 260 paires de bases, chaque séquence membre ayant une extrémité 5' et une extrémité 3' ;
   (iii) chaque séquence membre se lie à la séquence de biomarqueur tumoral d'intérêt à au moins 50 paires de bases de distance, à la fois à l'extrémité 5' et à l'extrémité 3', des régions abritant des variations du nombre de copies (CNV), des duplications segmentaires ou des éléments d'ADN répétitifs ; et
   (iv) la teneur en GC du pool de TACS est comprise entre 19 % et 80 %, telle que déterminée en calculant la teneur en GC de chaque membre du pool de TACS ;

   (c) isoler les membres de la bibliothèque de séquençage qui se lient au pool de TACS afin d'obtenir une bibliothèque enrichie ;
   (d) amplifier et séquencer la bibliothèque enrichie ; et
   (e) analyser statistiquement les séquences de la bibliothèque enrichie pour déterminer la présence ou l'absence d'au moins une séquence de biomarqueur tumoral, diagnostiquant ainsi le patient.

2. Procédé selon la revendication 1, dans lequel le pool de TACS comprend au moins 50 familles de TACS différentes.

3. Procédé selon l'une quelconque des revendications précédentes, dans lequel chaque famille de TACS comprend au moins 3 séquences membres.

4. Procédé selon l'une quelconque des revendications précédentes, dans lequel les TACS sont biotinylés et liés à des billes magnétiques recouvertes de streptavidine.

5. Procédé selon l'une quelconque des revendications précédentes, dans lequel le pool de TACS se lie à au moins une séquence de biomarqueur tumoral sélectionnée parmi les gènes répertoriés dans le tableau 1.

6. Procédé selon l'une quelconque des revendications précédentes, dans lequel l'amplification de la bibliothèque enrichie est réalisée en présence de séquences bloquantes qui inhibent l'amplification des séquences de type sauvage.

7. Procédé selon l'une quelconque des revendications précédentes utilisé pour déterminer et/ou détecter des profils mutationnels.

8. Kit comprenant un récipient contenant un pool de séquences de capture de cibles (TACS), dans lequel le pool de TACS comprend une pluralité de familles de TACS dirigées vers différentes séquences de biomarqueurs tumoraux d'intérêt,

   dans lequel chaque famille de TACS comprend une pluralité de séquences membres, dans lequel chaque séquence membre se lie à la même séquence de biomarqueur tumoral d'intérêt mais a des positions de départ et/ou d'arrêt différentes par rapport à un système de coordonnées de référence pour la séquence de biomarqueur tumoral d'intérêt, dans lequel les positions de départ et/ou d'arrêt sont décalées de 5 à 10 paires de bases, et dans lequel, en outre :

(i) chaque séquence membre au sein de chaque famille TACS a une longueur comprise entre 150 et 260 paires de bases, chaque séquence membre ayant une extrémité 5' et une extrémité 3' ;

(ii) chaque séquence membre se lie à la même séquence génomique d'intérêt à au moins 50 paires de bases, à la fois à l'extrémité 5' et à l'extrémité 3', des régions abritant des variations du nombre de copies (CNV), des duplications segmentaires ou des éléments d'ADN répétitifs ; et

(iii) la teneur en GC du pool de TACS est comprise entre 19 % et 80 %, telle que déterminée par calculant la teneur en GC de chaque membre au sein de chaque famille de TACS.

Fig. 1

FIGURE 1

FIG 2

EP 4 095 258 B1

| chromosome | start | stop | forward primer | reverse primer | SEQ ID NOs: |
|---|---|---|---|---|---|
| chr13 | 91049752 | 91050001 | TCT TCT CGT TCA AGA TGC CG | AAG GGT GGA AGC ACA TTG AC | 1, 2 |
| chr13 | 92177139 | 92177388 | AAA TTA CAT GCA AGG CTA CCA C | ATA GAG GAA CAA GCT GCA CA | 3, 4 |
| chr13 | 93071049 | 93071298 | ACA GGA AGA AAG GGG AGT TAC | CTC GTC CCT TGC ACA TCT TA | 5, 6 |
| chr13 | 94326973 | 94327222 | TTC AGG TTG TGT GAT GTG TC | AGG TGG GGA AGA ACA AAA CA | 7, 8 |
| chr13 | 94415507 | 94415756 | TAA GCA GAG GTT TTG TTG CC | GAG AGT AGG TGC AGG GAA AC | 9, 10 |
| chr13 | 95600034 | 95600283 | GTG AAG TAT TTG CTG CCA CC | ATC CCG CAT TCT TAA CCA CA | 11, 12 |
| chr13 | 112910529 | 112910778 | ATG GTC ATC TCA ACA GCA CA | GAC TAA GCA AAA GCA TCT CCC | 13, 14 |
| chr13 | 53328405 | 53328654 | TCC ACT GAC GTT GAG ATT CG | TTC TTA CAG GCT CAG GGT AT | 15, 16 |
| chr13 | 20533985 | 20534234 | CGA ACC TCC TTG ACC TCT TA | CCC ATA GCT TAA CCC CTA CAA | 17, 18 |
| chr13 | 20535728 | 20535977 | GTT ATG TTG ATA GGG GAA GCT T | TCT TCA TCT TAC TGT CTA GCA C | 19, 20 |
| chr13 | 20544227 | 20544476 | AAG CCC TCT ACT CCA TCT GT | TCA TAC CCT ATC CCT GTG ATC | 21, 22 |
| chr13 | 20567359 | 20567607 | TCA GAA CTC GTC AGT GGA AG | TCT TGC CCT TGA TTT GTT TCC | 23, 24 |
| chr13 | 20577159 | 20577407 | GAA GGG CCA GAC AGC TTA T | GCC TTT TAT CCA TAT GCC ACC | 25, 26 |
| chr13 | 20598772 | 20599021 | ATT TGC TTT GTT TTT GTC CCT | GCC CTC AAC TTT GCT TTT CA | 27, 28 |
| chr13 | 20600556 | 20600804 | GAT CAT TGC TTT GTT TGG ACC | AAA AAC CTG CAC TGT GTT CG | 29, 30 |
| chr13 | 20610779 | 20611027 | AGG AGA GGA AAA ATC TTG ACC | TTT TTA CAG CAA TCT TCA CTG C | 31, 32 |

FIGURE 2 (Page 1 of 80)

| chromosome | start | stop | forward primer | reverse primer | SEQ ID NOs: |
|---|---|---|---|---|---|
| chr13 | 20621336 | 20621585 | CTG CCT GCA GCC ATT ATT GT | TAA GCC GGA ATG ATT TGT AAG G | 33, 34 |
| chr13 | 20624392 | 20624641 | CCT TGG GGT ATG TTT GTT ATG T | TAC CCT TTG GCT TAA CAG CT | 35, 36 |
| chr13 | 20625461 | 20625710 | ACT TTT TCT GTA TCA GTC ACG G | AGT TGT CAT GTT GGG CTC AT | 37, 38 |
| chr13 | 20632609 | 20632857 | CTG CTT TTG TGT GTT CCC TT | TGT CCT AAG TTA CCT GTC TGA C | 39, 40 |
| chr13 | 20635196 | 20635444 | CCA GTT CCT GTT TTT CTG CC | GCC CTG GAA AGT ACT GTA ACA | 41, 42 |
| chr13 | 21127405 | 21127653 | GCC TTC ACT GAT CCT ACT TTC | TGT TAC AGC CAG GCT TTC AT | 43, 44 |
| chr13 | 21163998 | 21164247 | GGA TAT GGG GTA GGT TTT TGT | CAA CGA ACA CAG GGT TTA CA | 45, 46 |
| chr13 | 21170950 | 21171199 | ACT CAT AGA ACT GGG GCT TT | CCA GGA CTC TCT CTT TTC TTC | 47, 48 |
| chr13 | 21172515 | 21172764 | CTT TGT AGG TCC TCC AGA GA | GAC ACA TAA GAC CAC TTT AGG C | 49, 50 |
| chr13 | 21173774 | 21174022 | CCT GTT TTC AGT GGG TTG AA | GAT ATG TTC TGG AGG ACT GCT | 51, 52 |
| chr13 | 21182580 | 21182828 | CCA CGT TGT ACC TTT CCA TG | TGA TTC TCA CAG GCT CCT TG | 53, 54 |
| chr13 | 21203385 | 21203634 | GAG ATT CAA AAC AGT GGT GGC | TGA TGG AAG TTT CTA GGT CAG T | 55, 56 |
| chr13 | 21217173 | 21217422 | ACA ACA CTG TCC TTG GGT T | AAC ACT CTT GCT CCC TAT GT | 57, 58 |
| chr13 | 21228403 | 21228652 | TAA ATG TCC TGT GTG CTC GG | TTT CTC TCC CAG CTT GAT CTT | 59, 60 |
| chr13 | 21237418 | 21237666 | GAC AGG ACA CAT GGA GAG AG | CTG TGC AGA GAC GAA CTA AG | 61, 62 |
| chr13 | 21239719 | 21239968 | AAC TGA TGG TGA TTT GCA TGT | CTC TCG GAG CAA AGA CCT T | 63, 64 |

FIGURE 2 (Page 2 of 80)

| chromosome | start | stop | forward primer | reverse primer | SEQ ID NOs: |
|---|---|---|---|---|---|
| chr13 | 21245232 | 21245481 | GGC TTC TGG AAA CAT CTT GC | ACC TCA TAA GTA CGC CCA TC | 65, 66 |
| chr13 | 21251423 | 21251672 | CAA TAG AAG TAG GGG GTG AGG | TGA TCT TCC TTT GCT CCT GT | 67, 68 |
| chr13 | 21276601 | 21276850 | AGC TGG GAT GGG TTG TTT AT | ATG ACG ACG ATG TTG GAG AG | 69, 70 |
| chr13 | 21296989 | 21297237 | GCC AGT TGT CAG AAG AAT CC | TCC TTC AGC AAG CCT CTT TT | 71, 72 |
| chr13 | 21321012 | 21321261 | CAA CTG CAT TCC AAA ACA GC | TGA GGG TGA TAA CCT GTG AG | 73, 74 |
| chr13 | 21330138 | 21330386 | GAA GGG AAA CAG TGA GAA AGA | GGC TTG AAG TTT GTC TGT GA | 75, 76 |
| chr13 | 21348854 | 21349102 | TCA AGC GCC GTA AGT ATG TA | GGA TTT TCA CAT TGC TCA GC | 77, 78 |
| chr13 | 21351692 | 21351941 | TAA CCA TAA CAT CCA GGG CA | TAG CAT GAG AGT GAA CTG AGG | 79, 80 |
| chr13 | 21356527 | 21356775 | TTG TAA GCT GGC ACA CTG AA | CAG CCA CAT AGC CCA TAT CT | 81, 82 |
| chr13 | 21361621 | 21361869 | TTA AGA AAG TGC CGT GTT GC | TGC CCA TGA GTC TAC TTG TG | 83, 84 |
| chr13 | 21396343 | 21396591 | ATG GGT CCA TGA AGA GAA GC | AAG TGG ACT GAG GGA CAA TT | 85, 86 |
| chr13 | 21399744 | 21399992 | AGT TGT TTC CAG TAC TGC CA | TCA GAC TGA GCA TTA AAT CAC C | 87, 88 |
| chr13 | 21417352 | 21417601 | GTA TGC TTT CAA GTG ACG CC | TAG GTC TGG AAG AAT GCC AG | 89, 90 |
| chr13 | 21422981 | 21423229 | GAA GAC AAT GCA ATG AGG TGT | AGT ATC TTG GGC TTG TGA CA | 91, 92 |
| chr13 | 21607075 | 21607324 | AGA CCC AAT GAG AAC AGG AA | GCT CCA CTT CCA GTC TTT CT | 93, 94 |
| chr13 | 21631007 | 21631256 | TGA TGG AGG AAG TGT GAA GC | TTG GAA TAG TGA GCC TCC CT | 95, 96 |

FIGURE 2 (Page 3 of 80)

| chromosome | start | stop | forward primer | reverse primer | SEQ ID NOs: |
|---|---|---|---|---|---|
| chr13 | 22114658 | 22114907 | TGG CTT TTC TGT AGT TTG GG | TCT GGG GCT CTT TGT CTT TG | 97, 98 |
| chr13 | 22130956 | 22131205 | AGC AGC AAG AGT TGA GAA GA | ATT TTT CCT CCC CTG TAG CT | 99, 100 |
| chr13 | 22189915 | 22190164 | GGC TTT GAA AAA TCA CCA TGG | CTG GGC ACA CTG TAT TAC CA | 101, 102 |
| chr13 | 22270443 | 22270692 | AGA GGT TTC CAT CGT TGC TA | CAC CAC GTT TCT AAT GCA GA | 103, 104 |
| chr13 | 22396225 | 22396474 | AAC AGC CCC AAA CTT CCT AC | GAG TTG AAA AAG GTC CAC GC | 105, 106 |
| chr13 | 22609278 | 22609527 | ATC ACT GCC AAC AAG CCA TT | CAG AGG AAA GAC ACA GTG CT | 107, 108 |
| chr13 | 22691882 | 22692131 | TGA GTT GTG GGG GAT AAA GG | TCT TGG TTT TGA GGC TGT CA | 109, 110 |
| chr13 | 22765144 | 22765393 | TCT GGG CAC TTT CCT TAT GA | ATC AGC CTA ATT CTC CCC AC | 111, 112 |
| chr13 | 27175379 | 27175628 | GCA GTT TTG AGG GGA GAA GA | ATC CCC CAT CAT CCA TAC TC | 113, 114 |
| chr13 | 27405837 | 27406086 | TTC ATG ATG CCA CCT CCT C | CAT AGC TAG GCC TGT GAG TG | 115, 116 |
| chr13 | 27646720 | 27646969 | GCC CAA ATA CCC CTT CAG TA | TCA GCT TGC TCC TTC TCT G | 117, 118 |
| chr13 | 27674747 | 27674996 | GCA CCT CAA TCC CGT ACA AT | CTA GGT CCT CAG CAG TGT TT | 119, 120 |
| chr13 | 27969360 | 27969609 | GCG TTT TAA GCA GCT GTG TA | TCC CAG AGT TAA CAA TAC CCC | 121, 122 |
| chr13 | 27978231 | 27978480 | TCT CCA GAT CGA AAC AGC AT | CCA TTT GCA CTG CCG ATT TC | 123, 124 |
| chr13 | 28003481 | 28003730 | TCA TTC AAA GCC AAG ATG CC | ACT AGT CCC AAA AGC CTA CAC | 125, 126 |
| chr13 | 28073180 | 28073429 | ACA CGC TAC ATA GAC ACT GG | AAC AGC AGC GTC AGA ATA AC | 127, 128 |

FIGURE 2 (Page 4 of 80)

| chromosome | start | stop | forward primer | reverse primer | SEQ ID NOs: |
|---|---|---|---|---|---|
| chr13 | 28077809 | 28078058 | CAA TCG AGG TCA CAT TCA CC | AGG TCT TTA CGG GAA GGA AA | 129, 130 |
| chr13 | 28212786 | 28213035 | TGA CAC GCT GAA GAA AAT AGC | TGA GGG AGA AGT TTG GTA GG | 131, 132 |
| chr13 | 28302961 | 28303210 | GGA GGG AAT GGC AGA AGT AA | CCC TGT CTA AAG AGC CAT GT | 133, 134 |
| chr13 | 28325874 | 28326123 | GGA TTT TCA GAG CAG AGG TTG | GTT TGG AGT TTC GAT GCC TT | 135, 136 |
| chr13 | 28395893 | 28396142 | AAG AGT TGC CTG TAC CCT TC | ATG TTT TGG TCC TGG GAG AA | 137, 138 |
| chr13 | 28549591 | 28549840 | GTC TCT TTA CTG GGA GCG T | GGA AGT GGT TAG GGC AGA TT | 139, 140 |
| chr13 | 28592602 | 28592851 | ACA TTG CCC CTG ACA ACA TA | AGC CCA GTA AAG ATA AGA GGC | 141, 142 |
| chr13 | 28608293 | 28608542 | TCA TTA TCT GAG GAG CCG G | CCC TTC CCT TTC ATC CAA GA | 143, 144 |
| chr13 | 28617043 | 28617292 | GGC TCC ATA ATC TTC TGC AAT | CTG AAA CCT TCT CCT TAG CC | 145, 146 |
| chr13 | 28622471 | 28622720 | CGT ACA TCT GAT TTG TGG GT | ATT ATC AAC CTG ACC TG CA | 147, 148 |
| chr13 | 28623594 | 28623843 | TGC CAC TGA TGA TAC AAA AGC | AGG TGC AAA GCT GTT CAT G | 149, 150 |
| chr13 | 28941450 | 28941699 | GAA ACG TGT GGT GTC CTC TA | AAT GCC AAG ATT GTC CTT CA | 151, 152 |
| chr13 | 28958898 | 28959147 | AGT GTT TGG GGC TCT ATC AG | TGC GAA ACC TCA GTG ATC A | 153, 154 |
| chr13 | 28960375 | 28960624 | CTT TCC CTT TTG AGT CCT GC | CAC TTC CTT CAG CAC ACT TT | 155, 156 |
| chr13 | 29003985 | 29004234 | TCT CGC TTA CCT TGC TAC AT | CCG GCT CTC TAT GAA AGT GA | 157, 158 |
| chr13 | 29052686 | 29052935 | AAA GCG GGA ATT GGA ACT TT | ACT TGT CTG TCT GCC TGT TT | 159, 160 |

FIGURE 2 (Page 5 of 80)

| chromosome | start | stop | forward primer | reverse primer | SEQ ID NOs: |
|---|---|---|---|---|---|
| chr13 | 52293092 | 52293341 | GGG GAA CAT TGG GAA GAG AT | CTG ATG CGC TGA AAA CCA A | 161, 162 |
| chr13 | 52302656 | 52302905 | TCT ACC AGC TAC AAA CCC AT | TTC CAT GTG TTC TTC CTC CC | 163, 164 |
| chr13 | 53286404 | 53286653 | TCA GCC AAT ACC CAT AGC AG | AGC AGT AGG GTT AAC AGG AG | 165, 166 |
| chr13 | 53306303 | 53306552 | AAT CAG AGG AAG ATG GGT CG | GCA GCA ATG TTT CGG TGT A | 167, 168 |
| chr13 | 53497224 | 53497473 | GAG AAC TCC ACC CTG TCT TT | TAC TAA TGG CTG GGG GTA AC | 169, 170 |
| chr13 | 53548644 | 53548893 | GGG AGT GTG TGA ATG TGT CT | CTG ATG AGG CTA AAG GAC CA | 171, 172 |
| chr13 | 53652715 | 53652964 | AAG CTT TAC ATC ATG GCA CTG | CAG AGT TCT CCA TCC CAG AC | 173, 174 |
| chr13 | 53861872 | 53862121 | AAA GGT CCA TAG GCT CAC AT | CTG AGT TCC TCC TTT TGC CT | 175, 176 |
| chr13 | 53897429 | 53897678 | TTG ACC AAT GCC ATT AAG CC | GCA AAA GGT GGT GTT AGC TG | 177, 178 |
| chr13 | 53938205 | 53938454 | ACC AGG GAA ATG TTA GCT TCT | ATC CAC ATC CCA TGC CTA AG | 179, 180 |
| chr13 | 54626806 | 54627055 | TGC TCT GTT ATG GTT GGA GTT | TCT ATT CCT TTG GCA CCT CC | 181, 182 |
| chr13 | 54740174 | 54740423 | GAA GCG GCA GTA ATT CAG GA | AGG AAA GGA GAG CTT TGT CC | 183, 184 |
| chr13 | 54753834 | 54754083 | AAC TGT GTC CTA AGC AGT GA | CCT TTC AGC TTC CAA GTC CT | 185, 186 |
| chr13 | 54768238 | 54768487 | GTC ACC TCC AGA GCT TTC AT | GCT CTC TTC CTC CCA CTA AAA | 187, 188 |
| chr13 | 54773333 | 54773582 | GAA CAA TGC AAC CTG AGA ACT | ACT GCC TGT GTT TTC TTC CT | 189, 190 |
| chr13 | 54910969 | 54911218 | TGA TTG TCC TCT ACC ATG CAT | AAA GCA ATT TCT TCC CCA GC | 191, 192 |

FIGURE 2 (Page 6 of 80)

| chromosome | start | stop | forward primer | reverse primer | SEQ ID NOs: |
|---|---|---|---|---|---|
| chr13 | 54917347 | 54917596 | AGG CTT GAA ACA CCA CCT TTA | TGT CTG TTG CCA TTC CTT CT | 193, 194 |
| chr13 | 54926251 | 54926500 | TTG ATT TGT TGG GTG GTT GG | TGA CTG TGA CTT GTG CTT TC | 195, 196 |
| chr13 | 55009732 | 55009981 | TGC CAA TCT GAG GTT TTT CC | GAA TTA CAT TTC CCT GGG CG | 197, 198 |
| chr13 | 59785342 | 59785591 | ACT CTG CTT TAG GGC TTC TG | TGA AAC CGT CTT CCT TGT CT | 199, 200 |
| chr13 | 60086302 | 60086551 | GCA GAA AAG CTC CCA AAC AA | TTT AAA GCA GAG CAG GAC CT | 201, 202 |
| chr13 | 60668020 | 60668269 | GAA AAG AGG TGG AGA GGG AG | TTT ATG ACA CAC AGA GCA GC | 203, 204 |
| chr13 | 60707747 | 60707996 | AGG ACC CTT TTG CTG ATT TC | ATG TGT TTG ACC CTT TCC CT | 205, 206 |
| chr13 | 60975949 | 60976198 | CTG GCC CAA GTG CAT ACA TA | ATC CTG AAG TTG TTC CAC ATC | 207, 208 |
| chr13 | 61177164 | 61177413 | AAC CAG AGA GAC ACC TTG AC | GAC CCT GCT TTG TTA CTA GGA | 209, 210 |
| chr13 | 61495655 | 61495904 | TCC TCC CTA TCT CCT GTG AC | TGG ACA TGG ACA TTT CAA CG | 211, 212 |
| chr13 | 67720914 | 67721163 | ACT CTG CCA GAA AAG CCT AC | AAA AAT GCT TCC ACT TGC CT | 213, 214 |
| chr13 | 67799557 | 67799806 | CCT TTG TCT TGA AGC CTC CT | GCA CCT CCA ACA ACA TTC AA | 215, 216 |
| chr13 | 70101783 | 70102032 | TTG ACT GAG CAG AGT AGA GC | TTG GAA ATG GGG CTG GAG | 217, 218 |
| chr13 | 71512697 | 71512946 | TTC TAC CTA CAA GCA AAG AGA G | ACT GGT CTA TTG GGG GAA AAT | 219, 220 |
| chr13 | 71821654 | 71821903 | TCA CCA ACA GAG GAT CAA ACT | AGT GTT AGG AAA GCA GAG TG | 221, 222 |
| chr13 | 72405844 | 72406093 | CCG AGG GAT AAC ATA CAG CT | TGG ACA GGG TTT CAC AAG AT | 223, 224 |

FIGURE 2 (Page 7 of 80)

| chromosome | start | stop | forward primer | reverse primer | SEQ ID NOs: |
|---|---|---|---|---|---|
| chr13 | 74087455 | 74087704 | CAC TAG TCA CAG AAG CAG GT | CTC CTC TCC ATC TTT CCA GG | 225, 226 |
| chr13 | 74192477 | 74192726 | GTA GAA ACC CCG AGA CAA CT | ATA GGC TGA CTT CCA CAT CTC | 227, 228 |
| chr13 | 74260893 | 74261142 | CAA TTT GCT GTT CAG AGG CT | TGT GGG GGT CAA TTC TAA CG | 229, 230 |
| chr13 | 78218064 | 78218313 | TCA TGA TGT GGC TTA GTG GG | CCA ACG GGT AGT GGT AGA TT | 231, 232 |
| chr13 | 88033439 | 88033688 | TAC ACC CAC ATG CAT ACA CA | CTT ACC CCA CTT CTT CCT GA | 233, 234 |
| chr13 | 90954378 | 90954627 | GAA GTG TGC ATG GGA GAG T | CCT GTC ACA ACT GCC TTT G | 235, 236 |
| chr13 | 91290109 | 91290358 | TTT GGG TGG CTC TAT GTT AGG | TTT GCC ATT TTG TGA TGC CA | 237, 238 |
| chr13 | 91874767 | 91875016 | CTC CTT GAC TCA TTT CCC GT | GGA GTT TCA GGT TGG CAG AA | 239, 240 |
| chr13 | 92125914 | 92126163 | AGA GTA CCA CTG CCA AGA AA | ACA GCT TGC TTC AAA CTA CA | 241, 242 |
| chr13 | 92308079 | 92308328 | CTG CTA GTC TGT CAG GAG AG | TTG AAG GGG CAA AAT ACA GC | 243, 244 |
| chr13 | 92971372 | 92971621 | GGG CAG CAG TAT AAA CAT CC | GCC CCA AAT TGT AAC AAA GC | 245, 246 |
| chr13 | 94063232 | 94063481 | GAC ATT CCC TTC CAT TGA GC | TGA CGA AGA CTC CAA CAC AA | 247, 248 |
| chr13 | 94453836 | 94454085 | TTC CTG GTA AAT GTG CTG GT | AAG TCA GGG AAA TGA AGC TG | 249, 250 |
| chr13 | 94713643 | 94713892 | GGC CAG ATT TGC AGT GAT TT | GTA ACA CAG TGC TCC TTC TC | 251, 252 |
| chr13 | 95766126 | 95766375 | AGA ATC AAC AAC AAT GGC AGG | GGC CCC AAT TAG CTG ATT TC | 253, 254 |
| chr13 | 95814599 | 95814848 | CTC TGA GGA AAG CTT GTA GGA | CTG AGC AGG GAA AAA TCC AG | 255, 256 |

FIGURE 2 (Page 8 of 80)

| chromosome | start | stop | forward primer | reverse primer | SEQ ID NOs: |
|---|---|---|---|---|---|
| chr13 | 111956688 | 111956937 | AGG TTT TCG TTC TGC TTC AG | ACA AAG GAT TCA GGT GCA GT | 257, 258 |
| chr13 | 112517966 | 112518215 | CGG GTC AGT GAT TCT AGC T | ACA GGT TTT GCT CTT CAG GA | 259, 260 |
| chr13 | 112531519 | 112531768 | GTC ACT ATG GAA TGG GGG TT | GGC AAG TTT GTC TGG TTC ATT | 261, 262 |
| chr13 | 112986709 | 112986958 | GCT TCT TCC CCG CAA TAT GA | CCA TCT GCA TCT GTC TCC TT | 263, 264 |
| chr13 | 113000054 | 113000303 | CGG TTC AGA GTC AAT GCC TA | GCT CCT CTC CTT CTC CCT T | 265, 266 |
| chr13 | 21391429 | 21391678 | TAC CCA ACA AGC CAG AGA AAT | TGT ATA AGG GCA ATC GTG GT | 267, 268 |
| chr13 | 21394616 | 21394865 | AGA GGG AAA GTG CAA GGA AT | AAG GAA CCA GGT CAG ACA AG | 269, 270 |
| chr13 | 21404268 | 21404516 | GTT TTT CAG CAC ACT GTC CC | GTT AGA AGG CAA ACA TCA TGC | 271, 272 |
| chr13 | 21618233 | 21618482 | ACC ACA TTA CTC ACA ACC CT | TGC AGT CAT AGG AAA AGG CT | 273, 274 |
| chr13 | 21619521 | 21619769 | AAG CCC TTT TCA TCT CCA CA | GCA GTC AGA ATG GTT TGG C | 275, 276 |
| chr13 | 21629602 | 21629851 | CCA GAG CTG AGA CAA CTA CT | AGA CAT TGG TTT GGT TGG TTC | 277, 278 |
| chr13 | 21654127 | 21654376 | GAA GCA ATT CCT CAC ACC AC | ACA CAA ATG AAA GCC CGT AC | 279, 280 |
| chr13 | 22215795 | 22216044 | TTC AGT CAG AAT GAG GAG CC | GGT CTG CTG TTT CTC TTT GC | 281, 282 |
| chr13 | 22227261 | 22227510 | CTC CTC TCC CCT CTG ATT TT | AGA GCC TTA CCA AGC TGA AG | 283, 284 |
| chr13 | 22243011 | 22243260 | TTT TAA AGC GAC AGT CAC ACG | GGG ATG GTT ACT TAG TGG GG | 285, 286 |
| chr13 | 22255153 | 22255402 | TCC TCT GCC TTC TAC CCT TT | TTA CAC TCG CCT TCC AAA CA | 287, 288 |

FIGURE 2 (Page 9 of 80)

| chromosome | start | stop | forward primer | reverse primer | SEQ ID NOs: |
|---|---|---|---|---|---|
| chr13 | 22639387 | 22639636 | TGT TGT GCC TTT TGT TCT GG | GAG AGA GGA GGA GTT GGA AG | 289, 290 |
| chr13 | 27183101 | 27183350 | TTA GGA GGT AAG GCT GGA AAA A | ATC CAC GAC ATC CAA AAT CA | 291, 292 |
| chr13 | 27387690 | 27387939 | CTG TCA GCA ATT TCA GGT CAG | TTG TGC AAG AAG AAA CCT GC | 293, 294 |
| chr13 | 30466189 | 30466438 | AGA CAA AGG CTT CAC GGA AC | GGC CTT GCA TAA ACC ACA TT | 295, 296 |
| chr13 | 30977198 | 30977447 | TTC CTC TGT GTC TTG AAG GT | TTT GTA ATT GGT CCT CGC CT | 297, 298 |
| chr13 | 31061235 | 31061484 | ATC AAT GCA GGT GAG TGT GA | CAA GTA TTT CAT GGC GCT CC | 299, 300 |
| chr13 | 31205037 | 31205286 | CAC TCC ACA TAA GCC TCA GA | GTC AAC AGT ATC AGC TTC CAA | 301, 302 |
| chr13 | 32625308 | 32625557 | AGG ATG TAG TTG GGT GAG GA | TGG GCT TCT TTT TCA TTC CG | 303, 304 |
| chr13 | 34428102 | 34428351 | CAG TCA TCA CGG GGA GAT AC | TCA ACA AGC TCT CTG TTC AC | 305, 306 |
| chr13 | 36054899 | 36055148 | GAC AGA TAT TTG TGC AGG GT | GTG CAA ACA GTG ACC TCA AT | 307, 308 |
| chr13 | 36105686 | 36105935 | TCC TAG CCC TTA CCT TTC CT | GCT GGG CTG CTT TAA TTT CT | 309, 310 |
| chr13 | 40676702 | 40676951 | AGC CTG AAT GTC ACT GAT CA | GGA ATT GTG GGG TCA ATT GG | 311, 312 |
| chr13 | 40745383 | 40745632 | ACT CAT CAC TTC TGG CTG C | CTA GTG CTT CTA CCT CCA GAC | 313, 314 |
| chr13 | 49281723 | 49281972 | TCT TGT GTT TCC TGC CCT AT | AAC CAC ACA CTA ACA GGG AA | 315, 316 |
| chr13 | 53624527 | 53624776 | TTG CTG TGG ATG AGA ATG GA | TCT AGT TTG CCC TCT TTC CC | 317, 318 |
| chr13 | 60069277 | 60069526 | TGA GGG CAG AAA GAA ACA GA | CGA ATT GCT TCC TTG CTC TG | 319, 320 |

FIGURE 2 (Page 10 of 80)

| chromosome | start | stop | forward primer | reverse primer | SEQ ID NOs: |
|---|---|---|---|---|---|
| chr13 | 60654975 | 60655224 | GGG GTC ACA CAT CAC TTT TC | TCT ATC ACA GCA GGA AAT CAC T | 321, 322 |
| chr13 | 61151386 | 61151635 | ATA GGG TCA CAA TCC ACT GC | TCT TCG TGT TTC TCT AGC CC | 323, 324 |
| chr13 | 62064603 | 62064852 | ATA TTG AGC CCC GCA TGT TA | TTG AAG AGC TAA AGG GGG AG | 325, 326 |
| chr13 | 71479359 | 71479608 | GGT TGC AGG AGA AAG AAC AT | TGT CAC CGT ACT ACC TAA GC | 327, 328 |
| chr13 | 39233534 | 39233783 | TGC CAT GTA ATT GCC AAG AT | AGT ACG CTC CTT TGC AGA G | 329, 330 |
| chr13 | 39281667 | 39281916 | CCT GTT CTC CAT CCC TCT G | TTA CGG GGA CAC AAA ATG GT | 331, 332 |
| chr13 | 39343524 | 39343773 | TCA CAA ACT ACC CAA CAC CTA | CTG TGC TTT GCC CTT GAA G | 333, 334 |
| chr13 | 39444897 | 39445146 | CAG AAT TAG TTG GGG AGC TGT | AAG TCA ACC CAT ATG CCA CT | 335, 336 |
| chr13 | 39469458 | 39469707 | GCC ATC TCC TGA AAT AGT GC | ACA TTC AGG CTG TCA CAC AT | 337, 338 |
| chr13 | 39486237 | 39486486 | GCA AGT GTT CCC ATC TAG AA | ;TC ATG TCA CTA GTT TTA TAA GGC | 339, 340 |
| chr13 | 39541262 | 39541511 | ATT GAT ACC CCT CTC CCC AG | AGT AGT GAG GCT CCA AAG TG | 341, 342 |
| chr13 | 39597307 | 39597556 | AAG TAA GCT GTC TCC TGG C | TGG GAG GAG TTT GCT GTT TA | 343, 344 |
| chr13 | 39662550 | 39662799 | AGG TTG GTT GGC ATG AAG AA | TTC TAA GCC TGT GAC TGA CA | 345, 346 |
| chr13 | 39681445 | 39681694 | AGC AGA GTT TCA AGA CAA GC | TGA ACC TGA CTT TCC TTG GG | 347, 348 |
| chr13 | 39707053 | 39707302 | TTT TAC ACA GCA GGC CTC TT | GCC ATT CTA TCA TCT CGG GA | 349, 350 |
| chr13 | 39791099 | 39791348 | GCA ACT CCA AAT TAT CAG GGC | AGT CTC TCC CTG AAA CCC A | 351, 352 |

FIGURE 2 (Page 11 of 80)

| chromosome | start | stop | forward primer | reverse primer | SEQ ID NOs: |
|---|---|---|---|---|---|
| chr13 | 39882804 | 39883053 | CAG CAC CTT CCC TTA GCA AA | AAG AGT TGG CTT GGA GTT GA | 353,354 |
| chr13 | 39887397 | 39887646 | CTT GTT GTC TTG TAG CCC TG | CCC ACA ATT ATG AAA GGA GGT | 355,356 |
| chr13 | 40023278 | 40023527 | CAC AGA TAC AGA CGT CCA CA | TTG ACC AGG ACA AAT GAG GA | 357,358 |
| chr13 | 40038277 | 40038526 | AGC TCA GCA ATT AAA CAG TCC | CAC TTT GTT GGT CTG GGT CA | 359,360 |
| chr13 | 40071926 | 40072175 | TGT TGA GAG TGC CAG AGA TG | GGG ACT CTA GGT GGG GTT AA | 361,362 |
| chr13 | 40138914 | 40139163 | CTT CCA CCT TCT GCC AAT GA | GTT TAT GCC TTG GGA TTG CC | 363,364 |
| chr13 | 40171414 | 40171663 | TTC TGA CAT TTG CAA GCA CC | GTG TGG TAA GGA TGC TAG GA | 365,366 |
| chr13 | 40405751 | 40406000 | CTG TTG CTA GTT TCT TGG GC | GAA AGT GAC TCC TCC CTG AC | 367,368 |
| chr13 | 40483291 | 40483540 | TTT TCC AGT CCC AGC ACA T | CCA GGT TCT GTT CTC TGT CA | 369,370 |
| chr13 | 40562330 | 40562579 | TCT CTC TCT TCC TGA AAC AGC | GTG AGA CAT GGT TGC TGT TC | 371,372 |
| chr13 | 40590631 | 40590880 | AAC CTC TGC TTT GTG TAG TGA | CTT TCT CCT GCT CCA CCT AT | 373,374 |
| chr13 | 40633944 | 40634193 | GAG AGA ATG CAA GGT TCA GC | CTA TGT GTG TTC CAA CCC GA | 375,376 |
| chr13 | 40644042 | 40644291 | TAC AGC ATC AAA GAG GAA GC | GGG ACC TTC TAA CCA TGT GT | 377,378 |
| chr13 | 40661582 | 40661831 | GAG GGG ATG AGG GGA AAA AG | CGG GAT TTT GAA AAG GCA GA | 379,380 |
| chr13 | 40667939 | 40668188 | CAT GAC CTC TGA CGG ATC TG | GTG TTG TCT CTC AGC TCC TC | 381,382 |
| chr13 | 40684306 | 40684555 | AGG CAA TGA GGT CAA GGA C | GTT CTC TGG TTA AGG CCC TT | 383,384 |

FIGURE 2 (Page 12 of 80)

| chromosome | start | stop | forward primer | reverse primer | SEQ ID NOs: |
|---|---|---|---|---|---|
| chr13 | 40747946 | 40748195 | ATG ATG GCC CCA ACT TCT TC | CCT CTT CAC CTA TAA GCC CC | 385, 386 |
| chr13 | 40767281 | 40767530 | TTC TAG ACA CTG AGG GAG CA | TAG GGG ACA GTA AGC CAG AT | 387, 388 |
| chr13 | 41165017 | 41165266 | AAC GCT ACA CTT TAC GAG CT | ACG ATG GAC CTC TGT TGA AC | 389, 390 |
| chr13 | 41176869 | 41177118 | TCA ACA CTA CCT GCC AAT CA | ATT CCC ATC CAT CCA TCA CTC | 391, 392 |
| chr13 | 41573543 | 41573792 | TGC CGA CAC AAA AGA ATG C | TCG TTT TTG GAT GGT GGT TG | 393, 394 |
| chr13 | 41581284 | 41581533 | AAA GTG TTC CTC CCT GCT G | GAA GTT CCT CCA GTA GAC TCA | 395, 396 |
| chr13 | 41588844 | 41589093 | AGG AGC AAA ATA GTC TGG CT | TTG TTT GAG TCT GGG AGG AA | 397, 398 |
| chr13 | 41628316 | 41628565 | ACC ACT CTT GAA TCA TTG CAG | AAT ACT GTG AGA CTG CCA CC | 399, 400 |
| chr13 | 41633944 | 41634193 | CCA GCC AAT TTT CTC TTT CCC | CAG TCA CGG AAA GTA CCC TC | 401, 402 |
| chr13 | 41827812 | 41828061 | ACT GTC CCT ACT GCC AAT TT | ACC ATG TTT CCC TCT GTC AC | 403, 404 |
| chr13 | 42280338 | 42280587 | ATC TGG TTT GAA CTT GCC AAC | TTA CCA AGG GAC AGG ATG GA | 405, 406 |
| chr13 | 42349979 | 42350228 | CAC TCT GAA TAG CTC TCC CC | CCC TAG AGG TCA AGG TAT GG | 407, 408 |
| chr13 | 42589616 | 42589865 | TTA TCC GGG ACA GTT TCA GG | ATA GGC CCT GTG TGT TAG TT | 409, 410 |
| chr13 | 42773088 | 42773337 | GAA TCT TTT GGC CCA CAC TG | AGA AAG TCC CCT CCA TTT CT | 411, 412 |
| chr13 | 42811933 | 42812182 | GTC AGA CAC ACT TAG CTG GT | GCC AAT GCC AAA GTC AGT TA | 413, 414 |
| chr13 | 44211957 | 44212206 | ACA CAT TTC ACC TTC ACC CT | GAG GAC GAG TTG AAC AAA GC | 415, 416 |

FIGURE 2 (Page 13 of 80)

| chromosome | start | stop | forward primer | reverse primer | SEQ ID NOs: |
|---|---|---|---|---|---|
| chr13 | 44239246 | 44239495 | GCA CTA ATC CAG GGG CTT AA | ATA CTG GTC TCA AGG TAG CAC | 417, 418 |
| chr13 | 44247433 | 44247682 | CCC CTT ACC ACC ACT TCT AC | ACC AAG TGA AGC TGA GTT AAT G | 419, 420 |
| chr13 | 44359516 | 44359765 | TTC AGA TCC CTT AAG CAC GC | CCC AGA TAC ACT CCT GCT TC | 421, 422 |
| chr13 | 44388992 | 44389241 | ACC TAA GGC CTC AAA TTC CA | CAA ACA TGA GAG GGG GAG AA | 423, 424 |
| chr13 | 44493443 | 44493692 | CAG ACC ACG GGC ATA AGA AA | AAA CAC AGC AAT GAG GAA GG | 425, 426 |
| chr13 | 44557886 | 44558135 | TGG ATG TGT GGA TTT GGA GA | GAT ACT CCC CTG TGT TGC TT | 427, 428 |
| chr13 | 44959162 | 44959411 | CTG GCT GTC TTC TGG GAA A | GCT CTT ACT AGG ATG GCA GG | 429, 430 |
| chr13 | 45043950 | 45044199 | CAA GCA GAA CTG AGA AGA GTC | GTT TCC AGC AGC AAT CCT TT | 431, 432 |
| chr13 | 45115235 | 45115484 | AGT GGA ACG AGG ATT GTG TT | CTG TGC AGA AGG GTT AGC T | 433, 434 |
| chr13 | 45147386 | 45147635 | CTC CCA TCT GAA ACT GCT GA | AAA CCC CTG CTA CCC AAA AT | 435, 436 |
| chr13 | 47067063 | 47067312 | ACA TCA CAA CCA CCC TGA C | AAT GCC CAG ATG CTG TTT TC | 437, 438 |
| chr13 | 47120595 | 47120844 | GTG TTG ACC TGA TTT GCC AA | GAG TGG TTG TTC TCT CCA GAT | 439, 440 |
| chr13 | 47154937 | 47155186 | GAA CAA AGA GGA ACA GAG CC | CAG TCT AGA AGC TCA CCC AG | 441, 442 |
| chr13 | 47258834 | 47259083 | TTC ATG ATT CCA GGG TCC TC | TCT CCT CTA CCC CTA CAC TG | 443, 444 |
| chr13 | 47261849 | 47262098 | GGA CGG ATT TAG TGT ACA TTG G | GGT GTA AAT GTG GCC TCT CC | 445, 446 |
| chr13 | 47286641 | 47286890 | TTT CCT TCC AAC ACC ACA GA | ACA AAG CTA CAA ACT CTG GC | 447, 448 |

FIGURE 2 (Page 14 of 80)

| chromosome | start | stop | forward primer | reverse primer | SEQ ID NOs: |
|---|---|---|---|---|---|
| chr13 | 47314093 | 47314342 | TCC TTA GGG TTC TGC GAA AT | TTT CAC TGG GAG ACT GAT GC | 449, 450 |
| chr13 | 47623498 | 47623747 | GGA AAC TCC CTG CCT TCT AC | CTG TTC TGT TCC TGA GGC TA | 451, 452 |
| chr13 | 48562058 | 48562307 | TCT TCC AAA CAC CAG GTC TA | TGT GTA TCC ATT GCC TCA TCT | 453, 454 |
| chr13 | 48698166 | 48698415 | ACT CAA TGG AAG GAA GGG C | GAA GAG GGT GTG TGT AGG AC | 455, 456 |
| chr13 | 48762184 | 48762433 | AAG GAC TTG TGC TGT ATT GC | TGG TTG CTC TTC CTA GTT CC | 457, 458 |
| chr13 | 48806515 | 48806764 | GAC GGG AGC CAG TAT TCT AC | CAA TGT GGA GGA AGC TCT TG | 459, 460 |
| chr13 | 48811339 | 48811588 | GGG ATT GAG AGC TTG GTT CT | TTG CAC ACC CAA TAT GCT AC | 461, 462 |
| chr13 | 49119202 | 49119451 | CTC CCC ACC AAG ATG TTC AA | TCC AAG GTT TCT CTA GCG AC | 463, 464 |
| chr13 | 49479756 | 49480005 | GTT TTG GGT CAT GCA GTG TT | TCG CTA TTC TCC TTG CCA TA | 465, 466 |
| chr13 | 49505070 | 49505319 | GAC AAA AAC ACT TGC CAG AC | AAC AGC CTC TTT CCT TAG CA | 467, 468 |
| chr13 | 49530727 | 49530976 | AAC CAT GGC TTT GCA AGT AC | TTT TTG GCT CAG TGG GAT GT | 469, 470 |
| chr13 | 50387096 | 50387317 | GGA ACC CTC TGC TAT TTT GC | CTG TTC ATT CTT CTT CAG GGC | 471, 472 |
| chr13 | 50960526 | 50960775 | TAC TCC TTG TGT GAA CCC CT | TTC CCG AGC CCA TAA ACT AC | 473, 474 |
| chr13 | 51783208 | 51783457 | ACC TTT ACC CCA TAC CAT CC | TGC TCA GAT TTC AGC TTC CT | 475, 476 |
| chr13 | 51816719 | 51816968 | CAT TCC TTT GGT TGG TGT CC | GTC AGC GAT GTG GAT GTC TA | 477, 478 |
| chr13 | 51825241 | 51825490 | CTA ATG GGC CTG TTG TTC CT | AAC TGA CTC CAT GAC CTG TG | 479, 480 |

FIGURE 2 (Page 15 of 80)

EP 4 095 258 B1

| chromosome | start | stop | forward primer | reverse primer | SEQ ID NOs: |
|---|---|---|---|---|---|
| chr13 | 52025153 | 52025402 | CAA CCT ACC TGC CCA TAG TT | GAA GCT GCT ACT TGG TGA AC | 481, 482 |
| chr13 | 52249504 | 52249753 | CGT AGC AAA TTA TGG CGA GG | TTT TCC TCC TGT TCT GTT GC | 483, 484 |
| chr13 | 29111011 | 29111260 | GGT CAG AAG GGA AAG GGT TC | TTC TCA AAT GCA ACC ACT CC | 485, 486 |
| chr13 | 29192994 | 29193243 | CCA GAT TAA AAC GTG GTG CC | CTG GCC CTT CAA TTT CAT GC | 487, 488 |
| chr13 | 29233772 | 29234021 | CCC ACA ACT ATA GGT CGC AT | CCA GCA GTA CCG ATA TCA GAG | 489, 490 |
| chr13 | 29310139 | 29310388 | AGT TGT TCC ATT TGT ACC AGC | CAG AAG GCA GGA GAT GGA TT | 491, 492 |
| chr13 | 29563140 | 29563389 | TTG GCT GCA CTT TGA GTC A | AAG CAA CCA TTT TCC TGA GC | 493, 494 |
| chr13 | 29580073 | 29580322 | CAG ATG GCC CAT TGT AAC AA | TCA CCC TTC ATC TAC CCA CT | 495, 496 |
| chr13 | 29929175 | 29929424 | TAT TGA GGT TCC CGT GCT G | AAG CTG GTG ACC TTC TAC AG | 497, 498 |
| chr13 | 29934475 | 29934724 | AGA ATG TGA AGT GGC TCC AT | AAA ATT CTG GTT GGG GAG GA | 499, 500 |
| chr13 | 29939640 | 29939889 | TTT GGG TTT GTG TGT GTG TG | CCA TAC CTC ATC TGC TCT GT | 501, 502 |
| chr13 | 29957853 | 29958102 | AGG AAT CTC TCT CTG CCA AG | GGC TGT CCC TGA ACT ACT TT | 503, 504 |
| chr13 | 29995732 | 29995981 | TCT TCA AGG CAG GTC ATA GG | CTT GGC TTA AAC TCT GCT CC | 505, 506 |
| chr13 | 30003375 | 30003624 | GAA ACC TAA GAC GTT CCA CTG | GAC TTG AAC ACA CCC TCA GA | 507, 508 |
| chr13 | 30014954 | 30015203 | GAG TGA AGG GAT TGG AGC AA | AGG AGA AGA GAC CAT TGC AG | 509, 510 |
| chr13 | 30114022 | 30114271 | ATG TCT CAG GCT AGG TGT TC | TTA GCT AAG TCT GTG CGG AG | 511, 512 |

FIGURE 2 (Page 16 of 80)

| chromosome | start | stop | forward primer | reverse primer | SEQ ID NOs: |
|---|---|---|---|---|---|
| chr13 | 30145894 | 30146143 | ACA CTC ACA AAG CCC AGT TA | GAA GCA ACA CTG TAC ACG C | 513, 514 |
| chr13 | 30187253 | 30187502 | GTG CAG ACT CAT GTT ATG GC | TCT GAT AAA GGC TGG CTC AT | 515, 516 |
| chr13 | 30421518 | 30421767 | AGG ATC TCA AAG CAC CAC AG | TAA AAC AGT GCC GCT ACT TC | 517, 518 |
| chr13 | 30531947 | 30532196 | AAC GGG AAG AGG GAA ACT TT | AGT GCT ATG AGT CTT GGT CC | 519, 520 |
| chr13 | 30778718 | 30778967 | ATG TTC AAC AGA GTC AGG CT | GGA AAA CAT GCG GTG GTC TA | 521, 522 |
| chr13 | 30905760 | 30906009 | CAG TAA CAG TCC AGG GTC TT | GCA GAG AAA TGG GTT AAG GG | 523, 524 |
| chr13 | 31011560 | 31011809 | AGT CTG GGA GCC TAG AAT CA | GGT AGA GGT GGG TTA TCT GT | 525, 526 |
| chr13 | 31089936 | 31090185 | CAT TGT AGT TTC AGG ACA CCA A | CTG TAA ATC TCC GGG GGT G | 527, 528 |
| chr13 | 31227324 | 31227573 | ACC ACA GAA TGA CTT GCA GC | GGC GAG AAT GGA GAG AGA AA | 529, 530 |
| chr13 | 31430437 | 31430686 | TTT CAC GTG TAA CAG GAG CA | TCC CAA GCC AGG ATT CTT TT | 531, 532 |
| chr13 | 31575060 | 31575309 | TGC TAC AGG GAA AAT GGT CT | GAA CAA GTA CAA CCG TGC AG | 533, 534 |
| chr13 | 31641685 | 31641934 | TCC ACT GCT TAG TTT GCC TT | ACA AAT GCC CCA TAT CAA CC | 535, 536 |
| chr13 | 31718167 | 31718416 | ACA GGT GGG GAG AAA AGG TA | GGA AAG AGG CCT GGA GTA AT | 537, 538 |
| chr13 | 31723797 | 31724044 | CTG CCA CTA CTA CAC AGC TA | TTT CCA CTG GAT GTC GTC AT | 539, 540 |
| chr13 | 32249897 | 32250146 | ATG GGT CTC TGG AAT GCA TG | AGG ACA AAG TTT CAG CCT CT | 541, 542 |
| chr13 | 32296888 | 32297137 | AGT TCT CCA CAG CAC ATC AT | ACT CAG GAC ACG ACT TCA TAC | 543, 544 |

FIGURE 2 (Page 17 of 80)

| chromosome | start | stop | forward primer | reverse primer | SEQ ID NOs: |
|---|---|---|---|---|---|
| chr13 | 32311823 | 32312072 | TCT TTC ATC TCA GCT CTG CA | ACA TCT TTG GCT CAC TGG TT | 545, 546 |
| chr13 | 32316395 | 32316644 | CCG AAC AGT ATT TTG AGG GG | TCA CAG TGG GCT TCA TTC AG | 547, 548 |
| chr13 | 32321163 | 32321412 | TTT GGC TGT TTC CTG TTT CC | AGC TGG AAT CTA TGT AGG ATG G | 549, 550 |
| chr13 | 32348262 | 32348511 | ACC TAA CTT GCC TTG TCC TT | CTG CGG AAG GAT CTA GTC TT | 551, 552 |
| chr13 | 32565108 | 32565357 | ACA GGA GAA CAA GCA GCA TA | TGA GAA GTA TTC AGC ATT TCC C | 553, 554 |
| chr13 | 32578964 | 32579213 | CAG CCT AGT ATA TGG GAA CGT | CAC TCA CGG ACT TTT AGG C | 555, 556 |
| chr13 | 32584041 | 32584290 | ACC CAT ATG TAG TAT CGC TCT TG | | 557, 558 |
| chr13 | 32596887 | 32597136 | TAT GGG TTT TTC TGC TCC ACT | TCA CAC GCC AGG TTA TTA CA | 559, 560 |
| chr13 | 32651705 | 32651954 | AAA TGT GAG GGA GAG TCG TC | AAG TGG GTT TGC AGT TTG GA | 561, 562 |
| chr13 | 32760468 | 32760717 | GCA GGA CCC TTC AGC ATT A | ATG TAC GTG TGT GTC CAT GT | 563, 564 |
| chr13 | 32829312 | 32829561 | GAC TGG ATG ATG CAA AGG TG | AGG CGG GTT GGT CAA TAA TA | 565, 566 |
| chr13 | 32852622 | 32852871 | AAC ATT TGC AGG GGG ATC AA | CCA CAA ATC CCA TCA ACA CA | 567, 568 |
| chr13 | 32872003 | 32872252 | AAA GAT GCC TCC TTG TGT CT | TTT CAC AGT AAC ATC GGC AC | 569, 570 |
| chr13 | 33535420 | 33535669 | AAG TTA TCT GCC CAG GGA AA | CTG ACA GCC TGC ATT TGA TT | 571, 572 |
| chr13 | 33634729 | 33634978 | TCC TGG CTA GTT TTG CTG AA | TTT CCT GGA GTA AAG CGA TCT | 573, 574 |
| chr13 | 33660366 | 33660615 | CTC CTT GCT TGC CTT TAC AC | CCC ACA ATC ACC CAT CTC TA | 575, 576 |

FIGURE 2 (Page 18 of 80)

EP 4 095 258 B1

| chromosome | start | stop | forward primer | reverse primer | SEQ ID NOs: |
|---|---|---|---|---|---|
| chr13 | 33742309 | 33742558 | AGC CTT AAT TCC CCA TGC AT | TAT CTC ACT CCA CAG CTT CC | 577, 578 |
| chr13 | 33760763 | 33761012 | TTT TTC TGT GGA GTG TGG CT | ACA GGT AGT TTG GTG GTG TC | 579, 580 |
| chr13 | 33787480 | 33787729 | AAA GAG TCA ACC ATG CAC TG | GCT GTT GAA TGC CAG AAC TT | 581, 582 |
| chr13 | 33803219 | 33803468 | GGT GAA GCA GCC TGA ATA AA | ATA GGG TCG GTT TTG GTC TG | 583, 584 |
| chr13 | 33925858 | 33926107 | TCT TTG TAC CAA GCT GCC A | CAT CAT CCC TGT CAT TCC CA | 585, 586 |
| chr13 | 34286418 | 34286667 | GCT TCT ACT TTC CCC TCC AG | GTG GGC TAA GAA AAC ACC TC | 587, 588 |
| chr13 | 34301780 | 34302029 | AGA AAA GCC AAC CTC CTC TT | ATT GTG GTT TGT GGC ATG TG | 589, 590 |
| chr13 | 34380783 | 34381032 | CCA GGG TAC TAA AAG GGG AC | CCA GAT TCA GCC TGT ATT CC | 591, 592 |
| chr13 | 35504924 | 35505173 | TTG TGG GTC AAT GTC AAC AC | GCC CAT GGA AGT AAA CAG TC | 593, 594 |
| chr13 | 35532835 | 35533084 | AGA AAA GGT GGA GGA AGG GA | AGG TTT GAC ATA ATA GTG CTG C | 595, 596 |
| chr13 | 35538772 | 35539021 | GGA GTT GTT TAC AGG TGG ACT | AAT CCT TTC CCC ACT CAC TG | 597, 598 |
| chr13 | 36233165 | 36233414 | TAG CTT CCA ATT CAC AGG TCA | CAC AAA GCA GTT CCA TGT CC | 599, 600 |
| chr13 | 36343466 | 36343715 | TTA AAT GCG CCA AGT CCC TA | GGA CAA TTT CTC ACT TGC CA | 601, 602 |
| chr13 | 36348003 | 36348252 | ATT TCC TGG GTC AAG CTC TT | AAG GGG TGT TGT TAG ATG CT | 603, 604 |
| chr13 | 36380590 | 36380839 | CTT GGA CCA GGA ATG CTC TA | GCA TCA CAC ACA GCA GAT AC | 605, 606 |
| chr13 | 36386774 | 36387023 | AGG CAG TCA GAT CCA CCT AT | AAA ATG TCC GTC CCA GAT GA | 607, 608 |

FIGURE 2 (Page 19 of 80)

| chromosome | start | stop | forward primer | reverse primer | SEQ ID NOs: |
|---|---|---|---|---|---|
| chr13 | 36410707 | 36410956 | AAA ATT GCC TGC TGT TTG GA | GGG GGA AAA TGT GTT GTG TT | 609, 610 |
| chr13 | 36427833 | 36428082 | GCA CCA TCA TGA AAC CTC CT | AAC TGT TAG CTT CTC CAC CC | 611, 612 |
| chr13 | 36445018 | 36445267 | ATA GGC CAG TCT CAG GTA GA | CGA GTG TAG GTT CCG GTT TA | 613, 614 |
| chr13 | 36451093 | 36451342 | AGA TTG CAG CCT ACC CAA AG | AGA ATG CCC ATT TCA GGA GT | 615, 616 |
| chr13 | 36568336 | 36568585 | TTG ACT GAA GTG TTC CAG GT | ATA TGT GGT TTG AGG TCA GCT | 617, 618 |
| chr13 | 36576882 | 36577131 | GCT TCT TTC AAC CAT CCA CC | GGC TTT GGT CAC ATG GAG A | 619, 620 |
| chr13 | 36663599 | 36663848 | GCA GCA CTC AAC TAT TCC AC | TGA GGC AAG ATT CAG TGA CT | 621, 622 |
| chr13 | 37595672 | 37595921 | CAG GAG TTA TGG CAC CAG TG | ACT TCA TCT TGA CAG CAG CT | 623, 624 |
| chr13 | 38283224 | 38283473 | GAG AGT GTG GAG GCA GAA AA | CAC TTC CTC ATG ATG TTT TGG A | 625, 626 |
| chr13 | 38320894 | 38321143 | GCC CAA CTT ATT TTC CAG CT | GGC CAG CCC ACT TAT TTT TG | 627, 628 |
| chr13 | 38361974 | 38362223 | TCA AGC CCC TTA GAT TGA ACA | CTC AGG GTG GAG TTT CAA AC | 629, 630 |
| chr13 | 38380696 | 38380945 | GCT GGG CAT GTA GAA CTC AA | GAT CTT TCT TCC CCT CCT CC | 631, 632 |
| chr13 | 38789453 | 38789702 | GGG AAA TTG TCA AGG GCT TT | TGT ACA TCC ACC ACT TGT TTG | 633, 634 |
| chr13 | 39009512 | 39009761 | CTT AGT TGC TGT TGT GCT TCT | AGC GGT AGT AAG AAG GCA AA | 635, 636 |
| chr13 | 39096011 | 39096260 | AGT TGT AGC TGT ATC TGG GT | AGA CAG GTG ACC ATT TTC CC | 637, 638 |
| chr13 | 39202882 | 39203131 | AGC TGA GTC ATG TTT AAG GC | TGT GGA ACT TTT GAG CCA GA | 639, 640 |

FIGURE 2 (Page 20 of 80)

| chromosome | start | stop | forward primer | reverse primer | SEQ ID NOs: |
|---|---|---|---|---|---|
| chrX | 44004660 | 44004909 | CAA TTC AGA CTT TGC CCA AAC | CAT AAC GAA ATA GGG CCT TCC | 641, 642 |
| chrX | 44005572 | 44005821 | CAA TCA TTC CCA CAG TTC CAA | ATT TCT GCC TCT TCT CTT CCC | 643, 644 |
| chrX | 80622102 | 80622351 | CTG TGA TGG TCC ATT CAA GG | TCA TCA AGT CAC CTC TCC AC | 645, 646 |
| chrX | 81576088 | 81576337 | TAG CTG GAA ATT GCA AGG AG | TGG AAA ACT AGA CAG CAG CC | 647, 648 |
| chrX | 83458870 | 83459119 | TCT GTT CAC CTG AGC CTT T | GGA AAG GGG AAA AGG TGA CA | 649, 650 |
| chrX | 83461989 | 83462238 | TAA CTT GGA CTG TGA ACC CA | CTG TCC TCT GTC CCA CAT AA | 651, 652 |
| chrX | 83972195 | 83972444 | CGC AAC AGG ATG AAG GAA AT | TTT CTG AGT CCA TTC CCC AT | 653, 654 |
| chrX | 83973091 | 83973340 | GAC TCA CAC TCT GAA AGC CT | GGC CAT CCT GAT ATC TTC CA | 655, 656 |
| chrX | 84308155 | 84308404 | AAT TTA GGT AGC ACT GAC CCC | CAG AGA GAT GCA GAG GTT CA | 657, 658 |
| chrX | 84412933 | 84413182 | CTG GGG AAT TAG GAA GCA GA | TGT GTG TGA GCT AGC TGA AT | 659, 660 |
| chrX | 84530826 | 84531075 | ATG ACA AGG CTG GCT CAT C | GGT TTC CCA TCC TAC CAC AT | 661, 662 |
| chrX | 84531356 | 84531605 | TTA GTT TTG GCA TGT GGT GG | GGT GCT TTT GTT GCC TTA CT | 663, 664 |
| chrX | 84561384 | 84561633 | TGG TGA GGG AGT GTT CTT TT | ACT AGA AAG CAG GGT ACA GT | 665, 666 |
| chrX | 85583338 | 85583587 | TCA TTG GGG GAG TCA TTC AC | GCT TTT CCA ACT TC TGC TG | 667, 668 |
| chrX | 85584098 | 85584347 | GAA GTG GTG TGA TGA GGG TG | CAA CAG ACA AGT CAC CTC CT | 669, 670 |
| chrX | 85643367 | 85643616 | AAG TTA GGC CCT GTT AAG CA | GGT TCT TCC TGG ACT TCA AA | 671, 672 |

FIGURE 2 (Page 21 of 80)

| chromosome | start | stop | forward primer | reverse primer | SEQ ID NOs: |
|---|---|---|---|---|---|
| chrX | 85643847 | 85644096 | TGT TGG TCG GAG TCA GAA AT | GAA AGC AGT AGT TTC AGG TGT | 673, 674 |
| chrX | 85644765 | 85645014 | GTA AAA GAG GTT GGG ATG CC | TGA AAG ACT CTG TTG CCA TG | 675, 676 |
| chrX | 85682212 | 85682461 | CGT TGG ACA TGG ATC ATA CC | CAC TAC ACG CTC AGA ACA AA | 677, 678 |
| chrX | 85715710 | 85715959 | TCA CAA CAA GGG AAA TAG CCT A | AAG GCA AGC AAT AAT GAG GC | 679, 680 |
| chrX | 85764768 | 85765017 | TAG TCA GGT AAA CAA CGC CT | GGA CAG TCT GTG AAA ATT GCT | 681, 682 |
| chrX | 85988749 | 85988998 | TCT GTT TCT TGT TTG GCT GAG | ACA AAC AAC CCT TAA TGC CC | 683, 684 |
| chrX | 85989214 | 85989463 | ACA TAG GTC ACA CAA AGG GT | TGA AAC AGT GAA TCC GCA AT | 685, 686 |
| chrX | 85997574 | 85997823 | GTG TGA CAC TTT TCT GCC TT | AAG GGA AAT GTG GAT GCA GTA | 687, 688 |
| chrX | 87225841 | 87226090 | CTG GAA ATA GAA GGC CTT TGC | TTG AAG GGA AGC GGA AAG T | 689, 690 |
| chrX | 91517109 | 91517358 | TCT TGG TCT GGG AAT AAG CC | ATT CCC AGC TAA TGA TGC TCC | 691, 692 |
| chrX | 91517578 | 91517827 | TGC CCC TAT GAA CAA CAG AA | GCT CAC TTA CGC ATT AAC CA | 693, 694 |
| chrX | 94601042 | 94601291 | TGA ACG TCT TGC TTA CCC AC | ACA GGC AAA ATT CAG TTG GA | 695, 696 |
| chrX | 95282210 | 95282459 | GAA GGA AGG CAG AGG TCA A | AGG CTG AAT CAC GTC AAA AC | 697, 698 |
| chrX | 95700693 | 95700942 | CTT CCA CAA AGT CCT GCA AC | CCT CGT TCA CAT TTG ACG C | 699, 700 |
| chrX | 96016317 | 96016566 | ATG TGA ACC ATT GAG AGG CA | TGT CTG GGT TCA ACT GTT TG | 701, 702 |
| chrX | 96474722 | 96474971 | AAG AGA AAC TAC CCT GGC AA | ATT TTG CAT GCC TGT TGA GA | 703, 704 |

FIGURE 2 (Page 22 of 80)

| chromosome | start | stop | forward primer | reverse primer | SEQ ID NOs: |
|---|---|---|---|---|---|
| chrX | 96475190 | 96475439 | GCA TGT AGT TCA GTT CAG GC | GCT CTC CTC AAA ACC CAA GT | 705, 706 |
| chrX | 96522258 | 96522507 | ATG AAA TGT AAT GGG GTG CG | CTT AAT GAG GGG GCA CAA AG | 707, 708 |
| chrX | 96685785 | 96686034 | TTT TGG CAG TGA TGA CCT TG | TTT TCG TCC AGT CTT CCA CC | 709, 710 |
| chrX | 98226881 | 98227130 | TTC TTG GCT TTT CTG ACC CT | TAC AGG ACC GTC AGT GAG AG | 711, 712 |
| chrX | 98227467 | 98227716 | TTG AGA AAG ACC CCA ACA GAA | TTA GCT ACT GAC GCT TCA CC | 713, 714 |
| chrX | 98314697 | 98314946 | GAA AAT AAC ACA GTA GGG ATG C | GCA CAG ACA ACA TGC TAG TT | 715, 716 |
| chrX | 98331718 | 98331967 | GAA TGG AGA GGC AGT TTT CA | TTA GTC TGT TCA CTG GCA CA | 717, 718 |
| chrX | 102986351 | 102986600 | CAC CCT TTT CCT GTT TTG CA | CTT ATC AGC AGG GCA CAG T | 719, 720 |
| chrX | 112117885 | 112118134 | AGA AGA AAC TTG CAG TGT TGG | TTG CAT CAA ACA AAG CCA CA | 721, 722 |
| chrX | 116461543 | 116461792 | TGC AGC ATT ATT CTT TCT GGG | GAG AGA CAA GTC ACC CCT TC | 723, 724 |
| chrX | 116746667 | 116746916 | ACA CAC ATA TTA GGG AAC AGC | AGT CAA CTA CAA ATG GGG GA | 725, 726 |
| chrX | 117121287 | 117121536 | GAG TGT AGG TGC TTG GGT AT | GTG CCA AAA TCA ACG AAA GC | 727, 728 |
| chrX | 117494627 | 117494876 | CCT TAG AAT CCT AGC GCC TT | AAG GAG GGA GTA CAA AGT GAG | 729, 730 |
| chrX | 118400460 | 118400709 | TCA TGA GGT TGC CAG TGT TT | ATC ACA TTT TCA GCA CGA GG | 731, 732 |
| chrX | 119760145 | 119760394 | CCC CAT ACA TCA TCA CAT GC | CAG GGA GGG ATG ATT TGG AA | 733, 734 |
| chrX | 120436725 | 120436974 | GGG TAA TGC TTT CTT GGG GA | AGA ACT GAG AGG GGA GCA TA | 735, 736 |

FIGURE 2 (Page 23 of 80)

EP 4 095 258 B1

| chromosome | start | stop | forward primer | reverse primer | SEQ ID NOs: |
|---|---|---|---|---|---|
| chrX | 120437307 | 120437556 | AGT TGA GAA GGG AAG GCA AG | CAG TCA CCA ACA AAG GCT TT | 737, 738 |
| chrX | 121854317 | 121854566 | CTC CTG GTG GCT TAT TTT TGA | TCC CCA TCT CCC TAA CTC AT | 739, 740 |
| chrX | 121855104 | 121855353 | TCT GGA TTT TGG CTA CTC ATG A | TTT TTC TGC TGC ATC CAA GG | 741, 742 |
| chrX | 122863038 | 122863287 | GTA GTC CTC CTT TGC CCT TC | ACT GTA CGC CAT GAA AAA CA | 743, 744 |
| chrX | 123203756 | 123204005 | GAC ATG CAC AGA TCG AAA CC | GGC AAA TCA AGT GAG CTG AC | 745, 746 |
| chrX | 123989030 | 123989279 | CGC ATT TGA CAA CAG GGA TC | CGT GGG TGG AGA ATT TCA CA | 747, 748 |
| chrX | 124394967 | 124395216 | AAG CCA CCT GTT CTC TCT CA | ACT TAG GTC AGT TGC TTG GT | 749, 750 |
| chrX | 125455385 | 125455634 | ATT CCA ACC ATT CCG ACA CC | GAC TTC ATC AGC ACG TAC TT | 751, 752 |
| chrX | 125500101 | 125500350 | AAA GAA AAT GGT GAA CGT GC | TGC AGG CAA AAT TAG CAT GG | 753, 754 |
| chrX | 125500582 | 125500831 | TGA TCA GGG CTT TAG AGG TC | GAC TTA TCT GCT TTC ACC CC | 755, 756 |
| chrX | 127973758 | 127974007 | ACT CCC TAT TGT TCT CCC CT | CCC CAT GAA CCT AAG ACC AT | 757, 758 |
| chrX | 128274367 | 128274616 | CTC CTT GAC AGA TGT GAC CC | TCT GGA CAT GTC TTT GCG TA | 759, 760 |
| chrX | 128274689 | 128274938 | TTT TGG AGT CTG AGC CAC AA | CTC CAG GAC ATC TCA GCA AT | 761, 762 |
| chrX | 128275238 | 128275487 | TTG AAG TCC CGT TGC TGA T | CCT CTC GTG TGG GAA ATG TA | 763, 764 |
| chrX | 128694864 | 128695113 | TTG GGG TCA GTT CTA ACA GT | TAT CTC TGG CTA CCT CCT GT | 765, 766 |
| chrX | 131201343 | 131201592 | TTT TCA CCA CCT CTT CCC TC | CCC CAT CCC TGT ACC AAA G | 767, 768 |

FIGURE 2 (Page 24 of 80)

| chromosome | start | stop | forward primer | reverse primer | SEQ ID NOs: |
|---|---|---|---|---|---|
| chrX | 132160888 | 132161137 | ACC TGA CCA CAA GCT TTA CA | CTG CAG AGA TAT TCC ATG GC | 769, 770 |
| chrX | 133560531 | 133560780 | CAC ATA TTG GCG CAC AGT AC | AAA GCT GGG TTC TTA GGC TT | 771, 772 |
| chrX | 133847304 | 133847553 | GCC ATG CAC CGA TGA AAA AT | GCT GTT TTA GGG GCA CAT TT | 773, 774 |
| chrX | 133886077 | 133886326 | TTG TGA GGA GAT TTC TGG GC | AAG AGG CAA TGT GGA GGT TA | 775, 776 |
| chrX | 133886778 | 133887027 | CAC ACT AAG AGC ACT GGG AA | GCC CAA ACA ATC TGC CTT TTA | 777, 778 |
| chrX | 135279738 | 135279987 | ATG ACC TAG CAC ATC TTC CC | TGG CTT CAA ATA ACT GGG CT | 779, 780 |
| chrX | 135581031 | 135581280 | ACA TTT TCC CCA TTC CAT GC | AGA GCA CAC AGA ACA GAA CT | 781, 782 |
| chrX | 136941543 | 136941792 | GTC TGT CAA CCA CAC TTT GC | CTG GTG GAT TTC TCG TCA GA | 783, 784 |
| chrX | 138913313 | 138913562 | CTG TCT CTC CTT TTG CCA AA | TAG GTG TTT GTG TGA GGC TT | 785, 786 |
| chrX | 138913920 | 138914169 | AGT AAC CTG CGA CTC TCA GT | GCC TCA CTG CTC CTA TCT TT | 787, 788 |
| chrX | 139857034 | 139857283 | TAG CAT TTA AGG AGT GGG CT | CTC TAG CAG CTG TTC CTC C | 789, 790 |
| chrX | 139857318 | 139857567 | GGC CTC CTC AGT GAT TTG AA | CGT CGT ATC TCT GGC TTT GT | 791, 792 |
| chrX | 141400062 | 141400311 | CTT TCT TTG CCT CCC CTG TA | GTC CCC AAC CTC ATC TTT CA | 793, 794 |
| chrX | 141408144 | 141408393 | ACT TCC ATT TGT GTC AAC GG | TCT TCA AAG ATG GCT GCA AA | 795, 796 |
| chrX | 141408548 | 141408797 | TCT TGC TTT GGG TTA GAG GG | AGT ATA ACC AGA TAG CCG TGC | 797, 798 |
| chrX | 142517923 | 142518172 | AAC CAC ACC TCC ACA AGA AA | TGT CCT CAG GGC AAT AAA GT | 799, 800 |

FIGURE 2 (Page 25 of 80)

| chromosome | start | stop | forward primer | reverse primer | SEQ ID NOs: |
|---|---|---|---|---|---|
| chrX | 143466384 | 143466633 | CAA GAT ATG AGG GAG GGG AA | TTT GCT GCT TGA AGT GGA AC | 801, 802 |
| chrX | 147766073 | 147766322 | TCT AAC CTG GGC CCT TTC TT | GCT GCA ATG ACC TGA TTT CT | 803, 804 |
| chrX | 147869477 | 147869726 | TGC CCT TCC AGA ACT GTA AA | TCC CTC TCT CCT CCA AAT GA | 805, 806 |
| chrX | 147880766 | 147881015 | GCC AGG TCA CTT AAC AAA GC | TGC CAC AGT AGG TAT AGG TTG | 807, 808 |
| chrX | 147881811 | 147882060 | CAA TAA GGC GCC AAG TTC G | CCC TCG CCC TAA AGA AAC TA | 809, 810 |
| chrX | 151796091 | 151796340 | GTC ATC AGG GGA GCA AAT GT | CCC CTG AAT CCC TAC CTC AT | 811, 812 |
| chrX | 104102171 | 104102420 | AAC ATG CAA TCC CTG GAA TTC | TGT TGT ACA AGT GAG CCA TTC | 813, 814 |
| chrX | 104124449 | 104124698 | AGT TGT TTC AGG ACA GGA TCT | ACC AAG CAA TCA ACT CAC TCT | 815, 816 |
| chrX | 104234605 | 104234854 | TCC TCC TGC CTT TAA TAA GCT | GCC CAA TTT GTC TAG CCA ATA | 817, 818 |
| chrX | 104235195 | 104235444 | TTA CAA GGC ATC TGA CAG GAA | CTA CTG ATC CCA AAG AAG GCA | 819, 820 |
| chrX | 104291380 | 104291629 | TTA CTG GTA GGT TTG AGC ACA | GTG AAA GGT TCT ATC TGC CAA | 821, 822 |
| chrX | 104291786 | 104292035 | GAG CTA CGT TCT TTC TCA TCA C | GGC ATA CCG AGC ATA CAT AGA | 823, 824 |
| chrX | 104647680 | 104647929 | CAC CAA TTA AAG TGT GCT GCA | CAC CTG TTT CAC CAA ATC ACT | 825, 826 |
| chrX | 104648691 | 104648940 | TCC CTT CCA AAG TGC CTT ATA | TCT CAT GCT CTG ACA GAC AAG | 827, 828 |
| chrX | 106886942 | 106887191 | TGG TTG GTT TGG GAT ACT TGA | TTG TCC TGT TTC TCT TGT GAC | 829, 830 |
| chrX | 3343487 | 3343736 | GAA CCC AAA TCG ATC ATG CAT | CAC AGG ACT GCA TGC CTA TTA | 831, 832 |

FIGURE 2 (Page 26 of 80)

| chromosome | start | stop | forward primer | reverse primer | SEQ ID NOs: |
|---|---|---|---|---|---|
| chrX | 4013011 | 4013260 | AGA GTT AAA CGT GCA ATG TGG | GAC TCT CTC AGC ATC GAG TTT | 833, 834 |
| chrX | 5008870 | 5009119 | GTT CGT TGG TCA TAG TTG TTG T | CTG ATG GAA GGG CAT TAT CCA | 835, 836 |
| chrX | 5687782 | 5688031 | AGT TTA GCC AAA GGA TTC AGC | TTC TGG TTC CAT AAA TCC ATG C | 837, 838 |
| chrX | 5717003 | 5717252 | TCA ACC ATT TAG AAC CAC CTT G | GGG ATT ATT GTT GGC TAC TGA G | 839, 840 |
| chrX | 5808998 | 5809247 | AAT GTC CAC TTT AGC GGA GAG | AAT TGG TGA CCT AGG GAT CAG | 841, 842 |
| chrX | 5814187 | 5814436 | GGA GTA TTC TGT TCA TGT TGG G | GGG TTT GGT AAG GGA GAA TGA | 843, 844 |
| chrX | 5836574 | 5836823 | TTT CTA GAA TTG AGG AAG GGC A | AA GAC ATC CCA GTT ATG CAT TGT | 845, 846 |
| chrX | 5849776 | 5850025 | AGG ATA AGA CGA GGC ATC AAT | AGA TGG GAG GGA GAT TAG ACA | 847, 848 |
| chrX | 6895474 | 6895723 | TTC TGT GTT GAC ATG TAC CTC T | GAG TAG CAA CAA CAC ATG GAG | 849, 850 |
| chrX | 6965226 | 6965475 | TCC AAC ATT TCT CTC TGT CCC | TTC TCC TTC ATT AGC CAC ACA | 851, 852 |
| chrX | 6965573 | 6965822 | ATA ACG TGT ACT CCT CAG CC | AGT CTG CAC TGT ACT CTT CTG | 853, 854 |
| chrX | 7152743 | 7152992 | GCA CTT GGA GGA TGT AAA GAC | ACA AAT GGT TCA TGA TGG TGG | 855, 856 |
| chrX | 9529739 | 9529988 | TGT ATG CTT TAG GAC CCA GTT | GGT ACT CAC GTT TCA GTT TCC | 857, 858 |
| chrX | 9605323 | 9605572 | AAC TCC ACA GGA ATC TTT CTG A | GTT CAT TTC TAC AGT CCA GGC | 859, 860 |
| chrX | 9615158 | 9615407 | CAT TTC TCC TGG GAC CGA AT | CTC TCA CTG TGC TGC TTA AAG | 861, 862 |
| chrX | 9766329 | 9766578 | TTC TGA AGC TGA CGA AAT TCC | CAA AGA ATT CCA CAG AGA TGG G | 863, 864 |

FIGURE 2 (Page 27 of 80)

| chromosome | start | stop | forward primer | reverse primer | SEQ ID NOs: |
|---|---|---|---|---|---|
| chrX | 9793372 | 9793621 | GTT GCT TAG TCC TTG CTT CAC | TGA GCT ACA GAC AAG ATT GCA | 865, 866 |
| chrX | 9849298 | 9849547 | AGG TCA TTG GTC TGC AGT TAT | TCA CAT GGG ATC GAC ATA TGC | 867, 868 |
| chrX | 9850182 | 9850431 | AGC ATT TAG AGA ACA GCA GTC | CCA CAC AAT TTC CTG GCT ATG | 869, 870 |
| chrX | 10002581 | 10002830 | GTG TAA GAA GTG GTT GGG TTT | TCT TGC TTC TGG AGA GTT CTT | 871, 872 |
| chrX | 10301512 | 10301761 | AAA GGC AGA GCA GTG TAT TTA G | AGG TTA TGC AGA CTT CAG GAA | 873, 874 |
| chrX | 10354762 | 10355011 | AGA CAA GAG AAC AAT CAG GTG A | AAG CCA ATT CTG CCT CTC TAG | 875, 876 |
| chrX | 10375361 | 10375610 | CAA AGA AGC TCT AGG ACA GGA | TCC CTT CCT TAT TCT GGC AAC | 877, 878 |
| chrX | 10384260 | 10384509 | TGG TGG AGG AAA TCA ATG TTG | GCT TCA AAC ACT CTA AAG GGC | 879, 880 |
| chrX | 10398645 | 10398894 | CAC AAG GGA GGA AAC GTT CT | TGC CAT TAA TGA GAA GTG CTG | 881, 882 |
| chrX | 10572719 | 10572968 | TGA GAT TTA GTG CCA GCT AGA | TGG CAA TCC TGT TAA ACA ACT C | 883, 884 |
| chrX | 10579800 | 10580049 | AGG ATT AGT TTG GCT CCT CAG | CCC GAA CAT TGA TAA CAG AAG A | 885, 886 |
| chrX | 10580482 | 10580731 | CCT GCA CTA TTT CCT CAA AGC | GCA ACT CAG GAA AGA CTA CAT C | 887, 888 |
| chrX | 10627502 | 10627751 | CAA TTT CCT TCT CAC TGA GCC | TTA AGA AAG TAC CCA TCC TCC C | 889, 890 |
| chrX | 10645064 | 10645313 | ACG CTT CCC AAA TCT ATC TGG | GTC ATG CCT TAC AAC TTA GCA | 891, 892 |
| chrX | 10669664 | 10669913 | CTT ATT TGT GTG CCC AAT ACC A | AAT CTT TGC CAA GGT ATG AGC | 893, 894 |
| chrX | 10709674 | 10709923 | TTG CAG CAG GAA CAC CAT AAA | GCC ACT TAT ACC TCC AGA CAT | 895, 896 |

FIGURE 2 (Page 28 of 80)

EP 4 095 258 B1

| chromosome | start | stop | forward primer | reverse primer | SEQ ID NOs: |
|---|---|---|---|---|---|
| chrX | 10710087 | 10710336 | CTG TTC ATG TTG CTA CCA CAG | CCA CAA TCC TGA ATG CCA TG | 897, 898 |
| chrX | 11139863 | 11140112 | CTG CCT TAG ATT CAC TTT CGG | CTG CAA GGT ACA ACA CAA GTC | 899, 900 |
| chrX | 11658912 | 11659161 | CCA TCT GTG AGG TCT TCT TTG | ATC TCT GTG CCA GCA AGT ATT | 901, 902 |
| chrX | 11681358 | 11681607 | TCC TTG GTT GTG TAT TTA GCC | ATT GGG AAA CTG TCA CTG ATG | 903, 904 |
| chrX | 11686196 | 11686445 | AGA CTC AAC TCA CAT TGG CC | GCC CTA ATA GAG AAG CAA AGC | 905, 906 |
| chrX | 11686913 | 11687162 | GGT CTG ACT CTG TGG TTT GG | GGG AGC CAA TCA GAT AGA AGT | 907, 908 |
| chrX | 11687182 | 11687431 | TTT CAT TTC ATC CTG CCC ATG | GGG AAG TTG GGC TAT TTA ATG C | 909, 910 |
| chrX | 11917847 | 11918096 | TTC TGT TAT TCG CCA TCA GTC | AAC TGT GTA GAG CGA CCA AAT | 911, 912 |
| chrX | 12016474 | 12016723 | GAA TTG GGA ACT TGG GAA GC | CAG TAA GGC CAT GGT CTA GAT | 913, 914 |
| chrX | 12158815 | 12159064 | GAA CTT GGA AGA GGA CAG TGT | CTC AGA ACA TTT GCA CCT TCT | 915, 916 |
| chrX | 12485152 | 12485401 | AAC TGT CAT GTG TGT CTG CTA | ACC TGA TAC AAT GGA GCA TGT | 917, 918 |
| chrX | 12608017 | 12608266 | TGA TTC CTT CCA CCT ACC AAA | GGG ACC TAA ACT CCT TTG GAA | 919, 920 |
| chrX | 12724960 | 12725209 | GCC AAG GTC CAT TAT CTC AAG | TCT GCA GTG GTG TTA TCT AGT | 921, 922 |
| chrX | 12841791 | 12842040 | GAA CCT GCA TTG TCA TTC TCT | CAC TTA AGT TTC CAC GCC AG | 923, 924 |
| chrX | 12904558 | 12904807 | AAG AAC ATC AAC AAA CTC CAG G | GTA ATG GCG AGA GGT TAA AGC | 925, 926 |
| chrX | 12937788 | 12938037 | TCA ATT CTC TTT CAC ACG TGC | TTT AGG TAT CGA AGT TGG GTC A | 927, 928 |

FIGURE 2 (Page 29 of 80)

| chromosome | start | stop | forward primer | reverse primer | SEQ ID NOs: |
|---|---|---|---|---|---|
| chrX | 12938100 | 12938349 | AGG TGC TGG ATC TTG AAT TCA | GAT AAG TTT GGA AGC TGC ATC A | 929, 930 |
| chrX | 12992266 | 12992515 | TCC CTC TAC CCG AAT CTC TTA | AAG CTC TGC CAT TGA CTT TAC | 931, 932 |
| chrX | 12995926 | 12996175 | TGG GTT TAA AGG ACA CTA GCA | CCC TAC AGA ACC GAG GAA TC | 933, 934 |
| chrX | 12997273 | 12997522 | CGA AGG TCA CAC AGT TTA GTC | TCA ATC TTT GCA TAC ACA GCC | 935, 936 |
| chrX | 13293562 | 13293811 | ATG TGC CTT GTT GAT TGA TGG | GTA GGT TTA CAT GGA CAG ATG C | 937, 938 |
| chrX | 13338207 | 13338456 | AGA TGG TAT GTC ACA AAG CAC | TCT TCT GTT TAG TGC TGT GGT | 939, 940 |
| chrX | 13340422 | 13340671 | ACA CTT TGG AGA GCT TCA GAT | CAA GTT CAT TTC TTC CCT GCA | 941, 942 |
| chrX | 13351532 | 13351781 | GTG CCC AGA ATT ATT TGT GTC T | TGC AGG AAT ACA TGG TAG ACA | 943, 944 |
| chrX | 13624857 | 13625106 | GCT CTC TTG TGG AAA CGA TTA | CAT AGG CCT TCA TGT CTC TCA | 945, 946 |
| chrX | 15525635 | 15525884 | AGT TTG TTT CTC TGG CCT ACT | TAT GAG GGT GCA CTA ACA GAT | 947, 948 |
| chrX | 15543469 | 15543718 | ACC ACC TCA AAG ATT TCA TGG | GGT GCT ACT ACT GGT GTA TGT | 949, 950 |
| chrX | 15543853 | 15544102 | GTC TTC ATC TAT TTC GTG AGC C | GCC ATG CAA TAT CAA ATC CCA | 951, 952 |
| chrX | 15844810 | 15845059 | TCA TCC CAG ATT CAG AAT GCC | GAA ACC AAA GAC TAG TGC AGC | 953, 954 |
| chrX | 16860721 | 16860970 | CGT TCA ATG AAG TCC CTT GTC | ATG ACT AAC ACT CTG CCA AGT | 955, 956 |
| chrX | 16861729 | 16861978 | TTC ACA AGA ACT CTG CTG GAT | AAC AAA TGC ATC CCA GAC AGA | 957, 958 |
| chrX | 18307677 | 18307926 | GAA GCC TTC TAG TGG GAC TAA | GCA GAG AGG AGT ATG TGG TAT | 959, 960 |

FIGURE 2 (Page 30 of 80)

| chromosome | start | stop | forward primer | reverse primer | SEQ ID NOs: |
|---|---|---|---|---|---|
| chrX | 18600682 | 18600931 | GTT AGG GTC ATG GGT CAC TTT | ATG GGT CAT TCT ACG AAG CAA | 961, 962 |
| chrX | 19376384 | 19376633 | GCC TTT GTA GAG TGG ACT TCT | TGG TGT GTG TGG TGA TAA TTA G | 963, 964 |
| chrX | 20026004 | 20026253 | GGC TTC TTG ATA CTG CTT TCC | AGT GAC CCT CTG AAT AAC CTG | 965, 966 |
| chrX | 20627108 | 20627357 | AGG TGT GCA ATA CTC AAG GAA | TCA GCA AGT AAA CCT GAG ACC | 967, 968 |
| chrX | 21537202 | 21537451 | AAG GTC TTA GGA GTG AGG ACA | GTA CCT TCA CCC TCC AGA TC | 969, 970 |
| chrX | 21629967 | 21630216 | TCG TGC TAT TTC AGT CAG ATC T | TCA TCA AAT TGC CCA CTC CTA | 971, 972 |
| chrX | 21887062 | 21887311 | TGT CCA GCC GTA ACA TTT CAT | CAT CAG ACT GTC TTG CCT TTC | 973, 974 |
| chrX | 22202443 | 22202692 | AAT GGA CAT CTT TCA GGT CTG | CAT TCT TGC TGA CAT TTC CCA | 975, 976 |
| chrX | 23018625 | 23018874 | TCA AAT TGG GAT CGC ATT AGG | GAA GAT CAG GGT ATT GCT GAA A | 977, 978 |
| chrX | 23019006 | 23019255 | ATG CCT GGG TTT ATT CAT CTT G | TTT GTA GGT CAT TCA GCC TCC | 979, 980 |
| chrX | 24521525 | 24521774 | GGA GAA GTT TGG GTT TGA TCC | TGG CTA GGA TTC ACT TAG GAA A | 981, 982 |
| chrX | 24522047 | 24522296 | TGA TAG GAG CCA TCA GTT CTT | GCA AAC AGG GTG AAT TAT GCT | 983, 984 |
| chrX | 25401534 | 25401783 | AGC AGA TGT TGT TAG CTT TCC | AGA AGT CTG GGA AAC GAA GAG | 985, 986 |
| chrX | 25403300 | 25403549 | TTC TCT GTC ACT TCC ATG AGG | CAG ATG CTC CAT TAC TAG GTG | 987, 988 |
| chrX | 28689766 | 28690015 | CCA GTA ACT TAT TCT GCC AGA G | CAC ATG GAG AAA GGT GAA TCA | 989, 990 |
| chrX | 28693600 | 28693849 | TGA GAG ACA AGC TGC ATT ACA | TCC CAT CCA ATA CTG CCT TC | 991, 992 |

FIGURE 2 (Page 31 of 80)

| chromosome | start | stop | forward primer | reverse primer | SEQ ID NOs: |
|---|---|---|---|---|---|
| chrX | 28717026 | 28717275 | GCA CAG AAA TTA CAG TTC ATG G | TCT GGC TCA AAG GAT CAC ATG | 993, 994 |
| chrX | 28720131 | 28720380 | GCT CTC GTA TCT GAC AGT GAA | AAG ATC CCA TTG ACC CTG AAT | 995, 996 |
| chrX | 28808128 | 28808377 | CAG GCA TCT TGG TTT GTA GTG | CCA CAG GCT CTC TAG AAC TAA | 997, 998 |
| chrX | 28824813 | 28825062 | CGT GAT GAA CAG TGA TGA CTT | TTG AGA GGG TTT ACA AGG TCC | 999, 1000 |
| chrX | 28825844 | 28826093 | CGC CAT TTG TTC TCC TAT TCA | CTT CTC CTA CTC TGC ATT CTC A | 1001, 1002 |
| chrX | 29487025 | 29487274 | TTC GTT AGC TAC TGG GTA CTC | GAC ATT AGT GGA TTC AGG CCA | 1003, 1004 |
| chrX | 30847194 | 30847443 | CCA CCC TTT ACA CCT ATC CAA | GCT GAA GTG GAG GCA ATT AAC | 1005, 1006 |
| chrX | 32660941 | 32661190 | AGA GTG CAC AAA GGA GAA GAC | TAT AAC TGT TGA GTC TGC CCA | 1007, 1008 |
| chrX | 32949975 | 32950224 | TCT ACT GTG TCA AAG CAG ATT G | ACT GAG CTT ACA TTC ATG CAC | 1009, 1010 |
| chrX | 35971182 | 35971431 | TTC TTC CTA GCC TTC CTT TCC | GCC ACT TTC TCT GCA AAG AAT | 1011, 1012 |
| chrX | 35971544 | 35971793 | TCT CTG GCT GTG CAG TAA ATT | ACT CCT ACG GAC TCA AAA TCT | 1013, 1014 |
| chrX | 43599991 | 43600240 | AAA CTC CCA GCT TTA ATC CCT | GGC TCT CAT TAC AAT TGG CTG | 1015, 1016 |
| chrX | 43600262 | 43600511 | AAG AAT GGG TGA GTT GGG TTC | ATT GCT TTC AGT GGT GGA TTG | 1017, 1018 |
| chrX | 43807492 | 43807741 | GGA AAC TGA ATT GCC AAG TCT | GGA ATG AAA CAG AGG AGT CCC | 1019, 1020 |
| chrX | 63137429 | 63137678 | CTC CCA ACT TTT ATG CAG CC | AAG ATG CTA GAA ACC CAC AAG | 1021, 1022 |
| chrX | 63137928 | 63138177 | GCT CAG GGA ATA TCT TGG GAA | AAT GGG TGG GTT ACA GAG AG | 1023, 1024 |

FIGURE 2 (Page 32 of 80)

| chromosome | start | stop | forward primer | reverse primer | SEQ ID NOs: |
|---|---|---|---|---|---|
| chrX | 74269728 | 74269977 | ACA TTC AGC AAG TAG GAA GGA | GCA TTT GGA CAT GAA CAA GC | 1025, 1026 |
| chrX | 74270926 | 74271175 | CCC AGA AGA GCA GTA ACA AC | CAT TGG TGG GTG GAT AGC TG | 1027, 1028 |
| chrX | 74282022 | 74282271 | GAA AAA GGG GGA TAG GCA TT | ACC AGG AGG AGA AAA GCA AA | 1029, 1030 |
| chrX | 74329664 | 74329913 | CCC AAC AAC TGC AAT AAA AGG | CGG AAA ACA AAC CCT GAA GT | 1031, 1032 |
| chrX | 74344769 | 74345018 | ACC GAA ATT GCT TGC TCT TA | ACC TGC ATA TTG AGC CAT AC | 1033, 1034 |
| chrX | 74741441 | 74741690 | CCT TAG TGT GAC AGG ACA GG | TGT TCT TTC ACT TTT AGC CCC | 1035, 1036 |
| chrX | 76192017 | 76192266 | CCA AGA CAA CTA GGC CAA TG | TTT CTA GAA CCC TCA GCA ACT | 1037, 1038 |
| chrX | 76194203 | 76194452 | GAA GAG ATG ATG CAA AAG AGC | CCC CCA ACA GTT TTT AGT GGT | 1039, 1040 |
| chrX | 76221949 | 76222198 | TCC AAG CAA GGG ATC TCT TC | AAG GCA AAA CAC TCC CTT TT | 1041, 1042 |
| chrX | 76226569 | 76226818 | GAA TGG TCA GGG AAG GGT TT | CAG TGA TTG CCT CTA GAA AAG G | 1043, 1044 |
| chrX | 76234682 | 76234931 | AGT CTT CAG CCA TCT TCC TG | CTA AGC AGA TTG AAG CAG CT | 1045, 1046 |
| chrX | 76308591 | 76308840 | GCA TTT CCA GGC TTT ACA AGT | TGT CCC CAG GCT TAA GAA TC | 1047, 1048 |
| chrX | 76647025 | 76647274 | CTC TCT CTC CCT GGT CAG AT | AGC CAG AAT AAG CAA CTG TC | 1049, 1050 |
| chrX | 76711476 | 76711725 | CAG CAA TTC TCA GGC TCA GA | TTT CTC CTA TCC CAG CTT GC | 1051, 1052 |
| chrX | 76759387 | 76759636 | TCT GAA ACA AAG CCT CCT TAG | AGT ACA GAG GAT AAC AAG GGT | 1053, 1054 |
| chrX | 76778190 | 76778439 | AGG ATA AGG TTT CCC ATG CTC | CTC CCA TCA GTA CCC TCT CT | 1055, 1056 |

FIGURE 2 (Page 33 of 80)

| chromosome | start | stop | forward primer | reverse primer | SEQ ID NOs: |
|---|---|---|---|---|---|
| chrX | 76778847 | 76779096 | ATG TAT CTG AAG GAG CTC TGC | GGA ATG CCT AAA CCA TAC TGT | 1057, 1058 |
| chrX | 76875971 | 76876220 | GGC CTT CAT CAC AAA CAA CA | AGT GGT ATT TCA ATG CTC TAC C | 1059, 1060 |
| chrX | 76923678 | 76923927 | TGT GTC CCA TCT ACA AAG CC | GCA GTA CAT CGT CCT GGA A | 1061, 1062 |
| chrX | 76937441 | 76937690 | TCC TTG AAC TCT TTC CAA GC | CCT GAG CGA GAA GAA ATT TGT | 1063, 1064 |
| chrX | 76938848 | 76939097 | TGT TTT CCT GTC CAA GTC CA | GGA ACT GAA CAA GAA GTG GAG | 1065, 1066 |
| chrX | 76939481 | 76939730 | CAG CAT CCA TCG CTC GAA A | ACC TAC TCT TAT TCC GCA CT | 1067, 1068 |
| chrX | 76949897 | 76950146 | GGT ATT GGT GGG GGA AAT GA | TGA CAG CCT CTC TCT TCA AT | 1069, 1070 |
| chrX | 76951314 | 76951563 | GCA CTG CTG TAA AAG ATC TAT GAG | | 1071, 1072 |
| chrX | 77040328 | 77040577 | ACT CAA AGG CAC ATT TCG C | GCT GTT TTT CTG TGT GCT TC | 1073, 1074 |
| chrX | 77168344 | 77168593 | ATT CTA TTC CGA TCA CAG CCT T | ATG TAT TTC CTT TAG CGC CC | 1075, 1076 |
| chrX | 77634270 | 77634519 | CTA CCT GAC AAA TGG AGC TT | AAT TTT GCA AGA CTT CCG GT | 1077, 1078 |
| chrX | 78176076 | 78176325 | GAA ATG GCC ATG TGT ACT GAG | TCC CAG TTG TGA ACA TTT GC | 1079, 1080 |
| chrX | 78176555 | 78176804 | GAA GCC TCT CAA GCT ACA AG | ATG GGT TTT TGC ACA GAT GAC | 1081, 1082 |
| chrX | 78178901 | 78179150 | GGA TGA GAT TAG GGA GCA AAG T | GGG AGT CAG AAG GAG GTC A | 1083, 1084 |
| chrX | 78208245 | 78208494 | GGA AGT AAG AAG AGT GCT GC | CCT CTT TTT GCA TGA ACC TGA | 1085, 1086 |
| chrX | 78290291 | 78290540 | GCT CCT GAT TGA AGA AGT GT | CCT TAC CCT TTC CAC TCA GA | 1087, 1088 |

FIGURE 2 (Page 34 of 80)

| chromosome | start | stop | forward primer | reverse primer | SEQ ID NOs: |
|---|---|---|---|---|---|
| chrX | 78401753 | 78402002 | CTG CTC CTT TGT CTC CTG T | GAC TGG TAT AAT CTT GCC GTG | 1089, 1090 |
| chrX | 78403248 | 78403497 | TAA GGT GAG AGT GTG AGG AAG | TGT CTG CAT CTT GAT CTC TGG | 1091, 1092 |
| chrX | 78405536 | 78405785 | CCA GGG GAA CAT TTA CTC AGA | GGT TCC ACA GCA TTT GAG C | 1093, 1094 |
| chrX | 78411791 | 78412040 | AGG GTA CAT GTA AGG CAG CT | AGG AGC CCT TAA CTA TGG TG | 1095, 1096 |
| chrX | 78427326 | 78427575 | TTC TGT GGC ATT GTG TCT TG | TGG CTT GGG TAT TGC AGA TA | 1097, 1098 |
| chrX | 78430991 | 78431240 | CCC CAA ATT TAC CCC ACT CT | GGG GAC AGT AGA AGA TGA GT | 1099, 1100 |
| chrX | 78431372 | 78431621 | ATC CCT AGC ACT TTC AGG AC | AGT TGT TTC TGG ACG GAC TT | 1101, 1102 |
| chrX | 78437851 | 78438100 | TTA CCA ATC CAT CCA GCC TG | AGA ATT TCC CTG TCC ATA CCA | 1103, 1104 |
| chrX | 78526539 | 78526788 | AGA AGC TAA ACA GGT TGC CC | AGT GAG TGA ACT TGC CAT CA | 1105, 1106 |
| chrX | 78531686 | 78531935 | CAT AAG AGC ACA GCC AAG AT | GGC TTT TCT CTG CAC TGA TT | 1107, 1108 |
| chrX | 78996675 | 78996924 | TGT CCT GCC ACT TTT ACA TC | GCA AAG GAA ACA GGC TAA CTA | 1109, 1110 |
| chrX | 107682036 | 107682285 | GAG CTA AGC AGA ACC TAA GGA | ATG GCA GCT GAA TCG ATA TCT | 1111, 1112 |
| chrX | 107683930 | 107684179 | TTA AGC AAC AGG AAC CTA CCC | TAG GTG ATG GGC AAA TTC TCA | 1113, 1114 |
| chrX | 110366745 | 110366994 | GCC AAG GGT AAT CAT AGC AAC | GAA ATG CCT TCC CAC TTA CAA T | 1115, 1116 |
| chrX | 110367927 | 110368176 | TCA TAA TGA AAC CCT TGC TGC | ATT GGC AAA TGT ACC TGA AGC | 1117, 1118 |
| chrX | 110368330 | 110368579 | AAT CCT AGT GCA TGA GAC TCC | CAA TGA TTG CTC TTG TGC CAA | 1119, 1120 |

FIGURE 2 (Page 35 of 80)

EP 4 095 258 B1

| chromosome | start | stop | forward primer | reverse primer | SEQ ID NOs: |
|---|---|---|---|---|---|
| chrX | 110443307 | 110443556 | TAC TGC CTG CAT CAT TAC CAC | AGC TGT CCT CCT CTC CAT ATA | 1121, 1122 |
| chrX | 110466084 | 110466333 | ATT AAC AAT GAG GAG CCA GGT | GGC GTT CAA GTT ACT CGA TTG | 1123, 1124 |
| chrX | 110537791 | 110538040 | GAG AGA TGG TTG AGA AAT GCC | TAG TGA CTA GCT TTG GAG AGT | 1125, 1126 |
| chrX | 110538060 | 110538309 | ACT ACA ACC ACC AAT TAC AGC | AGC CAA TGA TCC CTT ATG ACT T | 1127, 1128 |
| chrX | 110538444 | 110538693 | AGT GAC ATT TCC AAG GGC TTT | AAC TAA ACT GGT AGG CAA TCG | 1129, 1130 |
| chrX | 110575740 | 110575989 | GGA GAT GGG AAG ACG ATT AGA | CAA TCA GAC CAC AGG AAG GAA | 1131, 1132 |
| chrX | 110576099 | 110576348 | AGT CTT TCA GTC TTA CAT GGG T | GAT GTG TTT ATT GCC TGT GGT | 1133, 1134 |
| chrX | 110950649 | 110950898 | TAG TTT CTG TGA TCC TGG CAG | GCT CCT TTC ATA GTT TCA GGG | 1135, 1136 |
| chrX | 110971144 | 110971393 | TTT CTC CCA GCT GTT CCT AG | ACA CAC TGC AGT TCT CAC TAT A | 1137, 1138 |
| chrX | 121597454 | 121597703 | TAC GGA ACT TCG AAT CAA CTC | TGA ATC AAG TGA CAT GAC AGC | 1139, 1140 |
| chrX | 135558463 | 135558712 | TCA AGT CAC CCT CAT TGT AGG | TTC TTA CAG TCC TCA GCA CTT | 1141, 1142 |
| chrX | 135570815 | 135571064 | TGT AAC GTG GAT GTG AGA TTG | CCA CTA GGC TGC ACT AAT GTA | 1143, 1144 |
| chrX | 135760826 | 135761075 | TTG AGC TAA GTC TGC ATC ACT | CAC TAG CTT CTG TAA CTG TGT G | 1145, 1146 |
| chrX | 135831981 | 135832230 | CTA GAG AAA GCA ACG CCT AAG | GTC TCA CAC TGC TCA TTT CCA | 1147, 1148 |
| chrX | 136044394 | 136044643 | AGC ACC TTC CAT AGC TTC TTT | CAC TAG GGT TCA TCA GCT GTT | 1149, 1150 |
| chrX | 136318161 | 136318410 | GTG TCT TCT GAT GGC CAA ATG | CAA CCA ACA TTA GAG TGA CCC | 1151, 1152 |

FIGURE 2 (Page 36 of 80)

| chromosome | start | stop | forward primer | reverse primer | SEQ ID NOs: |
|---|---|---|---|---|---|
| chrX | 136320360 | 136320609 | AAT CAG GTG AAA GGT ACC TCC | GCT GGT TGA GAG AGA GAG AGA | 1153, 1154 |
| chrX | 139380500 | 139380749 | CCT TCA CAA TTC AGG GAA CAA | ACA CCA GCC GAA TAC AGA TTT | 1155, 1156 |
| chrX | 139381074 | 139381323 | TGG ACC CAG TTC TAT GCA ATT | TGC TTC ATA CCT TTC TGC TTC | 1157, 1158 |
| chrX | 144897923 | 144898172 | AAT GAC CCT TAC AAC TCC GAA | ATT TCC CAT GCC TTT CAA CTC | 1159, 1160 |
| chrX | 144908837 | 144909086 | TTT GGG AAG TGA TTG TGA AGG | TCT CTC AGT AGG CGT CTT TAA | 1161, 1162 |
| chrX | 144958353 | 144958602 | TCA GCT GCA TAG ACC TTG TTT | TGA CTG CTA CGC TAG ACT TG | 1163, 1164 |
| chrX | 144959377 | 144959626 | TCA TTT GTT CTC ATT ACG GGC | GGA TAG AGG AAA CCC AGG TG | 1165, 1166 |
| chrX | 145799354 | 145799603 | TTC TAC CTG GGT TCT CTT GG | GGC TCT TTC AAA GTA TCC AGG | 1167, 1168 |
| chrX | 145922717 | 145922966 | TAA CTT CCT GAG CAC ACA TCA | AGA CTT TCT TTG TTG CCT TCA G | 1169, 1170 |
| chrX | 146994477 | 146994726 | GGG TAA ATT CAG GAA TGC ACA | GCC TCA ACA ATT CAG TCC AC | 1171, 1172 |
| chrX | 146995311 | 146995560 | ATT TCT TCT GGT GAG TTT GCG | CAT AAG TTG CTG GAA GAG AAC A | 1173, 1174 |
| chrX | 147540977 | 147541226 | CCT CTT CCT GAC ATG TTG TTG | TCG ACT GCT TTA AGT GAA GGA | 1175, 1176 |
| chrX | 149764787 | 149765036 | GTT GGT GCC AGA TTG TAA CTT | ACC AGT GAT TAG TGT TTC TCC T | 1177, 1178 |
| chrX | 150808513 | 150808762 | GCC TGG CTC AAT AAT AGT CCT | CAA GAC ACA CAC AAA CAC ACA | 1179, 1180 |
| chrX | 150809248 | 150809497 | ATG TTG CCT TCT CTA CGT TTG | GGT AAG AGT TGC CCT AAT GTC | 1181, 1182 |
| chrX | 150942801 | 150943050 | GAG TGG AGC GCT ACC TTT AT | GCT TGG CTT CTC ACA AAT GT | 1183, 1184 |

FIGURE 2 (Page 37 of 80)

| chromosome | start | stop | forward primer | reverse primer | SEQ ID NOs: |
|---|---|---|---|---|---|
| chrX | 79712826 | 79713075 | AGA ATG GGA AAT GGG AAG GA | GTT TGC CAG TAG AAA TGG GA | 1185, 1186 |
| chrX | 80374192 | 80374441 | CAC AAT ACA TGG GCT GCT TT | GTA AAG TGT TCA GAG GAC GG | 1187, 1188 |
| chrX | 80491243 | 80491492 | CCA CTC CAA CTC TGC TTT TAC | ACT AGG GAG AAG AAT TGG CAG | 1189, 1190 |
| chrX | 80514786 | 80515035 | GAT GTG GAT TGT CTT TGT TGC | CCA GTT CAT TCC AGC TTC CA | 1191, 1192 |
| chrX | 80553311 | 80553560 | AGC CTT TCA TTG CAC ATT TCA G | GAT CGC CAA CCT GTT TTA TAA G | 1193, 1194 |
| chrX | 104028154 | 104028403 | TCC CAC CAC AAG ACC AAT TT | GCT GTC AAA GTG GAG ATA ACC | 1195, 1196 |
| chrX | 104039199 | 104039448 | GAA GAT AGG TGG TGG AGT TCA | AAC GCT TCC ATC CAC CTA ATT | 1197, 1198 |
| chrX | 104101675 | 104101924 | CTG TTT GAA TGA AGT TGG CTG | ACC TGC CTG TCT TAC CAT TAA | 1199, 1200 |
| chr21 | 17021878 | 17022127 | TAT ACA TGG GTG GGA TTT GTC | GAT ACT TTC CTT TCT CCA GAT CT | 1201, 1202 |
| chr21 | 17046718 | 17046967 | TTT GTG ATG GAC CAT CTA ACC | GGC CGC AGT TTT TGA TTT AG | 1203, 1204 |
| chr21 | 17088576 | 17088825 | GAA GAT CTG GTG TCC CAC TA | TGG GAA GGA CGG TTT GTT A | 1205, 1206 |
| chr21 | 17134784 | 17135033 | ATC TGA AGA TCT CCG TGG TA | TGA ATC ATG CTG TGG AGA AC | 1207, 1208 |
| chr21 | 17161709 | 17161958 | ATG TAT GAA CCA TTT CCT GCT | ATA GAA GAG GTA CCC AGC AA | 1209, 1210 |
| chr21 | 17183683 | 17183932 | AGC ACA GAA TTG AAT GAA GGA A | CCC TGA CTA TGC TAA GTT GC | 1211, 1212 |
| chr21 | 17301983 | 17302232 | GCA TGA GTA AGG CTG AAG TG | TGC AAG CAA AAT GAA ACC AG | 1213, 1214 |
| chr21 | 17302814 | 17303063 | AAA GGC TTA TAT TGC TTT TGA ATC A | | 1215, 1216 |

FIGURE 2 (Page 38 of 80)

| chromosome | start | stop | forward primer | reverse primer | SEQ ID NOs: |
|---|---|---|---|---|---|
| chr21 | 17336649 | 17336898 | TTG CTG TTG TTG GGA TCA AG | CAG CTG CTC CTT CTT TAG C | 1217, 1218 |
| chr21 | 17346228 | 17346477 | GCT TCT GCA TCC ACC TAT CT | TAT ACT GCC AAA GGT GAC CT | 1219, 1220 |
| chr21 | 17348050 | 17348299 | AAA AGC TGC CTA AAA TGC CA | GAA GCA TAA TGA GAA CCT CCA | 1221, 1222 |
| chr21 | 17351309 | 17351558 | AGT CAG CAA GTT AGC AGA AA | AAC CCA ACT TGC AGA CAA TC | 1223, 1224 |
| chr21 | 17362239 | 17362488 | CCC ACA TTT ATC CCT TGT CC | ATC TGT TTG CTG TGT CAG AA | 1225, 1226 |
| chr21 | 17367713 | 17367962 | TGT CTG ATT CCA TCT TTC CC | TCC AGA TAT ATT CAA AAG GGA GA | 1227, 1228 |
| chr21 | 17506670 | 17506919 | CAG CCA CCA AAA CAC AAT G | AGG TTT TTA TCA AAG CCC CA | 1229, 1230 |
| chr21 | 17548792 | 17549041 | AAG GCC TGT TTT GTG TGT AG | GGG ACT TGA TGT TCT AAG CAA | 1231, 1232 |
| chr21 | 17591325 | 17591574 | ACA GCT CAC AGA TCT TTA AGC | CCT ACA TGA TAC GCA CAG TC | 1233, 1234 |
| chr21 | 17695933 | 17696182 | GGC ATT ATA AAG AGA TAG CTC CA | | 1235, 1236 |
| chr21 | 19087043 | 19087292 | TGT TTT TCC TTG CCC TGT AA | GTT TTT CTC TAC CCA GCA CA | 1237, 1238 |
| chr21 | 19178365 | 19178614 | TAA CCA GAT GAA TGA GGG CA | CTC TAA TTT GCC ACC CTC TTT | 1239, 1240 |
| chr21 | 22579319 | 22579568 | CAA CAA GCC AAA ACC ACA TC | GAA CGC TAA AGC TTT TCC CA | 1241, 1242 |
| chr21 | 22605212 | 22605461 | GTT GGT TCT TGA AGA CCT GA | TCT TTC TCC TGC CAA GTA GA | 1243, 1244 |
| chr21 | 22964667 | 22964916 | TGT GGT AAG TAG TCT CTA AAG A | ATA ACA CTG TCC TTC TGG GC | 1245, 1246 |
| chr21 | 22984832 | 22985081 | ACC AAA TTT CCA GAT CAC GG | GAG CCT ACT CTC TGA TAC GA | 1247, 1248 |

FIGURE 2 (Page 39 of 80)

| chromosome | start | stop | forward primer | reverse primer | SEQ ID NOs: |
|---|---|---|---|---|---|
| chr21 | 22985386 | 22985635 | AAT GAA TGG TCA TGG CTC AC | CCA GTT GTT CAC TTC TCT GAT | 1249, 1250 |
| chr21 | 36076596 | 36076845 | GCC CTG AAG CAC ATT AAA GT | GCC TTA AAA CCA AAC TGT GT | 1251, 1252 |
| chr21 | 36169368 | 36169617 | GCC TCC AGG TTT ATG ACA AC | CTG GAC TTC AAT CAC CCA AG | 1253, 1254 |
| chr21 | 36789088 | 36789337 | CAG CTC TAT TCC CCT TCT GA | TGA ACA AAT TGC TGT GCT GA | 1255, 1256 |
| chr21 | 37939587 | 37939836 | CCT TGG AAG GGA AAG TTG AT | CCC CCT AAA TGA AAG TGG TC | 1257, 1258 |
| chr21 | 37959766 | 37960015 | GTC CCA CCC TGC TCT TAG | | 1259, 1260 |
| chr21 | 37983316 | 37983565 | CAG AGT AAG ACA GTG GGA CA | TGA GTG CTT GGA ATT TTG CA | 1261, 1262 |
| chr21 | 38559539 | 38559788 | AAG TGT GGT GCA TAA AGG AT | GGT TGG CTA CTT CAT GGT AC | 1263, 1264 |
| chr21 | 38751525 | 38751774 | CTG TAA GAA GGA GGG TTT GG | TCA CTG CAT TCC TAG AAC CT | 1265, 1266 |
| chr21 | 39173262 | 39173511 | GAG GTT GAT GAG AGG TAG GG | ATA ACT CAG GCA AAA TGG GG | 1267, 1268 |
| chr21 | 39285267 | 39285516 | AGC CAG GGA TAT TGT TGA AG | CCT TCT GCT CTC ACT TTA CG | 1269, 1270 |
| chr21 | 39330340 | 39330589 | CAG GTA AGT GTG TGT TCC AG | GGA TAC TAG CAG AGG TGG AG | 1271, 1272 |
| chr21 | 39442392 | 39442641 | TTG ATT TCC ATG CAG AAG GG | TGT ACA CAA TAT GCC AGG AAC | 1273, 1274 |
| chr21 | 39499219 | 39499468 | TAA TTT GGC CTT AGG GGT TG | GCA CTT GAG TTA TGG GAC TT | 1275, 1276 |
| chr21 | 39541298 | 39541547 | TCC ATA AAA GGT GCT TAA AGC | TGT GAT ATG TAG TGT GTA TCA GT | 1277, 1278 |
| chr21 | 39563720 | 39563969 | TTG TAT CAC ACC ATC GTG GA | GAA TGT GTT CAA AGG AGG GT | 1279, 1280 |

FIGURE 2 (Page 40 of 80)

| chromosome | start | stop | forward primer | reverse primer | SEQ ID NOs: |
|---|---|---|---|---|---|
| chr21 | 39584873 | 39585122 | ACT GCT GAG AAC AAT CAT GC | GAT GAA GGG GAT TAC GGG AA | 1281, 1282 |
| chr21 | 46530377 | 46530731 | GGT GTG GAG AAT TTG TTT GC | TTT AGG AAG CAG CAC AAG AA | 1283, 1284 |
| chr21 | 46534043 | 46534292 | TGA TAT TAG GCG GTG GCT TA | GAG GGT GCT GGG GTT ATC | 1285, 1286 |
| chr21 | 46535763 | 46536012 | TCT CAC CTA AAA TCT GGG GC | CCC GAA AGC ACT TAC CTT TT | 1287, 1288 |
| chr21 | 46537454 | 46537703 | AGG ACT GTG ACA CTT TAT CTT T | TTC ATG AGC TGC AAT GTG TT | 1289, 1290 |
| chr21 | 46542089 | 46542338 | ATG CGA GGT AGA AAA TGA GAG | TGC TAT GAA AAG AGG GAC CA | 1291, 1292 |
| chr21 | 46556856 | 46557105 | TGT GGC TTT AAG GTT CTG AAG | GTC CTG GAT CTA CAC GTG AA | 1293, 1294 |
| chr21 | 46564465 | 46564714 | TCG TTG CTA TTC TGC TTT GA | GTA AAT CTG TGT GCC AGC AA | 1295, 1296 |
| chr21 | 46567255 | 46567504 | TCC CTT TTG TGG TTT CTT GG | TAA AAG AGG CGT GTG GAA AA | 1297, 1298 |
| chr21 | 46572969 | 46573218 | TTG CCG CAC TCT TCA TTA AT | CCA GTA GCT TGT GAT GTG TA | 1299, 1300 |
| chr21 | 46605329 | 46605578 | ACT GGC TTC TCC TCA TTA GT | TAC AGG CCT CTG AAA GAT GA | 1301, 1302 |
| chr21 | 32134809 | 32135058 | ACT GAT TTG CCA TGT AGA GC | AGA GCA TGC TAG ACG TCT TT | 1303, 1304 |
| chr21 | 32148136 | 32148385 | GAA TGT CAT ATT GCC TGC CA | CTG GGC TGT AAT TAA GGC TC | 1305, 1306 |
| chr21 | 32165994 | 32166243 | TCA CTC GCT TAG AAT GTT GC | AAC ACC TGC ACA CTT TGA AA | 1307, 1308 |
| chr21 | 32174173 | 32174422 | ACC ATT AAC TTC CTG CAA ACT | AAT GAA CTT TGT GGG CTG AA | 1309, 1310 |
| chr21 | 32179882 | 32180131 | TAA AGC CCC ATA CCA GGA TT | CCA CAC TCT CAC TGG TTC TA | 1311, 1312 |

FIGURE 2 (Page 41 of 80)

| chromosome | start | stop | forward primer | reverse primer | SEQ ID NOs: |
|---|---|---|---|---|---|
| chr21 | 32181384 | 32181633 | CCA AAA ATC ACC CAT ATG CG | CAC AGT GGA TAC CTC AGG AA | 1313, 1314 |
| chr21 | 32184463 | 32184712 | ATA ACC CAG GTG CTT CAA AG | CCA ACA CAG GAG GAA CTT TT | 1315, 1316 |
| chr21 | 32201994 | 32202243 | TCC ACA GCA GAA GTA ACG A | GAG AAA AGC CAA CAA AAA TGT G | 1317, 1318 |
| chr21 | 32208609 | 32208858 | AGG AAA GCT ATG AAG AAA GGG | TGT CAT TAC TAG AAG CAC CTT T | 1319, 1320 |
| chr21 | 32319600 | 32319849 | AGC TCC AGA GTG TCA GTA TT | TGG TGT GAG TTC AGG AGG GTT TA | 1321, 1322 |
| chr21 | 35034581 | 35034830 | TTG ATT TCC AGC ACT GAA CTT T | ACC ATT TCT GAC AGA ACA GA | 1323, 1324 |
| chr21 | 35060177 | 35060426 | TGT TTG ATT TTT CAG GCT GA | TGA TGC TTA AAC ACA TGC CT | 1325, 1326 |
| chr21 | 35070605 | 35070854 | CTG GTC ATT CCT GAG TGT CT | AAG GGA TAT GCA GCT TGT TC | 1327, 1328 |
| chr21 | 35093305 | 35093554 | TCC TGT TTT ACA CTT TTC TAA CTT T | | 1329, 1330 |
| chr21 | 35094930 | 35095179 | ACC TCA ACC TGT TTT AGC AC | GTT GTC ATT CAA ATG TCA CCA C | 1331, 1332 |
| chr21 | 35099418 | 35099667 | CAG TGT GTG TCA TGC CAA AT | CTC AGA TTC CAG AGC CCT C | 1333, 1334 |
| chr21 | 35138457 | 35138706 | GGT CTA TGT TAA TCT TGG GCC | ACA TTG CTA GCA GCT TTT GT | 1335, 1336 |
| chr21 | 35152877 | 35153126 | AGC CAG TCT GTA TCT AAA GGT | ACT TGC CAA GAA CAG TAT CTG | 1337, 1338 |
| chr21 | 35157173 | 35157422 | ACT CTT GGG GTT TCT TCA GT | TTC CTT CTT CCA GGT GAA CA | 1339, 1340 |
| chr21 | 35161795 | 35162044 | CGA ACC AAA AGC AAA ATC CTT | TGT TTC TCC TTC ATC TGG TC | 1341, 1342 |
| chr21 | 35296082 | 35296331 | AAT CTA TGG AGG TCA CTG GG | TGT CAC CTT GCA GAT ACA GG | 1343, 1344 |

FIGURE 2 (Page 42 of 80)

| chromosome | start | stop | forward primer | reverse primer | SEQ ID NOs: |
|---|---|---|---|---|---|
| chr21 | 35450331 | 35450580 | AGG TCC TTG TAG TTT GCT TG | ATT GTT CAC AGG GTC AAG TC | 1345, 1346 |
| chr21 | 35473022 | 35473271 | GTG AGA GCC AAT AGA GTG TG | ATG AGC CAG GAG AAT CAT CA | 1347, 1348 |
| chr21 | 35473971 | 35474220 | GCA TGG TGT GTG AAA GTG AT | CAT CAT TTC AAA GGG GCT CA | 1349, 1350 |
| chr21 | 35498164 | 35498413 | ATG CTG CTT TTG ACT GAT GT | TCT TCT CTA ACA CCC ACT CC | 1351, 1352 |
| chr21 | 35540943 | 35541192 | CGA AGC TGT ATT CCT GTC TC | ACC CTT ACC AAA GTA GCA TC | 1353, 1354 |
| chr21 | 35702965 | 35703214 | ATG GAC TAA CTG GAG AGC G | ATC ACG ACG CCT TGT TTA TT | 1355, 1356 |
| chr21 | 28574819 | 28575068 | ATG TCC TGC CAG TAA ACA CA | ACT TTC CTG CCA ACA ATC TC | 1357, 1358 |
| chr21 | 28586377 | 28586626 | TGC CAC TAA ACA CCT AAG GA | TTG ATA GTT GCA TCT GGG GA | 1359, 1360 |
| chr21 | 28597009 | 28597258 | TTG GAA ATT TTG GGG TCA GG | TTT GTT AAC TAA CGT GAT TCC A | 1361, 1362 |
| chr21 | 28616121 | 28616370 | TTC TGA CCT CCC TTA CTG AG | CCC ACT CTC CAT GTG TTC TT | 1363, 1364 |
| chr21 | 28631408 | 28631657 | TAT GTG CTT GCG ATG TGT T | TCC TGC TTC AAC TCA ATA CG | 1365, 1366 |
| chr21 | 28891355 | 28891604 | GAA CCT TAG GGC CAG TCT AT | ACC CCT CAT TTT CGT ATG TC | 1367, 1368 |
| chr21 | 28892223 | 28892472 | AGA ACC TGA TGT GTT TTC CTC | ACA GTT ATG GAG GAA TTG CG | 1369, 1370 |
| chr21 | 28919446 | 28919695 | ATG TGA GAG AAG CAA AAC CC | TCA AAC CTC TTT TAT CTG TCC C | 1371, 1372 |
| chr21 | 29012106 | 29012355 | GTA GTT GTC TTG AGG GCT TT | ATT TCA CGT AAC ACT CTG GT | 1373, 1374 |
| chr21 | 29021007 | 29021256 | CCT GGT CAA CAA CAT ATG GG | CTC TGG CAA AAC TTT CTG GAT | 1375, 1376 |

FIGURE 2 (Page 43 of 80)

| chromosome | start | stop | forward primer | reverse primer | SEQ ID NOs: |
|---|---|---|---|---|---|
| chr21 | 29753618 | 29753867 | AGA ATA TTG CAT TTG GCC AGA | GCT TCT CAT GCT CAC ACT G | 1377, 1378 |
| chr21 | 29796479 | 29796728 | AAA GCT ATG CAA ATA GTG GCA | AAT ATG ACT TGC CCT TTT GAA | 1379, 1380 |
| chr21 | 29812165 | 29812414 | AGT TCC CAA CAG AGG CTA AT | TCA CAG CCA GTT ACA CAG A | 1381, 1382 |
| chr21 | 29813966 | 29814215 | AGT TAT AGG TGA GGA AGG GC | CTA CAG TGC AGA AGA GTC CC | 1383, 1384 |
| chr21 | 29814951 | 29815200 | CAC TGC AAA AGA AGG AGG TT | TTG GTT TCA TGT GGC TTT GA | 1385, 1386 |
| chr21 | 29820028 | 29820277 | TCT CAA CAT CGC TGA TCT AGT | TCC TTC TCC CCA ACT TTC TT | 1387, 1388 |
| chr21 | 29825992 | 29826241 | AGG TAG CTG GAA AAG GAG AA | CTT TAG ATT CCA GGG CTC TTG | 1389, 1390 |
| chr21 | 29827616 | 29827865 | TGA AAT TGC CCA GAA TTG AGT | TGA AAG CGT GAA AAT CAG CT | 1391, 1392 |
| chr21 | 29841662 | 29841911 | ATT TTC CTG GAC TTC TGA CA | ACT CGA TCC CTA GGT AAT GT | 1393, 1394 |
| chr21 | 29884231 | 29884480 | TGT TAT TCC TCT TCC TGT CCA | ACA AGT GGG TAG GGA TGT TC | 1395, 1396 |
| chr21 | 29898255 | 29898504 | TAA CCA CAC AAC TAC AGC TT | GCA CAA GTT TTC AGG GAA TG | 1397, 1398 |
| chr21 | 30368230 | 30368479 | ATT CAA AAT GGG GAC GAG AG | GTG GAA AGT CTC GTC AGA AT | 1399, 1400 |
| chr21 | 30413906 | 30414155 | TCC CAG TTT GCT ACT CTG G | TCC ACC TCT GAG CAT AAC AT | 1401, 1402 |
| chr21 | 30415555 | 30415804 | TCT CAT TAT GTG AAG ATT GCT TTC | | 1403, 1404 |
| chr21 | 30428953 | 30429202 | TTA AGA TTA AGC AGT CTT CTT GG | | 1405, 1406 |
| chr21 | 30477650 | 30477899 | CAA CAG ATC TGA TTC TGC CC | CCA TCC CAC TTC TCC AGA TA | 1407, 1408 |

FIGURE 2 (Page 44 of 80)

| chromosome | start | stop | forward primer | reverse primer | SEQ ID NOs: |
|---|---|---|---|---|---|
| chr21 | 30528161 | 30528410 | CAT GTG TTT CAA AGT TGG CT | CCA CCT TCC TGC TTA AAG AA | 1409, 1410 |
| chr21 | 30550132 | 30550381 | ACT ATA GCA TTA GGG TGA GGG | CTC CTT ACT TGC ACT GAG TT | 1411, 1412 |
| chr21 | 30580738 | 30580987 | GAG ATT TGG ACA TGC TTT CA | GGG AAA ACT TAC GGG AAC TT | 1413, 1414 |
| chr21 | 30600446 | 30600695 | CCC ACC ACC AGT TGT CAT C | TAG AAA TGT TTA GGG TGC ATG A | 1415, 1416 |
| chr21 | 30628668 | 30628917 | GCC ACT CAC TTC CTA GAT AAT | TAC CCC TCT TTT CCA TCT GC | 1417, 1418 |
| chr21 | 30660609 | 30660858 | ACA GTA TCT CAG GGC CTT AT | GCA TTT TGA CAG GAA AGT GG | 1419, 1420 |
| chr21 | 30680391 | 30680640 | TCA GTA GTT CCT CAG ATG CTA | TAG GGC CAC AGT TTC TCA AT | 1421, 1422 |
| chr21 | 30720214 | 30720463 | AAT GCA TGA AAG TCC AGG AA | GAA TGA GAA ATC TGG CAG GA | 1423, 1424 |
| chr21 | 30782462 | 30782711 | ACA TGC ACT CTT GTC TTA TGC | TCA GAT GCC TGA TGA CCA TA | 1425, 1426 |
| chr21 | 30810199 | 30810448 | CAC ATA TAC TGG CTT TCT GGT C | AAT ATG CAG TGG GTA GGA GC | 1427, 1428 |
| chr21 | 30849402 | 30849651 | GCA CAT GTC ATT AGC AGG G | GAC AAC AGC TGA CTT CCA TT | 1429, 1430 |
| chr21 | 30868525 | 30868774 | ACA TGC TCA ATT ATG GAG CC | CAC TCT CTG GAA CAA ACA CA | 1431, 1432 |
| chr21 | 30988776 | 30989025 | ACA AAG ACA GGA ATA GGG CT | GTT TTG AGG CGG TTT CAT GA | 1433, 1434 |
| chr21 | 31012406 | 31012655 | CAA TCT GCT GAC TTG CTT CTT TTC A | | 1435, 1436 |
| chr21 | 31032720 | 31032969 | CTT TGG CTC AGA ATC TTC CAA | GAA CAC CTC AAA GTT GCT CA | 1437, 1438 |
| chr21 | 31085356 | 31085605 | TGT ATG AGG ACC AGC AGT AAA | AAG AAG CAA GGA CAA GGA TG | 1439, 1440 |

FIGURE 2 (Page 45 of 80)

| chromosome | start | stop | forward primer | reverse primer | SEQ ID NOs: |
|---|---|---|---|---|---|
| chr21 | 31494755 | 31495004 | TCT TTT CCC CTT GTG CAT AG | GAA ACA ACC ACC ACA ACA AA | 1441, 1442 |
| chr21 | 31560667 | 31560916 | GCT TAG TGT GTG TGA TCC GT | CCA CCA TGT TTA CAC CGT TT | 1443, 1444 |
| chr21 | 31605973 | 31606222 | ACT CAC CTT TCC CAA GAA GA | TTT ACC CAT GAA TTG CTG CA | 1445, 1446 |
| chr21 | 31621826 | 31622075 | TGG TAG TGG GAA GAG GTT GA | AGT TTG CAT TTG TTC AGG GA | 1447, 1448 |
| chr21 | 31653822 | 31654071 | GCA TGG AGA ACA AAA GCT GA | TTC GGA TGG CTT TGA TTG TC | 1449, 1450 |
| chr21 | 31670830 | 31671079 | TGT GAG CAA GAA ACT GAA GG | CAT TGT AAA TTA AAC GGC CTC | 1451, 1452 |
| chr21 | 31691347 | 31691596 | AGT ATC ACT TGT CCA GCT CA | CTA ATG CAA GCT GCT TCT CT | 1453, 1454 |
| chr21 | 31708936 | 31709185 | CAC AGG ACT AGG TAG GCT TT | TGT CTA GTG GTA ATC TGG GG | 1455, 1456 |
| chr21 | 31733443 | 31733692 | GTG TTA ACA GCT TTC CCT TCA | TCT GGA CAG TGG AGT TGA AA | 1457, 1458 |
| chr21 | 31768996 | 31769245 | TCA TGT ACA GAA AGA ATT AGC CT | | 1459, 1460 |
| chr21 | 31886189 | 31886438 | AGA GGC CAA GTG ACC AAA TA | AGG AGA GAC ATA ACT GGT CT | 1461, 1462 |
| chr21 | 31886808 | 31887057 | AAG CCA GTA ATT CAT CTT CCC | ATT GCA ATG TCT GTG GAT GT | 1463, 1464 |
| chr21 | 32119833 | 32120082 | CCA TGA CAT AAC ACA TCA CCA | AAG AAG TTG AGG TAG CAC G | 1465, 1466 |
| chr21 | 32125263 | 32125512 | ATA GAT TTC CTC CTG GGC TG | AGT GTC CTT TCC TCC AGT TC | 1467, 1468 |
| chr21 | 25795207 | 25795456 | CCA CCT CTG TAC CCA CTA TC | TTG AAC CTG AAA GGA ACT GTG | 1469, 1470 |
| chr21 | 25802273 | 25802522 | TTC CCT GGA AGA TAG CCA AT | GCT CAA TCA CCT GTT CCC TT | 1471, 1472 |

FIGURE 2 (Page 46 of 80)

| chromosome | start | stop | forward primer | reverse primer | SEQ ID NOs: |
|---|---|---|---|---|---|
| chr21 | 26355452 | 26355702 | TGG ACA CGT AAA AGA AGG TG | CAT TGA AGC TCA CTC TAA GGG | 1473, 1474 |
| chr21 | 26365770 | 26366019 | CTG AGA ACC TTG TCC AAC TG | CTA AAG TTT GGG TTA GGG GT | 1475, 1476 |
| chr21 | 26373440 | 26373689 | TTT CGC TAG TCT TTG CAC TT | TGC CTT ACA TTT TCT GTG GG | 1477, 1478 |
| chr21 | 26374774 | 26375023 | GCG GCA ATA ATT GTC ACA AA | GCC ATA AGA TTT CCC CAC TC | 1479, 1480 |
| chr21 | 26390279 | 26390528 | CAG AAA TGT GTC AGG CTA CA | ACA AAG CAA GAG GAT GAA ACA | 1481, 1482 |
| chr21 | 26390951 | 26391200 | AAC TCT TCA TTT TGA CGG GG | CAA AGC ACC ATC AAC ACT TA | 1483, 1484 |
| chr21 | 26402802 | 26403051 | TGG GTT GTG CTG TTG TTT AG | CAC TGC AAC TCC TAG AAT GA | 1485, 1486 |
| chr21 | 26496769 | 26497018 | GCT CAG CTC CTT TCA TCT G | TGT GGG GAA ATT GCT GTT TA | 1487, 1488 |
| chr21 | 26515400 | 26515649 | AGG ACA TTC AGC CTA TTT GC | GTA TGG GCA GCT GTA ACT TG | 1489, 1490 |
| chr21 | 26571662 | 26571911 | ACA CAG TAT CAA GGT CAA CA | TCA CAA GCC ACT GAA AAT GT | 1491, 1492 |
| chr21 | 26589786 | 26590035 | GCA GAA CCA CAG TCT ATG AG | TGT ATG TTA AGC TAG CCA ACA | 1493, 1494 |
| chr21 | 26604824 | 26605073 | AAT GCT CCA AGT TAT TCC AGA | TGT GTT ATT GAA CTT TGC CA | 1495, 1496 |
| chr21 | 26933670 | 26933919 | AAG AGA GAA GCG ACA AAA CC | TTC ATC CCT ACC TCA TCA CC | 1497, 1498 |
| chr21 | 26950703 | 26950952 | AAT GAG AAG GAA TTG GGT GC | AGA TCA CTT TTG GCT GTA ACC | 1499, 1500 |
| chr21 | 27045134 | 27045383 | AAT GAA GTG TTA GGG CCA TC | CAA GTG ACA ATC TCA GCC AA | 1501, 1502 |
| chr21 | 27398390 | 27398639 | GCT CAT CAC AGT TTA AGG AGT | GCA TTT TCA GAT GGT TCC CT | 1503, 1504 |

FIGURE 2 (Page 47 of 80)

| chromosome | start | stop | forward primer | reverse primer | SEQ ID NOs: |
|---|---|---|---|---|---|
| chr21 | 27441346 | 27441595 | ATG TGG AAG CAA GAG AAA GG | ATG GAG AGT TTG AGT GGA GT | 1505, 1506 |
| chr21 | 27465490 | 27465739 | TGA AAG GTG AAG TGG CTT TT | CTG CCA CTG GGT TTA TAG AAA A | 1507, 1508 |
| chr21 | 27485182 | 27485431 | GTA AGT AAG GGG TCC TAG CT | ACA CCT TTA CTC CTG TGG AT | 1509, 1510 |
| chr21 | 27486006 | 27486255 | TGT GAC TTC CAT GAA ACT GG | GAT GTA GGG CCT TAT CCA CA | 1511, 1512 |
| chr21 | 27502429 | 27502678 | GCT GAC AAA CTA ACC TTC CA | AGC TTG GTG ATC TTC AAA CA | 1513, 1514 |
| chr21 | 27520192 | 27520441 | AAA ACC TCC CAA AAC AGA CT | TGA AAT GGT GGG TAA TGC TC | 1515, 1516 |
| chr21 | 27539803 | 27540052 | AGT TTA GTG GCC ACG TGA AA | CAT GCA AGT TCA CGA GGT TA | 1517, 1518 |
| chr21 | 27623130 | 27623379 | GGC AGA AGT TTC AAT TCC CT | AGT CTG AGG AAG AAG CAA CT | 1519, 1520 |
| chr21 | 27623692 | 27623941 | AAA GCC TCT GTT TGC ACT TT | GAG TCC AAT CTT TTC CCA CA | 1521, 1522 |
| chr21 | 27763679 | 27763928 | TCA GTC AGC TTC TTG AGT CA | TCC ACT GCG TTC TTA TCC TT | 1523, 1524 |
| chr21 | 27894000 | 27894249 | TGT TTC ATT TGG GTC ATG GA | GGA CAG AAC AGC TAC AAA GG | 1525, 1526 |
| chr21 | 27926498 | 27926747 | TAC AGC ACT AGG ATC ACT CTG | CTG GCT TGT GAA TTA GAG GG | 1527, 1528 |
| chr21 | 28077177 | 28077426 | ATG CAT GTT TCT TGC AAA GG | GTA AGA TGG TGG GCA GGA T | 1529, 1530 |
| chr21 | 28122462 | 28122711 | GTA GGA TTC AGG GCA TTT CA | AGA AGG GCT CAA AAC ACA TC | 1531, 1532 |
| chr21 | 28161337 | 28161586 | AAC TGG AAC TGA GCG TGA G | TCA TGT AGG CTT TCT GAT TTT | 1533, 1534 |
| chr21 | 28180925 | 28181174 | TGT ATG CAG TTA CCT CCA GA | TGT AGC TCT TGA CCT AGC AA | 1535, 1536 |

FIGURE 2 (Page 48 of 80)

| chromosome | start | stop | forward primer | reverse primer | SEQ ID NOs: |
|---|---|---|---|---|---|
| chr21 | 28275754 | 28276003 | GGA TGT ATA CCA GAC CCC TT | TAA GTT CAC GGT GAA GTC AAC | 1537, 1538 |
| chr21 | 28291449 | 28291698 | CGA AAG ATG TTA GCA CCT CA | GTG GCT TCA ACT AAC TGG AC | 1539, 1540 |
| chr21 | 28294820 | 28295069 | AAT TGG CTT TGC AGT GTT TC | ACC CGT AAG TGT TTG AGT GA | 1541, 1542 |
| chr21 | 28295351 | 28295600 | GCT TGC TGG TAG GAG GTA TA | TGT AGA AGT AGG GTT TGC GT | 1543, 1544 |
| chr21 | 28295857 | 28296106 | CTG GAA ACG GAA GGA AGT TG | CAG GGG ACA TTT GAA GAT GG | 1545, 1546 |
| chr21 | 28311866 | 28312115 | CTG TGT TCA GTA AGT GGC TG | GAA ATT GTG CAG TGA AAG CA | 1547, 1548 |
| chr21 | 28334784 | 28335033 | GAC AGT GAA GTG TGA TCG TT | ATG TAA GAA GTG CGT TGC TTA | 1549, 1550 |
| chr21 | 28354814 | 28355063 | TGG TGA TGC TGT TTT GGA AA | AGA TCT GGA ATC TGA GAC TCC | 1551, 1552 |
| chr21 | 28415341 | 28415590 | TTC TTT CAG GCA GAA GAA ATG A | TAT AGG ATA AGG TCA AGC AGG T | 1553, 1554 |
| chr21 | 28433186 | 28433435 | AAG TTC CAT TAC TGT ATT GAA AAT T | | 1555, 1556 |
| chr21 | 28450226 | 28450475 | GGA CGT ACG TGC TTA TTT CA | TCA GTG AAT GAG GAA TCA TGC | 1557, 1558 |
| chr21 | 28474083 | 28474332 | CAG CCT GAT ATT CCC ATT GAG | TTC TCA GGA GTA CCA CAA GC | 1559, 1560 |
| chr21 | 28491062 | 28491311 | TCA TGA GGC TGA GTG AGT AT | GGA TGA AAC ACA GAA CCA TG | 1561, 1562 |
| chr21 | 28509439 | 28509688 | AAA GCT CTC CTA TCT CCA GT | CAG ATC CCT TTC ATT TTG CA | 1563, 1564 |
| chr21 | 28534176 | 28534425 | ACA AAG AGT CTG GAC TAT CCT | GGA AAA GTG CTC AAT TAG GC | 1565, 1566 |
| chr21 | 40042441 | 40042690 | TGC CTA AAA ATA CCC AAA GCT | CTG GCA GTT ATA GTC ACC AA | 1567, 1568 |

FIGURE 2 (Page 49 of 80)

| chromosome | start | stop | forward primer | reverse primer | SEQ ID NOs: |
|---|---|---|---|---|---|
| chr21 | 40427338 | 40427587 | CCA AGA CAC TCA CTC CAA AG | AGG AAG TCC TTA TGA TGC CA | 1569, 1570 |
| chr21 | 40509976 | 40510225 | CCA GGA AAA GGA GCA GTT TT | GCT GGT GAA GTT GGA GTT TT | 1571, 1572 |
| chr21 | 40531291 | 40531540 | GTT CAA CTC TTT CTG CGA AC | GCT GGG TTT AAG CCA CAT AT | 1573, 1574 |
| chr21 | 40578323 | 40578572 | CCA CTT CTT CTG TTT CCA AC | TGT AGC CTA AAT AGC AGC CT | 1575, 1576 |
| chr21 | 40601158 | 40601407 | GGG AAG GGC ATG CTA ATC A | AGG AAT TGC TTT TAT TTT AAC CA | 1577, 1578 |
| chr21 | 40630577 | 40630826 | TGT TCA ATC ACC TCT CCA TC | GCT GCT TTC AGG TTT TTG TG | 1579, 1580 |
| chr21 | 41280723 | 41280972 | AGA GGA GAG GCT AAG CTT TG | ATG GTG CAG AAA AGA GCA AT | 1581, 1582 |
| chr21 | 41607273 | 41607522 | ACA AGA GAG GAA GCT GTC AG | GTG TGG TGC CAT TTT CTT TC | 1583, 1584 |
| chr21 | 41629820 | 41630069 | AGC CCC TTT CTC CTT ATT CT | GAA CAA TAC TTT CTC CCC GG | 1585, 1586 |
| chr21 | 41703462 | 41703711 | AGG GAA GCA GGA TTT TAA CG | GAA ATG GCC ATG CTA GGA AT | 1587, 1588 |
| chr21 | 41729089 | 41729338 | AAG TTT CAC AAT ACC CAG GT | ACT TCC AGT AAC ATG GAT GC | 1589, 1590 |
| chr21 | 41761636 | 41761885 | TCG CAG AAT GGA CAA GTA CT | GAA ACA CCC AGA CTT GTA GC | 1591, 1592 |
| chr21 | 41839021 | 41839270 | GGC TCC CAG ATT TTG ATC AT | TTA AAT CTT TGT GTG CGT GT | 1593, 1594 |
| chr21 | 41908902 | 41909151 | CCT AGA ACT GCA AAA CAC CT | CCT CCA GGA ACT TTG TTC AG | 1595, 1596 |
| chr21 | 41933321 | 41933570 | TTC CAT TAT TTT CTC ACC GGC | AAA AAC CTT CAC AAA CCC CA | 1597, 1598 |
| chr21 | 41981868 | 41982117 | CAG TCC AAC AAA GAG GTC AC | GAG TGG ATA TTG TCT CGC TG | 1599, 1600 |

FIGURE 2 (Page 50 of 80)

| chromosome | start | stop | forward primer | reverse primer | SEQ ID NOs: |
|---|---|---|---|---|---|
| chr21 | 42029142 | 42029391 | TTG TGT GTG TTG AAG CCT AG | ACT AGC CCA CTA ATG TTG CT | 1601, 1602 |
| chr21 | 42030642 | 42030891 | AGT GGG GGT AGG AAG AAA AA | CGA TTA TCA GAA CAG ATG AGG T | 1603, 1604 |
| chr21 | 42080190 | 42080439 | GTT CCA ACA ATG TAA GGC AC | ATT GCA CAG CTG AAA ATC CT | 1605, 1606 |
| chr21 | 42251219 | 42251468 | GGA ACC CTC TAT GGT CAA AG | GGC ACA CTG ACC GTA TTT AT | 1607, 1608 |
| chr21 | 42559559 | 42559808 | ATT GCT GTG TAG TTC CTT GA | TGG TAG TGG GTC AGG AAT TT | 1609, 1610 |
| chr21 | 42601828 | 42602077 | TTT CTT GCC ACC ATT CTG AC | TCT CTT GAA AAG AAA GGC GG | 1611, 1612 |
| chr21 | 42851340 | 42851589 | CAC ACG TTC TAA CCA AGT GC | TGA ATT ACA CAG CAA AGC CC | 1613, 1614 |
| chr21 | 42872713 | 42872962 | TAG CCA CAT CTT AAC AGA CCT | CTG CCA ATG GGA TCG AAT TT | 1615, 1616 |
| chr21 | 43032728 | 43032977 | ATT CCT GAG GGT GAC ATG AA | GAG TGA CGC TGT TCA TTC TT | 1617, 1618 |
| chr21 | 43406661 | 43406910 | CCT GCC CCA TCA ACT TAA AA | GAT AGT AAC CGG GTG TAG CA | 1619, 1620 |
| chr21 | 46521300 | 46521549 | CTG GGT GCA GAG GAT CTC | | 1621, 1622 |
| chr21 | 16323548 | 16323797 | TTG AGT TGA ACT TTG CTT TAG A | TCT AAT AAG GGA TTG ATG GAG TT | 1623, 1624 |
| chr21 | 16330249 | 16330498 | GAA CTA GGA GAC ACT GGG TT | GGG AGA TTT CCT GCT TGT AG | 1625, 1626 |
| chr21 | 16332622 | 16332871 | CAA GAA TAG CTA ACT GGT GCT | AAT CCA TGC AGC TTC TCT CT | 1627, 1628 |
| chr21 | 16333315 | 16333564 | AGT TAC ACA CTG AAT CAT GGG | TGC ATT GTC TCT GGT TTG AA | 1629, 1630 |
| chr21 | 16335355 | 16335604 | CCC ATG TGG CTT CAC TAA TA | TCC TGA ATG CAT CCT TAA CC | 1631, 1632 |

FIGURE 2 (Page 51 of 80)

| chromosome | start | stop | forward primer | reverse primer | SEQ ID NOs: |
|---|---|---|---|---|---|
| chr21 | 16344361 | 16344610 | GAT TGT GGT CAC GTG GAG AG | TGG GAA AGG TGA GAA GGA TT | 1633, 1634 |
| chr21 | 16349606 | 16349855 | CAG ATC CAG GTA TTC GGA GA | TTC GGG ACC CAT ACC TAA AA | 1635, 1636 |
| chr21 | 16358650 | 16358899 | ACA ACA ACA ACC ATT ACC CA | TCT TTT CTG GAC CCA CAT GA | 1637, 1638 |
| chr21 | 15713251 | 15713500 | TGG CCA TAG TAC TGC TTG TA | GAC CTC TAC ATC TGT ATC TTC C | 1639, 1640 |
| chr21 | 15714357 | 15714606 | TGT CTC ACT GTT GGG AAC TT | CCC TTC ATT TTC TGT CCC AT | 1641, 1642 |
| chr21 | 15725977 | 15726226 | TTT GTC TGT ATC CTA TGC CC | TCA GCC TTG AGT ATT AGC CTA | 1643, 1644 |
| chr21 | 15731366 | 15731615 | GCT GAC AAA ATT GGA TCC CA | AAG TTT GCC ATG AAG GTC AT | 1645, 1646 |
| chr21 | 15816232 | 15816481 | AAA ATC CTC CTG AGT CCT CT | GAC TTC TGG TTG TTT CCT CA | 1647, 1648 |
| chr21 | 15843963 | 15844212 | TTC ACT GGG CTC TTC AGC TA | ATG TGT CTA TTG CCC TAC CT | 1649, 1650 |
| chr21 | 15858450 | 15858699 | AAA GGG CAG GAG TTA GGT AA | TGC GGG AAG TTC ACA TGA A | 1651, 1652 |
| chr21 | 15859015 | 15859264 | AGG CAT AAG AAA CCA GGT TG | GTG AAC TTC AGG CTG CTT A | 1653, 1654 |
| chr21 | 16002625 | 16002874 | GTA GTT CGG TCC AAT GTC AG | TGG TTT CGT CCC GTA AAT AG | 1655, 1656 |
| chr21 | 16030414 | 16030663 | GAT GGT CAC AAT TGC AGG TT | AAT TCC TTT CAA TGC TGG CT | 1657, 1658 |
| chr21 | 16063119 | 16063368 | GCC AAA GAT CTC AAT TGC CA | AGA AAG ACA CAT ATG CCA TGG | 1659, 1660 |
| chr21 | 16068358 | 16068607 | GAC CAA GAC TGT CTC TCC TT | TTG TTG GTG TCA GTT CTG AA | 1661, 1662 |
| chr21 | 16071495 | 16071744 | TAA TGG TCA AAT CCC TCT CAA A | TGT AGG AAC AGA TTA GGG CA | 1663, 1664 |

FIGURE 2 (Page 52 of 80)

| chromosome | start | stop | forward primer | reverse primer | SEQ ID NOs: |
|---|---|---|---|---|---|
| chr21 | 16081282 | 16081531 | CAT GTA GGC TGA AGA CTC CT | CTC CAA GCT GAT ATG CCA TC | 1665, 1666 |
| chr21 | 16115078 | 16115327 | TTT CTC TCC AAA CTG GTT GC | CCA CCC CTC ATT TCT TCC TT | 1667, 1668 |
| chr21 | 16122085 | 16122334 | TAC AGG CAA GAA ATA GTG TCT | GGG TCC TTC GTT TTC TGT TT | 1669, 1670 |
| chr21 | 16130688 | 16130937 | TTC AGC AAG AAT GGG GAT TC | TAG GAA ACA GGC TAA AAG GGA | 1671, 1672 |
| chr21 | 16133865 | 16134114 | CAA AGA GAG AGC CAT CAC AG | TAC TGT GTA GAA GGC AGT GT | 1673, 1674 |
| chr21 | 16139969 | 16140218 | TGA GAA CAC TGC TAT TTC TGC | GCT AAG GAC AAA GAA CCA CT | 1675, 1676 |
| chr21 | 16141592 | 16141841 | GCA TGG TCA GGA CAT TGG | | 1677, 1678 |
| chr21 | 16160517 | 16160766 | GAT TGA ATC AGG AGG GAA GC | TGG CTA AGA CCA GGA TTG TT | 1679, 1680 |
| chr21 | 16165861 | 16166110 | AGC TTA AAT GAT GAA GTG CTT TC | | 1681, 1682 |
| chr21 | 16189746 | 16189995 | TTC TCC TAC GTA TCT TGG CA | TGA TAG GCA GAT CAT TCC CC | 1683, 1684 |
| chr21 | 16190806 | 16191055 | ACC TGG GAC ATA ACC TTG AT | GCA AAT GGC AAA GGG AAA AC | 1685, 1686 |
| chr21 | 16192618 | 16192867 | CCA TTT TCC TAC TGC GTG TC | CAC GGC TAG TGC TCA TTT T | 1687, 1688 |
| chr21 | 16195809 | 16196058 | GAA CAT ACC AAA CCC ACT GG | CCG TAA TAC CCA AGT CAT CTG | 1689, 1690 |
| chr21 | 16198337 | 16198586 | ACC CAA TGA TGT ACA GTT CC | AGC AAA CTA AAA CAG CAA CTT C | 1691, 1692 |
| chr21 | 16203731 | 16203980 | ACC TAT TCG ACT TGA AAC TCA G | AGC CTG CTA TCT TCA CTG G | 1693, 1694 |
| chr21 | 16207035 | 16207284 | ATT TCT GCA CAA CTG TTC CA | ACC ACA TAT ATA GAG ACT TTG AA( | 1695, 1696 |

FIGURE 2 (Page 53 of 80)

| chromosome | start | stop | forward primer | reverse primer | SEQ ID NOs: |
|---|---|---|---|---|---|
| chr21 | 16209341 | 16209590 | GCT GTA ATG TGA CTA ACC CT | CTC CAG ACT CTG CAA GGA T | 1697, 1698 |
| chr21 | 16211270 | 16211519 | TTT CCC GAG GTT CAC AGA TA | TTT CCT GTT GCT CTT GAT CA | 1699, 1700 |
| chr21 | 16219907 | 16220156 | GAA CTT GTG TGA CCC AAA AC | CCA CAA AGA TGA AGG CCA AG | 1701, 1702 |
| chr21 | 16230785 | 16231034 | CAT TGC ACT GTG ATG TCA TG | CCA TAC CTT AGT TCT CAG GGT | 1703, 1704 |
| chr21 | 16236026 | 16236275 | GCA CTG GAA ATT GAC ATC AC | GTA AGA GAG AGC TGG GAC AA | 1705, 1706 |
| chr21 | 16241139 | 16241388 | GGG AGA GGC TGA AAG AAG AA | TGT GAC CAT CCT ATC CAC AA | 1707, 1708 |
| chr21 | 16242812 | 16243061 | TGT ATC ACT TCC TCA TGC CA | AGA ACC AGT TGT ACG AGT TC | 1709, 1710 |
| chr21 | 16246944 | 16247193 | CCA AGA GTT TCC TGT TTC CA | AGT CTT CTC CCT TCC TTG TC | 1711, 1712 |
| chr21 | 16265484 | 16265733 | ATG ACA CAT ACA TCC ATT TAC A | GCC CTT TTC TCT CTT TGA CC | 1713, 1714 |
| chr21 | 16273734 | 16273983 | CTC AAA CTG CCC AGT GAT TT | TCT TGT TCC AAG TAT TCC TGG | 1715, 1716 |
| chr21 | 16275865 | 16276114 | TCC TAG CTT GCC AAA GAA AT | ACC ACT TTA GCC CAT CTC TT | 1717, 1718 |
| chr21 | 16290234 | 16290483 | CCT CCT CTC CAG GCA TTT TA | GTG GTG GAT TAT TGA GCT GG | 1719, 1720 |
| chr21 | 16295877 | 16296126 | AAC AGA GTA GCA CAG AGA GT | TGC TTA ATG GGA TCA TTG ACC | 1721, 1722 |
| chr21 | 16296687 | 16296936 | TTC AGA GAG ACA GAC AGC AT | CCC ACA TAG TGC AAA AGA CA | 1723, 1724 |
| chr21 | 16298842 | 16299091 | AGA GGA AAA TCA CAA GCA GT | GTA TGT GTG AAG TAG CCG AG | 1725, 1726 |
| chr21 | 16316213 | 16316462 | TTT GAA AAC CCA ACA GAC CT | TGG TCC TAA CTC AGA CCT TT | 1727, 1728 |

FIGURE 2 (Page 54 of 80)

| chromosome | start | stop | forward primer | reverse primer | SEQ ID NOs: |
|---|---|---|---|---|---|
| chr21 | 16319836 | 16320085 | AAT CCA CAC ACC AAC AGA GG | AAC ATG AAG GGA AGG TTG TG | 1729, 1730 |
| chr18 | 64634331 | 64634580 | TGT TCA CAT CTG TTG GTT TGC | TAT ACT TCA ACT TGC AGG CAG | 1731, 1732 |
| chr18 | 64645925 | 64646174 | GTT ACT CGG TGG GTG ATA TTT | GCC AAA GAC AAT GAG AGA GTC | 1733, 1734 |
| chr18 | 64975938 | 64976187 | AAG AAA CAC CAG CAT CAG TTC | ATT TAG CAG CCA TGA CCA GTA | 1735, 1736 |
| chr18 | 64996573 | 64996822 | AGG TTT AGA GGT GAG TGA ACA | TGG GCC AAT TCC TAA TCC ATT | 1737, 1738 |
| chr18 | 65066425 | 65066674 | AGA AAC TTC ACT GTC TTC CAC T | CGA TCT CAT GAA TAA GTC TGA CC | 1739, 1740 |
| chr18 | 65073729 | 65073978 | TGT GAT GGA CAT TGG TAC CTG | TGG AAA GCA GAC TAA CAG TGA | 1741, 1742 |
| chr18 | 65074170 | 65074419 | ATA TCA TCT GCC TGT CCC AAC | GGG ACC CTA TGT AGA GAT TGT | 1743, 1744 |
| chr18 | 66343957 | 66344206 | GAG TGC TCT GTG TTT GTT TCA | GGA GCC CAA GGA TGT ATT AGA | 1745, 1746 |
| chr18 | 66344406 | 66344655 | TCA GTG GTG AGC TCT TGA ATA T | CTA ATA GCT GGT TCT GCA CAC | 1747, 1748 |
| chr18 | 66585247 | 66585496 | ACT CTC TCT TCA CAC ATG CAA | GAT GAA ATG AAT GCT GAC TCT C | 1749, 1750 |
| chr18 | 66596489 | 66596738 | GTG TTG AAG TCA GTA AAG CCT | AAA CTG AAG CTT CGA GAA CCC | 1751, 1752 |
| chr18 | 66601231 | 66601480 | TGC TTT CAC ATG GCA CTA GAT | TGC CGA CAA CTA CTT TAG GTA | 1753, 1754 |
| chr18 | 69966208 | 69966457 | GGA GAG AGA AAT CCC AAC TGA | TGT ATC CAA TCA CCT GTC AGA | 1755, 1756 |
| chr18 | 70088939 | 70089188 | GGG AGA TGT CAA CAC TAG GTC | CAG CAC CTT AAG CAG AAA TCA | 1757, 1758 |
| chr18 | 70093364 | 70093613 | ATA TGA CAT GGT GGC TCT CC | AAG CCC TTC ATC CAT TTC TCT | 1759, 1760 |

FIGURE 2 (Page 55 of 80)

| chromosome | start | stop | forward primer | reverse primer | SEQ ID NOs: |
|---|---|---|---|---|---|
| chr18 | 70107935 | 70108184 | AAC ATA GAG CCA TGG GAG GTA | ATC TTG GTG CCA TCT TAA GGT | 1761, 1762 |
| chr18 | 70198868 | 70199117 | GAT AGC CTT CAA ATC ATG CCT | AGC AAA CCA ATC GCA AAC TAG | 1763, 1764 |
| chr18 | 70200128 | 70200377 | GAA AGC GGG TGA ACA ACA ATA | AAT CAT CAT CTT CAT CAG CTC G | 1765, 1766 |
| chr18 | 70200907 | 70201156 | GAT AGA GAG CAC AAA GAG CAT | CCT TTC GCC TTG CTT ATA TGG | 1767, 1768 |
| chr18 | 70674976 | 70675225 | AAC AAG AGG AAT AGG AGC CAG | ACT ACT CAA CAG CCT ACC AAA | 1769, 1770 |
| chr18 | 70682967 | 70683216 | TGG GTG CTG ATA GTA ACA AAG | TGG AGC TGG GAA CTT TAA TGT | 1771, 1772 |
| chr18 | 73541191 | 73541440 | ACT ATT GAA CTG TTG GCT TCG | GAA GAG AGA GAG AAT GCG TGT | 1773, 1774 |
| chr18 | 73548315 | 73548564 | TCC ACT AAA GAG CAA CCA AAC | GAC ATG GAT ATT CTG GTG CCA | 1775, 1776 |
| chr18 | 73605288 | 73605537 | TGA AGT GGT CAG TAA CAA TGG | CTG TTG CAA GAT GAC CCA AAT | 1777, 1778 |
| chr18 | 73704485 | 73704734 | ATT TCA GAG CTC CTT TGT CCT | AGA TAC ACA CAC GTT CAC AAA C | 1779, 1780 |
| chr18 | 73752546 | 73752795 | GGC AAA GAA ATC TGG TGT TCA | GAT GGC AAT GCT TGA TAA CGA | 1781, 1782 |
| chr18 | 73764755 | 73765004 | TTG CTG GTT GAT AGG CAT TTG | TTC CAT GAA GTT CCT CAA GAC T | 1783, 1784 |
| chr18 | 73768420 | 73768669 | ATT CCC GCA ATT GTG AGA TTC | CCA CCC ACA TAC CCT GAA ATT | 1785, 1786 |
| chr18 | 73775027 | 73775276 | AAA GAG GTA CAG AAC TCA GAC C | GTA ACA CAC GGA TGC TGA AG | 1787, 1788 |
| chr18 | 73788751 | 73789000 | GTC TTC ATG AAC GTT GCC AAT | TGG ACA CGA GGC TAT TTG TAG | 1789, 1790 |
| chr18 | 73851108 | 73851357 | CTT CTC AGG GCT CTT TGT GTA | GGC TTA AGA AGA AGA GTG GTT | 1791, 1792 |

FIGURE 2 (Page 56 of 80)

| chromosome | start | stop | forward primer | reverse primer | SEQ ID NOs: |
|---|---|---|---|---|---|
| chr18 | 73854713 | 73854962 | GCA ACA GAA ACC AAG ATT CCT | TAG TTT CCT TTG GCC TTC TCC | 1793, 1794 |
| chr18 | 73871422 | 73871671 | TCA TTG TCT ACC TCA AAG AGC A | CTA GGG TTC CCA GTT CAC AAA | 1795, 1796 |
| chr18 | 73876103 | 73876352 | CTC TGA AGG AAC AAA GGA TGG | GAA ACA ACA TTG AGG GCA TTG | 1797, 1798 |
| chr18 | 73879805 | 73880054 | CAT TAG AAT GCG GTG GTT TCA | GAG CTC TTT GAA GTA GAA GCA | 1799, 1800 |
| chr18 | 73884248 | 73884497 | GAT GTC TGG GCT GAG GTT TAA | CCA TTC CAA CAA AGC TTC CG | 1801, 1802 |
| chr18 | 73931865 | 73932114 | TTC CTC TTG AAG ATG CAC TGG | CTA CTT GCC CTA TTG TGT CGA | 1803, 1804 |
| chr18 | 73938014 | 73938263 | CCA GCA TGT GAG GAA TTG AAC | CCA GGG TGT TTG AAG GTA GAA | 1805, 1806 |
| chr18 | 73943212 | 73943461 | CCC ACT TAG TCA TCC ACA CAT | CTG GAG GTA AGA AGG AAT GCA | 1807, 1808 |
| chr18 | 73947628 | 73947877 | GTT TCA CAC ACC AGA AGA GAG | ATG TGG GAC TCT TTG CTC TC | 1809, 1810 |
| chr18 | 73964168 | 73964417 | CCA TAC ACC TGC TCT GAC ATT | ATG AAT ACA GCT TTG CAT GGC | 1811, 1812 |
| chr18 | 73966321 | 73966570 | ATC AGT AAC AGT CCC ATT GCT | GTT AAA GCA TTC ACA GCC CTC | 1813, 1814 |
| chr18 | 73974071 | 73974320 | CAT GAG GCA TTT GAT CCA TGG | CTG GGA CTT GTC TAT CCT CCT | 1815, 1816 |
| chr18 | 73985549 | 73985798 | ACC CTG TTT CAC TGA ACA ACT | ATG TCT GTC CAA GTG AAC AGT | 1817, 1818 |
| chr18 | 73987174 | 73987423 | CCC TGT AAT GAG AGC GTT ATT | AAA GTA TCC AGA CCC AGA ACC | 1819, 1820 |
| chr18 | 48466086 | 48466335 | GAG AGA ATG GGT TAA ATC TGC C | ATA GGC CAG CAC TCC AAA TAA | 1821, 1822 |
| chr18 | 48928528 | 48928777 | CTG CCT GAC TTA GCC TTA AAT | CAA ATC AAG TCC CAT GGT AGG | 1823, 1824 |

FIGURE 2 (Page 57 of 80)

| chromosome | start | stop | forward primer | reverse primer | SEQ ID NOs: |
|---|---|---|---|---|---|
| chr18 | 48929078 | 48929327 | AAC CAG AAT GTT ACT AGC CCA | CTT TCC GTC TTT ATA GGC AGC | 1825, 1826 |
| chr18 | 48951524 | 48951773 | CAA TCC TGT GTG TTT AGT GGA | AGA AAT CGT GTT CAC AGC CTA | 1827, 1828 |
| chr18 | 48951856 | 48952105 | TGT GGG AAG CAT TGA CTC TT | CCG TAA ATG AAG TGG CTT GAA | 1829, 1830 |
| chr18 | 48959882 | 48960131 | TCC TGT GAG AAA TGG AGC TTT | CCT TTC TTG CAA CCT TGA GAT | 1831, 1832 |
| chr18 | 48960304 | 48960553 | GTT GCC AAG CTT AAA TAC CTG T | CTC TAG ATG CTC AAC CTC AGG | 1833, 1834 |
| chr18 | 49004516 | 49004765 | CCA CAA CAA CAT AAA CAC TGC | AAT AAC AGT CCA CCA GAA CCA | 1835, 1836 |
| chr18 | 49006425 | 49006674 | TCT TCC AGG CAT ATT CAT TGC | TCC AAG GAC CTG CAA ATG TTA | 1837, 1838 |
| chr18 | 49017695 | 49017944 | ATC CAT TCT CTC TAC TTG GGA | AGG TTA CAT CAT TCA CCC ACA | 1839, 1840 |
| chr18 | 49148010 | 49148259 | GGC TAG AGG GTG ATT ATA AGC T | ATG AAG ACA ATG ACA TCT GCG | 1841, 1842 |
| chr18 | 49149627 | 49149876 | TAC TCA TCC CGA TTT CTT CCC | CAC TTG TCA TGG TTT AGG GAC | 1843, 1844 |
| chr18 | 49149933 | 49150182 | TTC TCT TTC TCT TCT GGG CAG | ACC TCC ACC TTA TTG CTT CAA | 1845, 1846 |
| chr18 | 49150459 | 49150708 | TTG TGA GAC TCA AGG CCA TTT | GAA TTG CAA AGG ATG GGT AGG | 1847, 1848 |
| chr18 | 50239403 | 50239652 | TTG GTA GAG AGA GGC CAT TTG | CTG CAT TGT GAG TCC ATG TAA | 1849, 1850 |
| chr18 | 50242789 | 50243038 | GGA GGA AGC TCT TGA AGA CAT | TCC TAT CTT CAT CCC TCT TCC | 1851, 1852 |
| chr18 | 50243069 | 50243318 | AGC ATC TTC CGT TTA ACT CCA | TTC CAT TTA GCC TCC CAT CTG | 1853, 1854 |
| chr18 | 50243367 | 50243616 | CTG CCT GCC AAG TAT GTT CT | TAG CTT TAT GGG CCT TGT TCT | 1855, 1856 |

FIGURE 2 (Page 58 of 80)

| chromosome | start | stop | forward primer | reverse primer | SEQ ID NOs: |
|---|---|---|---|---|---|
| chr18 | 50247229 | 50247478 | CTA CTC CTT GTG TCA TTG GCT | CCA CCT CTC AAA CCC AGA TTT | 1857, 1858 |
| chr18 | 50308727 | 50308976 | TGA TTC TGA GAC ACG TGC TTA | GTC AGC GTA TTT GGG ATT GAA T | 1859, 1860 |
| chr18 | 50309031 | 50309280 | CTT GAG GAC CTT TCA TGC TTG | AAA TCT GCC ACC CAT TTC TTC | 1861, 1862 |
| chr18 | 50338747 | 50338996 | TTG TGA TTT CAG GTA GGA GGG | TAT TCT GAG TTC TAC CCA GGT | 1863, 1864 |
| chr18 | 50423284 | 50423533 | TTC CCT CAG AGA CAG TAT CCT | TAC ATG AGA CCC AGA AAC AGA | 1865, 1866 |
| chr18 | 50424422 | 50424671 | AAA GGA GGT CTG GCT TTG AA | CTT CTT GGC AGA CTA TCA GGA | 1867, 1868 |
| chr18 | 50424789 | 50425038 | GCA TAA ACC TGG ACT GTG AAA | AAG CTG CTA AAT CTG TAG GGA | 1869, 1870 |
| chr18 | 50448837 | 50449086 | CAC TCA GCG ATT CTC CTC AC | CTG ACC AGA CCT GTT GAC TAA | 1871, 1872 |
| chr18 | 50449492 | 50449741 | AAA GCC AGA CAC AGA CTA GTT | TGA TAT GTT CAG TTT GCC TAC C | 1873, 1874 |
| chr18 | 50514079 | 50514328 | ATT CCT GGG ACC ACA AGC AT | AGC TTG AGT TTC TTG CTG GG | 1875, 1876 |
| chr18 | 50519889 | 50520138 | TAT TGC CTC ATG TGG TTG TG | TTC CCG CAA AGT AGA AGC TAT | 1877, 1878 |
| chr18 | 56064244 | 56064493 | GTG TTG ACT TGA AAG GAA TCA C | AAA CGC ATA CAA ACA GGA GAC | 1879, 1880 |
| chr18 | 56148666 | 56148915 | CAG GTT AGG AAT GAC AGT GGG | CTT CTA AAC CCA TCA CCT GCT | 1881, 1882 |
| chr18 | 56149037 | 56149286 | TTA GGT TAC CCA GGG ACG TTA | CAC CCA AAC TCA CAG GTA CAA | 1883, 1884 |
| chr18 | 56244171 | 56244420 | CGG TTT GCT TTC TGA ACA ACA | TGA ATT CTG AGA TCG AGA GCC | 1885, 1886 |
| chr18 | 56245242 | 56245491 | CAT TTG GGA CCC TTT GAA ACT | AGA TTA ACT GTT GCC TCA CTG | 1887, 1888 |

FIGURE 2 (Page 59 of 80)

| chromosome | start | stop | forward primer | reverse primer | SEQ ID NOs: |
|---|---|---|---|---|---|
| chr18 | 56247063 | 56247312 | GGT TAT CTC TGG GCA AAG TTC | CAA GAC AGT GCA TTC CAT GG | 1889, 1890 |
| chr18 | 56527049 | 56527298 | TAG AGA GCA GAG AAC AAA CCC | CCA AGG AAA GAG TTG AGA AGG | 1891, 1892 |
| chr18 | 56528011 | 56528260 | ATA AGG GCA TTT GGA GGG AAA | TGA GTG CAG TCG ATA AGG AAG | 1893, 1894 |
| chr18 | 56532190 | 56532439 | AAT GCA CAC TTA GAC ACC ACA | AAC ACC GAG AAA GAG AGA GAG | 1895, 1896 |
| chr18 | 56532882 | 56533131 | GGA TTG CTA CCC AGG AGA TAA | TGT ACT GCT TTC GTC TTA TGC | 1897, 1898 |
| chr18 | 56534208 | 56534457 | AGA GGT TCT GTG TAT GAG TGT | CAT ATT CGC ACT GTA TAG CCG | 1899, 1900 |
| chr18 | 56601487 | 56601736 | TAG AGA ATT GTA CGC TGG ACA | CTG ATG GAT TCT CTG GTG TGA | 1901, 1902 |
| chr18 | 56633131 | 56633380 | TCT GGA GAA ATG CAC AAG AGA | TCA AGG AGA AGA GAG AGG GTA | 1903, 1904 |
| chr18 | 56635259 | 56635508 | TGG GTT AGA ACA TGG TGC TTA | TCT CCC AAA GCA GAC AAA GAC | 1905, 1906 |
| chr18 | 56719296 | 56719545 | ATC GCA TCA CAC CCT TAC TAT | AGT CAG TTG TTA CGT GCA AAG | 1907, 1908 |
| chr18 | 58037828 | 58038077 | GGA AAT TTA GCT TGA CAT GGC | AAA GGT TTG TTC ATC CTC CCT | 1909, 1910 |
| chr18 | 58038328 | 58038577 | TTT GTA AAT CCA CAG TGC CTA C | ACA GAG CAC GCA ATA TAG GAA | 1911, 1912 |
| chr18 | 58039111 | 58039360 | GGT ACT GGA GAG CAT AGA AGA | CTG CAT TCA CCC ATG TAC TTT | 1913, 1914 |
| chr18 | 58040290 | 58040539 | AAA CAA GCT ATC TTC AGG CAG | TCC AGC CAT ACC ATG TCT ATC | 1915, 1916 |
| chr18 | 59424085 | 59424334 | CGA ACA ATC AGA GAC TCG ACT | CTC ACT AGG GAA GAA CAG CAG | 1917, 1918 |
| chr18 | 59424765 | 59425014 | CTG GTT GAC AAT CTG CAA GTT | TGC TGG GTC TGA GTG TTA TAA A | 1919, 1920 |

FIGURE 2 (Page 60 of 80)

| chromosome | start | stop | forward primer | reverse primer | SEQ ID NOs: |
|---|---|---|---|---|---|
| chr18 | 59441324 | 59441573 | ACC ATC CAA GTC GTC TTC ATA | GTT GTG TGA ATG GTG CTG TTA | 1921, 1922 |
| chr18 | 59499718 | 59499967 | ATA GCT ACG CAT ACC CTG TAG | GGC CCA GAA GAC TCT TGT AAT | 1923, 1924 |
| chr18 | 59500226 | 59500475 | AGC AGA AGA AAC AGT AAG GCA | CGA CCT ACA TCA GCT AAT GGT | 1925, 1926 |
| chr18 | 59507920 | 59508169 | ACC CAG GGA CCT ATT TGT TC | AAG GGA AGA ATA ACA ATG GTG C | 1927, 1928 |
| chr18 | 59543044 | 59543293 | CAA GGG CTC AGG TCT TCA TTA | ATG GGA GTA TGG GAG TAG GAA | 1929, 1930 |
| chr18 | 59556453 | 59556702 | TCA CTG TGA CTT GGA GAC TAA | TTG GTG GCT TGC AGA GAT TT | 1931, 1932 |
| chr18 | 59614976 | 59615225 | TGC TCT GCT TCA CTG TGA TTA | TCA CAC GAT CAT CAT ACT CAC A | 1933, 1934 |
| chr18 | 59625553 | 59625802 | GAT GAA TGA CTA ATA GCC CAC G | GGT AGC AGA TGA CTA GAC GAT | 1935, 1936 |
| chr18 | 59631090 | 59631339 | CCA TGT TTA GTT TGG TGC TGT | ACG CCT CTG TCA TTT CCT AAC | 1937, 1938 |
| chr18 | 59631779 | 59632028 | AGC CAA GTG AGG TGC TAA AT | CAG TTG ACT CAA TGG TGC AAT | 1939, 1940 |
| chr18 | 59632388 | 59632637 | TCA GGG AGA AAT GAT GTC ACC | ATA GGT TAC AGA TTG CCA CGT | 1941, 1942 |
| chr18 | 59695718 | 59695967 | CAG TAG CTG GCA AGA ATC ATC | GAT GCT GCT ATC AAA GGA ACA | 1943, 1944 |
| chr18 | 59696087 | 59696336 | CAA CAC TGC TAG AAT TCC CAA | AAG ACA AAG AGA TGG AAG GCA | 1945, 1946 |
| chr18 | 59696563 | 59696812 | CCT GAG AAG CAC CTG ATT GTA | AAT ACC CTC TTC CCT TCC TCA | 1947, 1948 |
| chr18 | 59712419 | 59712668 | TGT TGT CAG AAA TCC CAG GAA | CCA CCG TCA ATA TTT ATC AGC T | 1949, 1950 |
| chr18 | 59756979 | 59757228 | AAC TGG AGC CAT ATA ACG ATG | GCC TCA GTC CAA ATC TTA GAT | 1951, 1952 |

FIGURE 2 (Page 61 of 80)

| chromosome | start | stop | forward primer | reverse primer | SEQ ID NOs: |
|---|---|---|---|---|---|
| chr18 | 59770789 | 59771038 | GAA ATG GTG CCC TAT TGT TGA | CCT TAG GAT TCT CAA AGA GTG T | 1953, 1954 |
| chr18 | 59805993 | 59806242 | TCT TAC ATG CAG TCA TAC TCC T | GCA GTA CAG ATT CTT GAA CAG T | 1955, 1956 |
| chr18 | 59813876 | 59814125 | GTC CCT CAG TAA CAC CAT CTT A | AGA GGA TTA GAT GTC TTG CTG T | 1957, 1958 |
| chr18 | 59855411 | 59855660 | GAA GCA AGA GGA TCA GGC AAT | TAC TGC AGG CAA TTC AGG TAA | 1959, 1960 |
| chr18 | 59909054 | 59909303 | AGT GTT TCA GAG GCT TGA AAG | GAA GAG GTC CAG TAA GTG AGG | 1961, 1962 |
| chr18 | 59909336 | 59909585 | ATC CGG CAT CCT TTA AAC TCT | TTG TGA GTC CTT GTC TCC TTG | 1963, 1964 |
| chr18 | 59910140 | 59910389 | AGG CTA GGA AGA AAT GGG AAA | GAC GAC TAA GAC ATT GCA TCA | 1965, 1966 |
| chr18 | 59910602 | 59910851 | ACA CAT ATG CTC TGT CTC TCA | AGA AGT CTC TCT CCG TTG TTT | 1967, 1968 |
| chr18 | 59973803 | 59974052 | AGT TAG TTA TCA CCT CGT CCC | CTT ATG TGC ATC AAC TGT GCT | 1969, 1970 |
| chr18 | 59996728 | 59996977 | TGT GTA TTT CCC TCT AGT TGC A | AGT GTC TCT CAG AAT CAG GAC | 1971, 1972 |
| chr18 | 60010541 | 60010790 | AGC CTC TTT CTA CAT CGT TCG | TTT ATT TCC CTA CGC AAA GCC | 1973, 1974 |
| chr18 | 60011381 | 60011630 | TTA CCT GTG CAG AAG AGT GAC | AGC CTT GGA TGA CTG AGT TGA | 1975, 1976 |
| chr18 | 60240694 | 60240943 | TCT TGT GTT CTA GCG TGT TTG | TTG GTT TCT ATT CTG CAC TGC | 1977, 1978 |
| chr18 | 60378353 | 60378602 | TGT GGT TAG TCA GAA ATG TGG | TTA GAG CTT GCT AGT ATC GGG | 1979, 1980 |
| chr18 | 60384593 | 60384842 | GGG TCC TGA TGA GTC TTT GTC | GAA ATC CCA AAC TGC CTG AAA | 1981, 1982 |
| chr18 | 60384979 | 60385228 | TGG TGC CTT TGT TTA TTC AGC | ATC CTT CTT GTG AAC CTT CCT | 1983, 1984 |

FIGURE 2 (Page 62 of 80)

| chromosome | start | stop | forward primer | reverse primer | SEQ ID NOs: |
|---|---|---|---|---|---|
| chr18 | 60389544 | 60389793 | TGT TAT GTG CCA GGG TTT AAC | ACC AGA TGC ATG TGA TTA AAG G | 1985, 1986 |
| chr18 | 60401084 | 60401333 | ATT GGT TCC AGA TAC AGT CGA | GTG TAC TCT AGG CTA CTG TCA | 1987, 1988 |
| chr18 | 60412199 | 60412448 | GGA AGG AAG TAC AGC ATG GAT | GTC AGC AGC AAG TAA AGG TTC | 1989, 1990 |
| chr18 | 60420053 | 60420302 | CCT TAT TGA AGC TGA CCA TGC | CAT CTA GTC AAG GGT TCC ACA | 1991, 1992 |
| chr18 | 60434561 | 60434810 | CAG TGG GAA ATG TGC TTA CAT | CAA GTC ATG CTC CAA ACT GTT | 1993, 1994 |
| chr18 | 60449047 | 60449296 | AAA GGG CCT ATA TTC ACC AGA | AGG ACT TAG GAC AAC AGA GAA | 1995, 1996 |
| chr18 | 60452471 | 60452720 | AGA GCC ATT TAA GAC TCT CTG T | TGC CCA ACA CCA TCT CTA ATA | 1997, 1998 |
| chr18 | 60456757 | 60457006 | TAC CTT GTT CTC TGC CTC AAT | GCC TTC ATC ACT CAG AAC TTC | 1999, 2000 |
| chr18 | 60467147 | 60467396 | TTT AGA AGG ATG TGG ACA GGG | AGG GTA TCT ATT CTC CGG ACA | 2001, 2002 |
| chr18 | 60469757 | 60470006 | AGC AGG ACA TGG ACT TCA AA | ATT TGC CCA AGT AAG TTC CAC | 2003, 2004 |
| chr18 | 60514168 | 60514417 | GCG GCT CTT GTT TCT GAA ATC | TGG AAG TAC ATG GGA TGC ATT | 2005, 2006 |
| chr18 | 60537888 | 60538137 | ACA GGT AGG AGT TCA GAG ACA | CTT GAA GAG TTC CAA TGC CAA | 2007, 2008 |
| chr18 | 60544380 | 60544629 | TTT CCT ATT CTG CTC TTC TGC T | CGC TGC TGT TTA AAT CGA TCA | 2009, 2010 |
| chr18 | 60544742 | 60544991 | AAA TGC TGC TCA GGG TTA GAG | GCT CTG CTT TGC TCA AAT TCT | 2011, 2012 |
| chr18 | 60545407 | 60545656 | CAA CTT TAC TCT GCA CAG CTC | TGA GCT CCA GAA TTA GAT GTG T | 2013, 2014 |
| chr18 | 60611934 | 60612183 | CAG TGC CAC TAC AAA GAA ATC A | AAC ACC TCC TTT CTC ACT ACA G | 2015, 2016 |

FIGURE 2 (Page 63 of 80)

| chromosome | start | stop | forward primer | reverse primer | SEQ ID NOs: |
|---|---|---|---|---|---|
| chr18 | 60617081 | 60617330 | AGA AGC AGA GGT TGG ATA TGG | ACA AAC TTC ATT CAC CGC AG | 2017, 2018 |
| chr18 | 60617641 | 60617890 | CCA CAA TCC CAT AGT CAC CAT | AAC TAC GCC ACC CAA CTA AA | 2019, 2020 |
| chr18 | 60630438 | 60630687 | GCT GGT TCT TGT TGC TGA TAA | GCT TCA GTG TAA CCA TGA CTC | 2021, 2022 |
| chr18 | 60630849 | 60631098 | TCC CTC ACG ACT TAT GTT TGA | TGC ACA ATT AAG CTA CTT CTC C | 2023, 2024 |
| chr18 | 60639840 | 60640089 | CTA CCT TCT CCA GTG CAC TAT | CAA CAC CAA ACT TGC CTG AAT | 2025, 2026 |
| chr18 | 60640300 | 60640549 | GGA CCT CTC TTT GAA ATG GAC | TCC ACA ATT TCT ACA GCA ACC | 2027, 2028 |
| chr18 | 60776944 | 60777193 | TGG GTT GTG TTT CTC TGA CTT | GTG TCA TTT GAT TGG TGC TCT | 2029, 2030 |
| chr18 | 60813738 | 60813987 | TAC AAG CCT CCT TTA ACC CTT | GGG TGT TTC AGT AGG TTA GGA T | 2031, 2032 |
| chr18 | 60814585 | 60814834 | AAA GGG TTT GAT ACA GTT GGG | TGG GAT TCT AAT GTC TGG TGC | 2033, 2034 |
| chr18 | 60938666 | 60938915 | AGG TAT TGG AGA GCA AGA AAG A | AGA GGT GGT TGG TTG GTT | 2035, 2036 |
| chr18 | 60939202 | 60939451 | GCA AAC TGG AGC TAA AGT CAT | GGG CAT CCT GTC TGA AAT ATG | 2037, 2038 |
| chr18 | 60950541 | 60950790 | TTG GTT GCT AGC TCT CAA ATG | AAG AAG CGC AGA TAC AGT ACA | 2039, 2040 |
| chr18 | 60953225 | 60953474 | TTC AAT GCC CTT ACT TCT CCT | CAC AGC AAG TTT GAA CCT AGT | 2041, 2042 |
| chr18 | 60958474 | 60958723 | GCT TGA GGC GCA TAT GAT TG | AGT GCA AAT GAT GAC CTG TTG | 2043, 2044 |
| chr18 | 60960879 | 60961128 | CAT ATG GTG CTT GTT CTG GG | GAA CTG TTG CAT GAG AGG TAC | 2045, 2046 |
| chr18 | 60965583 | 60965832 | CAA ACT CAC CAC CCT TCA TTC | CTT TGT CCT TCT CTG TTG TGT | 2047, 2048 |

FIGURE 2 (Page 64 of 80)

| chromosome | start | stop | forward primer | reverse primer | SEQ ID NOs: |
|---|---|---|---|---|---|
| chr18 | 60970051 | 60970300 | CCT TAG CCC TGC AAA TAA CAC | TTT CTT CTA GAG TCC AGA GGT G | 2049, 2050 |
| chr18 | 60974839 | 60975088 | TCA CAG ATA CGG ACA AGC TC | ATT CTC TTC TCT CTT CCA GCC | 2051, 2052 |
| chr18 | 60984378 | 60984627 | GCC ACA GGT ATC AAT CAC TTC | TAT GGC TTT GCT ACC TTG TCA | 2053, 2054 |
| chr18 | 61055997 | 61056246 | GCT TCT GTG GCA CTA ATC AAG | CTA TTT CTC TGG CTC TTG ACC | 2055, 2056 |
| chr18 | 61088669 | 61088918 | GCC TTA TTG ACT TAC TGG ACT G | TTC TTA AAC CTC TGT GTG GCT | 2057, 2058 |
| chr18 | 61090462 | 61090711 | GTC AGG GAT TAG AGG CAG AAC | TGG ACA AAC AAG AAC TGG GTA | 2059, 2060 |
| chr18 | 61100325 | 61100574 | CAA TCA CTT GGT CAG ATA GTG T | CCA TGA TCA CTG AAA CCA ACA | 2061, 2062 |
| chr18 | 61154459 | 61154708 | AGT GGA GAG GTT GAG TAT AGT G | CCA TAT GCC CTG CTC TTT AAG | 2063, 2064 |
| chr18 | 61162901 | 61163150 | TGA GAA GGG AGG AAG AAT GTG | GGG TGG ACA AAG CAA TTC AAA | 2065, 2066 |
| chr18 | 61170629 | 61170878 | AAG GTC AAA CTC TCC ATT CCA | CAG CAT TCA ATT CAT CCT TGT G | 2067, 2068 |
| chr18 | 61197492 | 61197741 | GGA GAT GTC TTT GCC CTG ATT | AAG TAG AAG CAA GCC CTG AAT | 2069, 2070 |
| chr18 | 61213452 | 61213701 | GGT CCT CGT TTG TCC TTA AGA | GTG TGG TAG GGA TGA GAA TTA T | 2071, 2072 |
| chr18 | 61213900 | 61214149 | AGT ATT GCT TTG AGG GCT CTA | TCA GGT AAG CTT CCC TCC AC | 2073, 2074 |
| chr18 | 61216749 | 61216998 | AGC TAT CAT GTA AGT CAC TCC C | ACT GGT TGT AGA AAG GAC CTC | 2075, 2076 |
| chr18 | 61232928 | 61233177 | AGG AAA TTC AGT ACC TCA GCT | AGT AAG AGG CCA GAA GTC AGA | 2077, 2078 |
| chr18 | 61236079 | 61236328 | CAA CCT TCT CAT TGT TGA AGC T | GCA GTG CAG GCC TAT ATA TAG | 2079, 2080 |

FIGURE 2 (Page 65 of 80)

| chromosome | start | stop | forward primer | reverse primer | SEQ ID NOs: |
|---|---|---|---|---|---|
| chr18 | 61253277 | 61253526 | GGT GAC TTT GCT TTC CCA AG | AAA TCT CTG AGT CGG CCA TAA | 2081, 2082 |
| chr18 | 61259478 | 61259727 | GCT GAC AAA CGG AGG GAG AG | AGG CAT GGC AAA CTT ACT TG | 2083, 2084 |
| chr18 | 61264533 | 61264782 | TGT TCT TCA TCA GGC ACA ATG | CAT TTC ACT TTC GAG GAT GGT | 2085, 2086 |
| chr18 | 61289440 | 61289689 | ACC ATT TGT GTG ATC CAG AAC | GTC AGA CTA AAG TGA GGA CCA | 2087, 2088 |
| chr18 | 61368889 | 61369138 | CTT GTC TTG AGT GCG GTA CA | CCA GCC CTA CCT AAA GTG AAT | 2089, 2090 |
| chr18 | 61370663 | 61370912 | GCC TCT CAG TTT CCT CTT ATA G | CCC TTT CAC AAG ACT CTT CTC | 2091, 2092 |
| chr18 | 63685629 | 63685878 | CCT AGA TCA GTG CAG AGA ATT TAG T | | 2093, 2094 |
| chr18 | 63711583 | 63711832 | CTT TAG TTT GGA GGC CTC ATT C | CCA AAT GTA GAA CAG GAT CAG C | 2095, 2096 |
| chr18 | 63721183 | 63721432 | CTG ACA TCG ATG GAA TTC TGG | TAG CAG TAG GTG TGG CTT TC | 2097, 2098 |
| chr18 | 64379625 | 64379874 | ACA CTG CAC GAA TGG AAG ATC | TTG GAT TCT CTT GGT TGT GAG | 2099, 2100 |
| chr18 | 64398368 | 64398617 | ATA AAC TTG GTG CTC AGT GGT | CAA CGC TTT GGT ATA GTT TGT G | 2101, 2102 |
| chr18 | 64410092 | 64410341 | ACA AGC ATT ACA GAA TTC GGC | CCA GGT GCC ATC GTT AAA GAA | 2103, 2104 |
| chr18 | 64412606 | 64412855 | ACT GAC AGA TTC TCA CCT ATA TCA G | | 2105, 2106 |
| chr18 | 64417267 | 64417516 | GGA TCA AAG CCA CTC TAG ACT | GGA TAA GTC AAC TAC CAT GGT T | 2107, 2108 |
| chr18 | 64421562 | 64421811 | TTG AGA TGG CAT CAA GTT CAA G | AAT GGA ATT ACT CAG CTG TGG | 2109, 2110 |
| chr18 | 64426871 | 64427120 | ACT GGA TTC ATG CGT TAT CAA G | GGC AGA AAC TGA TAG AGA CTG | 2111, 2112 |

FIGURE 2 (Page 66 of 80)

| chromosome | start | stop | forward primer | reverse primer | SEQ ID NOs: |
|---|---|---|---|---|---|
| chr18 | 64436287 | 64436536 | TAT TCT TGT GTG GAC CCT GTG | ACC ATG TTC TGA GTA CCT CTT | 2113, 2114 |
| chr18 | 64593727 | 64593976 | TGA TAT TGC ATG AAA GTC CCT G | ,AG TCC TGA ATC AAT GTC TAA CAC | 2115, 2116 |
| chr18 | 24962594 | 24962843 | TCA CAC ATG AGG AGT AGA CA | TGG TCC CTG TGC TTT GAT AT | 2117, 2118 |
| chr18 | 24964748 | 24964997 | CAC AGC AGG AGA CAT GAG AA | TGG CTT TTC TTT CCT CGG TA | 2119, 2120 |
| chr18 | 25003553 | 25003802 | TTT CCT CCT GGC TTG ATC AC | CCA AGG CTG CTT TAA TTC CA | 2121, 2122 |
| chr18 | 25033345 | 25033594 | CAA AGG AGA GAA GTG ACC CA | CAA ACT ATC GCT GAG GAC CT | 2123, 2124 |
| chr18 | 25037434 | 25037683 | CTA CGA GTG AAA CAG AGT GC | GTC TGC TGC CAT TGA GTT AT | 2125, 2126 |
| chr18 | 25052992 | 25053241 | AGA CTA AAA GCC TCC AAG CC | GCT CAC CCT CTC TTC TCT CT | 2127, 2128 |
| chr18 | 25080042 | 25080291 | TAG AAT ATG TCA CCC AGC CC | ACC TGT TTC TCC CAG TTA CA | 2129, 2130 |
| chr18 | 25124915 | 25125164 | ACA GAA TCA TCC CAT AGC CA | GCC CAT GAA AGA GAA ACC AG | 2131, 2132 |
| chr18 | 25128057 | 25128306 | CGC CAT TCT GTG CTT AAT TTG | CTC CAT CAG TGC AGA AGT CC | 2133, 2134 |
| chr18 | 25140257 | 25140506 | GTG AAG CAA GAG AAA GCA AGA | ACA GTC AGC AGC CCT AAA AT | 2135, 2136 |
| chr18 | 25140722 | 25140971 | TGA TTC GGC TGC AGG TTA TT | GCC TCC TTC ACA TAA TGC AG | 2137, 2138 |
| chr18 | 25147759 | 25148008 | CTG GCT TCA AAT GCA TCT GA | TTG TCA ACA GAG AGT CAG CT | 2139, 2140 |
| chr18 | 25157828 | 25158077 | ACT GGG AAA TTG GAA TTC GC | CTG AAA TGG TCT GGG AGT CT | 2141, 2142 |
| chr18 | 25160244 | 25160493 | GGT AAA ACT GCC TGG AAA CT | TCA AAG ACA GAG TGA GTG GA | 2143, 2144 |

FIGURE 2 (Page 67 of 80)

| chromosome | start | stop | forward primer | reverse primer | SEQ ID NOs: |
|---|---|---|---|---|---|
| chr18 | 25166354 | 25166603 | AGA CCA CGG GCT CTA TCT AT | TTT GGG GGT TAC ACT TCA TAG | 2145, 2146 |
| chr18 | 25172986 | 25173235 | CAT CTT GCT TAT TGG CTT ACG A | AGC CAG CAG AAT AAT ACC AGG | 2147, 2148 |
| chr18 | 25174972 | 25175221 | GAG GTA GAG GCA GTG TCT TG | ACC TCA TCT TTT GTC AGC CT | 2149, 2150 |
| chr18 | 25175640 | 25175889 | TAG TCC TTG AAC TCC CTG GT | ACA GTT CCA TAG GCA GGT TT | 2151, 2152 |
| chr18 | 25182855 | 25183104 | CCA GCT TAG CGT CTG TTT TT | GCA GTT CCA GAT CCA ATA TGC | 2153, 2154 |
| chr18 | 25198531 | 25198780 | GAC CAC AAC TAT CAA GAG CAC | TTT GGG AAA GAT GGG AGA GC | 2155, 2156 |
| chr18 | 25201886 | 25202135 | CCA AAG AAA GGT TGA AGC CC | TTG CAG GTA AGG TAC AGA AGA | 2157, 2158 |
| chr18 | 25209395 | 25209644 | AAT AAT GTG CAC TGT GAT GGC | CTT CAG CTG CAT CTT GAG C | 2159, 2160 |
| chr18 | 25213880 | 25214129 | TTT AAG GGT CTG ATG GTT GC | GGC ACT TCA AAA ACA AAC CC | 2161, 2162 |
| chr18 | 25216888 | 25217137 | GAA AAT GCC CAT CGT CTC AA | AGG GTT TTA TGG TCT CCT GG | 2163, 2164 |
| chr18 | 25228617 | 25228866 | ATT CCT TGT CTT TCC CCC TC | TCA ACA CGG AGA ACT GAA AAC | 2165, 2166 |
| chr18 | 25231535 | 25231784 | TCA TTT CTC TAG CCC AAA GAT G | TCC GTG TAA ATG AAC AAA GCA C | 2167, 2168 |
| chr18 | 25245662 | 25245911 | TTG GTT TGT TGA CTT CAG CC | TTC AGG GAA TGG TTT GCA TT | 2169, 2170 |
| chr18 | 25246300 | 25246549 | GCT CAG TGA CAG TTG GGA TT | GAG ATG CCA TTC CCA AAA GG | 2171, 2172 |
| chr18 | 25254843 | 25255092 | GTC TTG TCT CTC TTC TTC CAC T | CCC TAC CAT AGT GCC AGA TG | 2173, 2174 |
| chr18 | 25265476 | 25265725 | TTT AGA GCA GGT GGA AAC GA | CAG CTT TCA GTG ACA GAG GA | 2175, 2176 |

FIGURE 2 (Page 68 of 80)

| chromosome | start | stop | forward primer | reverse primer | SEQ ID NOs: |
|---|---|---|---|---|---|
| chr18 | 25278532 | 25278781 | CTT GGG TTT TTA TCG GTT GCT | CCA AAG GCA GAT GAG TGT TT | 2177, 2178 |
| chr18 | 25279010 | 25279259 | GTT GCC TGG ATT GCT CTA AA | GCC TGG GAT AGA AAT GGG AA | 2179, 2180 |
| chr18 | 25283817 | 25284066 | AAA TCA CGA CGT AGG AAA CC | GGA AAG GAA AGG AAG CTG TG | 2181, 2182 |
| chr18 | 25292042 | 25292291 | TCA CCT TGG AGC AGG TCA TA | TCA GAG AGG TCT TGC TGA AG | 2183, 2184 |
| chr18 | 25295429 | 25295678 | CGA AGA AGG TCT GGG AGA TG | TCT CTC TGT TGC TTG TTT CCT | 2185, 2186 |
| chr18 | 25330345 | 25330594 | CAC CAT TGT TTC ATC AGG ACT | CGC ATG TGG TAG ATC ATC AG | 2187, 2188 |
| chr18 | 25332963 | 25333212 | TTG CAT CAT CAG CTC ACA TAC | TAT AAC ACC CTC ACC TCC CA | 2189, 2190 |
| chr18 | 25333284 | 25333533 | AGA CCT GGA AAA TGA TGG GT | CAC CCA AAT CAC CTT GCT ATG | 2191, 2192 |
| chr18 | 25345252 | 25345501 | CCT GGA AGT GTG TAA CAA GC | CAA CTA CCG TGG ATT CCG TT | 2193, 2194 |
| chr18 | 25384702 | 25384951 | CTC CCT AGC AAA AAC TTC TCA | TCT ATC ATG AGT CGC TTC CA | 2195, 2196 |
| chr18 | 25510767 | 25511016 | TGC CTT ATT CAC TGT GCA AC | TTT CTG TCA CTT TCT GGG CT | 2197, 2198 |
| chr18 | 25519108 | 25519357 | TGA TGG CCT AGT GAG TTT CC | CGT GTG TTT CTA GTG CAT TGT | 2199, 2200 |
| chr18 | 25531724 | 25531973 | TGC AAT GTA ACA AAA GCG TG | GGG CTC AGA GGG AAT ATC AG | 2201, 2202 |
| chr18 | 25543168 | 25543417 | GCC ACA TTT GCT TTC ACA CA | CCG ACG AAT GGA TGA AAG AC | 2203, 2204 |
| chr18 | 25547425 | 25547674 | ACG TCA TTG GGT TCA TGG C | ACC CAA ATT CCA TGC CTA CT | 2205, 2206 |
| chr18 | 25557343 | 25557592 | ATT TTA CCC CCT TAG GCA CC | TGT ACA GCA GTC TCC AGA AA | 2207, 2208 |

FIGURE 2 (Page 69 of 80)

| chromosome | start | stop | forward primer | reverse primer | SEQ ID NOs: |
|---|---|---|---|---|---|
| chr18 | 25564483 | 25564732 | TCT TCT ACA CAG CCC TTC AG | GCC CTC TTA CCC TTT CTC AT | 2209, 2210 |
| chr18 | 25583087 | 25583336 | ATT GGG ATC GTC AGC ATC AA | CAT TCA AAG ATC CAG ACC AGG | 2211, 2212 |
| chr18 | 25585083 | 25585332 | TGA TTG TCT TGT CCA CTG GT | ATC TGT GAT TGC TGC CCT C | 2213, 2214 |
| chr18 | 25589902 | 25590151 | ACA TCA CAC TTC ATG CCC TT | CTA GAA ACT CCC AGG ACA GA | 2215, 2216 |
| chr18 | 25601966 | 25602215 | ATG AAG GCA TTA GGA GGG AG | CAT GTG TGG AAA GGA TTG GT | 2217, 2218 |
| chr18 | 25611986 | 25612235 | AAA GTG GAG AAG TGG CAG AT | TAT GAA TGA ACC GTG GCT CA | 2219, 2220 |
| chr18 | 25617498 | 25617747 | CCT TAG GAT TCT GAG AGG TGA G | AAG TTG AGT CGT TTG TCC CA | 2221, 2222 |
| chr18 | 25629105 | 25629354 | AGG GCT TCT GAT TGA TTT GC | TAG TGT TTC AGG AGC GTG TT | 2223, 2224 |
| chr18 | 25631180 | 25631429 | CAG GGT AGT CGG GAT TTC TC | CCA GGA CAA GCA GAC ATT TT | 2225, 2226 |
| chr18 | 25643639 | 25643888 | CCC GGT AAT GAT CTA CAG CA | GCT GCT GGT GAT TTT TGA AGA | 2227, 2228 |
| chr18 | 25645371 | 25645620 | AAC GGC ACT TGG TTC ACT A | CTC TGG GCA AAC AAG AAA CC | 2229, 2230 |
| chr18 | 25665025 | 25665274 | ACT CTG AAC TCC TCC TCC TG | GTG TGT GTT TGT GGA AGT GT | 2231, 2232 |
| chr18 | 25666614 | 25666863 | TTT GCT CAC ACA CAA GAC AC | AAA GCC CAA TCT CTC TGG TTA | 2233, 2234 |
| chr18 | 25667057 | 25667306 | ACG ACT GCA TCC TTT TCA TG | TAG GCG GGC TTA TTG TGT TT | 2235, 2236 |
| chr18 | 25670741 | 25670990 | CCA TCA TAG CCT ACA AAT ACC C | TCA ATG TAA ACT GCC CGG AG | 2237, 2238 |
| chr18 | 25673208 | 25673457 | AGA GAG TTG AAA ATA TCC CCC A | ATG GGA GTC GAA TGG TGT AAA | 2239, 2240 |

FIGURE 2 (Page 70 of 80)

123

| chromosome | start | stop | forward primer | reverse primer | SEQ ID NOs: |
|---|---|---|---|---|---|
| chr18 | 25681018 | 25681267 | ACT TTC TTG AAC ACC CCA GT | TAA CAT TTG AGG GCA TGG GA | 2241, 2242 |
| chr18 | 25682079 | 25682328 | TGC CTG TGT CCT ACT TTT CC | CAG AAT ACC CTC ACT GTG CT | 2243, 2244 |
| chr18 | 25689906 | 25690155 | TGA CTG CCC AAG AAT GTA CA | GGA GAT AAC AGC AGA GGT CC | 2245, 2246 |
| chr18 | 25700540 | 25700789 | CCG AAC ACG CTG TAT GTA TT | TGT GGG TGA TAT CTG TGT CT | 2247, 2248 |
| chr18 | 25703648 | 25703897 | GGA AGG GAA TTG AAG CAC AG | GCC ATC CTG TAA CTG AAT GC | 2249, 2250 |
| chr18 | 25707188 | 25707437 | TCA TCC TAT CCA CCA ACC TG | GTA TTT TCC CTT TGC CGC AG | 2251, 2252 |
| chr18 | 25716100 | 25716349 | AAT GAA CTG GCC CTG ACT TA | ATT ACA GCA AAG AAC GTG GC | 2253, 2254 |
| chr18 | 25722951 | 25723200 | TAA GAT ACC ATA CCG CAG CT | TCT GTG TGT TTT GCA TTG GT | 2255, 2256 |
| chr18 | 25725055 | 25725304 | TCA GCG TTC ATG GTA CCA ATA | GTG ACA GTT TTC CAA GGC AT | 2257, 2258 |
| chr18 | 25748105 | 25748354 | GTT TCC CAA CCA ACA AAC AAG | GCA TTA CTT TTT CGC ACA CT | 2259, 2260 |
| chr18 | 26214441 | 26214690 | TCT ATC GGG ATG GAG AGT GA | GGG GAT TGT TTT AAG CAG GC | 2261, 2262 |
| chr18 | 26236788 | 26237037 | GTT CAG ACA GGT GGA CTA GG | CCA TTG TTC TCA CCA ACT CT | 2263, 2264 |
| chr18 | 26261336 | 26261585 | AAG CAA CCT GGG AAA TTG TG | CCT GAA ACA CAA GCA GCA G | 2265, 2266 |
| chr18 | 26265937 | 26266186 | GGG TTA AGG TTG CTG GGT TA | AGC TGC CTA TTT GAT TGG TG | 2267, 2268 |
| chr18 | 26285286 | 26285535 | GTG AAA TGT GGT TGT AGT GCA | CAG TAC AGT CAG CCT TCC TT | 2269, 2270 |
| chr18 | 26289006 | 26289255 | GTG ACT GCC TTG CTT CAT TT | TGC ATT CAA ACT ACC CCA AG | 2271, 2272 |

FIGURE 2 (Page 71 of 80)

| chromosome | start | stop | forward primer | reverse primer | SEQ ID NOs: |
|---|---|---|---|---|---|
| chr18 | 26308405 | 26308654 | CAG TTC TGG AGC CTT CTA CT | CCG AAA AGA GGC AAG CAA TT | 2273, 2274 |
| chr18 | 26308962 | 26309211 | TTA GGG CAG GAT GTA CAG AA | GTC ACC TCA ACC TAA CTC CA | 2275, 2276 |
| chr18 | 26312054 | 26312303 | GCT GGT GAC CTT CAT TCA AG | GCA ACA GTC TAC CCG TCT AG | 2277, 2278 |
| chr18 | 26316149 | 26316398 | TAA AAC CAT GTT CGG GGC A | ATC AAT GCT CTG ACC TCC TG | 2279, 2280 |
| chr18 | 47100905 | 47101154 | CTA CCT GTC CGT TTC CCT TAC | ATA CAT CAG GCC TCC AGA ATT | 2281, 2282 |
| chr18 | 47130278 | 47130527 | GAC AAA GAT GAC TGG AGG TGA | CAC ATC TTT AGA GCT CAG GTG A | 2283, 2284 |
| chr18 | 47366936 | 47367185 | GGG TGG ATG GTG AGA TAT GTG | CCA TCA CTT CAC AAT CCA CAC | 2285, 2286 |
| chr18 | 47371131 | 47371380 | CTT CAA ACC TGA TCC ATG TGC | AAT CCT GCA GTC ATC TTC CC | 2287, 2288 |
| chr18 | 47689868 | 47690117 | GCA GAA ACA GCA TGA ATC TCC | CAA GTC TGG TTT GTG AGA AGC | 2289, 2290 |
| chr18 | 47690267 | 47690516 | TGA CTT CAA ACA TCC CAT CCA | GGG AGC TTC TGT AGT CTT TGA | 2291, 2292 |
| chr18 | 47703689 | 47703938 | GTG GGA TGA GTT CTA GAG GAA | TAA GGA GAG CAG GAC TTA CAG | 2293, 2294 |
| chr18 | 47708139 | 47708388 | AGT CAC ACA CAT ACA CAC AGT | CCA ACG GTT CAT TTG TCG TAT | 2295, 2296 |
| chr18 | 47717445 | 47717694 | ACG ACT TCC CTG TGT AAC TTA | CCT TGC TCT GTT AAT GGG TTT | 2297, 2298 |
| chr18 | 47738531 | 47738780 | CCT TCT CTT GTC TCT AGT GCC | AAC AAT GCT AAA CGG GAA TCC | 2299, 2300 |
| chr18 | 47777438 | 47777687 | CTG GAA ATA CAC ACA CAC CTG | GCA GAG TTC ATA GAA GGG TCA | 2301, 2302 |
| chr18 | 47794212 | 47794461 | ATT TGT AAA CCA CCC ACT TCG | AAA CCG AGA CGA CCA CCT AAT | 2303, 2304 |

FIGURE 2 (Page 72 of 80)

| chromosome | start | stop | forward primer | reverse primer | SEQ ID NOs: |
|---|---|---|---|---|---|
| chr18 | 47799400 | 47799649 | TCA CTG TGC TGA CAA ATC CTA | GAG TGA TGA TGA GCC ATG ATG | 2305, 2306 |
| chr18 | 47802662 | 47802911 | CCC ATC ATC ATC CCT TCA GAC | GGA TAT TGG AGA TAG CAG GCA | 2307, 2308 |
| chr18 | 39874986 | 39875235 | TCA TCA CTT TAT CCT CCC AGT | GTT TGG CAC TGC AAC TAG ATA | 2309, 2310 |
| chr18 | 39891490 | 39891739 | ATG GGC ACA GGT AAA GAG TTT | TGA AAT AAG GGA AGC CAC ACA | 2311, 2312 |
| chr18 | 42204240 | 42204489 | TTT GTA AGC TGA GTG TGA GGT | TCC TTA GTG TGC CAA TTA GCC | 2313, 2314 |
| chr18 | 42313146 | 42313395 | TTA CTG TTT GAA TGC CAG CTC | TTA ATG TGG AGA GAC AGG CC | 2315, 2316 |
| chr18 | 42313939 | 42314188 | TCC CTT CTC CCA TCA CAA TTC | CAA TGC ATC TTA CTC ACC CTT | 2317, 2318 |
| chr18 | 42314302 | 42314551 | GAA GAC TGC ATG TGT GTC CTA | AGT ATG GAA GTG GGA ATT GGA | 2319, 2320 |
| chr18 | 42349923 | 42350172 | TTC TCA CTC TCA ACT GAA CCA | TTG CTT CCA CAG AAA CTC TTC | 2321, 2322 |
| chr18 | 42407165 | 42407414 | TCA GGG AGC TTC TAA TTA AGG A | AAC CGA CCT ATT CCA AAG TCT | 2323, 2324 |
| chr18 | 42418033 | 42418282 | AAG ATG ATC CCA GGC TTA AGG | TGT GAA GCG AAT ACA GCT CAA | 2325, 2326 |
| chr18 | 42449702 | 42449951 | GTT GAG GTT TGC TGA TCT TGG | GTT CTA ACT ACA CCA GGC TCT | 2327, 2328 |
| chr18 | 42450400 | 42450649 | CAA AGA TAG ATT CGC ACA CCA | GTA TGA GTG TAG GTG TGG AGG | 2329, 2330 |
| chr18 | 42461427 | 42461676 | GAG CTG GAC AAA TTA AAT GGC | AAT CTG GAT CTA GCG AAG GAC | 2331, 2332 |
| chr18 | 42462834 | 42463083 | CCA GTG CAT TTG GTT TGA CA | TAA TGA GAA GGC AGG ATG AGG | 2333, 2334 |
| chr18 | 42468595 | 42468844 | TAT GTG AAT CCT CTG TGT GGC | AAG ACA ACT CTC TAG GCC TCA | 2335, 2336 |

FIGURE 2 (Page 73 of 80)

| chromosome | start | stop | forward primer | reverse primer | SEQ ID NOs: |
|---|---|---|---|---|---|
| chr18 | 42468938 | 42469187 | AGC TTC TCT CTC ATT CTG CTT | GTT TAT GGT TGT CCC TGG AGA | 2337, 2338 |
| chr18 | 42644003 | 42644252 | GAC TCC CGA TTT CAT TTG CTG | TCC ACC TTC TGA TCA CAC AAT | 2339, 2340 |
| chr18 | 42644494 | 42644743 | TCC CAA TCG TTG TGA AAC ATA C | AAG ATC AGG TAC CAA GGC ATT | 2341, 2342 |
| chr18 | 42645387 | 42645636 | TCT TTA CAG GAA GTT GGG ACC | ATG ATG TGA AGT CCA TGG TGA | 2343, 2344 |
| chr18 | 42741059 | 42741308 | TAA GTT CAG ATC AGG GAG CAG | TCC AAA TTG ACT TCC ATG AGC | 2345, 2346 |
| chr18 | 42745733 | 42745982 | GTT GAA AGT CTT ACA GAA CGC T | GTG GTT TCA GGA ATT TGG AGG | 2347, 2348 |
| chr18 | 42746460 | 42746709 | CAA CAT AGG CAC ATT GTC CTC | TAT TTG CTG CTT CAT TCT TCC C | 2349, 2350 |
| chr18 | 42746911 | 42747160 | GTC AGG CCT CAT AAC TCT CTT | AAG TCA TTA CGT CCC ACA CTG | 2351, 2352 |
| chr18 | 42747473 | 42747722 | GTT GTG TGG CTT TCC TTA TCA | AAA GTC ATC AGA AGG GTA GCA | 2353, 2354 |
| chr18 | 43796659 | 43796908 | CCT GCA GCT CTG TGT AAA TTT | AAT GAT GCC GAA CAG TGA GTA | 2355, 2356 |
| chr18 | 43820471 | 43820720 | CTT TGG CCA GTT CTT TCT CTC | AAT GTA AGA CAG GGA CAG AGA | 2357, 2358 |
| chr18 | 43841913 | 43842162 | GTG GCC CAG CAT TAT TTG TT | TGA ATT CCA CAG TCC AGT CAA | 2359, 2360 |
| chr18 | 43842202 | 43842451 | TCT TGG TGT GAC TTG CTA ACA | AAT GCC TTC AAA GAC AGT GAC | 2361, 2362 |
| chr18 | 43842647 | 43842896 | TTT CTG GCT GAG ATA AGA CCC | CAC ACC TGC AAT TGA GAT GAA | 2363, 2364 |
| chr18 | 43845701 | 43845950 | ACA ATT CCG TGG TAT ACA GCT | GTC ATG ATG ATG CAA CAG CTA | 2365, 2366 |
| chr18 | 43846353 | 43846602 | TGA TTG TGC CCT AAC CAA ACT | TTT CAC GGT AAG AGG AGC AAA | 2367, 2368 |

FIGURE 2 (Page 74 of 80)

| chromosome | start | stop | forward primer | reverse primer | SEQ ID NOs: |
|---|---|---|---|---|---|
| chr18 | 43917652 | 43917901 | TGT CGT ATC CTG CTG TTT AGA | TTC TCT CTA GGC AGG TGA ACT | 2369, 2370 |
| chr18 | 43918519 | 43918768 | TCA GGA GTA AAG TCA GGA CCT | ACC CTT TGA AAG AAC CAG GAA | 2371, 2372 |
| chr18 | 43918998 | 43919247 | TGA GCT GAT TTA CTG TGA CAC | AAT GAG CCA CTG TTC TCT AGG | 2373, 2374 |
| chr18 | 43919544 | 43919793 | TAG CAC CTT GAC TTC AGG ATT | ATT TGC ACA TTA GGG CCT CAA | 2375, 2376 |
| chr18 | 44086123 | 44086372 | TGA GGT TGG AAA GGG TCA ATT | GAA CTT CCC TGC TTC CTT CT | 2377, 2378 |
| chr18 | 44094299 | 44094548 | CAG CTT TCC TTC CTC TTC TCT | AAA TAC TTG GCT GTG ACC ATG | 2379, 2380 |
| chr18 | 44167214 | 44167463 | ACA GTG AGA GGA AAG AAC AGC | AAG ACC CTT GAG AAC TTC CAA | 2381, 2382 |
| chr18 | 44167791 | 44168040 | GGA AAT AAA TTG TGA GCT GGC | ATG TGT AAA GAC GTC CTG GAA | 2383, 2384 |
| chr18 | 44168296 | 44168545 | AGA CAG CCC TTC AAT CCA TAC | TCT ATG TGG AGG GAT TTG ACA | 2385, 2386 |
| chr18 | 44173512 | 44173761 | CCT ACA TCC CTT CCT CCT TTC | TTC CTG AAG TTT ATG GTG CAA C | 2387, 2388 |
| chr18 | 44174626 | 44174875 | TCA CCC ATC TTC CAA TTA GCT | AAG GTT GAA TGA GGA TCA AGC | 2389, 2390 |
| chr18 | 44857765 | 44858014 | TTT CTA AGC ACA AAC TGA CAC C | GAG GGA AGA ACA CAA CAC ATG | 2391, 2392 |
| chr18 | 45110525 | 45110774 | GAT GTT TGC ACT GGA GGG ATA | CAG ACT AGC CTA CAA TCC TCC | 2393, 2394 |
| chr18 | 45197621 | 45197870 | GAA GAC TAA ATG TTG GCC GAA | CAT TCA GGT TCT TAA GGG CTG | 2395, 2396 |
| chr18 | 45197871 | 45198120 | GTA GAG AGA GGG AGG ATC ACA | GAA AGG CAG ACG ATG AAA GAG | 2397, 2398 |
| chr18 | 45198760 | 45199009 | CTT GCC ATG AAG TTT GAC CAG | ATC TCC ATC AGC ACA GGA ATT | 2399, 2400 |

FIGURE 2 (Page 75 of 80)

| chromosome | start | stop | forward primer | reverse primer | SEQ ID NOs: |
|---|---|---|---|---|---|
| chr18 | 45234886 | 45235135 | AGG ATG AGC ATT TGT AAC CTG | GCA GTG TGA CAT CTG TGA ATG | 2401, 2402 |
| chr18 | 45235523 | 45235772 | TGT CGC TTT CAA ATT ACC CAC | AGA CTT TCC TGT TCC TCT TCA | 2403, 2404 |
| chr18 | 45238370 | 45238619 | CAT ACA AGT GCT CTG TTA GGC | TTT ACA ATG AAC TAG CCA GGC | 2405, 2406 |
| chr18 | 45335896 | 45336145 | AGG ACT TGG AAC CAG AAA GAC | TAA CAT CTG CCT GAA AGC TTC | 2407, 2408 |
| chr18 | 45336353 | 45336602 | AAA GAG GGC TGA TAT CGT CTG | CTC GTG TGT GCA ATT TGG AAT | 2409, 2410 |
| chr18 | 45357461 | 45357710 | CTC ACT GCA AAC TAT GGA ACC | GGA GAG ATG GAG AAG ACC TTT | 2411, 2412 |
| chr18 | 45357745 | 45357994 | TAT TCT GCC CAT CTT CTT CCT | AAT GTA TTA CTG TGC TCC CGT | 2413, 2414 |
| chr18 | 45358502 | 45358751 | GGA GAC AGC CCA AAC ATA GA | ACC ACC TGC CAC TGT ATA AAT | 2415, 2416 |
| chr18 | 45362379 | 45362628 | AGC AAT GGT GAA GTT CTG GAT | AAT TCT TCT TCT GGT GCA AGG | 2417, 2418 |
| chr18 | 45397588 | 45397837 | CTG GTC AGT GAG AGA AGG GAA | TTG CCC TTT GAA CTG TTG ATC | 2419, 2420 |
| chr18 | 45864183 | 45864432 | TTT CTC CAC TGG CAT GAA CT | AAG CAC ACT AAG GCC TGA TAA | 2421, 2422 |
| chr18 | 46464382 | 46464631 | CAT GAT CAC AAT TCC AAG CCA | TCA GTA TCC ATT CAG CAT CCA | 2423, 2424 |
| chr18 | 46473428 | 46473677 | ACC CAG TCA AGT TAC AGT CTT | TAA CCG AAG CCC ATA CTC TG | 2425, 2426 |
| chr18 | 46474545 | 46474794 | TGT AAA GCA TAT CAA GGG AAC G | TCC TTA CTC CAG ATA CCC GAT | 2427, 2428 |
| chr18 | 46481797 | 46482046 | TGC AGA GAT ATG TTC CCG TAT | TCT GGC TTC TTT CTT GGA GAG | 2429, 2430 |
| chr18 | 46483016 | 46483265 | AGA AGA CAG TAC AAG GAA GGC | GCA ACA TTC ATT TCA TCC TGC | 2431, 2432 |

FIGURE 2 (Page 76 of 80)

EP 4 095 258 B1

| chromosome | start | stop | forward primer | reverse primer | SEQ ID NOs: |
|---|---|---|---|---|---|
| chr18 | 46589014 | 46589263 | TGT TTG CCA TTT GTT CTC CTC | CAT CAC GAC ATC CAT TTC CAC | 2433, 2434 |
| chr18 | 46599847 | 46600096 | TTG AAG GCA AGA GAA GTT TGG | GTG GTT ATT ATC GGT GGG TGA | 2435, 2436 |
| chr18 | 46601866 | 46602115 | GCC AAG GAA ATG TAG GGA AAG | CTG CCA TTC CTT GTT TCC AA | 2437, 2438 |
| chr18 | 46983278 | 46983527 | AAC CTT CAC ACC TAG AGA CAG | ATT TGG ACT TGA AGC AGC CT | 2439, 2440 |
| chr18 | 47013103 | 47013352 | GCA TAA CAG GGA AAG TCA CCT | CTG TAT TCT TTG TCC ACC ACC | 2441, 2442 |
| chr18 | 47016802 | 47017051 | AGG ATG TTA GTG GTT TGG GTA | GAC AGG ACA CCT TGG ATT GAT | 2443, 2444 |
| chr18 | 286722 | 286971 | TCT GAC ACT GAC CTT CAA CT | GAG TAA TTC CCC CGA TGC AG | 2445, 2446 |
| chr18 | 298393 | 298642 | GAA ACA TTG CTT TCC CTC CA | TGA GAA TCA TTG AGC CAA ACC | 2447, 2448 |
| chr18 | 338127 | 338376 | TTC CAC ACA TCT CTT CTC CG | AAT TGT GAG CGT TAG AGT GC | 2449, 2450 |
| chr18 | 340427 | 340676 | GGG AGC CTT GAA AAC CTG AA | CCA GTG GGT CTT AAC ATT GAG | 2451, 2452 |
| chr18 | 387135 | 387384 | ATT GGT AGC GTT GTC AGC A | AAA AGG CTA GTA GAG GGT GC | 2453, 2454 |
| chr18 | 391175 | 391424 | TTC CTG CAT CTT GTA GAC CC | TCG CTG AAG AAC TGA GAC AC | 2455, 2456 |
| chr18 | 434440 | 434689 | GGG CTA ATG TTT TGC TTC CA | GGG AAG TGA TTG GAG AGA GG | 2457, 2458 |
| chr18 | 457527 | 457776 | TGT TGA TTA GAG CTT CCC CC | CCG CTT GGT ATA GAG TGC TG | 2459, 2460 |
| chr18 | 460384 | 460633 | AGA GGT TTT CTT CCC CGT G | TTG AGG TTG TCA GGA AAG CT | 2461, 2462 |
| chr18 | 469281 | 469530 | TAG TGC CCT CTA TTG TGC CT | TCT TGG GAA AGG GTC ATT CT | 2463, 2464 |

FIGURE 2 (Page 77 of 80)

| chromosome | start | stop | forward primer | reverse primer | SEQ ID NOs: |
|---|---|---|---|---|---|
| chr18 | 572716 | 572965 | ACT GCT GGA CTT TGA AAT GC | TCT CTT CCA TGC ACT CCA C | 2465, 2466 |
| chr18 | 598352 | 598601 | CAA ACA GTG AGA TGT GGC TG | GAT CTG ACC CTC TGC TCA C | 2467, 2468 |
| chr18 | 615413 | 615662 | TTG CTT CCT GAA AAC TGG TTC | CCT GAA AGA TGC ATG GTT GG | 2469, 2470 |
| chr18 | 618280 | 618529 | ATT CCA ATC ACG TCT CTG CA | GCC ACA GAA CAA CCA GAT TC | 2471, 2472 |
| chr18 | 901111 | 901360 | ACA ATC TCA CAG CCT GGA AA | TGG GGT TAA GAG CTC AAG AG | 2473, 2474 |
| chr18 | 939383 | 939632 | TCA GAT GGG TGA GGT TCT TG | AGT GTG GAT GAC TTC TGC AA | 2475, 2476 |
| chr18 | 26321283 | 26321532 | CTG CTC CTT CCC TCC AAT TA | GCC CCA TTA TCC TCC TTT GT | 2477, 2478 |
| chr18 | 26337865 | 26338114 | AAA TGC CAG TCC TGT AAA GG | CAA ATC AGA CCC ACT AAG CAC | 2479, 2480 |
| chr18 | 26345717 | 26345966 | TGT CCC ATT GCT TAG GAA GT | TGT TTT GGA CTG CTT CAC TC | 2481, 2482 |
| chr18 | 26365387 | 26365636 | TGT GTG ATC CAG AGA CCC TA | ATT ATC ATG CTC ACT CCT CCA | 2483, 2484 |
| chr18 | 26367518 | 26367767 | ACC AAT GTA GAC TTA GCG GG | GAC ATA GCA CGG GAG GAG TA | 2485, 2486 |
| chr18 | 26392500 | 26392749 | ACT CTC ATA TTG CCC CAC TT | GCT AGT GGC GTT TTA GGA AA | 2487, 2488 |
| chr18 | 26406499 | 26406748 | CCA GGG ATT GAT GTA CTG GT | TCG CTT GGA AGT CAT AGC C | 2489, 2490 |
| chr18 | 26711590 | 26711839 | AAT TCT GGT CTA TCT GGC GT | CAG AGC TGC TTT GAA GAT AAT CC | 2491, 2492 |
| chr18 | 26729342 | 26729591 | GCC AGC CCT TTT CAC ATA TT | GAA GCC TGA TAG ATG TGC CT | 2493, 2494 |
| chr18 | 26733981 | 26734230 | AAC TAC ACC ATC CCC TGT TT | TAC CTC TGC CTC CAA TTG TC | 2495, 2496 |

FIGURE 2 (Page 78 of 80)

| chromosome | start | stop | forward primer | reverse primer | SEQ ID NOs: |
|---|---|---|---|---|---|
| chr18 | 26739336 | 26739585 | CCA TTT CAA ACA TGC TGG TC | CCC ATG TAG ACA AAG TGC TT | 2497, 2498 |
| chr18 | 26750685 | 26750934 | AGA TTA TAA GAA GGC AGG GAA C | GGC AGG TTT GTC TTA CAG TT | 2499, 2500 |
| chr18 | 26752487 | 26752736 | GTA GAG GGC TTA AAA CAT GTC C | TGT GAG ATT GCA TTC CCC TT | 2501, 2502 |
| chr18 | 26757573 | 26757822 | ACA AGA ACA CAG TCG TTA AGC | CGG GTC AGA GAA AGA TCA GG | 2503, 2504 |
| chr18 | 27244230 | 27244479 | TCT CTC CTT CAC TCC CTT CA | AAA CAT TTG TAA CCA CTC CCT G | 2505, 2506 |
| chr18 | 35076347 | 35076596 | TGT CCA CCC CTC TTT GAT TG | CCT GTA ATA TGG GAC TCC TGG | 2507, 2508 |
| chr18 | 35147912 | 35148161 | TTT AGC TTC TCC TGC CTT TG | CCA AAC CAC ACA CAC AAA CT | 2509, 2510 |
| chr18 | 35282655 | 35282904 | AGA AGC AAT TCA CCA GGT CA | AGG AGA AGG ACA TTT CAC AGG | 2511, 2512 |
| chr18 | 35292141 | 35292390 | TGG AGT CAG AAG TGT GTG TT | AGG CCG ATA AGA CAA GGT TC | 2513, 2514 |
| chr18 | 35304247 | 35304496 | TCT GGT GTC AAA GCT TAG GG | GTT TCC CAT AGA GCC CTG G | 2515, 2516 |
| chr18 | 35311302 | 35311551 | TGC CGA TGA TGT GTG TTT TG | CTC CAC TCT CTC CAA CCA AC | 2517, 2518 |
| chr18 | 36463709 | 36463958 | TGT CCC TTC CTA ATC CCA AA | AAA CTG TGA AAG GAC GAG GA | 2519, 2520 |
| chr18 | 36473033 | 36473282 | AGG ATG TTT AAG TTG CAG CA | TCC CTT GCT TTT GTA CCA GG | 2521, 2522 |
| chr18 | 36475403 | 36475652 | TAT GCA GTT TTA CCC CCT CC | CAT GTG TGT ACT GTG CCT CA | 2523, 2524 |
| chr18 | 36479145 | 36479394 | TTC TGT GTG GTC TCC TCT TG | TCT TTC ACT GTC ACT ATG GGG | 2525, 2526 |
| chr18 | 36482038 | 36482287 | ATG GAG GGA CAA GTG AGA CA | AAA TGC AGC TTC CCA ACA TC | 2527, 2528 |

FIGURE 2 (Page 79 of 80)

| chromosome | start | stop | forward primer | reverse primer | SEQ ID NOs: |
|---|---|---|---|---|---|
| chr18 | 36483959 | 36484208 | GGG GAA CAT GGA GCT GTA AA | GAG ACT TTC TGG AGG ACG AA | 2529, 2530 |
| chr18 | 36485761 | 36486010 | GGA CCC CCT ACC ACA TTT AC | GAA ACG TAA TTT AGT GAC TGG C | 2531, 2532 |
| chr18 | 36487627 | 36487876 | AGA TGG AGA AAT GTG CAG AGA | ATG TGT CTA TTG CTA CCT GTG A | 2533, 2534 |
| chr18 | 36489135 | 36489384 | ATG ACT GCA TCC AAG AGC A | CTC CTT CAT TTT GCT GGT GG | 2535, 2536 |
| chr18 | 36531244 | 36531493 | CAG AAT TTC CAG GCA GTT GT | GGT GAT CAT TTG TCT GCA CA | 2537, 2538 |
| chr18 | 36554006 | 36554255 | GAA TCC AGA AGC TCA GTC CTT | TGA AGG GAT GAA GGC AGA AG | 2539, 2540 |
| chr18 | 36558050 | 36558299 | AGC CCT GGA ATC TTG ACA TT | ATT GTG TTG TCC TTC CGT TT | 2541, 2542 |
| chr18 | 36569619 | 36569868 | GTG CAT TAT ACG GAT GGC C | CTG AAG CAT CAC TGG CAT TG | 2543, 2544 |
| chr18 | 1017928 | 1018177 | GGT AGA GGG TCC TGT GAT TC | AAG CTT GTA GTC TGG GTA GC | 2545, 2546 |
| chr18 | 241538 | 241787 | GTA ACT GCT AGC CAC TGA GT | TCC CTC TGT ACT ATG TAG CAT G | 2547, 2548 |
| chr18 | 242831 | 243080 | GCC TTT TTG GGA ATC CTA GT | TTC ATT TCC CTT TGT TGC CC | 2549, 2550 |

FIGURE 2 (Page 80 of 80)

Fig. 3

sequence of interest

single TACS

sequence of interest

family of TACS

Fig. 4A

Fig 4B

Average fold-increase: 54.7%

Fig 5

Fig. 5 Cont.

5 %

Fig. 6

Fig. 6 cont

Fig 7

Observed pattern of somatic SNVs

Fig 8

Fig 9

Fragment based scores for chromosome 21

Fig 10

| chromosome | start | stop | gene |
|---|---|---|---|
| chr1 | 115256347 | 115256588 | NRAS |
| chr1 | 115256398 | 115256647 | NRAS |
| chr1 | 115256410 | 115256649 | NRAS |
| chr1 | 115256442 | 115256691 | NRAS |
| chr1 | 115256467 | 115256726 | NRAS |
| chr1 | 115256470 | 115256715 | NRAS |
| chr1 | 115258538 | 115258778 | NRAS |
| chr1 | 115258570 | 115258813 | NRAS |
| chr1 | 115258607 | 115258846 | NRAS |
| chr1 | 115258659 | 115258901 | NRAS |
| chr3 | 178916725 | 178916970 | PI3KCA |
| chr3 | 178916766 | 178917010 | PI3KCA |
| chr3 | 178916782 | 178917028 | PI3KCA |
| chr3 | 178916822 | 178917068 | PI3KCA |
| chr3 | 178917417 | 178917566 | PI3KCA |
| chr3 | 178917417 | 178917618 | PI3KCA |
| chr3 | 178917420 | 178917590 | PI3KCA |
| chr3 | 178917420 | 178917604 | PI3KCA |
| chr3 | 178921331 | 178921578 | PI3KCA |
| chr3 | 178921339 | 178921585 | PI3KCA |
| chr3 | 178921347 | 178921596 | PI3KCA |
| chr3 | 178921364 | 178921608 | PI3KCA |
| chr3 | 178927888 | 178928114 | PI3KCA |
| chr3 | 178927898 | 178928114 | PI3KCA |
| chr3 | 178927974 | 178928182 | PI3KCA |
| chr3 | 178935873 | 178936102 | PI3KCA |
| chr3 | 178935930 | 178936133 | PI3KCA |
| chr3 | 178935944 | 178936197 | PIK3CA |
| chr3 | 178935995 | 178936197 | PI3KCA |
| chr3 | 178936030 | 178936279 | PI3KCA |
| chr3 | 178951914 | 178952159 | PI3KCA |
| chr3 | 178951915 | 178952140 | PI3KCA |
| chr3 | 178951921 | 178952140 | PI3KCA |
| chr3 | 178951921 | 178952159 | PI3KCA |
| chr3 | 178951948 | 178952109 | PIK3CA |
| chr3 | 178952004 | 178952152 | PIK3CA |
| chr3 | 178952010 | 178952109 | PIK3CA |
| chr3 | 178952109 | 178952152 | PIK3CA |

Fig. 10 cont

| chromosome | start | stop | gene |
|---|---|---|---|
| chr7 | 55241468 | 55241710 | EGFR |
| chr7 | 55241542 | 55241789 | EGFR |
| chr7 | 55241576 | 55241746 | EGFR |
| chr7 | 55242269 | 55242500 | EGFR |
| chr7 | 55242272 | 55242520 | EGFR |
| chr7 | 55242280 | 55242532 | EGFR |
| chr7 | 55242319 | 55242558 | EGFR |
| chr7 | 55242332 | 55242584 | EGFR |
| chr7 | 55242505 | 55242271 | EGFR |
| chr7 | 55248852 | 55249080 | EGFR |
| chr7 | 55248896 | 55249120 | EGFR |
| chr7 | 55248933 | 55249182 | EGFR |
| chr7 | 55248937 | 55249187 | EGFR |
| chr7 | 55248961 | 55249208 | EGFR |
| chr7 | 55259335 | 55259571 | EGFR |
| chr7 | 55259337 | 55259586 | EGFR |
| chr7 | 55259356 | 55259602 | EGFR |
| chr7 | 55259368 | 55259570 | EGFR |
| chr7 | 55259391 | 55259640 | EGFR |
| chr12 | 25378488 | 25378688 | KRAS |
| chr12 | 25378503 | 25378751 | KRAS |
| chr12 | 25378546 | 25378778 | KRAS |
| chr12 | 25378554 | 25378783 | KRAS |
| chr12 | 25380153 | 25380359 | KRAS |
| chr12 | 25380166 | 25380337 | KRAS |
| chr12 | 25380167 | 25380326 | KRAS |
| chr12 | 25380167 | 25380359 | KRAS |
| chr12 | 25398080 | 25398329 | KRAS |
| chr12 | 25398145 | 25398394 | KRAS |
| chr12 | 25398153 | 25398397 | KRAS |
| chr12 | 25398159 | 25398408 | KRAS |
| chr12 | 25398186 | 25398433 | KRAS |

**EP 4 095 258 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 7888017 B
- US 8008018 B
- US 8195415 B
- US 8296076 B
- US 8682594 B
- US 20110201507 A
- US 20120270739 A
- WO 2016189388 A
- US 20160340733 A
- WO 2014130890 A

### Non-patent literature cited in the description

- **CHIU, R. W. et al.** *Proc. Natl. Acad. Sci. USA*, 2008, vol. 105, 20458-20463
- **FAN, H.C. et al.** *Proc. Natl. Acad. Sci. USA*, 2008, vol. 105, 16266-162710
- **PALOMAKI, G.E. et al.** *Genet. Med.*, 2011, vol. 13, 913-920
- **EHRICH, M. et al.** *Am. J. Obstet. Gynecol.*, 2011, vol. 204, 205e1-11
- **CHEN, E.Z. et al.** *PLoS One*, 2011, vol. 6, e21791
- **SEHNERT, A.J. et al.** *Clin. Chem.*, 2011, vol. 57, 1042-1049
- **PALOMAKI, G.E. et al.** *Genet. Med.*, 2012, vol. 14, 296-305
- **BIANCHI, D.W. et al.** *Obstet. Gynecol.*, 2012, vol. 119, 890-901
- **ZIMMERMAN, B. et al.** *Prenat. Diag.*, 2012, vol. 32, 1233-1241
- **NICOLAIDES, K.H. et al.** *Prenat. Diagn.*, 2013, vol. 33, 575-579
- **SPARKS, A.B. et al.** *Prenat. Diagn.*, 2012, vol. 32, 3-9
- **KOUMBARIS, G. et al.** *Clinical chemistry*, 2016, vol. 62 (6), 848-855
- **MEYER, M ; KIRCHNER, M.** *Cold Spring Harb. Protoc.*, 2010, vol. 6, pdbprot5448
- **LIAO, G.J. et al.** *PLoS One*, 2012, vol. 7, e38154
- **MARICIC, T. et al.** *PLoS One*, 2010, vol. 5, e14004
- **TEWHEY, R. et al.** *Genome Biol.*, 2009, vol. 10, R116
- **TSANGARAS, K. et al.** *PLoS One*, 2014, vol. 9, e109101
- **EICHLER. E.E.** *Trends Genet.*, 2001, vol. 17, 661-669
- **TODD, J. et al.** *Nature Reviews Genet.*, 2012, vol. 13, 36-46
- **JIANG et al.** *Proceedings of the National Academy of Sciences*, 2015, vol. 112 (11), E1317-E1325
- **CHAN, K.C.** *Clin. Chem.*, 2004, vol. 50, 88-92
- **FORBES, S.A. et al.** *Curr. Protocol Hum. Genetic*, 2016, vol. 91
- **FORBES, S.A. et al.** *Nucl. Acids Res.*, 2017, vol. 45, D777-D783
- **PRIOR et al.** *Cancer Res.*, 2012, vol. 72, 2457-2467
- **SHAR, N.A. et al.** *Mol. Canc.*, 2016, vol. 15, 76
- **MARTIN, M. et al.** *EMB.netJournal*, 2011, vol. 17, 1
- **LI, H. ; DURBIN, R.** *Bioinformatics*, 2009, vol. 25, 1754-1760
- **TARASOV, ARTEM et al.** Sambamba: fast processing of NGS alignment formats.. *Bioinformatics*, 2015, vol. 31 (12), 2032-2034
- **LI, H. et al.** *Bioinformatics*, 2009, vol. 25, 2078-2079
- Python Software Foundation. *Python*, 2015
- The R Foundation. *The R Project for Statistical Computing*, 2015